# EUROPEAN PATENT APPLICATION

(11) **EP 1 284 298 A2**
(43) Date of publication of application: **19.02.2003**
(21) Application number: 02255229.3
(22) Date of filing: 26.07.2002
(51) Int. Cl.: C12Q 1/68, A01K 67/027

(54) **Identification and use of molecules implicated in pain**

(30) Priority: 27.07.2001 GB 0118354; 07.02.2002 GB 0202883
(71) Applicant: WARNER-LAMBERT COMPANY, Morris Plains, New Jersey 07950 (US)
(72) Inventor: Brooksbank, Robert Alan, Cambridgeshire, CB1 3AW (GB); Dixon, Alistair Kerr,, Cambridgeshire, CB1 2LL (GB); Lee, Kevin, c/o Pfizer Limited UK, Sandwich, Kent CT13 9NJ (GB); Pinnock, Robert Denham, Pfizer Global Res. and Dev, Ann Arbor, MI 48105 (US)
(74) Representative: Hayles, James Richard

(57) **Abstract**

The invention relates to the use of:
(a) an isolated gene sequence that is down-regulated in the spinal cord of a mammal in response to mechanistically distinct first and second models of neuropathic or central sensitization pain;
(b) an isolated gene sequence comprising a nucleic acid sequence of any of Tables I to VI;
(c) an isolated gene sequence having at least 80% sequence identity with a nucleic acid sequence of any of Tables I to VI;
(d) an isolated nucleic acid sequence that is hybridizable to any of the gene sequences according to (a), (b) or (c) under stringent hybridisation conditions;
(e) a recombinant vector comprising a gene sequence or nucleic acid sequence according to any one of (a) to (d);
(f) a host cell containing the vector according to (e);
(g) a non-human animal having in its genome an introduced gene sequence or nucleic acid sequence or a removed or down-regulated gene sequence or nucleic acid sequence according to any one of (a) to (d);
(h) an isolated polypeptide comprising an amino acid sequence at least 90% identical to an amino acid sequence encoded by a nucleotide sequence according to any one of (a) to (d), or a variant polypeptide thereof with sequential amino acid deletions from the C terminus and/or the N-terminus;
(i) an isolated polypeptide encoded by a nucleotide sequence according to any one of (a) to (d); or
(j) an isolated antibody that binds specifically to a polypeptide according to (h) or (i);
in the screening of compounds for the treatment of pain, or for the diagnosis of pain.

The invention also relates to the use of naturally occurring compounds such as peptide ligands of the expression products of the above gene sequences and their associated signal transduction pathways for use in the treatment of pain.

SEQUENCE LISTING

<110> Warner-Lambert Company
      Lee, Kevin
      Dixon, Alistair
      Brooksbank, Robert
      Pinnock, Robert

<120> Identification and Use of Molecules Implicated in Pain

<130> WL-A-018199

<150> GB 0118354.0
<151> 2001-07-27

<160> 117

<170> PatentIn Ver. 2.1

<210> 1
<211> 531
<212> PRT
<213> Rattus norvegicus

<220>
<223>  Pyruvate kinase M1 subunit

<400> 1
Met Pro Lys Pro Asp Ser Glu Ala Gly Thr Ala Phe Ile Gln Thr Gln
  1               5                  10                  15

Gln Leu His Ala Ala Met Ala Asp Thr Phe Leu Glu His Met Cys Arg
             20                  25                  30

Leu Asp Ile Asp Ser Ala Pro Ile Thr Ala Arg Asn Thr Gly Ile Ile
         35                  40                  45

Cys Thr Ile Gly Pro Ala Ser Arg Ser Val Glu Met Leu Lys Glu Met
     50                  55                  60

Ile Lys Ser Gly Met Asn Val Ala Arg Leu Asn Phe Ser His Gly Thr
 65                  70                  75                  80

His Glu Tyr His Ala Glu Thr Ile Lys Asn Val Arg Ala Ala Thr Glu
                 85                  90                  95

Ser Phe Ala Ser Asp Pro Ile Leu Tyr Arg Pro Val Ala Val Ala Leu
            100                 105                 110

Asp Thr Lys Gly Pro Glu Ile Arg Thr Gly Leu Ile Lys Gly Ser Gly
        115                 120                 125

Thr Ala Glu Val Glu Leu Lys Lys Gly Ala Thr Leu Lys Ile Thr Leu
    130                 135                 140

Asp Asn Ala Tyr Met Glu Lys Cys Asp Glu Asn Ile Leu Trp Leu Asp
145                 150                 155                 160

Tyr Lys Asn Ile Cys Lys Val Val Glu Val Gly Ser Lys Ile Tyr Val
                165                 170                 175

Asp Asp Gly Leu Ile Ser Leu Gln Val Lys Glu Lys Gly Ala Asp Tyr
            180                 185                 190

Leu Val Thr Glu Val Glu Asn Gly Gly Ser Leu Gly Ser Lys Lys Gly
        195                 200                 205

Val Asn Leu Pro Gly Ala Ala Val Asp Leu Pro Ala Val Ser Glu Lys
    210                 215                 220

Asp Ile Gln Asp Leu Lys Phe Gly Val Glu Gln Asp Val Asp Met Val
225                 230                 235                 240

Phe Ala Ser Phe Ile Arg Lys Ala Ala Asp Val His Glu Val Arg Lys
                245                 250                 255

Val Leu Gly Glu Lys Gly Lys Asn Ile Lys Ile Ile Ser Lys Ile Glu
            260                 265                 270

Asn His Glu Gly Val Arg Arg Phe Asp Glu Ile Leu Glu Ala Ser Asp
        275                 280                 285

Gly Ile Met Val Ala Arg Gly Asp Leu Gly Ile Glu Ile Pro Ala Glu
    290                 295                 300

Lys Val Phe Leu Ala Gln Lys Met Met Ile Gly Arg Cys Asn Arg Ala
305                 310                 315                 320

Gly Lys Pro Val Ile Cys Ala Thr Gln Met Leu Glu Ser Met Ile Lys
                325                 330                 335

Lys Pro Arg Pro Thr Arg Ala Glu Gly Ser Asp Val Ala Asn Ala Val
            340                 345                 350

Leu Asp Gly Ala Asp Cys Ile Met Leu Ser Gly Glu Thr Ala Lys Gly
        355                 360                 365

Asp Tyr Pro Leu Glu Ala Val Arg Met Gln His Leu Ile Ala Arg Glu
    370                 375                 380

Ala Glu Ala Ala Val Phe His Arg Leu Leu Phe Glu Glu Leu Ala Arg
385                 390                 395                 400

Ala Ser Ser Gln Ser Thr Asp Pro Leu Glu Ala Met Ala Met Gly Ser
                405                 410                 415

Val Glu Ala Ser Tyr Lys Cys Leu Ala Ala Ala Leu Ile Val Leu Thr
            420                 425                 430

Glu Ser Gly Arg Ser Ala His Gln Val Ala Arg Tyr Arg Pro Arg Ala
        435                 440                 445

Pro Ile Ile Ala Val Thr Arg Asn Pro Gln Thr Ala Arg Gln Ala His
    450                 455                 460

Leu Tyr Arg Gly Ile Phe Pro Val Leu Cys Lys Asp Ala Val Leu Asp
465                 470                 475                 480

Ala Trp Ala Glu Asp Val Asp Leu Arg Val Asn Leu Ala Met Asn Val
                485                 490                 495

Gly Lys Ala Arg Gly Phe Phe Lys Lys Gly Asp Val Val Ile Val Leu

            500                 505                 510

Thr Gly Trp Arg Pro Gly Ser Gly Phe Thr Asn Thr Met Arg Val Val
        515                 520                 525

Pro Val Pro
    530

<210> 2
<211> 531
<212> PRT
<213> Rattus norvegicus

<220>
<223> Pyruvate kinase M2 subunit

<400> 2
Met Pro Lys Pro Asp Ser Glu Ala Gly Thr Ala Phe Ile Gln Thr Gln
1               5                   10                  15

Gln Leu His Ala Ala Met Ala Asp Thr Phe Leu Glu His Met Cys Arg
            20                  25                  30

Leu Asp Ile Asp Ser Ala Pro Ile Thr Ala Arg Asn Thr Gly Ile Ile
        35                  40                  45

Cys Thr Ile Gly Pro Ala Ser Arg Ser Val Glu Met Leu Lys Glu Met
    50                  55                  60

Ile Lys Ser Gly Met Asn Val Ala Arg Leu Asn Phe Ser His Gly Thr
65                  70                  75                  80

His Glu Tyr His Ala Glu Thr Ile Lys Asn Val Arg Ala Ala Thr Glu
                85                  90                  95

Ser Phe Ala Ser Asp Pro Ile Leu Tyr Arg Pro Val Ala Val Ala Leu
            100                 105                 110

Asp Thr Lys Gly Pro Glu Ile Arg Thr Gly Leu Ile Lys Gly Ser Gly
        115                 120                 125

Thr Ala Glu Val Glu Leu Lys Lys Gly Ala Thr Leu Lys Ile Thr Leu
    130                 135                 140

Asp Asn Ala Tyr Met Glu Lys Cys Asp Glu Asn Ile Leu Trp Leu Asp
145                 150                 155                 160

Tyr Lys Asn Ile Cys Lys Val Val Glu Val Gly Ser Lys Ile Tyr Val
                165                 170                 175

Asp Asp Gly Leu Ile Ser Leu Gln Val Lys Glu Lys Gly Ala Asp Tyr
            180                 185                 190

Leu Val Thr Glu Val Glu Asn Gly Gly Ser Leu Gly Ser Lys Lys Gly
        195                 200                 205

Val Asn Leu Pro Gly Ala Ala Val Asp Leu Pro Ala Val Ser Glu Lys
    210                 215                 220

Asp Ile Gln Asp Leu Lys Phe Gly Val Glu Gln Asp Val Asp Met Val

225                 230                 235                 240

Phe Ala Ser Phe Ile Arg Lys Ala Ala Asp Val His Glu Val Arg Lys
                245                 250                 255

Val Leu Gly Glu Lys Gly Lys Asn Ile Lys Ile Ile Ser Lys Ile Glu
            260                 265                 270

Asn His Glu Gly Val Arg Arg Phe Asp Glu Ile Leu Glu Ala Ser Asp
        275                 280                 285

Gly Ile Met Val Ala Arg Gly Asp Leu Gly Ile Glu Ile Pro Ala Glu
    290                 295                 300

Lys Val Phe Leu Ala Gln Lys Met Met Ile Gly Arg Cys Asn Arg Ala
305                 310                 315                 320

Gly Lys Pro Val Ile Cys Ala Thr Gln Met Leu Glu Ser Met Ile Lys
                325                 330                 335

Lys Pro Arg Pro Thr Arg Ala Glu Gly Ser Asp Val Ala Asn Ala Val
            340                 345                 350

Leu Asp Gly Ala Asp Cys Ile Met Leu Ser Gly Glu Thr Ala Lys Gly
        355                 360                 365

Asp Tyr Pro Leu Glu Ala Val Arg Met Gln His Leu Ile Ala Arg Glu
    370                 375                 380

Ala Glu Ala Ala Ile Tyr His Leu Gln Leu Phe Glu Glu Leu Arg Arg
385                 390                 395                 400

Leu Ala Pro Ile Thr Ser Asp Pro Thr Glu Ala Ala Ala Val Gly Ala
                405                 410                 415

Val Glu Ala Ser Phe Lys Cys Cys Ser Gly Ala Ile Ile Val Leu Thr
            420                 425                 430

Lys Ser Gly Arg Ser Ala His Gln Val Ala Arg Tyr Arg Pro Arg Ala
        435                 440                 445

Pro Ile Ile Ala Val Thr Arg Asn Pro Gln Thr Ala Arg Gln Ala His
    450                 455                 460

Leu Tyr Arg Gly Ile Phe Pro Val Leu Cys Lys Asp Ala Val Leu Asp
465                 470                 475                 480

Ala Trp Ala Glu Asp Val Asp Leu Arg Val Asn Leu Ala Met Asn Val
                485                 490                 495

Gly Lys Ala Arg Gly Phe Phe Lys Lys Gly Asp Val Val Ile Val Leu
            500                 505                 510

Thr Gly Trp Arg Pro Gly Ser Gly Phe Thr Asn Thr Met Arg Val Val
        515                 520                 525

Pro Val Pro
    530

<210> 3
<211> 11788

<212> DNA
<213> Rattus norvegicus

<220>
<223> Pyruvate kinase

<400> 3
   cccgggcgag cgccgggagg gtggagagtc accgggcggg gctggaggaa tgtccgggac 60
   ctataaatct gggcaacgcc ctggtaggcc agggcagatg gggcacctgg gcagaattcc 120
   aaaatgggat tatgtagcct ctgaggtcct aaagcaacag gtggcggacc acccggggat 180
   ctaggggtgg tggcggcggt ggacccgagg gcgggtcctg cctcctcacc acttccccat 240
   tggccatcag aatgacccat gcgcaatttt ggtttgcaat gtccttccgc cacggaaggt 300
   agtccccctc aaaagggcaa cctgcttgtc ccgcctaccc tgcgactctc tcagaaggtg 360
   cgggtgcctg ttgagaggcg gggctctgct agctgctgcc cggattgggc gaggggcggg 420
   gctgcggagg gattgcggcg gcccgcagca gtgataacct tgaggcccag tctgcgcagc 480
   cccgcacagc agcgacccgt cctaagtcga cagacgtcct ctttaggtat tgcaacagga 540
   tctgaagtac gcccgaggtg agcggggaga acctttgcca ttctgtggcc cagagccaat 600
   gccatagtgg atcactagtg ggtggctgca atgtcaggtg gcagagccag gcttgggtgc 660
   atgttcttta ggtatccaga aaagccgatg tccagggtag agtgcgggtc tttgggaagg 720
   cagggaagag caaggagatg tggagtgcag gctgtgtgct caggagtttg aaaggagcct 780
   cgtggcctga aaattgattc aagtctctgg tattctgtga ggtacatccc aggttctcgc 840
   ttactcagcc aactatccag ctcagtgagt gcttagctgt aagtcgtaat aaagtaaaag 900
   caaaggtcag gacagctgtt gggattgtga cctgggtgat taattgcctg gaacagatgt 960
   tcagactact gtgtcgtggt ccctatcaga cagagaagga gactctagat agccagaact 1020
   ggggctggga aagctgggtc taatgtcagc tcttgggaag agtagtgact ttcaaggtca 1080
   acctcggttg cacagtggat ttcaggccag catagactag ctactgtgtg tgattccatc 1140
   tcctaggtta ctgaagactc cttggtgagg ttgtaatgac tttggagcca tttcaatgtc 1200
   cttgtgcaaa atttgctttg tgtgtcttgt acatggtgct gcttttttgc ttcttgtcca 1260
   gaaaaaaact tgttgcctgt aatttggacc ttattcctgt gtcggataga ggctaaggaa 1320
   tacatttagg tgatacctca aacctaggac tttaaaaaga aaggacttgg gacagggttc 1380
   gtctcttggc agcaggatcg tttaggtcat ggcgaacatg ataaagaggc tatgtcagtg 1440
   ccaaagcagg tttgacattg tcgtgatagg cttcccagat gttcgtgtgc tgacacatta 1500
   gctacgtgct ctgcaggagt ggggaggttt ccttgtgtgg gaaggtatgt tctctctagg 1560
   gttggtgtga agctcttcaa tagatggcag ccgttgtaag aagctctgct taaactggca 1620
   gccagggtgt gtatgtgcac tgggaaggaa gctacttttg ttcaccaagg tctttgctgt 1680
   tcgttcatag tcacaggagc cctttgccca atttgagtag tacccacata accagcacgt 1740
   gccaggcctc tgagtgactt gtgcacaaag cagtcttatt gggttaatgt gtaagcggga 1800
   gacagtctcc tatgccaaat ggttcattaa gcactgccta gtctaattga actcttcaaa 1860
   tctccgggat ccaggatctc agaaaccatg cccaagccag acagcgaagc agggactgcc 1920
   ttcattcaga cccagcagct ccatgcagcc atggctgaca ccttcctgga acacatgtgc 1980
   cgcctggaca ttgactccgc acccatcacg gcccgcaaca ctgggatcat ctgtaccatt 2040
   ggtgagtgag tgtggccccc ttcgcagggc ttctgcctgg tttgaaaggc gtaataacca 2100
   ttgcagggct aacctaggtt ctgagagaca tgtccacact tttagaggaa cgtattggtg 2160
   tacttcttct gcacttggtg aggttcaggg tggtctagtg gcctttgcag gagtagttca 2220
   gggacttcag atctttttcc tgtgctgtca ctccactttc tgcagaattt atagaaaatg 2280
   atagtgtcct tccttacata taaattaaga gagtccttcc ttacatataa atcaagagag 2340
   ttgactagca cagtagtctt tctggacttt ggaaagacct ttctctaaat ttgtggaggt 2400
   attaaaaaca aaggacaagg agttgccttt gatagtagca gatttcatat actttcctac 2460
   ctgagaatca tgattttctt cctgtggctc taaatgtttc ttggtaggcc ctgcttcccg 2520
   atctgtggag atgctgaagg agatgattaa gtctgggatg aatgtggctc ggctgaattt 2580
   ctctcatgga acccatgagg tgagtggcag cttgatccag gaggttcggg acttgctgct 2640
   gctgtgctcc aggcttctca gtgaatggac gttgctctac ttacatttag atcccctctc 2700
   tccctcttcc cctctggcca acttcattgg catcaacaac ctgcctgctt tgtcgccggc 2760
   caggttatcc ctaccatcaa gcttcaccag ggctttctta ttctatgtgc accttcgatg 2820
   aggggctgtg gctgtctgtc ttggatgagg ggctgtggct gtctgtcttg gttttaacca 2880
   ttcctggaga cggtggaatg aatcgtagct ccctcgagct cttggcctaa ttgatcatct 2940
   ttggctccag ggtggctcag ggcatgggaa ctggacttgg gtgtggagaa cacctacctg 3000
   tcaatctccc cttctttctc tccagtacca tgcagagact atcaagaatg tccgtgcagc 3060
   cacagaaagc tttgcatctg atcccattct ctaccgacct gttgcggtgg ctctggatac 3120
   aaagggacct gagatccgga ctggactcat caagggcgtg agtatccagg agtttaggtc 3180
   tttaaatgag aatatttttc atctgcctgg taggaattat tatagatgca catttttggt 3240
   atgtgaataa catacttaag tctcactctg gggacctggt ttgttgtttg ttttgtttgt 3300
   ttccctcaat aaacaaattc aggatttaca gaaaggtgat cggtttcttg gggctttgag 3360
   ccagagtttg agcgccgcca tcagggtgtt ggcgtccaca gtcacacgcc tctgctgtct 3420
   ttaatctaga gcggcaccgc agaggtggag ctgaagaagg gagccacact gaagatcacc 3480
   ctggacaacg cctacatgga gaagtgcgac gagaacatcc tgtggctgga ctataagaac 3540
   atctgcaagg tggtggaggt gggcagcaag atctacgtgg acgatgggct catctccctg 3600
   caggtgaagg agaaaggtat gtgtggtgta cagtccacgg cccaatgcca ctcccatccc 3660
   cagaactctg gtaagcactt aacctagcat gtatgaattg gtctcccaag gtcaaaagtt 3720
   taaggtggtt gttggtctgc atccctggcc tgtctgaaac actgcctgag aaaaaataga 3780
   caaataacct acaaaggcct atgtgtacac ctctaccctt tagttccagc actcgggaat 3840
   cagcaggtgt gtgagttctc atgtgtaagg actactcctg tatgcctaga atgagtctag 3900
   agttctcttg gcttctcaca aactgagata gatggtcttg atccctttca cacaggtgct 3960
   gactacctgg tgacagaagt ggaaaatggt ggctccttgg gcagcaagaa gggcgtgaac 4020
   ctgcctggtg ctgctgtgga cctccctgct gtgtcagaaa aggacatcca ggacctgaag 4080
   tttggggtgg agcaggacgt ggacatggtg tttgcgtctt tcatccgcaa ggcggctgac 4140
   gtgcatgagg ttaggaaggt cctgggagag aagggcaaga acatcaagat catcagcaaa 4200
   atcgagaacc atgaaggtgt ccgcaggtga aggtccactc ctaccgtgtg cctggggtgt 4260
   ggccttgagg gcacctctgt cagggcccag gaaagctctg tccattcttt ggttgtactt 4320
   cctgttctat agcatctttc tttttatcag gtttgatgag attttggagg ccagcgatgg 4380
   aatcatggta gctcgtggtg acctgggcat tgagattccg gcagagaagg tcttcctagc 4440
   tcagaagatg atgattggac gatgcaaccg agctgggaag ccagtcatct gcgccaccca 4500
   ggcatgtgct atcccttcct tctgtgttct ccacctagga gacctggtct tgacctggcc 4560
   tttaggtaca cgtacccgca catagctatg acctgcgtac ctgtgcaagc ttcggggaat 4620
   tgccctggaa tcatcactga agatgtcctg ctcttccatt atttagtgac tttcatttag 4680
   cggtggtctc ttacttaata aaaacccttg tttgtcccct cctagatgct ggagagcatg 4740
   atcaagaagc cacgccccac ccgtgctgaa ggcagtgacg tggccaatgc agtcctagat 4800
   ggagctgact gcatcatgct gtccggagaa acagccaaag gggactaccc tctggaggct 4860
   gttcgcatgc agcacctggt gagtaagtcc tcagagtcct ggggtagaag agctctgctg 4920
   gagaggcctc tgtccagtct tgttacattg ctcccgtcac agcaaggaga gtgaggtttg 4980
   tggagtgtgc ttgagtttca ttgtgctttc actgcctgca cctgcccctt tgtcctgctc 5040
   tggggattac ataggccaca tctggctaaa atatcaaggt cctaggatgc agtcaaggga 5100
   tgccttcctt gtggacaccc agagggcctg ggtacctcta ttctaaagga gccaagagtt 5160
   tgttcagcta ttcgccttgt tacctctcat ttggtctcct gtggtctgcc catgggcaat 5220
   gcttctctcc actgcagctg tagccatact gagctgcttt aagctggccg tgcatggtgc 5280
   ctgtgacatg ggacttcctc ccttgctgtg ccagacccaa ctcggggcta caaatagctc 5340
   tgggggtggg gaatgggtgc taatttagca ggttctgtct ggtctaggaa ttataaagac 5400
   ttctcaggca tattatgtgc tcttttgatt aagtcttctg gtttcagtaa gatagggtct 5460
   ggcgcaagct tgtaattcca acaatcaaat caagcctgcg gcaggaagat ggcttgagat 5520
   taggctggac tagaatgaga catcttgtag aagcaacagg cgtcagtgac ctgtccctgc 5580
   ggactttcca cccaggcctc cgtcttctgt gttctgctcc agaacttctt ccggagcaga 5640
   acaattatct ggggcatcag ttagggaccg ttgcctgata aaggcatggt ggattagctt 5700
   tttgggggtt ttctattccc ttttcagcca acaaagaacc acagtatctt ttgtggctcc 5760
   agtttcccac aagccccccc aaacttaaga caagtgaaag aaaagaagga agccccttct 5820
   gggttaggca tcttaccttg ctttatatgt atatgagagt tgagtagatt ccatgatctt 5880
   tccatggatt tatctgaatt atagctgtag ccagatgttt gcctatattg aaacagacca 5940
   gcgctttgac tcggctggtc atgacctgtt ctgattggaa ttgtgactgg tcctcatagc 6000
   ttctcatagc tgcaggcatt ctctcccaga agacttttcc cttccttcaa tctcccccac 6060
   agtagctgcc ttcctggttt acaccaaaac cccttttcta cttacctggc ctcacagtgg 6120
   tcaggaacag gactttgacc aggtattcta aagcatgtag tcacataaat gtatttttgg 6180
   atagctcaag aagaccttgc ccaaagtggc ggttgatgag attaattaca attaattata 6240
   tgtgtatgag ttagttttgg tgtcctgtcc ttttctcatg ccctggtctt tcctatgaac 6300
   agacacctta gaacctcgag gctgggattg catggccctg ctcagaagat gagtcacaga 6360
   gtccgggtta gactgtggct accccctcag ggatacaaat ttctctatca gttaacactt 6420
   aggacagctt ctcccctttc tttatctgtt tgctgtttcc tcctgtgtct aactgattca 6480
   gttcaaaacc tgtaattaaa agctagacat cccagctgtg gttgcttcct gtccatgcct 6540
   cttgtccttt gggcttgcct gcattccatg cttaaccaaa agattacttt ccacttgcaa 6600
   atctgctaat gctaccaata aaatcgagtg ctggtttcat atcattcctg gaattgcact 6660
   ctctaaaact ttatttcttt atttctaatg cttggtttta cttgaacagg tggtctgtat 6720
   ataacacatt tgctatcctg taaccgtttt aattattgca gtttgaatct gtgtcttgaa 6780
   aggcctgtgt gctttcccag gcgtctgcct cctcacatgg ctgttcagtg taccgtgttc 6840
   aagtgagcca gtcgaccact gttctgttta gaacttgtgc actgcaactt tggctctttt 6900
   gaccctcacc cccagctttc agagctgccc gagtgtttcc actgtaagcc aagtgcaagc 6960
   gctcactctt gtgtgtaggc ggagttggat gccttgtaac cacatagcca tgtgaggagg 7020
   ggacgccttt ttcttcctgt aagctgtgtc aggaggcagt gtggtcaagc ggaagtgtag 7080
   ttggctccac cttggcatct ttccatgcca gggtcccttt ctcagcttta atcaaaaaca 7140
   aaagaaatga tggatggtgc atgcctagca cttgggaggc tgaggcaaag aaaatagtgg 7200
   tgttttgagg ctgaccatgg caagttcaag gccagtaggt gctatggtaa gatcctatct 7260
   caaacctgtg tggggttgag aattggcttt ttttgtttgt ttgtttttag aaacaggaaa 7320
   tcccaaaagg atactatctt tccagaaggg aaggaaggat ctgtccgtgt gtttgaacag 7380
   aaaagagagc cacccaaaat cccacatata ataccattcc aggctttgac catcctgcct 7440
   ctgtatctgc atggagaaga aaagattaac ctaagagttc cttcctctca ttagttccct 7500
   gtctttccat gtgttgtctc ttgtttttgc cttcatcctt cttccttatc cttcctaccc 7560
   taaaccttac agatagctcg agaggctgag gcagccgtgt tccaccgcct gctgtttgaa 7620
   gagcttgcgc gagcctccag tcaatccaca gaccccctgg aggccatggc catgggcagc 7680
   gtggaggcct cttataaatg tttagcagca gctttgatag ttctgacgga gtccggcagg 7740
   tagggccctg agggcaggta tcattataag ataaccagct tctcacacaa ctagggccca 7800
   ctgtgtgcct gagcctgggc atagcctctc tcctgcagga aggcagccaa ggaggtcacg 7860
   atagggcagg accaaaggat tccctagtgg gtacagtgga agtcacaggc actggttcag 7920
   gatggttccc tgtggagttt ctaatcttgc tcaagtttca gaaacgtgtt agtgaactca 7980
   tctttctcct ggctttttgt gcccaggaca tgttccttcc cagttgcctg tgactcttct 8040
   ccttcatttg tgacaaagct ctgacaaagc cctgtccccc ttcctcgtcc ctctggacgg 8100
   atgttgctcc cctagattgc ccgagaggca gaggctgcca tctaccactt gcagttattc 8160
   gaggaactcc gccgcctggc gcccattacc agcgacccca cagaagctgc cgccgtgggt 8220
   gccgtggagg cctccttcaa gtgctgcagt ggggccatta tcgtgctcac caagtctggc 8280
   aggtaggagg cggcagtggc tccctgggga tcccagagca actctgggct gattttaaga 8340
   cccctggctg ccatcaaagg actccaggaa gcactcccag gtacatcaga ttaggtggct 8400
   ccagtgccag tcagtgcagg cctgcctcag ggcctgaagc atatatacag ttttgcttag 8460
   ttacggtgtg gaagctgggc atgggtacat gcctttaatc ctcccagcat tcgggcagaa 8520
   gaggtgggca aatgtctgca ttcgtggaca gtgctggtct acaaagcaag ttccaggaca 8580
   gagccctagc tcacaaaaat taactcagct ggtaaatact acttgctaca ccaagttaga 8640
   cggatggagg agggagaaag gtaacttagc aagcgtgccc aaggacatat acagtaacac 8700
   tttttctcat cccacttgga gggagctcaa tggataaagt gttggcaatc tggatttgga 8760
   tctggcacct gtgggttttt gttgttgttt gggtttttgt tttttttttt ttttttaagt 8820
   cacctggaga agtacgttag gcctttagga gcatggggtt ttggttttta agatttattt 8880
   attatatatg aactacactc tccctatctt cagtcgcacc agaagagggt gattacagat 8940
   gattgtgtaa gccaccatgt ggttgctggg agttgaactc aggacctctg gaagagcagt 9000
   tagtgctctt aaccactgag ccatctctcc agccagcact gttctttcag aagcccaaca 9060
   catacatgac agctcacact atctaactgc agtcctgggg ggaatctaat gccctcttct 9120
   aacttccagc cgtacatgtt tatatgatgc atgtggtcag gatacccaga cagctggagg 9180
   gaaaggaagg tgcccaacac acctgtgtgc ctgtaatccc agcactcatg gtacagaggt 9240
   agaaagatca caagataggt ctaatctgag caaattctgc aacagcctag atacagaaag 9300
   ccaaacacat aaaaatcagt gtgggctgag gatgttgacc tttgtgtgaa ttggccatga 9360
   acattgtatg cagctggaag caagggatgg ggtgctgaaa aatggggacc ttaaaactaa 9420
   ttttctgaag tcctgaagtt tcgagagctc caagatacaa gttctatgct aatcctggac 9480
   tttctctgaa gagttcctgg ttccttcaaa gcccatgtag gccagctaga agcagggtat 9540
   tttctgcctg tggaatggcc acatccccta ataccttgtg tgtcctcaag atgcctagtt 9600
   aattcagtaa gcctgaaaca gatctgatag aagtaaacat atcatttgct atcggttgag 9660
   gagagagggg ctccctacat tcccaaagga tcttgattgg ccagatatta accatgcctg 9720
   gtcacaccta gattttccaa aaaaccatag aaattcagag gtttctttta gcaccaagtt 9780
   cagctgataa gactcccaaa ggagcagtat agtactctct ggcaaatact gtccactcca 9840
   gggtctctgc agataccaga agtcaggagc taggcacatg gtgcttggat tgtaaaagtt 9900
   gctggcagct acaggatggt tctggtgaga ttaggccaga gctgcctcac tgcctgatgg 9960
   aagggttcac agtgtgggag ggatcctgcc agccgtggtc ctatgggact gcccacactg 10020
   agatcaggac aatgagttaa aaggacaggg acaggtctga gggggtggcc aggcactaaa 10080
   cagtaattgt aagtgggcaa acctgtggcc tagaggtgaa ttagagggtg ctcctttggc 10140
   tgactgaaag ggtctcgtga cacaatggcc attcttccca ccttctcagg agtgctcacc 10200
   aagtggcccg gtaccgccca agggctccta tcattgctgt gacacgcaat ccccagacag 10260
   cccgccaggc ccatctgtac cgtggcatct tccctgtgct gtgtaaggat gccgtactgg 10320
   atgcctgggc tgaggacgtt gatcttcgtg tgaacttggc catgaatgtt ggtatgtagc 10380
   tggaagcaag ggatggggtg ctgggaaatg gggagcctaa aactaccatt tcctgaagcc 10440
   ctcacccaat tttgggccca aggcaaaatt aattgcctca ttaggctctt gtaagattaa 10500
   ttggagctac tgccctgtgg ggtggggcac acaagctgtt gtggtctttg ttcctatata 10560
   aggttggata tcaagagaca aggaagcagc tgaccctgaa cttgggcaag gctggccact 10620
   ctagagtcta aactgaatgg tgtccaaagg tccctgcagt ctgagttttg cctcagccct 10680
   tgtttaagct aggagacttg caccaccttg tggtaaaaag aagtaactgg caacctgctc 10740
   tcctacctga attagaagca atagggcatt tgttatcctg cctggttagg cttgtgtctg 10800
   ggctgggaga aagcaccacc ttgtggtagg agagagtact ggttgctcag ttaggactgg 10860
   gtagggcttt gcagttatcc ccaagtgtta tatacctcta ctcaccaacc tccttctccc 10920
   ctcaggcaag gcccgaggct tcttcaagaa gggagatgtg gtcattgtgc tgactggatg 10980
   gcgccctggc tctggcttca ccaacaccat gcgtgtagtg cctgtaccat gatgatcctc 11040
   tggagcttct cttctagccc ctgtcccttc ccctccccta tcctatccat taggccagca 11100
   acgttgtagt gctcactctg ggccatagtg tggcgctggt gggctgggac accaggaaaa 11160
   attaatgcct ctgaaacatg caatagagcc cagctatttt tcatggccct acttgagcca 11220
   ggggtgaagg aggaatgcag gattggaaac cctctgactt tatcacagaa gggcagcatt 11280
   atctctgtgt tctttgctcc tgtagaaagt tttccagaga attcccagcc ctggcctgga 11340
   atcaggagac agcaagaaca gaggctgggg gcccagggtt cccatgtaga tgacttttgg 11400
   ccctgtccct gacttgcttt cccaacagct ttggcctctc tcctcgtgca ctccactgct 11460
   gtccctgcag atgttccact ctccacctcg tactctgcag cgtctccagg cctgttgcta 11520
   tagtgcccac ctgaatgtca ataaacagca gcggaacgac ctgtgttctt ttcttcctgg 11580
   ggggcgtggt tgaggctatc ctctgagcag tcgaaaatga caaccggcct aaaggctcag 11640
   cctggagccg agtatatagg ttgcctcctc caacagcacc aagtggggct atctatgtgt 11700
   tggaaagctc actgttttat ttcaggacag actggaaagg tctgggacag gttcagctag 11760
   cacctgtaat ggtgggtaga ccggaggg 11788

<210> 4
<211> 446
<212> PRT
<213> Rattus norvegicus

<220>
<223> Dopamine receptor D1

<400> 4
Met Ala Pro Asn Thr Ser Thr Met Asp Glu Ala Gly Leu Pro Ala Glu
1               5                   10                  15

Arg Asp Phe Ser Phe Arg Ile Leu Thr Ala Cys Phe Leu Ser Leu Leu
            20                  25                  30

Ile Leu Ser Thr Leu Leu Gly Asn Thr Leu Val Cys Ala Ala Val Ile
        35                  40                  45

Arg Phe Arg His Leu Arg Ser Lys Val Thr Asn Phe Phe Val Ile Ser
    50                  55                  60

Leu Ala Val Ser Asp Leu Leu Val Ala Val Leu Val Met Pro Trp Lys
65                  70                  75                  80

Ala Val Ala Glu Ile Ala Gly Phe Trp Pro Phe Gly Ser Phe Cys Asn
                85                  90                  95

Ile Trp Val Ala Phe Asp Ile Met Cys Ser Thr Ala Ser Ile Leu Asn
            100                 105                 110

Leu Cys Val Ile Ser Val Asp Arg Tyr Trp Ala Ile Ser Ser Pro Phe
        115                 120                 125

Gln Tyr Glu Arg Lys Met Thr Pro Lys Ala Ala Phe Ile Leu Ile Ser
    130                 135                 140

Val Ala Trp Thr Leu Ser Val Leu Ile Ser Phe Ile Pro Val Gln Leu
145                 150                 155                 160

Ser Trp His Lys Ala Lys Pro Thr Trp Pro Leu Asp Gly Asn Phe Thr
                165                 170                 175

Ser Leu Glu Asp Thr Glu Asp Asp Asn Cys Asp Thr Arg Leu Ser Arg
            180                 185                 190

Thr Tyr Ala Ile Ser Ser Ser Leu Ile Ser Phe Tyr Ile Pro Val Ala
        195                 200                 205

Ile Met Ile Val Thr Tyr Thr Ser Ile Tyr Arg Ile Ala Gln Lys Gln
    210                 215                 220

Ile Arg Arg Ile Ser Ala Leu Glu Arg Ala Ala Val His Ala Lys Asn
225                 230                 235                 240

Cys Gln Thr Thr Ala Gly Asn Gly Asn Pro Val Glu Cys Ala Gln Ser
                245                 250                 255

Glu Ser Ser Phe Lys Met Ser Phe Lys Arg Glu Thr Lys Val Leu Lys
            260                 265                 270

Thr Leu Ser Val Ile Met Gly Val Phe Val Cys Cys Trp Leu Pro Phe
        275                 280                 285

Phe Ile Ser Asn Cys Met Val Pro Phe Cys Gly Ser Glu Glu Thr Gln
    290                 295                 300

Pro Phe Cys Ile Asp Ser Ile Thr Phe Asp Val Phe Val Trp Phe Gly
305                 310                 315                 320

Trp Ala Asn Ser Ser Leu Asn Pro Ile Ile Tyr Ala Phe Asn Ala Asp
                325                 330                 335

Phe Gln Lys Ala Phe Ser Thr Leu Leu Gly Cys Tyr Arg Leu Cys Pro
            340                 345                 350

Thr Thr Asn Asn Ala Ile Glu Thr Val Ser Ile Asn Asn Asn Gly Ala
        355                 360                 365

Val Val Phe Ser Ser His His Glu Pro Arg Gly Ser Ile Ser Lys Asp
    370                 375                 380

Cys Asn Leu Val Tyr Leu Ile Pro His Ala Val Gly Ser Ser Glu Asp
385                 390                 395                 400

Leu Lys Lys Glu Glu Ala Gly Gly Ile Ala Lys Pro Leu Glu Lys Leu
                405                 410                 415

Ser Pro Ala Leu Ser Val Ile Leu Asp Tyr Asp Thr Asp Val Ser Leu
            420                 425                 430

Glu Lys Ile Gln Pro Val Thr His Ser Gly Gln His Ser Thr
        435                 440                 445


<210> 5
<211> 960
<212> PRT
<213> Mus musculus

<220>
<223>  GABA-B1a receptor

<400> 5

Met Leu Leu Leu Leu Leu Leu Leu Leu Phe Leu Arg Pro Leu Gly Ala
1               5                   10                  15

Gly Gly Ala Gln Thr Pro Asn Val Thr Ser Glu Gly Cys Gln Ile Ile
            20                  25                  30

His Pro Pro Trp Glu Gly Gly Ile Arg Tyr Arg Gly Leu Ile Arg Asp
        35                  40                  45

Gln Val Lys Ala Ile Asn Phe Leu Pro Val Asp Tyr Glu Ile Glu Tyr
    50                  55                  60

Val Cys Arg Gly Glu Arg Glu Val Val Gly Pro Lys Val Arg Lys Cys
65                  70                  75                  80

Leu Ala Asn Gly Ser Trp Thr Asp Met Asp Thr Pro Ser Arg Cys Val
                85                  90                  95

Arg Ile Cys Ser Lys Ser Tyr Leu Thr Leu Glu Asn Gly Lys Val Phe
            100                 105                 110

Leu Thr Gly Gly Asp Leu Pro Ala Leu Asp Gly Ala Arg Val Asp Phe
        115                 120                 125

Arg Cys Asp Pro Asp Phe His Leu Val Gly Ser Ser Arg Ser Ile Cys
    130                 135                 140

Ser Gln Gly Gln Trp Ser Thr Pro Lys Pro His Cys Gln Val Asn Arg
145                 150                 155                 160

Thr Pro His Ser Glu Arg Arg Ala Val Tyr Ile Gly Ala Leu Phe Pro
                165                 170                 175

Met Ser Gly Gly Trp Pro Gly Gly Gln Ala Cys Gln Pro Ala Val Glu
            180                 185                 190

Met Ala Leu Glu Asp Val Asn Ser Arg Arg Asp Ile Leu Pro Asp Tyr
        195                 200                 205

Glu Leu Lys Leu Ile His His Asp Ser Lys Cys Asp Pro Gly Gln Ala
    210                 215                 220

Thr Lys Tyr Leu Tyr Glu Leu Leu Tyr Asn Asp Pro Ile Lys Ile Ile
225                 230                 235                 240

Leu Met Pro Gly Cys Ser Ser Val Ser Thr Leu Val Ala Glu Ala Ala
                245                 250                 255

Arg Met Trp Asn Leu Ile Val Leu Ser Tyr Gly Ser Ser Ser Pro Ala
            260                 265                 270

Leu Ser Asn Arg Gln Arg Phe Pro Thr Phe Phe Arg Thr His Pro Ser
        275                 280                 285

Ala Thr Leu His Asn Pro Thr Arg Val Lys Leu Phe Glu Lys Trp Gly
    290                 295                 300

Trp Lys Lys Ile Ala Thr Ile Gln Gln Thr Thr Glu Val Phe Thr Ser
305                 310                 315                 320

Thr Leu Asp Asp Leu Glu Glu Arg Val Lys Glu Ala Gly Ile Glu Ile
                325                 330                 335

Thr Phe Arg Gln Ser Phe Phe Ser Asp Pro Ala Val Pro Val Lys Asn
            340                 345                 350

Leu Lys Arg Gln Asp Ala Arg Ile Ile Val Gly Leu Phe Tyr Glu Thr
        355                 360                 365

Glu Ala Arg Lys Val Phe Cys Glu Val Tyr Lys Glu Arg Leu Phe Gly
    370                 375                 380

Lys Lys Tyr Val Trp Phe Leu Ile Gly Trp Tyr Ala Asp Asn Trp Phe
385                 390                 395                 400

Lys Thr Tyr Asp Pro Ser Ile Asn Cys Thr Val Glu Glu Met Thr Glu
                405                 410                 415

Ala Val Glu Gly His Ile Thr Thr Glu Ile Val Met Leu Asn Pro Ala
            420                 425                 430

Asn Thr Arg Ser Ile Ser Asn Met Thr Ser Gln Glu Phe Val Glu Lys
        435                 440                 445

Leu Thr Lys Arg Leu Lys Arg His Pro Glu Glu Thr Gly Gly Phe Gln
    450                 455                 460

Glu Ala Pro Leu Ala Tyr Asp Ala Ile Trp Ala Leu Ala Leu Ala Leu
465                 470                 475                 480

Asn Lys Thr Ser Gly Gly Gly Gly Arg Ser Gly Val Arg Leu Glu Asp
                485                 490                 495

Phe Asn Tyr Asn Asn Gln Thr Ile Thr Asp Gln Ile Tyr Arg Ala Met
            500                 505                 510

Asn Ser Ser Ser Phe Glu Gly Val Ser Gly His Val Val Phe Asp Ala
        515                 520                 525

Ser Gly Ser Arg Met Ala Trp Thr Leu Ile Glu Gln Leu Gln Gly Gly
    530                 535                 540

Ser Tyr Lys Lys Ile Gly Tyr Tyr Asp Ser Thr Lys Asp Asp Leu Ser
545                 550                 555                 560

Trp Ser Lys Thr Asp Lys Trp Ile Gly Gly Ser Pro Pro Ala Asp Gln
                565                 570                 575

Thr Leu Val Ile Lys Thr Phe Arg Phe Leu Ser Gln Lys Leu Phe Ile
            580                 585                 590

Ser Val Ser Val Leu Ser Ser Leu Gly Ile Val Leu Ala Val Val Cys
        595                 600                 605

Leu Ser Phe Asn Ile Tyr Asn Ser His Ala Arg Tyr Ile Gln Asn Ser
    610                 615                 620

Gln Pro Asn Leu Asn Asn Leu Thr Ala Val Gly Cys Ser Leu Ala Leu
625                 630                 635                 640

Ala Val Val Phe Pro Leu Gly Leu Asp Gly Tyr His Ile Gly Arg Ser
                645                 650                 655

Gln Phe Pro Phe Val Cys Gln Ala Arg Leu Trp Leu Leu Gly Leu Gly

            660                 665                 670

Phe Ser Leu Gly Tyr Gly Ser Met Phe Thr Lys Ile Trp Trp Val His
        675                 680                 685

Thr Val Phe Thr Lys Lys Glu Glu Lys Lys Glu Trp Arg Lys Thr Leu
    690                 695                 700

Glu Pro Trp Lys Leu Tyr Ala Thr Val Gly Leu Leu Val Gly Met Asp
705                 710                 715                 720

Val Leu Thr Leu Ala Ile Trp Gln Ile Val Asp Pro Leu His Arg Thr
                725                 730                 735

Ile Glu Thr Phe Ala Lys Glu Glu Pro Lys Glu Asp Ile Asp Val Ser
            740                 745                 750

Ile Leu Pro Gln Leu Glu His Cys Ser Ser Lys Lys Met Asn Thr Trp
        755                 760                 765

Leu Gly Ile Phe Tyr Gly Tyr Lys Gly Leu Leu Leu Leu Leu Gly Ile
    770                 775                 780

Phe Leu Ala Tyr Glu Thr Lys Ser Val Ser Thr Glu Lys Ile Asn Asp
785                 790                 795                 800

His Arg Ala Val Gly Met Ala Ile Tyr Asn Val Ala Val Leu Cys Leu
                805                 810                 815

Ile Thr Ala Pro Val Thr Met Ile Leu Ser Ser Gln Gln Asp Ala Ala
            820                 825                 830

Phe Ala Phe Ala Ser Leu Ala Ile Val Phe Ser Ser Tyr Ile Thr Leu
        835                 840                 845

Val Val Leu Phe Val Pro Lys Met Arg Arg Leu Ile Thr Arg Gly Glu
    850                 855                 860

Trp Gln Ser Glu Thr Gln Asp Thr Met Lys Thr Gly Ser Ser Thr Asn
865                 870                 875                 880

Asn Asn Glu Glu Glu Lys Ser Arg Leu Leu Glu Lys Glu Asn Arg Glu
                885                 890                 895

Leu Glu Lys Ile Ile Ala Glu Lys Glu Glu Arg Val Ser Glu Leu Arg
            900                 905                 910

His Gln Leu Gln Ser Arg Gln Gln Leu Arg Ser Arg Arg His Pro Pro
        915                 920                 925

Thr Pro Pro Asp Pro Ser Gly Gly Leu Pro Arg Gly Pro Ser Glu Pro
    930                 935                 940

Pro Asp Arg Leu Ser Cys Asp Gly Ser Arg Val His Leu Leu Tyr Lys
945                 950                 955                 960

<210> 6
<211> 4365

<212> DNA
<213> Mus musculus

<220>
<223> GABA-B1a receptor

<400> 6
   taacttgcgg ccgccgtcgc cccggggaag gagagacggg ggtggggttt gcgggtagcg 60
   atcgcgaagg ggagagaccc cggccgggct ggagcctgga ttcctgtgga agaagaacag 120
   ggggagggga agctggagga ccgggaggga gaacggggag ccgcggccgg gcctgggccc 180
   ttgaggcccg gggagagccg cggagcggga ccggccgccg agatgctgct gctgctgctt 240
   ctgcttctct tcctccgccc cctgggcgct ggcggggctc agacccccaa cgtcacctcg 300
   gaaggttgcc agattataca tccgccctgg gaaggtggca tcaggtaccg tggcttgatt 360
   cgcgaccagg tgaaggccat caatttcctg cctgtggact atgagattga atatgtgtgc 420
   cggggcgaac gcgaggtggt ggggcccaag gtgcgcaagt gcctggccaa cggctcctgg 480
   acggatatgg acacacccag tcgctgtgtc cgaatctgct ccaagtctta tttgaccctg 540
   gaaaatggga aggttttcct gacgggtggg gacctcccag ctctggatgg agcccgggtg 600
   gatttccgat gtgaccctga cttccatctg gtgggcagct cccggagcat ctgtagtcag 660
   ggccagtgga gcacccccaa gccccactgc caggtgaatc gaacgccaca ctcagaacgg 720
   cgtgcagtat acatcggggc gctgtttccc atgagcgggg gctggccggg gggccaggcc 780
   tgccagcctg cggtggagat ggcgctggag gacgttaaca gccgcagaga catcctgccg 840
   gactacgagc tcaagcttat ccaccacgac agcaagtgcg acccaggcca agccaccaag 900
   tacttgtatg aactactcta caacgacccc atcaagatca tcctcatgcc cggctgcagc 960
   tctgtgtcca cactggtagc cgaggctgcc cggatgtgga accttattgt gctctcatat 1020
   ggctccagct caccagcctt gtcaaaccga cagcggtttc caacgttctt tcggacacat 1080
   ccatccgcca cactccacaa tcccacccgg gtgaaactct tcgaaaagtg gggctggaag 1140
   aagattgcca ccatccagca gactaccgag gtcttcacct caacactgga tgacctggag 1200
   gagcgagtga aagaggctgg gattgagatc acttttcgac agagtttctt ctcagatcca 1260
   gctgtgcctg ttaaaaacct gaagcgtcaa gatgctcgaa tcatcgtggg acttttctat 1320
   gagaccgaag cccggaaagt tttttgtgag gtctataagg aacggctctt tgggaagaag 1380
   tatgtctggt ttctcatcgg gtggtatgct gacaactggt tcaaaaccta tgacccgtca 1440
   atcaattgta cagtagaaga gatgactgag gcggtggagg gccatatcac cacggagatt 1500
   gtcatgctga accctgccaa cacccgaagc atttccaaca tgacatcaca ggaatttgtg 1560
   gagaaactaa ccaagcggct gaaaagacac cctgaggaga ctggaggctt ccaggaggca 1620
   ccactggcct atgatgctat ttgggccttg gctttggcct tgaacaagac ctctggagga 1680
   ggtggccgtt caggagtgcg cctggaggac tttaactaca acaaccagac cattacagac 1740
   caaatctacc gggccatgaa ctcctcctcc tttgagggtg tttctggcca cgtggtcttt 1800
   gatgccagcg gctcccggat ggcatggacg cttatcgagc agctacaggg cggcagctac 1860
   aagaagatcg gctactacga cagcaccaag gatgatcttt cctggtccaa aacagacaag 1920
   tggatcggag ggtctccccc agccgaccag accttggtca tcaagacatt ccgtttcctg 1980
   tcacagaaac tctttatctc cgtctcagtt ctctccagcc tgggcattgt tcttgctgtt 2040
   gtctgtctgt cctttaacat ctacaactcc cacgctcgtt atatccagaa ttcccagccc 2100
   aacctgaaca atctgactgc tgtgggctgc tcactggcac tggctgttgt cttccctctc 2160
   gggctggatg gttaccacat agggagaagc cagttcccgt ttgtctgcca ggcccgcctt 2220
   tggctcttgg gcttgggctt tagtctgggc tatggctcta tgttcaccaa gatctggtgg 2280
   gtccacacag tcttcacgaa gaaggaggag aagaaggagt ggaggaagac cctagagccc 2340
   tggaaactct atgccactgt gggcctgctg gtgggcatgg atgtcctgac tcttgccatc 2400
   tggcagattg tggacccctt gcaccgaacc attgagactt ttgccaagga ggaaccaaag 2460
   gaagacatcg atgtctccat tctgccccag ttggagcact gcagctccaa gaagatgaat 2520
   acgtggcttg gcattttcta tggttacaag gggctgctgc tgctgctggg aatctttctt 2580
   gcttacgaaa ccaagagcgt gtccactgaa aagatcaatg accacagggc cgtgggcatg 2640
   gctatctaca atgtcgcggt cctgtgtctc atcactgctc ctgtgaccat gatcctttcc 2700
   agtcagcagg acgcagcctt tgcctttgcc tctctggcca tcgtgttctc ttcctacatc 2760
   actctggttg tgctctttgt gcccaagatg cgcaggctga tcacccgagg ggaatggcag 2820
   tctgaaacgc aggacaccat gaaaacagga tcatccacca acaacaacga ggaagagaag 2880
   tcccgactgt tggagaagga aaaccgagaa ctggaaaaga tcatcgctga gaaagaggag 2940
   cgcgtctctg aactgcgcca tcagctccag tctcggcagc aactccgctc acggcgccac 3000
   cccccaacac ccccagatcc ctctgggggc cttcccaggg gaccctctga gccccctgac 3060
   cggcttagct gtgatgggag tcgagtacat ttgctttaca agtgaggggg catggagaag 3120
   gatcaagcca gtaggggagg gaagggtctg ggaagagggt gggggcctgg gaggagggta 3180
   aggactccta tctccaacct ggagagcaca cgctccaatc cccctcttat aaatacatgt 3240
   cgctctgtgc atctggggtt atttgggtct ccagtactct gggaaacaga ctgttttctt 3300
   tctcccctat aattttatat ctccacttca caggttttgt ttgaaccctg cttggagtta 3360
   ttattcactc atggctccag aggggcatct catttttctc cggtagcctg tcttgtacag 3420
   ttaccacagc aactcctgtc atttcagcag caggggtctt cctacactag cagggctctc 3480
   gctctctcca tttttcagcc tcagaatctc cttccattat tcttctcctt ctacatgtct 3540
   ccatggcttc ctctcccagg ggactcgttc tacacacata cacacacaca cacacacaca 3600
   cacacacaca cacacacaca cacaccccgc atcctgccct ctcctaggca gctgcatgtc 3660
   gtcctgtaca aatgtgctcg cttctgagtg ctttgtgcgg ccgttcactt gtgctgtctg 3720
   cataagctgc gtctgtgagt gcacggtggt ttgtgggtgc gtgaagtggc atgctccggt 3780
   aggtgtgtgt gtgtgtgccc acgcgtgcgc ccgtgtgcat gcgttcgtgt tgccctgact 3840
   ggctgtctca gccttctgag taattgggat tccagttgtc tgtctagctc atgtcctgtc 3900
   ttcttccagt agagccgtga acacccaaca cacacagtta atcgggctcc ccccagtcca 3960
   tgttttctga gccatccaaa aactctcctt ggccttaggt tcatctgcaa atgttccctc 4020
   tgttctttgc tctcgtgcgt ccaccttcat tctcttcagt catttctcag atctgctgcg 4080
   tcgtggtttc ctttccttca ttatcatcgt cattattttt cagaacttaa gggaaaaaga 4140
   aatggggaca ggttggaggc tgtttccagt ggaatagtgg gtgcgcgtcc tgaccaaatg 4200
   aaggcacgga cagatggact gacggggcgg gaggcggcgt ccctttcaca ccgtggtgtc 4260
   tcttgggggg gaaggatctc cctgaatctc aataaagcag tgaacagtaa aaaaaaaaaa 4320
   aaaaaaaaaa aaaaaaaaaa aaaaaaaaaa aaaaaaaaaa aaaaa 4365

<210> 7
<211> 582
<212> PRT
<213> Rattus norvegicus

<220>
<223> Differentiation-associated Na-dependent inorganic phosphate
cotransporter

<400> 7
Met Glu Ser Val Lys Gln Arg Ile Leu Ala Pro Gly Lys Glu Gly Ile
1               5                   10                  15

Lys Asn Phe Ala Gly Lys Ser Leu Gly Gln Ile Tyr Arg Val Leu Glu
            20                  25                  30

Lys Lys Gln Asp Asn Arg Glu Thr Ile Glu Leu Thr Glu Asp Gly Lys
        35                  40                  45

Pro Leu Glu Val Pro Glu Lys Lys Ala Pro Leu Cys Asp Cys Thr Cys
    50                  55                  60

Phe Gly Leu Pro Arg Arg Tyr Ile Ile Ala Ile Met Ser Gly Leu Gly
65                  70                  75                  80

Phe Cys Ile Ser Phe Gly Ile Arg Cys Asn Leu Gly Val Ala Ile Val
                85                  90                  95

Asp Met Val Asn Asn Ser Thr Ile His Arg Gly Gly Lys Val Ile Lys
            100                 105                 110

Glu Lys Ala Lys Phe Asn Trp Asp Pro Glu Thr Val Gly Met Ile His
        115                 120                 125

Gly Ser Phe Phe Trp Gly Tyr Ile Ile Thr Gln Ile Pro Gly Gly Tyr
    130                 135                 140

Ile Ala Ser Arg Leu Ala Ala Asn Arg Val Phe Gly Ala Ala Ile Leu
145                 150                 155                 160

Leu Thr Ser Thr Leu Asn Met Leu Ile Pro Ser Ala Ala Arg Val His
                165                 170                 175

Tyr Gly Cys Val Ile Phe Val Arg Ile Leu Gln Gly Leu Val Glu Gly
            180                 185                 190

Val Thr Tyr Pro Ala Cys His Gly Ile Trp Ser Lys Trp Ala Pro Pro
        195                 200                 205

Leu Glu Arg Ser Arg Leu Ala Thr Thr Ser Phe Cys Gly Ser Tyr Ala
    210                 215                 220

Gly Ala Val Ile Ala Met Pro Leu Ala Gly Ile Leu Val Gln Tyr Thr
225                 230                 235                 240

Gly Trp Ser Ser Val Phe Tyr Val Tyr Gly Ser Phe Gly Met Val Trp
                245                 250                 255

Tyr Met Phe Trp Leu Leu Val Ser Tyr Glu Ser Pro Ala Lys His Pro
            260                 265                 270

Thr Ile Thr Asp Glu Glu Arg Arg Tyr Ile Glu Glu Ser Ile Gly Glu
        275                 280                 285

Ser Ala Asn Leu Leu Gly Ala Met Glu Lys Phe Lys Thr Pro Trp Arg
    290                 295                 300

Lys Phe Phe Thr Ser Met Pro Val Tyr Ala Ile Ile Val Ala Asn Phe
305                 310                 315                 320

Cys Arg Ser Trp Thr Phe Tyr Leu Leu Leu Ile Ser Gln Pro Ala Tyr
                325                 330                 335

Phe Glu Glu Val Phe Gly Phe Glu Ile Ser Lys Val Gly Met Leu Ser
            340                 345                 350

Ala Val Pro His Leu Val Met Thr Ile Ile Val Pro Ile Gly Gly Gln
        355                 360                 365

Ile Ala Asp Phe Leu Arg Ser Lys Gln Ile Leu Ser Thr Thr Thr Val
    370                 375                 380

Arg Lys Ile Met Asn Cys Gly Gly Phe Gly Met Glu Ala Thr Leu Leu
385                 390                 395                 400

Leu Val Val Gly Tyr Ser His Thr Arg Gly Val Ala Ile Ser Phe Leu
                405                 410                 415

Val Leu Ala Val Gly Phe Ser Gly Phe Ala Ile Ser Gly Phe Asn Val
            420                 425                 430

Asn His Leu Asp Ile Ala Pro Arg Tyr Ala Ser Ile Leu Met Gly Ile
        435                 440                 445

Ser Asn Gly Val Gly Thr Leu Ser Gly Met Val Cys Pro Ile Ile Val
    450                 455                 460

Gly Ala Met Thr Lys Asn Lys Ser Arg Glu Glu Trp Gln Tyr Val Phe
465                 470                 475                 480

Leu Ile Ala Ala Leu Val His Tyr Gly Gly Val Ile Phe Tyr Ala Leu
                485                 490                 495

Phe Ala Ser Gly Glu Lys Gln Pro Trp Ala Asp Pro Glu Glu Thr Ser
            500                 505                 510

Glu Glu Lys Cys Gly Phe Ile His Glu Asp Glu Leu Asp Glu Glu Thr
        515                 520                 525

Gly Asp Ile Thr Gln Asn Tyr Ile Asn Tyr Gly Thr Thr Lys Ser Tyr
    530                 535                 540

Gly Ala Thr Ser Gln Glu Asn Gly Gly Trp Pro Asn Gly Trp Glu Lys
545                 550                 555                 560

Lys Glu Glu Phe Val Gln Glu Ser Ala Gln Asp Ala Tyr Ser Tyr Lys
                565                 570                 575

Asp Arg Asp Asp Tyr Ser
            580

<210> 8
<211> 3982
<212> DNA
<213> Rattus norvegicus

<220>
<223> Differentiation-associated Na-dependent inorganic phosphate
cotransporter

<400> 8
agacagtaag gttcttttgc ttttttccct tacacaagga ttcgatgacg tttttggtca 60
atctgattaa aagacagcgg atttggttgc gttaagactt caaaaccggg aatttacgtt 120
gtttttcggt gaggtgactt ccagaacggg gactcatcag cacccgccca aataccacgg 180
cactgcgcgc gccctcggcc accggatcct ccccttccaa tgagactttg tgactgtgtg 240
taccaattct cctattagga aacccgtggg ctgaatgcag ctattccgtt gtactctctt 300
tctcgctctc cctcccctct ccaactcaca gccttgctga aaagctcatc tctgctgaga 360
agaaaacgtt ctaccttaac ctattaagac tatgcgcaga actcgccttt catagccatc 420
acaatttaaa tctggtaagg ctggacacga gtctttacaa gaatggagtc ggtaaaacaa 480
aggattttgg ccccggggaa agaggggata aagaattttg ctggaaaatc cctcggacag 540
atctacaggg tgctggagaa gaagcaggat aaccgagaga ccatcgagct gacagaggac 600
ggcaagcccc tggaggtgcc tgagaagaag gctccgctat gcgactgtac gtgcttcggc 660
ctgccgcgcc gctacatcat agccatcatg agcggcctcg gcttctgcat ctcctttggt 720
atccgctgta acctgggtgt ggccattgtg gacatggtca acaacagcac catccaccgg 780
ggtggcaaag ttatcaagga gaaagccaag tttaactggg accccgagac tgtggggatg 840
attcacgggt cgttcttctg gggctatatc atcacgcaga ttccgggcgg atacatcgca 900
tcgcgactgg ctgctaaccg ggtctttggg gctgccatac tgcttacctc taccctcaat 960
atgctgatcc catctgcagc cagagtgcat tatggatgcg tcatctttgt tagaatattg 1020
caaggacttg tggagggcgt cacctaccca gcctgtcacg ggatatggag caagtgggcc 1080
cctcctttgg agaggagtag gttggctacc acctccttct gtggttccta tgctggagca 1140
gtcattgcaa tgcccctagc tggtatcctg gtgcagtaca ctggatggtc ttcagtattt 1200
tacgtatatg gaagctttgg tatggtctgg tatatgttct ggcttctggt gtcttacgag 1260
agccccgcaa agcatccaac cataacagac gaagaacgta ggtacataga agagagcatc 1320
ggggagagcg caaatctgtt aggagcaatg gagaaattca agaccccatg gaggaagttt 1380
ttcacatcca tgcccgtcta tgcgataatt gttgcaaact tctgcaggag ttggactttt 1440
tatttactgc tcatcagtca accagcttat ttcgaggagg tttttggatt tgaaatcagc 1500
aaggttggca tgttgtctgc ggtcccacac ctggtcatga caatcattgt gcctatcggg 1560
gggcaaattg cagactttct aaggagcaag caaattcttt caacaactac agtgcgaaag 1620
atcatgaact gcgggggttt tggcatggaa gccacactgc ttctggttgt tggctactct 1680
catactagag gggtggccat ctccttcttg gtgcttgcag tgggattcag tggatttgct 1740
atctctggtt tcaatgtgaa ccacttggat attgccccga gatatgccag tatcttaatg 1800
ggcatttcaa atggtgttgg cacgctgtcg ggaatggtct gcccgatcat tgttggtgca 1860

atgacgaaga acaagtcccg tgaagaatgg cagtatgtct tcctcatcgc tgcactggtc 1920
cactatggtg gagtcatatt ttatgcacta tttgcctcag gagagaagca accttgggca 1980
gaccctgagg aaacaagcga agaaaagtgt ggcttcattc atgaagatga actggatgaa 2040
gaaacggggg acatcactca gaattacata aattacggta ccaccaaatc ctacggcgcc 2100
acctcacagg agaacggagg ctggcctaac ggctgggaga aaaaggaaga atttgtgcaa 2160
gaaagtgcgc aagacgcgta ctcctataag gaccgagatg attattcata acgaagctag 2220
ttgctggatt cctttgtagt gtttgtgatt aaattaattg tgattgcaca aaatcatttt 2280
aagaaatgtg gtgtaaacat gtaaacacat caaccaagca agtcttgctg ttcaaaaaat 2340
aataataata tgaattcaaa acagaccgtg agagtcccat caagtgcaat ctgtggcggc 2400
agtcacgtga cgccatttcc attcaggcca ttcgtccttt tcgtttgtga tttaaaggtt 2460
tcctgtagaa ataagtaggt attcgttgga tccatcacca cgttagagag tacaactaca 2520
acagttggca catgtcatcc tacggaagtt aggaagccaa agctactgga ttatgtgaac 2580
tgcatttatt tatttattac actggactgc aaaatatccc agggaaatcc tgtctagaga 2640
catagtagaa ctggaaagat ggctagattg ggtactgacg ataatcattg tgtgtatatc 2700
atggagtggc tatatctttt aattggagaa ctatattgta tagctagcaa aattgtactg 2760
aattattact aggagtgcac agtgtgtgat attttgtgat cttccaaaag cttatcttgc 2820
agtgttttgt gaaacgcttg ggcacaaaca cttattttta tgaacaagag cttgtaaagg 2880
gaggagtatg ctccatgctc tcccattcac tacctgacag tatcaaacct tcacatttca 2940
atgaaatcca acgtccatgt aacatatcac atgacttttt ttgcaaaaaa gaatataaga 3000
agaaatagac ttcaatgtat tttttattac aactttgtac tggttgtaac ttgcattagg 3060
aaaaatgatt aatatatgta taatcgtaaa gaatctaata aaaatttact atgaagatat 3120
agcccttaaa atgcaatatt aacaacaaaa atatatagaa aatttagata atcttccttg 3180
ataactagag actatatgga actcacacca caaagctata tataatatga aaagataaac 3240
aatagagatt gtatatgtag acgattttat gacctaatgt cccatttaag aggtatttgt 3300
cttgagtata tagtacaaag tatattaaaa ttatatctac atccctgtat atcttataca 3360
tatccactca cacaaacata acaaatactt ttcacacaga accaaaaaca agcatacacc 3420
taatgttggg tttggggatt gcaatttcta ctttcataga gtcatagaat tttagatggg 3480
aaaaaaaaag gcattttgct cgtcatttct taatataatt aattcaacag gaactgcaac 3540
atttgtgtac caagcaataa gtgcgaagca taaacctgct gtgtgtaaac tatccccata 3600
ctgcttgtgg tagcactgat ttctttcttt taaagaactt aacatcggag ctctttacaa 3660
tgttttgcgc tgataagaat gcacatccca atttaacgca aagtgtcacc tggtgtgttt 3720
acctgtctgt tttgggtatt tggtctgttt ggtgtcctgt gctcttgact ggaggccctg 3780
ctactgcgaa tataaaacgt gaagtttgtt tctaaatgca aaccactcct gaccttaaga 3840
aactaaagtc cctctctgct ttgtgtctcc aagtactatc atgtgaccat aacccttgct 3900
gtgctgagta aaaagatgtg aactgtcatt ttgttgctgc gaagcaagtg ttaataaaat 3960
gttctattta aaaaaaaaaa aa 3982

<210> 9
<211> 854
<212> PRT
<213> Homo sapiens

<220>
<223> Vacuolar assembly protein VSP41 gene

<400> 9
Met Ala Glu Ala Val Glu Gln Glu Thr Gly Ser Leu Glu Glu Ser Thr
1               5                   10                  15

Asp Glu Ser Glu Glu Glu Glu Ser Glu Glu Glu Pro Lys Leu Lys Tyr
            20                  25                  30

Glu Arg Leu Ser Asn Gly Val Thr Glu Ile Leu Gln Lys Asp Ala Ala
        35                  40                  45

Ser Cys Met Thr Val His Asp Lys Phe Leu Ala Leu Gly Thr His Tyr
    50                  55                  60

Gly Lys Val Tyr Leu Leu Asp Val Gln Gly Asn Ile Thr Gln Lys Phe
65                  70                  75                  80

Asp Val Ser Pro Val Lys Ile Asn Gln Ile Ser Leu Asp Glu Ser Gly
                85                  90                  95

Glu His Met Gly Val Cys Ser Glu Asp Gly Lys Val Gln Val Phe Gly
            100                 105                 110

Leu Tyr Ser Gly Glu Glu Phe His Glu Thr Phe Asp Cys Pro Ile Lys
        115                 120                 125

Ile Ile Ala Val His Pro His Phe Val Arg Ser Ser Cys Lys Gln Phe
    130                 135                 140

Val Thr Gly Gly Lys Lys Leu Leu Leu Phe Glu Arg Ser Trp Met Asn
145                 150                 155                 160

Arg Trp Lys Ser Ala Val Leu His Glu Gly Glu Gly Asn Ile Arg Ser
                165                 170                 175

Val Lys Trp Arg Gly His Leu Ile Ala Trp Ala Asn Asn Met Gly Val
            180                 185                 190

Lys Ile Phe Asp Ile Ile Ser Lys Gln Arg Ile Thr Asn Val Pro Arg
        195                 200                 205

Asp Asp Ile Ser Leu Arg Pro Asp Met Tyr Pro Cys Ser Leu Cys Trp
    210                 215                 220

Lys Asp Asn Val Thr Leu Ile Ile Gly Trp Gly Thr Ser Val Lys Val
225                 230                 235                 240

Cys Ser Val Lys Glu Arg His Ala Ser Glu Met Arg Asp Leu Pro Ser
                245                 250                 255

Arg Tyr Val Glu Ile Val Ser Gln Phe Glu Thr Glu Phe Tyr Ile Ser
            260                 265                 270

Gly Leu Ala Pro Leu Cys Asp Gln Leu Val Val Leu Ser Tyr Val Lys
        275                 280                 285

Glu Ile Ser Glu Lys Thr Glu Arg Glu Tyr Cys Ala Arg Pro Arg Leu
    290                 295                 300

Asp Ile Ile Gln Pro Leu Ser Glu Thr Cys Glu Glu Ile Ser Ser Asp
305                 310                 315                 320

Ala Leu Thr Val Arg Gly Phe Gln Glu Asn Glu Cys Arg Asp Tyr His
                325                 330                 335

Leu Glu Tyr Ser Glu Gly Glu Ser Leu Phe Tyr Ile Val Ser Pro Arg
            340                 345                 350

Asp Val Val Val Ala Lys Glu Arg Asp Gln Asp Asp His Ile Asp Trp
        355                 360                 365

Leu Leu Glu Lys Lys Lys Tyr Glu Glu Ala Leu Met Ala Ala Glu Ile
    370                 375                 380

Ser Gln Lys Asn Ile Lys Arg His Lys Ile Leu Asp Ile Gly Leu Ala
385                 390                 395                 400

Tyr Ile Asn His Leu Val Glu Arg Gly Asp Tyr Asp Ile Ala Ala Arg

405                 410                 415

Lys Cys Gln Lys Ile Leu Gly Lys Asn Ala Ala Leu Trp Glu Tyr Glu
            420                 425                 430

Val Tyr Lys Phe Lys Glu Ile Gly Gln Leu Lys Ala Ile Ser Pro Tyr
        435                 440                 445

Leu Pro Arg Gly Asp Pro Val Leu Lys Pro Leu Ile Tyr Glu Met Ile
    450                 455                 460

Leu His Glu Phe Leu Glu Ser Asp Tyr Glu Gly Phe Ala Thr Leu Ile
465                 470                 475                 480

Arg Glu Trp Pro Gly Asp Leu Tyr Asn Asn Ser Val Ile Val Gln Ala
                485                 490                 495

Val Arg Asp His Leu Lys Lys Asp Ser Gln Asn Lys Thr Leu Leu Lys
            500                 505                 510

Thr Leu Ala Glu Leu Tyr Thr Tyr Asp Lys Asn Tyr Gly Asn Ala Leu
        515                 520                 525

Glu Ile Tyr Leu Thr Leu Arg His Lys Asp Val Phe Gln Leu Ile His
    530                 535                 540

Lys His Asn Leu Phe Ser Ser Ile Lys Asp Lys Ile Val Leu Leu Met
545                 550                 555                 560

Asp Phe Asp Ser Glu Lys Ala Val Asp Met Leu Leu Asp Asn Glu Asp
                565                 570                 575

Lys Ile Ser Ile Lys Lys Val Val Glu Glu Leu Glu Asp Arg Pro Glu
            580                 585                 590

Leu Gln His Val Tyr Leu His Lys Leu Phe Lys Arg Asp His His Lys
        595                 600                 605

Gly Gln Arg Tyr His Glu Lys Gln Ile Ser Leu Tyr Ala Glu Tyr Asp
    610                 615                 620

Arg Pro Asn Leu Leu Pro Phe Leu Arg Asp Ser Thr His Cys Pro Leu
625                 630                 635                 640

Glu Lys Ala Leu Glu Ile Cys Gln Gln Arg Asn Phe Val Glu Glu Thr
                645                 650                 655

Val Tyr Leu Leu Ser Arg Met Gly Asn Ser Arg Ser Ala Leu Lys Met
            660                 665                 670

Ile Met Glu Glu Leu His Asp Val Asp Lys Ala Ile Glu Phe Ala Lys
        675                 680                 685

Glu Gln Asp Asp Gly Glu Leu Trp Glu Asp Leu Ile Leu Tyr Ser Ile
    690                 695                 700

Asp Lys Pro Pro Phe Ile Thr Gly Leu Leu Asn Asn Ile Gly Thr His
705                 710                 715                 720

Val Asp Pro Ile Leu Leu Ile His Arg Ile Lys Glu Gly Met Glu Ile
                725                 730                 735

Pro Asn Leu Arg Asp Ser Leu Val Lys Ile Leu Gln Asp Tyr Asn Leu
            740                 745                 750

Gln Ile Leu Leu Arg Glu Gly Cys Lys Lys Ile Leu Val Ala Asp Ser
        755                 760                 765

Leu Ser Leu Leu Lys Lys Met His Arg Thr Gln Met Lys Gly Val Leu
    770                 775                 780

Val Asp Glu Glu Asn Ile Cys Glu Ser Cys Leu Ser Pro Ile Leu Pro
785                 790                 795                 800

Ser Asp Ala Ala Lys Pro Phe Ser Val Val Val Phe His Cys Arg His
                805                 810                 815

Met Phe His Lys Glu Cys Leu Pro Met Pro Ser Met Asn Ser Ala Ala
            820                 825                 830

Gln Phe Cys Asn Ile Cys Ser Ala Lys Asn Arg Gly Pro Gly Ser Ala
        835                 840                 845

Ile Leu Glu Met Lys Lys
    850

<210> 10
<211> 770
<212> PRT
<213> Rattus norvegicus

<220>
<223>  Git 1

<400> 10
Met Ser Arg Lys Gly Pro Arg Ala Glu Val Cys Ala Asp Cys Ser Ala
  1               5                  10                  15

Pro Asp Pro Gly Trp Ala Ser Ile Ser Arg Gly Val Leu Val Cys Asp
             20                  25                  30

Glu Cys Cys Ser Val His Arg Ser Leu Gly Arg His Ile Ser Ile Val
         35                  40                  45

Lys His Leu Arg His Ser Ala Trp Pro Pro Thr Leu Leu Gln Met Val
     50                  55                  60

His Thr Leu Ala Ser Asn Gly Ala Asn Ser Ile Trp Glu His Ser Leu
 65                  70                  75                  80

Leu Asp Pro Ala Gln Val Gln Ser Gly Arg Arg Lys Ala Asn Pro Gln
                 85                  90                  95

Asp Lys Val His Pro Ile Lys Ser Glu Phe Ile Arg Ala Lys Tyr Gln
            100                 105                 110

Met Leu Ala Phe Val His Lys Leu Pro Cys Arg Asp Asp Asp Gly Val
        115                 120                 125

Thr Ala Lys Asp Leu Ser Lys Gln Leu His Ser Ser Val Arg Thr Gly
    130                 135                 140

Asn Leu Glu Thr Cys Leu Arg Leu Leu Ser Leu Gly Ala Gln Ala Asn

145                 150                 155                 160

Phe Phe His Pro Glu Lys Gly Thr Thr Pro Leu His Val Ala Ala Lys
                165                 170                 175

Ala Gly Gln Thr Leu Gln Ala Glu Leu Leu Val Val Tyr Gly Ala Asp
            180                 185                 190

Pro Gly Ser Pro Asp Val Asn Gly Arg Thr Pro Ile Asp Tyr Ala Arg
        195                 200                 205

Gln Ala Gly His His Glu Leu Ala Glu Arg Leu Val Glu Cys Gln Tyr
    210                 215                 220

Glu Leu Thr Asp Arg Leu Ala Phe Tyr Leu Cys Gly Arg Lys Pro Asp
225                 230                 235                 240

His Lys Asn Gly His Tyr Ile Ile Pro Gln Met Ala Asp Arg Ser Arg
                245                 250                 255

Gln Lys Cys Met Ser Gln Ser Leu Asp Leu Ser Glu Leu Ala Lys Ala
            260                 265                 270

Ala Lys Lys Lys Leu Gln Ala Leu Ser Asn Arg Leu Phe Glu Glu Leu
        275                 280                 285

Ala Met Asp Val Tyr Asp Glu Val Asp Arg Arg Glu Asn Asp Ala Val
    290                 295                 300

Trp Leu Ala Thr Gln Asn His Ser Thr Leu Val Thr Glu Arg Ser Ala
305                 310                 315                 320

Val Pro Phe Leu Pro Val Asn Pro Glu Tyr Ser Ala Thr Arg Asn Gln
                325                 330                 335

Gly Arg Gln Lys Leu Ala Arg Phe Asn Ala Arg Glu Phe Ala Thr Leu
            340                 345                 350

Ile Ile Asp Ile Leu Ser Glu Ala Lys Arg Arg Gln Gln Gly Lys Ser
        355                 360                 365

Leu Ser Ser Pro Thr Asp Asn Leu Glu Leu Ser Ala Arg Asn Gln Ser
    370                 375                 380

Asp Leu Asp Asp Gln His Asp Tyr Asp Ser Val Ala Ser Asp Glu Asp
385                 390                 395                 400

Thr Asp Gln Glu Pro Leu Pro Ser Ala Gly Ala Thr Arg Asn Asn Arg
                405                 410                 415

Ala Arg Ser Met Asp Ser Ser Asp Leu Ser Asp Gly Ala Val Thr Leu
            420                 425                 430

Gln Glu Tyr Leu Glu Leu Lys Lys Ala Leu Ala Thr Ser Glu Ala Lys
        435                 440                 445

Val Gln Gln Leu Met Lys Val Asn Ser Ser Leu Ser Asp Glu Leu Arg
    450                 455                 460

Lys Leu Gln Arg Glu Ile His Lys Leu Gln Ala Glu Asn Leu Gln Leu
465                 470                 475                 480

Arg Gln Pro Pro Gly Pro Val Pro Val Pro Ser Leu Pro Ser Glu Arg
                485                 490                 495

Ala Glu His Thr Leu Met Gly Pro Gly Gly Ser Thr His Arg Arg Asp
            500                 505                 510

Arg Gln Ala Phe Ser Met Tyr Glu Pro Gly Ser Ala Leu Lys Pro Phe
        515                 520                 525

Gly Gly Ala Pro Gly Asp Glu Leu Ala Thr Arg Leu Gln Pro Phe His
    530                 535                 540

Ser Thr Glu Leu Glu Asp Asp Ala Ile Tyr Ser Val His Val Pro Ala
545                 550                 555                 560

Gly Leu Tyr Arg Ile Arg Lys Gly Val Ser Ala Ser Ser Val Thr Phe
                565                 570                 575

Thr Pro Ser Ser Pro Leu Leu Ser Ser Ser Gln Glu Gly Ser Arg His
            580                 585                 590

Ala Ser Lys Leu Ser Arg His Gly Ser Gly Ala Glu Ser Asp Tyr Glu
        595                 600                 605

Asn Thr Gln Ser Gly Glu Pro Leu Leu Gly Leu Glu Gly Lys Arg Phe
    610                 615                 620

Leu Glu Leu Ser Lys Glu Asp Glu Leu His Ala Glu Leu Glu Ser Leu
625                 630                 635                 640

Asp Gly Asp Pro Asp Pro Gly Leu Pro Ser Thr Glu Asp Val Ile Leu
                645                 650                 655

Lys Thr Glu Gln Val Thr Lys Asn Ile Gln Glu Leu Leu Arg Ala Ala
            660                 665                 670

Gln Glu Phe Lys His Asp Ser Phe Val Pro Cys Ser Glu Lys Ile His
        675                 680                 685

Leu Ala Val Thr Glu Met Ala Ser Leu Phe Pro Lys Arg Pro Ala Leu
    690                 695                 700

Glu Pro Val Arg Ser Ser Leu Arg Leu Leu Asn Ala Ser Ala Tyr Arg
705                 710                 715                 720

Leu Gln Ser Glu Cys Arg Lys Thr Val Pro Pro Glu Pro Gly Ala Pro
                725                 730                 735

Val Asp Phe Gln Leu Leu Thr Gln Gln Val Ile Gln Cys Ala Tyr Asp
            740                 745                 750

Ile Ala Lys Ala Ala Lys Gln Leu Val Thr Ile Thr Thr Arg Glu Lys
        755                 760                 765

Lys Gln
    770

<210> 11
<211> 3236
<212> DNA
<213> Rattus norvegicus

<220>
<223>  Git 1


<400> 11
gcccgcgccc cccacgctgg cccagagggc tgcggccgct tcgccgctga ggatgtcccg 60
gaaggggccg cgagcggagg tgtgtgcgga ctgcagcgcc cctgaccctg gctgggcatc 120
tatcagcaga ggtgtgctgg tttgtgacga gtgctgcagt gtgcaccgga gcctgggacg 180
acacatctcc attgtcaaac accttcgcca cagcgcctgg cctcctacgc tgctacagat 240
ggtgcacacg cttgccagca acggggccaa ctctatctgg gagcattccc tgctggaccc 300
tgcacaagtg caaagtggcc ggcgcaaagc caacccccaa gacaaagtcc accccatcaa 360
gtcagagttc atcagggcaa agtaccagat gctggcgttt gtgcacaagc ttccctgccg 420
tgatgatgat ggggtcaccg ccaaagacct cagcaagcaa ctgcactcga gtgtgcgaac 480
aggcaacctg gagacctgtc tgcgcctgct ttccctgggt gcccaggcca acttcttcca 540
cccagaaaag ggcactacac ctctacacgt ggctgccaag gcagggcaga cactgcaggc 600
tgagctgcta gtagtgtatg gggctgaccc aggctcccct gatgtcaatg gccgcacacc 660
cattgactat gccaggcagg cggggcacca tgaactggca gaaaggctag ttgagtgcca 720
gtatgagctc actgaccggt tggccttcta cctctgtgga cggaagcctg atcacaagaa 780
tgggcattac atcataccac agatggctga cagatctcgg caaaagtgca tgtctcagag 840
tttggatctg tctgaattgg ccaaagctgc caagaagaag ctacaagcac tcagcaaccg 900
gctctttgag gagctcgcca tggatgtgta tgacgaagtg gaccggagag aaaatgatgc 960
agtgtggcta gctacccaga atcatagcac cctggtgaca gaacgcagtg ctgtgccctt 1020
cctgccagtc aaccctgaat actcagccac tcggaatcag ggacgacaga aattggctcg 1080
ttttaatgcc cgagagtttg ccaccttgat catcgacatc ctcagtgagg ccaaacggag 1140
gcagcagggc aagagtctga gcagccccac agacaacctc gagctgtctg cacggaacca 1200
aagtgacctg gacgaccagc acgactacga tagtgtggct tctgacgaag acacagacca 1260
ggagcccttg cccagtgctg gcgccacgcg gaacaatcgt gccaggagca tggactcctc 1320
agatctgtcg gatggggctg tgacgctgca ggagtacctg gagctgaaga aggctctggc 1380
tacctccgag gccaaagtgc agcagctcat gaaagtcaac agcagtctga gtgacgagct 1440
tcggaagctg cagagggaga tccacaaact gcaggcagag aacctgcagc tccggcagcc 1500
accaggacca gtgcctgtac cctcacttcc cagtgaacgg gcagaacaca cactcatggg 1560
ccctggtgga agtacccatc gcagggaccg ccaggccttc tctatgtatg agccaggctc 1620
cgccctgaag ccctttggag gtgcacctgg ggatgagctc gccacacggc tccagccttt 1680
ccacagcaca gagctggaag atgatgccat ctattcagta catgtccctg ctggccttta 1740
ccggatccgg aagggggtgt ctgcctcttc tgtgaccttc actccctcct ccccactgct 1800
gtcaagctcc caagaaggaa gtcgccatgc gagcaagctt tcacgccacg gcagtggcgc 1860
agagagtgac tatgagaaca cacagagtgg agagcctctg ctgggacttg aagggaagcg 1920
attcctagag ctgagcaagg aggatgagct gcacgctgag ctggagagct tagatggaga 1980
cccagacccc gggctcccca gcacagaaga tgtcatccta aagacagagc aggtcaccaa 2040
gaacattcag gagctattgc gggcagccca ggagttcaaa catgacagct ttgtgccctg 2100
ttcagaaaag atccatttgg ctgtgactga gatggcctct ctcttcccaa agaggccagc 2160
cctggagcct gtgcgcagtt cactgcggct gctcaacgcc agcgcctacc ggctgcagag 2220
tgaatgccgg aagacagtgc ctccagaacc tggcgcccct gtggacttcc agctactgac 2280
tcagcaggtg atccagtgcg cctatgacat cgccaaggct gccaagcagc tggtcaccat 2340
caccacccga gagaagaaac agtgaccact ttccctgccc ttacccacac cctgggacct 2400
cactggccat gggagctggg ccactccaga cactaaccca cccccaacag agccactggc 2460
acaagtgccc ttagtgccac cattctcctc tggcatccag gtgccctggt gtccacaccc 2520
ccttaagccc caaggatggg aatgtgaggt gacaggacca tctgtccccc acattccacg 2580
cccctccctc tgtacatagc atccctgccc tgctgatacg caggagcctc aaggagtccc 2640
tcccagctct tcacctgcct tccagggccc cctcagcaaa gggtgggaca gggacactcc 2700
aagtggggca gccccgccta ctgcacccta tccccatgag ccagttcagc cctggggggt 2760
cgagcagagg caccccctcc tttgtgcatt atctmtgggt gtytytgccc cgtagccagc 2820
agcccccccg ctgctctttc atgacaaatg gcccctgcca gagcacaggc cccaacttgt 2880
gtgccagggt ccccagcagc aaacactgga acgtctttct ctcccctacc ccttaatttt 2940
aactttgtgg taactgagtg tccctgcgtt cttacaactg agtgtgtgag cggcagtgcc 3000
gtgccagaga cctggtccac ctggagctct gaggggcagg ggaaccagag tagtgggacc 3060
agcattggaa gtcagcttcc ctctacccaa cctgggacgg ggactgtcag ggtcaccagc 3120
tgtggtccag ttgctacccc tacttgtatc cctatcctat aaacttaaaa ggaaaaccac 3180
ttccctccat ctctccccat cccctgtggg aagtgtgctg accaggcaga ggacag 3236

<210> 12
<211> 136
<212> PRT
<213> Homo sapiens

<220>
<223> Histone H3.3


<400> 12
Met Ala Arg Thr Lys Gln Thr Ala Arg Lys Ser Thr Gly Gly Lys Ala
1               5                   10                  15

Pro Arg Lys Gln Leu Ala Thr Lys Ala Ala Arg Lys Ser Ala Pro Ser
            20                  25                  30

Thr Gly Gly Val Lys Lys Pro His Arg Tyr Arg Pro Gly Thr Val Ala
        35                  40                  45

Leu Arg Glu Ile Arg Arg Tyr Gln Lys Ser Thr Glu Leu Leu Ile Arg
    50                  55                  60

Lys Leu Pro Phe Gln Arg Leu Val Arg Glu Ile Ala Gln Asp Phe Lys
65                  70                  75                  80

Thr Asp Leu Arg Phe Gln Ser Ala Ala Ile Gly Ala Leu Gln Glu Ala
                85                  90                  95

Ser Glu Ala Tyr Leu Val Gly Leu Phe Glu Asp Thr Asn Leu Cys Ala
            100                 105                 110

Ile His Ala Lys Arg Val Thr Ile Met Pro Lys Asp Ile Gln Leu Ala
        115                 120                 125

Arg Arg Ile Arg Gly Glu Arg Ala
    130                 135


<210> 13
<211> 1039
<212> DNA
<213> Homo sapiens

<220>
<223> Histone H3.3


<400> 13
   tgttcgcagc cgccgccgcg ccgccgtcgc tctccaacgc cagcgccgcc tctcgctcgc 60
   gtaagtaagg aggtctctgc gagctccagc cgaagagaag gggtaccatg ggctcgtaca 120
   aagcagactg cccgcaaatc gaccggtggt aaagcaccca ggaagcaact ggctacaaaa 180
   gccgctcgca agagtgcgcc ctctactgga ggggtgaaga aacctcatcg ttacaggcct 240
   ggtactgtgg cgctccgtga aattagacgt tatcagaagt ccactgaact tctgattcgc 300
   aaacttccct tccagcgtct ggtgcgagaa attgctcagg actttaaaac agatctgcgc 360
   ttccagagcg cagctatcgg tgctttgcag gaggcaagtg aggcctatct ggttggcctt 420
   tttgaagaca ccaacctgtg tgctatccat gccaaacgtg taacaattat gccaaaagac 480
   atccagctag cacgccgcat acgtggagaa cgtgcttaag aatccactat gatgggaaac 540
   atttcattct caaaaaaaaa aaaaaaattt ctcttcttcc tgttattggt agttctgaac 600
   gttagatatt ttttttccat ggggtcaaag gtacctaagt atatgattgc gagtggaaaa 660
   ataggggaca gaaatcaggt attggcagtt tttccatttt catttgtgtg tgaattttta 720
   atataaatgc ggagacgtaa agcattaatg caagttaaaa tgtttcagtg aacaagtttc 780
   agcggttcaa ctttataata attataaata aacctgttaa atttttctgg acaatgccag 840
   catttggatt tctttaaaac aagtaaattt cttattgatg gcaactaaat ggtgtttgta 900
   gcatttttat catacagtag attccatcca ttcactatac ttttctaact gagttgtcct 960
   acatgcaagt acatgttttt aatgttgtct gtcttctgtg ctgttcctgt aagtttgcta 1020
   ttaaaataca ttaaactat 1039

<210> 14
<211> 520
<212> PRT
<213> Rattus norvegicus

<220>
<223> Cytosolic 3-hydroxy 3-methylglutaryl coenzyme A synthase

<400> 14
Met Pro Gly Ser Leu Pro Leu Asn Ala Glu Ala Cys Trp Pro Lys Asp
1               5                   10                  15

Val Gly Ile Val Ala Leu Glu Ile Tyr Phe Pro Ser Gln Tyr Val Asp
            20                  25                  30

Gln Ala Glu Leu Glu Lys Tyr Asp Gly Val Asp Ala Gly Lys Tyr Thr
        35                  40                  45

Ile Gly Leu Gly Gln Ala Arg Met Gly Phe Cys Thr Asp Arg Glu Asp
    50                  55                  60

Ile Asn Ser Leu Cys Leu Thr Val Val Gln Lys Leu Met Glu Arg Asn
65                  70                  75                  80

Ser Leu Ser Tyr Asp Cys Ile Gly Arg Leu Glu Val Gly Thr Glu Thr
                85                  90                  95

Ile Ile Asp Lys Ser Lys Ser Val Lys Ser Asn Leu Met Gln Leu Phe
            100                 105                 110

Glu Glu Ser Gly Asn Thr Asp Ile Glu Gly Ile Asp Thr Thr Asn Ala
        115                 120                 125

Cys Tyr Gly Gly Thr Ala Ala Val Phe Asn Ala Val Asn Trp Ile Glu
    130                 135                 140

Ser Ser Ser Trp Asp Gly Arg Tyr Ala Leu Val Val Ala Gly Asp Ile
145                 150                 155                 160

Ala Ile Tyr Ala Ser Gly Asn Ala Arg Pro Thr Gly Gly Val Gly Ala
                165                 170                 175

Val Ala Leu Leu Ile Gly Pro Asn Ala Pro Val Ile Phe Asp Arg Gly
            180                 185                 190

Leu Arg Gly Thr His Met Gln His Ala Tyr Asp Phe Tyr Lys Pro Asp
        195                 200                 205

Met Leu Ser Glu Tyr Pro Val Val Asp Gly Lys Leu Ser Ile Gln Cys
    210                 215                 220

Tyr Leu Ser Ala Leu Asp Arg Cys Tyr Ser Val Tyr Arg Lys Lys Ile
225                 230                 235                 240

Arg Ala Gln Trp Gln Lys Glu Gly Lys Asp Lys Asp Phe Thr Leu Asn

                245                 250                 255

Asp Phe Gly Phe Met Ile Phe His Ser Pro Tyr Cys Lys Leu Val Gln
            260                 265                 270

Lys Ser Leu Ala Arg Met Phe Leu Asn Asp Phe Leu Asn Asp Gln Asn
        275                 280                 285

Arg Asp Lys Asn Ser Ile Tyr Ser Gly Leu Glu Ala Phe Gly Asp Val
    290                 295                 300

Lys Leu Glu Asp Thr Tyr Phe Asp Arg Asp Val Glu Lys Ala Phe Met
305                 310                 315                 320

Lys Ala Ser Ala Glu Leu Phe Asn Gln Lys Thr Lys Ala Ser Leu Leu
                325                 330                 335

Val Ser Asn Gln Asn Gly Asn Met Tyr Thr Ser Ser Val Tyr Gly Ser
            340                 345                 350

Leu Ala Ser Val Leu Ala Gln Tyr Ser Pro Gln Gln Leu Ala Gly Lys
        355                 360                 365

Arg Ile Gly Val Phe Ser Tyr Gly Ser Gly Leu Ala Ala Thr Leu Tyr
    370                 375                 380

Ser Leu Lys Val Thr Gln Asp Ala Thr Pro Gly Ser Ala Leu Asp Lys
385                 390                 395                 400

Ile Thr Ala Ser Leu Cys Asp Leu Lys Ser Arg Leu Asp Ser Arg Thr
                405                 410                 415

Cys Val Ala Pro Asp Val Phe Ala Glu Asn Met Lys Leu Arg Glu Asp
            420                 425                 430

Thr His His Leu Ala Asn Tyr Ile Pro Gln Cys Ser Ile Asp Ser Leu
        435                 440                 445

Phe Glu Gly Thr Trp Tyr Leu Val Arg Val Asp Glu Lys His Arg Arg
    450                 455                 460

Thr Tyr Ala Arg Arg Pro Ser Thr Asn Asp His Ser Leu Asp Glu Gly
465                 470                 475                 480

Val Gly Leu Val His Ser Asn Thr Ala Thr Glu His Ile Pro Ser Pro
                485                 490                 495

Ala Lys Lys Val Pro Arg Leu Pro Ala Thr Ser Gly Glu Pro Glu Ser
            500                 505                 510

Ala Val Ile Ser Asn Gly Glu His
        515                 520

<210> 15
<211> 3275
<212> DNA
<213> Rattus norvegicus

<220>
<223> Cytosolic 3-hydroxy 3-methylglutaryl coenzyme A synthase

<400> 15
cccagggtcc gatcgcgttt ggtgcctgaa ggaggaaccg gtgacagacc tggagactac 60
agttctctgt ccttcacaca gctctttcac catgcctggg tcacttcctt tgaatgcaga 120
ggcttgctgg ccaaaagatg tgggaatcgt tgcccttgaa atctactttc cttctcagta 180
tgttgatcaa gctgagttgg aaaaatacga tggtgtagat gctggaaagt ataccattgg 240
cctgggccag gccaggatgg gcttctgcac ggatcgcgaa gacatcaact ctctctgcct 300
gactgtggtt cagaaactga tggagagaaa tagcctttcc tatgactgca ttgggcggct 360
ggaagtcggg acagagacaa tcatcgacaa atcaaaatcc gtgaagtcta atttgatgca 420
gctgtttgag gagtctggga atacagatat agaaggaata gatacaacta atgcatgcta 480
tgggggcaca gccgcagtct tcaatgctgt gaactggatc gaatccagct cttgggatgg 540
acgatacgct ttggtagttg caggagacat cgctatatat gcctcaggaa acgccaggcc 600
tacaggtgga gttggagctg tggctctgct aattgggcca aatgctcctg taatttttga 660
ccgagggctt cgtgggacac acatgcagca tgcctacgac ttttacaagc ctgacatgct 720
ctctgaatac cctgtggtag atggaaaact ctccatacag tgctacctca gcgcattgga 780
ccgctgctat tctgtctacc gcaaaaagat ccgggcccag tggcagaaag agggaaagga 840
taaagatttt accctgaatg attttggctt catgatcttt cactcgccat actgtaaact 900
ggtgcagaaa tctctagcta ggatgttcct gaatgacttt cttaacgatc aaaacagaga 960
caaaaacagt atttacagtg ggctggaagc ctttggggat gtgaaattag aagatactta 1020
cttcgacaga gatgtggaaa aggcatttat gaaggctagt gctgagctat tcaaccagaa 1080
aacaaaggca tctttgcttg tatcgaatca aaatggaaac atgtacacat cctctgtata 1140
cggttccctt gcttctgttc tggcacagta ctcacctcaa cagttggccg ggaagaggat 1200
tggagtgttc tcttacggtt ctggcttggc tgccacactc tactccctta aagtcacaca 1260
agatgccaca ccaggatctg ctcttgacaa aataacagca agtttatgtg accttaagtc 1320
aaggcttgac tcaagaacgt gtgtggcacc agacgtcttt gctgaaaaca tgaagctcag 1380
agaggacaca catcacttag ccaactatat tccccagtgt tcaatagatt cactcttcga 1440
aggaacatgg tatctagtca gagtggatga aaagcacaga agaacttacg cccggcgtcc 1500
ctccacaaat gaccacagtt tggatgaagg agtgggactt gtgcattcaa acacagctac 1560
agagcatatt ccaagcccgg ctaagaaagt gccaagactt cctgcgacct cgggcgaacc 1620
tgagtcggct gtcatcagta acggggagca ctgagagccg tggccttcac agaggctcgg 1680
ggctggatgg ggtatgggaa acgttagagg aatggatgtc ttgggacaat tttacagatt 1740
acgtgttgct taaaaatgta atgtaactga cacagagccc agaaaactgt tgtgtttttg 1800
gagaagtctc gctgagctcc taacacactt cctgctgtgg gctggccaat ggtgaatgta 1860
ctgcgatggt gttaagggct ctgcagaacg tcatacctcg ctgcatgttt acacgcatgc 1920
gggttaggct tcaaactcgg tctgaactga gtgcttctga ctgcaaaggc agaggtactg 1980
ctgtccagtt taaaaaattg tttttttttt ttttaatgtg taagaatttt tatacttaaa 2040
taaaaaaaag tacctgtagc ttttggggga aaaaaaaaaa cctttttcta ggttggggat 2100
tgtggaattt aaatgttaca cataaactct gcttaatggc aaggcaaaca tttatctttt 2160
tcgaagattt ataaatcctg aagagaaaaa aagagggtat ggttctagga tctggatgaa 2220
ccatcagtga gaaaggttag tcataatcaa gtgagcagaa ggatgctggc gttgagcagg 2280
cctctgtcac agcaaccagg gctctgtggg cacagctgag ggaactttct ggccaggtgc 2340
ccgtgactgc tgctcagctg cactgagatg cagtggagct gctgcacgga agcttgctgt 2400
ggtgctgaac gccttacctg cggataaagt gtaaagtagg agggatgggc agggcactat 2460
taggttacag tgttacagac ccaggttata gacttgacag ctcaaactca ccagacacct 2520
ttttcccttt gtggtttgtg tattttttgt gttttgtttg ttttttttta tattgttcaa 2580
tttaaaaaat ttagaaaatt ttaaccttac gttttcacat agtgtgatta gccaaaagga 2640
atttcacttc aagatctaga aatagaattc ataacatttt ttcctaaact ttgactttta 2700
aaacaacgaa aattaccaca tgagatgaac aagaaaattc attagaaagt tctctgggtg 2760
atttttggtg ctgaactgac atgagcctca tagactgtaa aacagaggta gttgaaacta 2820
atgtacagaa ctacattttt taattttatt tgcatttaat tctgtgaagt ttcagttatc 2880
taaaataaac acataaacgt gtaatgtttc agattgcaag gtgagatgta atgtagcatt 2940
tgtaagatat tcttgtcaat attaactggt aggattttga tttgtacagt tttaattggt 3000
taaaatgatc tcattttaac atccactgct atagatgaat gatgtaagct cagatttaat 3060
gaatggtggg gaaatggtgc atgtaatttt ttcgcaagta tcgagagttc tgtatgtttt 3120
gaaaagaata atttaacgtt tgggttgcca ggaagggggc tttcccagag tccattgcca 3180
ggcgttgggc aagcctcgca atgttggcac ggagcgttaa ccacacctta ctaatagcaa 3240
ggggaataac tttgaaataa agttttagac aaata 3275

<210> 16
<211> 353
<212> PRT

<213> Rattus norvegicus

<220>
<223> farnesyl pyrophosphate synthetase

<400> 16
Met Asn Gly Asp Gln Lys Leu Asp Val His Asn Gln Glu Lys Gln Asn
1               5                   10                  15

Phe Ile Gln His Phe Ser Gln Ile Val Lys Val Leu Thr Glu Asp Glu
            20                  25                  30

Leu Gly His Pro Glu Lys Gly Asp Ala Ile Thr Arg Ile Lys Glu Val
        35                  40                  45

Leu Glu Tyr Asn Thr Val Gly Gly Lys Tyr Asn Arg Gly Leu Thr Val
    50                  55                  60

Val Gln Thr Phe Gln Glu Leu Val Glu Pro Arg Lys Gln Asp Ala Glu
65                  70                  75                  80

Ser Leu Gln Arg Ala Leu Thr Val Gly Trp Cys Val Glu Leu Leu Gln
                85                  90                  95

Ala Phe Phe Leu Val Leu Asp Asp Ile Met Asp Ser Ser His Thr Arg
            100                 105                 110

Arg Gly Gln Ile Cys Trp Tyr Gln Lys Pro Gly Ile Gly Leu Asp Ala
        115                 120                 125

Ile Asn Asp Ala Leu Leu Leu Glu Ala Ala Ile Tyr Arg Leu Leu Lys
    130                 135                 140

Phe Tyr Cys Arg Glu Gln Pro Tyr Tyr Leu Asn Leu Leu Glu Leu Phe
145                 150                 155                 160

Leu Gln Ser Ser Tyr Gln Thr Glu Ile Gly Gln Thr Leu Asp Leu Ile
                165                 170                 175

Thr Ala Pro Gln Gly Gln Val Asp Leu Gly Arg Tyr Thr Glu Lys Arg
            180                 185                 190

Tyr Lys Ser Ile Val Lys Tyr Lys Thr Ala Phe Tyr Ser Phe Tyr Leu
        195                 200                 205

Pro Ile Ala Ala Ala Met Tyr Met Ala Gly Ile Asp Gly Glu Lys Glu
    210                 215                 220

His Ala Asn Ala Leu Lys Ile Leu Leu Glu Met Gly Glu Phe Phe Gln
225                 230                 235                 240

Ile Gln Asp Asp Tyr Leu Asp Leu Phe Gly Asp Pro Ser Val Thr Gly
                245                 250                 255

Lys Val Gly Thr Asp Ile Gln Asp Asn Lys Cys Ser Trp Leu Val Val
            260                 265                 270

Gln Cys Leu Leu Arg Ala Thr Pro Gln Gln Arg Gln Ile Leu Glu Glu
        275                 280                 285

Asn Tyr Gly Gln Lys Asp Pro Glu Lys Val Ala Arg Val Lys Ala Leu
    290                 295                 300

Tyr Glu Glu Leu Asp Leu Arg Ser Val Phe Phe Lys Tyr Glu Glu Asp
305                 310                 315                 320

Ser Tyr Asn Arg Leu Lys Ser Leu Ile Glu Gln Cys Ser Ala Pro Leu
                325                 330                 335

Pro Pro Ser Ile Phe Leu Glu Leu Ala Asn Lys Ile Tyr Lys Arg Arg
            340                 345                 350

Lys

<210> 17
<211> 1271
<212> DNA
<213> Rattus norvegicus

<220>
<223> farnesyl pyrophosphate synthetase

<400> 17
ttatatttgg gttctgccta ctgagccggg agtctgggaa ctacaactcc cagagtgctg 60
agcggatgca cgctctgctt ttaggtgtaa gccgcaaaca tcttggaccc cgggagaatc 120
cgcgttgaag cacagagcat ttagctcctc tgtcagaatg aatggggacc agaaactgga 180
tgttcataac caagaaaagc agaatttcat ccagcacttc tcccagattg tcaaggtgct 240
gactgaggat gaactgggac acccagagaa gggagatgct attacccgga tcaaagaggt 300
cctggagtac aacactgtag gaggcaagta caatcggggt ctgacggtgg tacagacctt 360
ccaggaactg gtggaaccaa ggaaacagga tgctgagagc ctacagcggg ccctgacggt 420
gggctggtgt gtagaactgc tccaggcttt cttcctcgtg ttagatgaca tcatggactc 480
ttcccacact cgccgggggc agatctgctg gtatcagaag ccgggcatag gcttggatgc 540
catcaacgat gctctgcttc tggaagccgc tatctaccgc ctgcttaagt tctactgcag 600
ggagcagccc tactacctca acctgctgga gctctttcta cagagttcct atcagactga 660
gatcgggcag actctcgacc tcatcacagc accccagggc caagtggatc ttggtagata 720
cactgaaaag aggtacaaat ctatcgtcaa gtacaagaca gctttctact ctttctacct 780
gcctatcgcg gctgccatgt acatggctgg aattgatggg gagaaggaac acgctaatgc 840
cctgaagatc ctgctggaga tgggcgagtt cttccagatc caggacgact accttgatct 900
ctttggagac cccagtgtga ccggaaaggt cggcactgac atccaggaca acaaatgcag 960
ctggctggtg gttcagtgtc tgctacgagc cactcctcag cagcgccaga tcttagagga 1020
gaattatggg cagaaggacc cagaaaaagt ggcgcgggtg aaagcactgt acgaggagct 1080
ggatctgcgg agtgtgttct tcaagtacga ggaagacagt tacaaccgcc tcaagagtct 1140
catagagcag tgctccgcgc ccctgccccc atccatcttc ctggaactag caaacaagat 1200
ctacaagcgg agaaagtaac ctcgaattgt agaggctgcg agggaggggt ctcaataaat 1260
tattgttcaa c                                                      1271

<210> 18
<211> 152
<212> PRT
<213> Rattus norvegicus

<220>
<223> Bendless protein

<400> 18
Met Ala Gly Leu Pro Arg Arg Ile Ile Lys Glu Thr Gln Arg Leu Leu
1               5                   10                  15

Ala Glu Pro Val Pro Gly Ile Lys Ala Glu Pro Asp Glu Ser Asn Ala
            20                  25                  30

Arg Tyr Phe His Val Val Ile Ala Gly Pro Gln Asp Ser Pro Phe Glu
        35                  40                  45

Gly Gly Thr Phe Lys Leu Glu Leu Phe Leu Pro Glu Glu Tyr Pro Met
    50                  55                  60

Ala Ala Pro Lys Val Arg Phe Met Thr Lys Ile Tyr His Pro Asn Val
65                  70                  75                  80

Asp Lys Leu Gly Arg Ile Cys Leu Asp Ile Leu Lys Asp Lys Trp Ser
                85                  90                  95

Pro Ala Leu Gln Ile Arg Thr Val Leu Leu Ser Ile Gln Ala Leu Leu
            100                 105                 110

Ser Ala Pro Asn Pro Asp Asp Pro Leu Ala Asn Asp Val Ala Glu Gln
        115                 120                 125

Trp Lys Ser Asn Glu Ala Gln Ala Ile Glu Thr Ala Arg Ala Trp Thr
    130                 135                 140

Arg Leu Tyr Ala Met Asn Asn Ile
145                 150


<210> 19
<211> 459
<212> DNA
<213> Rattus norvegicus

<220>
<223> Bendless protein


<400> 19
atggccgggc tgccccgcag gatcatcaag gaaacccagc gtttgctggc agaaccagtt 60
cctggcatta aagcagaacc agatgagagc aacgcccgtt attttcatgt ggtcattgct 120
ggtccccagg attccccctt tgagggaggg acttttaaac ttgaactatt ccttccagaa 180
gaatacccaa tggcagcacc taaagtacgt ttcatgacca aaatttatca tcctaatgta 240
gacaagttgg gaagaatatg tttagatatt ttgaaagata agtggtcccc agcactgcag 300
atccgaacag ttctgctatc aatccaggct ttgctaagtg ctcctaatcc agatgatcca 360
ttagcaaatg atgtagctga gcagtggaag agcaacgaag cccaagccat agaaacagca 420
agagcatgga ctaggctata tgccatgaat aatatttaa                        459


<210> 20
<211> 2505
<212> PRT
<213> Rattus norvegicus

<220>
<223> Fatty acid synthase


<400> 20
Met Glu Glu Val Val Ile Ala Gly Met Ser Gly Lys Leu Pro Glu Ser
1               5                   10                  15

Glu Asn Leu Gln Glu Phe Trp Ala Asn Leu Ile Gly Gly Val Asp Met
            20                  25                  30

Val Thr Asp Asp Asp Arg Arg Trp Lys Ala Gly Leu Tyr Gly Leu Pro
        35                  40                  45

Lys Arg Ser Gly Lys Leu Lys Asp Leu Ser Lys Phe Asp Ala Ser Phe
    50                  55                  60

Phe Gly Val His Pro Lys Gln Ala His Thr Met Asp Pro Gln Leu Arg
65                  70                  75                  80

Leu Leu Leu Glu Val Ser Tyr Glu Ala Ile Val Asp Gly Gly Ile Asn
                85                  90                  95

Pro Ala Ser Leu Arg Gly Thr Asn Thr Gly Val Trp Val Gly Val Ser
            100                 105                 110

Gly Ser Glu Ala Ser Glu Ala Leu Ser Arg Asp Pro Glu Thr Leu Leu
        115                 120                 125

Gly Tyr Ser Met Val Gly Cys Gln Arg Ala Met Met Ala Asn Arg Leu
    130                 135                 140

Ser Phe Phe Phe Asp Phe Lys Gly Pro Ser Ile Ala Leu Asp Thr Ala
145                 150                 155                 160

Cys Ser Ser Ser Leu Leu Ala Leu Gln Asn Ala Tyr Gln Ala Ile Arg
                165                 170                 175

Ser Gly Glu Cys Pro Ala Ala Ile Val Gly Gly Ile Asn Leu Leu Leu
            180                 185                 190

Lys Pro Asn Thr Ser Val Gln Phe Met Lys Leu Gly Met Leu Ser Pro
        195                 200                 205

Asp Gly Thr Cys Arg Ser Phe Asp Asp Ser Gly Asn Gly Tyr Cys Arg
    210                 215                 220

Ala Glu Ala Val Val Ala Val Leu Leu Thr Lys Lys Ser Leu Ala Arg
225                 230                 235                 240

Arg Val Tyr Ala Thr Ile Leu Asn Ala Gly Thr Asn Thr Asp Gly Cys
                245                 250                 255

Lys Glu Gln Gly Val Thr Phe Pro Ser Gly Glu Ala Gln Glu Gln Leu
            260                 265                 270

Ile Arg Ser Leu Tyr Gln Pro Gly Gly Val Ala Pro Glu Ser Leu Glu
        275                 280                 285

Tyr Ile Glu Ala His Gly Thr Gly Thr Lys Val Gly Asp Pro Gln Glu
    290                 295                 300

Leu Asn Gly Ile Thr Arg Ser Leu Cys Ala Phe Arg Gln Ser Pro Leu
305                 310                 315                 320

Leu Ile Gly Ser Thr Lys Ser Asn Met Gly His Pro Glu Pro Ala Ser
                325                 330                 335

Gly Leu Ala Ala Leu Thr Lys Val Leu Leu Ser Leu Glu Asn Gly Val
            340                 345                 350

Trp Ala Pro Asn Leu His Phe His Asn Pro Asn Pro Glu Ile Pro Ala
        355                 360                 365

Leu Leu Asp Gly Arg Leu Gln Val Val Asp Arg Pro Leu Pro Val Arg
370                 375                 380

Gly Gly Ile Val Gly Ile Asn Ser Phe Gly Phe Gly Gly Ala Asn Val
385                 390                 395                 400

His Val Ile Leu Gln Pro Asn Thr Gln Gln Ala Pro Ala Pro Ala Pro
                405                 410                 415

His Ala Ala Leu Pro His Leu Leu His Ala Ser Gly Arg Thr Met Glu
            420                 425                 430

Ala Val Gln Gly Leu Leu Glu Gln Gly Arg Gln His Ser Gln Asp Leu
        435                 440                 445

Ala Phe Val Ser Met Leu Asn Asp Ile Ala Ala Thr Pro Thr Ala Ala
    450                 455                 460

Met Pro Phe Arg Gly Tyr Thr Val Leu Gly Val Glu Gly His Val Gln
465                 470                 475                 480

Glu Val Gln Gln Val Pro Ala Ser Gln Arg Pro Leu Trp Phe Ile Cys
                485                 490                 495

Ser Gly Met Gly Thr Gln Trp Arg Gly Met Gly Leu Ser Leu Met Arg
            500                 505                 510

Leu Asp Ser Phe Arg Glu Ser Ile Leu Arg Ser Asp Glu Ala Leu Lys
        515                 520                 525

Pro Leu Gly Val Lys Val Ser Asp Leu Leu Leu Ser Thr Asp Glu His
    530                 535                 540

Thr Phe Asp Asp Ile Val His Ser Phe Val Ser Leu Thr Ala Ile Gln
545                 550                 555                 560

Ile Ala Leu Ile Asp Leu Leu Thr Ser Met Gly Leu Lys Pro Asp Gly
                565                 570                 575

Ile Ile Gly His Ser Leu Gly Glu Val Ala Cys Gly Tyr Ala Asp Gly
            580                 585                 590

Cys Leu Ser Gln Arg Glu Ala Val Leu Ala Ala Tyr Trp Arg Gly Gln
        595                 600                 605

Cys Ile Lys Asp Ala Asn Leu Pro Ala Gly Ser Met Ala Ala Val Gly
    610                 615                 620

Leu Ser Trp Glu Glu Cys Lys Gln Arg Cys Pro Pro Gly Val Val Pro
625                 630                 635                 640

Ala Cys His Asn Ser Glu Asp Thr Val Thr Ile Ser Gly Pro Gln Ala
                645                 650                 655

Ala Val Asn Glu Phe Val Glu Gln Leu Lys Gln Glu Gly Val Phe Ala
            660                 665                 670

Lys Glu Val Arg Thr Gly Gly Leu Ala Phe His Ser Tyr Phe Met Glu
        675                 680                 685

Gly Ile Ala Pro Thr Leu Leu Gln Ala Leu Lys Lys Val Ile Arg Glu

690                 695                 700

Pro Arg Pro Arg Ser Ala Arg Trp Leu Ser Thr Ser Ile Pro Glu Ala
705                 710                 715                 720

Gln Trp Gln Ser Ser Leu Ala Arg Thr Ser Ser Ala Glu Tyr Asn Val
                725                 730                 735

Asn Asn Leu Val Ser Pro Val Leu Phe Gln Glu Ala Leu Trp His Val
            740                 745                 750

Pro Glu His Ala Val Val Leu Glu Ile Ala Pro His Ala Leu Leu Gln
        755                 760                 765

Ala Val Leu Lys Arg Gly Val Lys Pro Ser Cys Thr Ile Ile Pro Leu
    770                 775                 780

Met Lys Arg Asp His Lys Asp Asn Leu Glu Phe Phe Leu Thr Asn Leu
785                 790                 795                 800

Gly Lys Val His Leu Thr Gly Ile Asp Ile Asn Pro Asn Ala Leu Phe
                805                 810                 815

Pro Pro Val Glu Phe Pro Val Pro Arg Gly Thr Pro Leu Ile Ser Pro
            820                 825                 830

His Ile Lys Trp Asp His Ser Gln Thr Trp Asp Ile Pro Val Ala Glu
        835                 840                 845

Asp Phe Pro Asn Gly Ser Ser Ser Ser Ser Ala Thr Val Tyr Asn Ile
    850                 855                 860

Asp Ala Ser Ser Glu Ser Ser Asp His Tyr Leu Val Asp His Cys Ile
865                 870                 875                 880

Asp Gly Arg Val Leu Phe Pro Gly Thr Gly Tyr Leu Tyr Leu Val Trp
                885                 890                 895

Lys Thr Leu Ala Arg Ser Leu Ser Leu Ser Leu Glu Glu Thr Pro Val
            900                 905                 910

Val Phe Glu Asn Val Thr Phe His Gln Ala Thr Ile Leu Pro Arg Thr
        915                 920                 925

Gly Thr Val Pro Leu Glu Val Arg Leu Leu Glu Ala Ser His Ala Phe
    930                 935                 940

Glu Val Ser Asp Ser Gly Asn Leu Ile Val Ser Gly Lys Val Tyr Gln
945                 950                 955                 960

Trp Glu Asp Pro Asp Ser Lys Leu Phe Asp His Pro Glu Val Pro Ile
                965                 970                 975

Pro Ala Glu Ser Glu Ser Val Ser Arg Leu Thr Gln Gly Glu Val Tyr
            980                 985                 990

Lys Glu Leu Arg Leu Arg Gly Tyr Asp Tyr Gly Pro His Phe Gln Gly
        995                 1000                1005

Val Tyr Glu Ala Thr Leu Glu Gly Glu Gln Gly Lys Leu Leu Trp Lys
    1010                1015                1020

Asp Asn Trp Val Thr Phe Met Asp Thr Met Leu Gln Ile Ser Ile Leu
1025                1030                1035                1040

Gly Phe Ser Lys Gln Ser Leu Gln Leu Pro Thr Arg Val Thr Ala Ile
                1045                1050                1055

Tyr Ile Asp Pro Ala Thr His Leu Gln Lys Val Tyr Met Leu Glu Gly
            1060                1065                1070

Asp Thr Gln Val Ala Asp Val Thr Thr Ser Arg Cys Leu Gly Val Thr
        1075                1080                1085

Val Ser Gly Gly Val Tyr Ile Ser Arg Leu Gln Thr Thr Ala Thr Ser
    1090                1095                1100

Arg Arg Gln Gln Glu Gln Leu Val Pro Thr Leu Glu Lys Phe Val Phe
1105                1110                1115                1120

Thr Pro His Val Glu Pro Glu Cys Leu Ser Glu Ser Ala Ile Leu Gln
                1125                1130                1135

Lys Glu Leu Gln Leu Cys Lys Gly Leu Ala Lys Ala Leu Gln Thr Lys
            1140                1145                1150

Ala Thr Gln Gln Gly Leu Lys Met Thr Val Pro Gly Leu Glu Asp Leu
        1155                1160                1165

Pro Gln His Gly Leu Pro Arg Leu Leu Ala Ala Ala Cys Gln Leu Gln
    1170                1175                1180

Leu Asn Gly Asn Leu Gln Leu Glu Leu Gly Glu Val Leu Ala Arg Glu
1185                1190                1195                1200

Arg Leu Leu Leu Pro Glu Asp Pro Leu Ile Ser Gly Leu Leu Asn Ser
                1205                1210                1215

Gln Ala Leu Lys Ala Cys Ile Asp Thr Ala Leu Glu Asn Leu Ser Thr
            1220                1225                1230

Leu Lys Met Lys Val Val Glu Val Leu Ala Gly Glu Gly His Leu Tyr
        1235                1240                1245

Ser His Ile Ser Ala Leu Leu Asn Thr Gln Pro Met Leu Gln Leu Glu
    1250                1255                1260

Tyr Thr Ala Thr Asp Arg His Pro Gln Ala Leu Lys Asp Val Gln Thr
1265                1270                1275                1280

Lys Leu Gln Gln His Asp Val Ala Gln Gly Gln Trp Asp Pro Ser Gly
                1285                1290                1295

Pro Ala Pro Thr Asn Leu Gly Ala Leu Asp Leu Val Val Cys Asn Cys
            1300                1305                1310

Ala Leu Ala Thr Leu Gly Asp Pro Ala Leu Ala Leu Asp Asn Met Val
        1315                1320                1325

Ala Ala Leu Lys Asp Gly Gly Phe Leu Leu Met His Thr Val Leu Lys
    1330                1335                1340

Gly His Ala Leu Gly Glu Thr Leu Ala Cys Leu Pro Ser Glu Val Gln
1345                1350                1355                1360

Pro Gly Pro Ser Phe Leu Ser Gln Glu Glu Trp Glu Ser Leu Phe Ser
                1365                1370                1375

Arg Lys Ala Leu His Leu Val Gly Leu Lys Lys Ser Phe Tyr Gly Thr
            1380                1385                1390

Ala Leu Phe Leu Cys Arg Arg Leu Ser Pro Gln Asp Lys Pro Ile Phe
        1395                1400                1405

Leu Pro Val Glu Asp Thr Ser Phe Gln Trp Val Asp Ser Leu Lys Ser
    1410                1415                1420

Ile Leu Ala Thr Ser Ser Ser Gln Pro Val Trp Leu Thr Ala Met Asn
1425                1430                1435                1440

Cys Pro Thr Ser Gly Val Val Gly Leu Val Asn Cys Leu Arg Lys Glu
                1445                1450                1455

Pro Gly Gly His Arg Ile Arg Cys Ile Leu Leu Ser Asn Leu Ser Ser
            1460                1465                1470

Thr Ser His Val Pro Lys Leu Asp Pro Gly Ser Ser Glu Leu Gln Lys
        1475                1480                1485

Val Leu Glu Ser Asp Leu Val Met Asn Val Tyr Arg Asp Gly Ala Trp
    1490                1495                1500

Gly Ala Phe Arg His Phe Gln Leu Glu Gln Asp Lys Pro Glu Glu Gln
1505                1510                1515                1520

Thr Ala His Ala Phe Val Asn Val Leu Thr Arg Gly Asp Leu Ala Ser
                1525                1530                1535

Ile Arg Trp Val Ser Ser Pro Leu Lys His Met Gln Pro Pro Ser Ser
            1540                1545                1550

Ser Gly Ala Gln Leu Cys Thr Val Tyr Tyr Ala Ser Leu Asn Phe Arg
        1555                1560                1565

Asp Ile Met Leu Ala Thr Gly Lys Leu Ser Pro Asp Ala Ile Pro Gly
    1570                1575                1580

Lys Trp Ala Ser Arg Asp Cys Met Leu Gly Met Glu Phe Ser Gly Arg
1585                1590                1595                1600

Asp Lys Cys Gly Arg Arg Val Met Gly Leu Val Pro Ala Glu Gly Leu
                1605                1610                1615

Ala Thr Ser Val Leu Leu Ser Pro Asp Phe Leu Trp Asp Val Pro Ser
            1620                1625                1630

Ser Trp Thr Leu Glu Glu Ala Ala Ser Val Pro Val Val Tyr Thr Thr
        1635                1640                1645

Ala Tyr Tyr Ser Leu Val Val Arg Gly Arg Ile Gln His Gly Glu Thr
    1650                1655                1660

Val Leu Ile His Ser Gly Ser Gly Gly Val Gly Gln Ala Ala Ile Ser
1665                1670                1675                1680

Ile Ala Leu Ser Leu Gly Cys Arg Val Phe Thr Thr Val Gly Ser Ala

1685                1690                1695

Glu Lys Arg Ala Tyr Leu Gln Ala Arg Phe Pro Gln Leu Asp Asp Thr
        1700                1705                1710

Ser Phe Ala Asn Ser Arg Asp Thr Ser Phe Glu Gln His Val Leu Leu
    1715                1720                1725

His Thr Gly Gly Lys Gly Val Asp Leu Val Leu Asn Ser Leu Ala Glu
    1730                1735                1740

Glu Lys Leu Gln Ala Ser Val Arg Cys Leu Ala Gln His Gly Arg Phe
1745                1750                1755                1760

Leu Glu Ile Gly Lys Phe Asp Leu Ser Asn Asn His Pro Leu Gly Met
                1765                1770                1775

Ala Ile Phe Leu Lys Asn Val Thr Phe His Gly Ile Leu Leu Asp Ala
            1780                1785                1790

Leu Phe Glu Gly Ala Asn Asp Ser Trp Arg Glu Val Ala Glu Leu Leu
        1795                1800                1805

Lys Ala Gly Ile Arg Asp Gly Val Val Lys Pro Leu Lys Cys Thr Val
    1810                1815                1820

Phe Pro Lys Ala Gln Val Glu Asp Ala Phe Arg Tyr Met Ala Gln Gly
1825                1830                1835                1840

Lys His Ile Gly Lys Val Leu Val Gln Val Arg Glu Glu Glu Pro Glu
                1845                1850                1855

Ala Met Leu Pro Gly Ala Gln Pro Thr Leu Ile Ser Ala Ile Ser Lys
            1860                1865                1870

Thr Phe Cys Pro Glu His Lys Ser Tyr Ile Ile Thr Gly Gly Leu Gly
        1875                1880                1885

Gly Phe Gly Leu Glu Leu Ala Arg Trp Leu Val Leu Arg Gly Ala Gln
    1890                1895                1900

Arg Leu Val Leu Thr Ser Arg Ser Gly Ile Arg Thr Gly Tyr Gln Ala
1905                1910                1915                1920

Lys His Val Arg Glu Trp Arg Arg Gln Gly Ile His Val Leu Val Ser
                1925                1930                1935

Thr Ser Asn Val Ser Ser Leu Glu Gly Ala Arg Ala Leu Ile Ala Glu
            1940                1945                1950

Ala Thr Lys Leu Gly Pro Val Gly Gly Val Phe Asn Leu Ala Met Val
        1955                1960                1965

Leu Arg Asp Ala Met Leu Glu Asn Gln Thr Pro Glu Leu Phe Gln Asp
    1970                1975                1980

Val Asn Lys Pro Lys Tyr Asn Gly Thr Leu Asn Leu Asp Arg Ala Thr
1985                1990                1995                2000

Arg Glu Ala Cys Pro Glu Leu Asp Tyr Phe Val Ala Phe Ser Ser Val
                2005                2010                2015

Ser Cys Gly Arg Gly Asn Ala Gly Gln Ser Asn Tyr Gly Phe Ala Asn
        2020                2025                2030

Ser Thr Met Glu Arg Ile Cys Glu Gln Arg Arg His Asp Gly Leu Pro
    2035                2040                2045

Gly Leu Ala Val Gln Trp Gly Ala Ile Gly Asp Val Gly Ile Ile Leu
2050                2055                2060

Glu Ala Met Gly Thr Asn Asp Thr Val Val Gly Gly Thr Leu Pro Gln
2065                2070                2075                2080

Arg Ile Ser Ser Cys Met Glu Val Leu Asp Leu Phe Leu Asn Gln Pro
                2085                2090                2095

His Ala Val Leu Ser Ser Phe Val Leu Ala Glu Lys Lys Ala Val Ala
            2100                2105                2110

His Gly Asp Gly Glu Ala Gln Arg Asp Leu Val Lys Ala Val Ala His
        2115                2120                2125

Ile Leu Gly Ile Arg Asp Leu Ala Gly Ile Asn Leu Asp Ser Ser Leu
    2130                2135                2140

Ala Asp Leu Gly Leu Asp Ser Leu Met Gly Val Glu Val Arg Gln Ile
2145                2150                2155                2160

Leu Glu Arg Glu His Asp Leu Val Leu Pro Ile Arg Glu Val Arg Gln
                2165                2170                2175

Leu Thr Leu Arg Lys Leu Gln Glu Met Ser Ser Lys Ala Gly Ser Asp
            2180                2185                2190

Thr Glu Leu Ala Ala Pro Lys Ser Lys Asn Asp Thr Ser Leu Lys Gln
        2195                2200                2205

Ala Gln Leu Asn Leu Ser Ile Leu Leu Val Asn Pro Glu Gly Pro Thr
    2210                2215                2220

Leu Thr Arg Leu Asn Ser Val Gln Ser Ser Glu Arg Pro Leu Phe Leu
2225                2230                2235                2240

Val His Pro Ile Glu Gly Ser Ile Thr Val Phe His Ser Leu Ala Ala
                2245                2250                2255

Lys Leu Ser Val Pro Thr Tyr Gly Leu Gln Cys Thr Gln Ala Ala Pro
            2260                2265                2270

Leu Asp Ser Ile Pro Asn Leu Ala Ala Tyr Tyr Ile Asp Cys Ile Lys
        2275                2280                2285

Gln Val Gln Pro Glu Gly Pro Tyr Arg Val Ala Gly Tyr Ser Phe Gly
    2290                2295                2300

Ala Cys Val Ala Phe Glu Met Cys Ser Gln Leu Gln Ala Gln Gln Gly
2305                2310                2315                2320

Pro Ala Pro Ala His Asn Asn Leu Phe Leu Phe Asp Gly Ser His Thr
                2325                2330                2335

Tyr Val Leu Ala Tyr Thr Gln Ser Tyr Arg Ala Lys Leu Thr Pro Gly
            2340                2345                2350

Cys Glu Ala Glu Ala Glu Ala Glu Ala Ile Cys Phe Phe Ile Lys Gln
        2355                2360                2365

Phe Val Asp Ala Glu His Ser Lys Val Leu Glu Ala Leu Leu Pro Leu
    2370                2375                2380

Lys Ser Leu Glu Asp Arg Val Ala Ala Ala Val Asp Leu Ile Thr Arg
2385                2390                2395                2400

Ser His Gln Ser Leu Asp Arg Arg Asp Leu Ser Phe Ala Ala Val Ser
                2405                2410                2415

Phe Tyr Tyr Lys Leu Arg Ala Ala Asp Gln Tyr Lys Pro Lys Ala Lys
            2420                2425                2430

Tyr His Gly Asn Val Ile Leu Leu Arg Ala Lys Thr Gly Gly Thr Tyr
        2435                2440                2445

Gly Glu Asp Leu Gly Ala Asp Tyr Asn Leu Ser Gln Val Cys Asp Gly
    2450                2455                2460

Lys Val Ser Val His Ile Ile Glu Gly Asp His Arg Thr Leu Leu Glu
2465                2470                2475                2480

Gly Arg Gly Leu Glu Ser Ile Ile Asn Ile Ile His Ser Ser Leu Ala
                2485                2490                2495

Glu Pro Arg Val Ser Val Arg Glu Gly
            2500                2505


<210> 21
<211> 9143
<212> DNA
<213> Rattus norvegicus

<220>
<223> Fatty acid synthase

<400> 21
agagagacgg cagcagcgtc ccgtccagtt cgcctgccgc gctcctcgct tgtcgtctgc 60
ctccagatcc cagacagaga agagccatgg aggaggtggt gatagccggt atgtccggga 120
aattgcccga gtcagagaac ctgcaggagt tctgggccaa cctcattggc ggtgtggaca 180
tggtcacaga cgatgacagg aggtggaagg ctgggctcta tgggttgcct aagcggtctg 240
gaaagctgaa ggatctgtcc aagttcgacg cctccttttt tggggtccac cccaagcagg 300
cacacacaat ggacccgcag ctccggctgc tgctggaagt cagctatgaa gctattgtgg 360
acggaggtat caacccggcc tcactccgag gaacaaacac tggtgtctgg gtgggtgtga 420
gtggttccga ggcgtcggag gccctgagca gagatcctga gactcttctg ggctacagca 480
tggtgggctg ccagagagca atgatggcca accggctctc tttcttcttc gacttcaaag 540
gacccagcat tgccctggac acagcctgct cctctagcct actggcacta cagaatgcct 600
atcaggctat ccgcagtggg gagtgccctg ctgccattgt gggcgggatc aacctgctgc 660
taaagcctaa cacctctgtg cagttcatga agctaggcat gctcagcccc gatggcacct 720
gcagatcctt tgatgattca gggaacgggt attgccgtgc tgaggctgtc gtggcagttc 780
tgctgactaa gaagtccttg gctcggcgag tctatgccac tattctgaat gccgggacga 840
acacagatgg ctgcaaggag caaggcgtga cattcccctc tggagaagcc caggaacaac 900
tcatccgttc tctgtatcag ccgggcggtg tggcccccga gtctcttgaa tatattgaag 960
cccatggcac gggcaccaag gtgggggacc cccaggaact gaacggcatt actcggtccc 1020
tgtgtgcttt ccgccagagc cctttgttaa ttggctccac caaatccaac atgggacacc 1080
ctgagcctgc ctcggggctt gcagccctga ccaaggtgct gttatcccta gaaaatgggg 1140
tttgggcccc caacctgcat ttccacaacc ccaaccctga aatcccagca cttcttgatg 1200
ggcggctgca ggtggtcgat aggcccctgc ctgttcgtgg tggcatcgtg ggcatcaact 1260

cgtttggctt cggaggtgcc aatgttcacg tcatcctcca gcccaacaca cagcaggccc 1320
cagcacctgc cccacatgct gccctaccgc atttgctgca tgccagtgga cggaccatgg 1380
aggcagtgca gggcctgctg gaacagggcc gccagcacag tcaggacttg gcctttgtga 1440
gcatgctcaa tgacattgca gcaaccccta cagcagccat gcccttcaga ggttacactg 1500
tgttaggtgt tgagggccat gtccaggaag tgcagcaagt gcctgccagc cagcgcccac 1560
tctggttcat ctgctcaggg atgggcacac agtggcgtgg aatggggctg agccttatgc 1620
gcctggacag tttccgtgag tccatcctgc gctctgatga ggctctgaag cccttgggag 1680
tcaaagtgtc agacctgctg ctgagcactg atgagcacac ctttgatgac atcgtgcatt 1740
cctttgtgag cctcaccgcc atccagattg ccctcatcga cctgctgacg tctatggggc 1800
tgaaacctga tggcatcatt gggcactcct tgggagaggt tgcctgtggc tatgcagatg 1860
gctgtctctc ccagagagag gctgtgcttg cagcctactg gagaggccag tgcattaagg 1920
atgccaacct tccggctgga tccatggcag ctgttggttt gtcctgggaa gaatgtaaac 1980
aacgctgccc tcctggtgtg gtgcctgcct gccacaactc tgaggacact gtgaccatct 2040
ctggacctca ggctgcagtg aatgaatttg tggagcagct aaagcaagag ggcgtgtttg 2100
ccaaggaggt gcgaacaggt ggcctggcct tccactccta cttcatggaa ggaattgccc 2160
ccacgctgct gcaggctctc aagaaggtga tccgggagcc acggccacgc tcagcacgct 2220
ggctcagcac ctctatccct gaggcccagt ggcagagcag cctggcccgc acatcttctg 2280
ctgagtacaa cgtcaacaac ctggtgagcc ctgtgctctt ccaggaagca ctgtggcacg 2340
tccccgagca cgccgtggtg ctggagattg caccccatgc actgttgcag gctgtcctga 2400
agcgaggcgt gaagcctagc tgcaccatca tccccttgat gaagagggac cataaagata 2460
acttggagtt cttcctcacc aacctcggca aggtgcacct cacaggcatc gacatcaacc 2520
ctaatgcctt gttcccacct gtggaattcc cggttccccg agggactcct ctcatctccc 2580
ctcacatcaa gtgggaccac agtcagactt gggatatccc agttgctgaa gacttcccca 2640
acggttccag ctcctcctca gctacagtct acaacattga cgccagttcc gagtcatctg 2700
accactacct ggtcgaccac tgcattgacg gccgtgtcct cttccctggc actggctacc 2760
tgtacctggt gtggaagaca ctggctcgaa gcctgagctt gtccctagaa gagacccctg 2820
tggtgtttga gaacgtgaca tttcatcagg ccaccatcct gcccaggaca ggaaccgtgc 2880
ctctggaggt gcggctgcta gaggcctcac atgcatttga ggtgtctgac agtggcaacc 2940
tgatagtgag cgggaaagtg taccagtggg aagaccctga ctccaagtta ttcgaccacc 3000
cagaagtccc gatccccgcc gagtccgagt ctgtctcccg cttgacgcag ggagaagtat 3060
acaaggagct gcggctacgt ggctatgact atggccctca tttccagggc gtctatgagg 3120
ccaccctcga aggtgagcaa ggcaagctgc tctggaaaga caactgggtg accttcatgg 3180
acacaatgct gcagatatcc atcctgggct tcagcaagca gagtctgcag ctacccaccc 3240
gtgtgactgc catctatatt gaccctgcaa cccacctgca gaaggtgtac atgctggagg 3300
gagacactca agtggctgac gtgaccacga gccgctgtct gggcgtgacc gtctctggtg 3360
gtgtctacat ttcgagacta cagacaacag caacctcacg gcggcagcag gaacagctgg 3420
tccccaccct ggagaagttt gtcttcacac cccatgtgga gcctgagtgc ctgtctgaga 3480
gtgctatcct gcagaaagag ctgcagctgt gcaagggtct ggcaaaggct ctgcagacca 3540
aggccaccca gcaagggctg aagatgacag tgcctgggct agaggacctt ccccagcatg 3600
gactgcctcg actcttggct gctgcctgcc agctgcagct caacggaaac ctgcaactgg 3660
agttaggtga ggtactggct cgagagaggc tcctgctgcc agaagaccct ctgatcagtg 3720
gcctccttaa ctcccaggcc ctcaaggcct gcatagacac agccctggag aacttgtcta 3780
ctctcaagat gaaggtggtg gaggtgctgg ctggagaagg ccacttgtat tcccacatct 3840
cagcactgct caacacccag cctatgctgc aactggagta tacagccacc gaccggcacc 3900
cccaggccct gaaggatgtt cagaccaagc tgcagcagca tgatgtagca cagggccagt 3960
gggacccttc tggtcctgct cctaccaacc tgggtgctct tgaccttgtg gtgtgcaact 4020
gtgcgttagc caccctgggg gatccagccc tggccctgga caacatggta gctgccctca 4080
aggatggtgg tttcctgcta atgcacacag tgctcaaagg acatgccctt ggggagaccc 4140
tggcctgcct cccttctgag gtgcagcctg ggcccagctt cttaagccag gaagagtggg 4200
agagcctgtt ctcaaggaag gcactgcacc tggtgggcct taaaaagtca ttctacggta 4260
ctgcgctgtt cctgtgccgc cgtctcagcc cacaggacaa gcccatcttc ctgcctgtgg 4320
aggatactag tttccagtgg gtggactctc tgaagagcat tctggccaca tcctcctccc 4380
agcctgtgtg gctaacagcc atgaactgcc ccacctcagg tgtggtaggc ttggtgaact 4440
gtctccgaaa agagccgggt ggacaccgga ttcggtgtat cctgctgtcc aacctcagca 4500
gcacatctca cgtccccaag ctggaccctg gctcttcaga gctacagaag gtgctagaga 4560
gtgatctggt gatgaacgtg tacagggacg gtgcctgggg tgccttccgt cacttccagt 4620
tagagcagga caagcccgag gagcagacag cacatgcctt tgtaaacgtc cttacccgag 4680
gggaccttgc ctccatccgc tgggtctctt ctcccctgaa acacatgcag ccgccctcga 4740
gctcaggagc acagctctgc actgtctact atgcctcact gaacttccga gatatcatgc 4800
tggccacggg caagctgtcc cctgatgcca ttccaggtaa atgggccagc cgggactgca 4860
tgcttggcat ggagttctca ggccgtgata agtgcggccg gcgtgtgatg gggctggtac 4920
ccgcagaagg cctggccacc tcagtcctgt tatcacccga cttcctctgg gatgtaccct 4980

ctagctggac cctggaggag gcggcttctg tgcctgttgt ctacaccacc gcctactact 504[?]
ccttagtagt gcgtggtcgt attcagcacg gggaaactgt gctcattcac tcgggctccg 510[?]
gtggtgtggg ccaagcggcc atttccattg cccttagcct gggctgccga gtcttcacca 516[?]
ctgtgggctc cgctgagaag cgagcttacc tccaggccag attccctcag ctggatgaca 522[?]
ccagctttgc taactctcga gacacatcgt ttgagcagca tgtgttactg cacacaggtg 528[?]
gcaaaggggt ggacctggtc ctcaactccc tggcagaaga gaagctgcag gccagtgtgc 534[?]
ggtgcttggc tcagcatggc cgcttcctag agatcggcaa atttgatctt tctaacaacc 540[?]
accctctggg catggccatc ttcttgaaga acgtcacttt ccatgggatc ctgctggatg 546[?]
cactttttga gggggccaac gacagctggc gggaggtggc agagctgctg aaggccggca 552[?]
tccgtgatgg ggttgtgaag cctctcaagt gtacagtgtt tcccaaggcc caggtggagg 558[?]
acgccttccg atacatggct caaggaaaac atattggcaa agtccttgtc caggtacggg 564[?]
aggaggagcc cgaggctatg ctgccagggg ctcagcccac cctgatttcc gccatctcca 570[?]
agaccttctg cccagagcat aagagttaca tcatcactgg tggcctaggt ggctttggcc 576[?]
tggaactggc ccggtggctt gtgcttcgtg gggcccaaag gcttgtacta acttcccgat 582[?]
ctggaatccg cacaggctac caagccaagc acgttcggga gtggaggcgc cagggcatcc 588[?]
atgtgctagt gtcgacaagc aatgtcagtt cactggaggg ggcccgtgct ctcatcgctg 594[?]
aagccacaaa gcttgggccc gttggaggtg tcttcaacct ggccatggtt ttaagggatg 600[?]
ccatgctgga gaaccagact ccagaactct tccaggatgt caacaagccc aagtacaatg 606[?]
gcaccctgaa ccttgacagg gcgacccggg aagcctgtcc tgagctggac tactttgtgg 612[?]
ccttctcctc tgtaagctgc gggcgtggta atgctggcca atccaactat ggcttcgcca 618[?]
actctaccat ggagcgtatt tgcgaacagc gccggcacga tggcctccca ggtcttgccg 624[?]
tgcaatgggg tgccattggt gacgtgggca ttatcttgga agcgatgggt accaatgaca 630[?]
cagtcgttgg cggcacactg ccacagcgca tctcctcctg catggaggtg ctggacctct 636[?]
tcctgaatca gccccacgca gtcctgagca gttttgtgct ggctgagaag aaagctgtgg 642[?]
cccatggtga tggtgaagcc cagagggatc tggtgaaagc agtggcacac atcctaggca 648[?]
tccgcgacct cgcagggatt aacctggaca gctcgctggc agacctcggc ctggactcgc 654[?]
tcatgggtgt ggaagtgcgc cagatcctgg aacgtgaaca tgatctggtg ctacccattc 660[?]
gtgaagtacg gcaactcaca ctgcggaagc ttcaggaaat gtcctccaag gctggctcag 666[?]
acactgagtt ggcagccccc aagtccaaga atgatacatc cctgaagcag gcccagctga 672[?]
atctgagtat cctgctggtg aaccctgagg gccctacctt aacacgactc aactcagtgc 678[?]
agagctctga gcggcctctg ttcctggtgc accccattga aggttccatc actgtgttcc 684[?]
acagcctggc tgccaagctc agtgtgccca cctacggtct gcagtgcacc caagcggccc 690[?]
ccctggacag cattccaaac ctggctgcct actacattga ttgcatcaag caggtgcagc 696[?]
ctgaggggcc ctaccgagtg gctgggtatt cttttggagc ttgtgtagcc ttcgagatgt 702[?]
gctcccagct gcaggcccag cagggcccag cccccgccca caacaacctc ttcttgtttg 708[?]
atggctcaca cacctacgta ttggcgtaca cccagagcta ccgggcaaag ctgaccccag 714[?]
gctgtgaggc tgaggctgaa gctgaagcca tatgcttctt cattaagcag tttgttgatg 7200
cagagcatag caaggtgcta gaggccctgc taccactgaa gagcctggag gaccgggttg 7260
ctgctgctgt ggacctcatc actagaagcc accagagcct ggaccgccgt gacctgagct 7320
ttgctgccgt gtccttctac tacaagcttc gagccgccga ccagtataaa cccaaggcca 7380
agtaccacgg caatgtgatc ctgctgcggg ccaagacagg tggcacctac ggcgaggact 7440
tgggtgccga ttacaacctg tcccaggtgt gtgatgggaa ggtgtctgtg cacatcattg 7500
agggtgacca ccgtacgctg ctggagggca ggggcctgga gtctatcatc aacatcatcc 7560
acagctccct ggctgagcct cgagtgagtg tacgggaggg ctagacctgc ctaccatgaa 7620
gccacgaccc acaccggcca ccagagatgc tccgatcccc accacaccct gagtgcaggg 7680
actggggagg gtcctgctgg tgggaccccc tcaccccagt ggcccagcac caccccctcc 7740
cctggtggct gctacaaaca ggaccatcac atgtgtccca gccacttagt ggggttccca 7800
gagccactga cttggaggca ccctggtctg tgaagagtca gtggaggcca gcaagagcca 7860
aactgagcct tttctgccaa gtgacatttg tcacactggt tgtttctcca ttaaattctc 7920
atatttattg cattgctggg aaagaccgcc caccccaggg ttaactcatt ccagaacccc 7980
taaagtggga aaagccatgt ggggaaggct gctggctgga gccccttttt gtcttagccc 8040
tgtacccgct cactgcaggg cagggtatgg agagggctgg ttcgcgggga acgaggaccc 8100
cagcagacac tgtagcccat ggcccttggt ccccagcact cccggctgca cccatgatgc 8160
agggcctacc agactctgcg gaccgcaccg ggcactcact gtatttgttt tccaagattc 8220
aaattgctgc ttgggttttg aatttactgc agctgtcagt gtaaagaaac atgtctgaac 8280
tgtgtccttt ttacaccaac ctggtaaaaa tgctcttgat gctgtcccgt tgccacaatt 8340
aaactgcacg tgagctctgg cttccgttca gtctctttcc agtcccagac ctgagtcccc 8400
agagcctcca cagctcttac agtgagaatc aaattggccc actccttgga aggcgtggca 8460
ttctgtcaga gtaaaaggaa agtagagtgt gctgattcac gttcagcgtg tggggctggc 8520
tagagacctt ggcactgtag tgaacagaat gtgtccacct ttaagtcacc ctgaaggcat 8580
caccatagct acagcctcac ccaggggtag agaatagtac tgtctacttg ttgactacct 8640
ggcagttggt gccagcccct atagaggaaa acagcagtgt gtggccactg tgagaagcat 8700

atccctggaa acaggtgacc agagcagagg gctaacgcct acctgagtca cacaaaactg 8760
accaggcttg agtgtccaga agagtctatc agaaggccac agcattcagt cctatccaca 8820
gagagcagca gactaagttg tctccttgcc agcttagaaa actgcagtgc tggggtacag 8880
gtagggtgtt caggaggtcc gggccccagt gattagtcta agactgaagc atctggttgg 8940
ctgtggtccc acctagaaaa ttcttaaagc tcttgtcatg tacttcctgg gaaggaccta 9000
ccctgtctca ataatgtctc tagctcgttg gagtctactg actcaaacat ttataaagtg 9060
tcctagaaag gcctgactcc cctacaaggc tgtgtgatcc ttcaaactca catatgtgag 9120
ccaataaaac cttgagactc tag 9143

<210> 22
<211> 373
<212> PRT
<213> Rattus norvegicus

<220>
<223> Glutamine synthetase

<400> 22
Met Ala Thr Ser Ala Ser Ser His Leu Asn Lys Gly Ile Lys Gln Met
1               5                   10                  15

Tyr Met Asn Leu Pro Gln Gly Glu Lys Ile Gln Leu Met Tyr Ile Trp
            20                  25                  30

Val Asp Gly Thr Gly Glu Gly Leu Arg Cys Lys Thr Arg Thr Leu Asp
        35                  40                  45

Cys Asp Pro Lys Cys Val Glu Glu Leu Pro Glu Trp Asn Phe Asp Gly
    50                  55                  60

Ser Ser Thr Phe Gln Ser Glu Gly Ser Asn Ser Asp Met Tyr Leu His
65                  70                  75                  80

Pro Val Ala Met Phe Arg Asp Pro Phe Arg Arg Asp Pro Asn Lys Leu
                85                  90                  95

Val Phe Cys Glu Val Phe Lys Tyr Asn Arg Lys Pro Ala Glu Thr Asn
            100                 105                 110

Leu Arg His Ser Cys Lys Arg Ile Met Asp Met Val Ser Ser Gln Arg
        115                 120                 125

Pro Trp Phe Gly Met Glu Gln Glu Tyr Thr Leu Met Gly Thr Asp Gly
    130                 135                 140

His Pro Phe Gly Trp Pro Ser Asn Gly Phe Pro Gly Pro Gln Gly Pro
145                 150                 155                 160

Tyr Tyr Cys Gly Val Gly Ala Asp Lys Ala Tyr Gly Arg Asp Ile Val
                165                 170                 175

Glu Ala His Tyr Arg Ala Cys Leu Tyr Ala Gly Ile Lys Ile Thr Gly
            180                 185                 190

Thr Asn Ala Glu Val Met Pro Ala Gln Trp Glu Phe Gln Ile Gly Pro
        195                 200                 205

Cys Glu Gly Ile Arg Met Gly Asp His Leu Trp Val Ala Arg Phe Ile
    210                 215                 220

Leu His Arg Val Cys Glu Asp Phe Gly Val Ile Ala Thr Phe Asp Pro

225                 230                 235                 240

Lys Pro Ile Pro Gly Asn Trp Asn Gly Ala Gly Cys His Thr Asn Phe
                245                 250                 255

Ser Thr Lys Ala Met Arg Glu Glu Asn Gly Leu Arg Cys Ile Glu Glu
            260                 265                 270

Ala Ile Asp Lys Leu Ser Lys Arg His Gln Tyr His Ile Arg Ala Tyr
        275                 280                 285

Asp Pro Lys Gly Gly Leu Asp Asn Ala Arg Arg Leu Thr Gly Phe His
    290                 295                 300

Glu Thr Ser Asn Ile Asn Asp Phe Ser Ala Gly Val Ala Asn Arg Ser
305                 310                 315                 320

Ala Ser Ile Arg Ile Pro Arg Ile Val Gly Gln Glu Lys Lys Gly Tyr
                325                 330                 335

Phe Glu Asp Arg Arg Pro Ser Ala Asn Cys Asp Pro Tyr Ala Val Thr
            340                 345                 350

Glu Ala Ile Val Arg Thr Cys Leu Leu Asn Glu Thr Gly Asp Glu Pro
        355                 360                 365

Phe Gln Tyr Lys Asn
    370

<210> 23
<211> 2793
<212> DNA
<213> Rattus norvegicus

<220>
<223> Glutamine synthetase

<400> 23
acagccgaga atgggagtag ggcggagtgt ttgagcagca cacccatttc ctctccgctc 60
ttcgtctcgt tctcgtggcc tgtccaccca tccatcatct gccggccacc gctctgaaca 120
ccttccacca tggccacctc agcaagttcc cacttgaaca aaggcatcaa gcagatgtac 180
atgaacctgc cccagggcga gaagatccaa ctcatgtata tctgggttga tggtaccggg 240
gaagggctac gctgcaagac ccgtactctg gactgtgacc ccaagtgtgt agaagagtta 300
cccgagtgga actttgatgg ttctagtacg tttcagtctg aaggctccaa cagcgacatg 360
tacctccatc ctgtggccat gtttcgagac cccttccgca gagaccccaa caagctggtg 420
ttctgcgaag tattcaagta taaccggaag cccgcagaga ccaacctgag gcacagctgt 480
aagcgtataa tggacatggt gagcagccag cgcccctggt ttggaatgga acaggagtat 540
actctcatgg gaacagacgg ccaccctttc ggctggcctt ctaatggctt ccctggaccc 600
caaggaccct attactgcgg tgtgggagct gacaaggctt atggccgaga tatcgtggag 660
gctcactacc gggcctgctt gtatgctgga atcaagatca cagggacaaa tgccgaggtt 720
atgcctgccc agtgggaatt ccagatagga ccctgcgaag ggatccgcat gggagatcat 780
ctctgggtag cccgttttat cttgcatcgg gtatgcgaag actttggggt gatagcaacc 840
tttgacccca agcccattcc agggaactgg aatggggcag gctgccacac caactttagc 900
accaaggcca tgcgggagga gaatggtctg aggtgcattg aggaggccat tgataaactg 960
agcaagaggc accagtacca catccgtgcc tacgacccca aggggggcct ggacaacgcc 1020
cgccgtctga ctggattcca cgaaacctcc aacatcaacg acttttccgc tggcgttgcc 1080
aaccgcagcg ccagtatccg cattccccgg attgtcggcc aggagaagaa gggttacttt 1140
gaagaccgtc ggccttctgc caattgcgac ccctatgcgg tgacggaagc catcgtccgc 1200
acgtgtctcc tcaacgaaac tggcgacgag cccttccaat acaagaacta agcggactcg 1260
acttccagtg atcttgagcc cttcctagtt caccccactc ccaactgttc cctctcccac 1320

tggtccccac tgtaactcaa aaggatggaa taccaaggtc tttttattcc ttgcgcccag 1380
ttaatttttg cctttattgg tcagaataga ggggtcaggt tcttaatctc tacacaccca 1440
accccttctt tcctagctag ctttccagtg ggggaacggg agggggtggg gaagggtaac 1500
ccaccgcttc atctcagcgg gaatgcatgt cctgtaggca tagctgtcac aaatcgggtg 1560
tacttgtggt gagggaggac tggttttttt tttccttcag gataattgaa agggcaggcc 1620
caacagctta gattaacatt ttctctgtca gtagagagct gttatttctt ccggtgaaac 1680
cagctttcta ttgaagtctg gtgaggagtt ggaggttggt ctcttggctt ccttagctta 1740
gggaagggga gttcaccctc ccttcatgaa acacagttca cctgacaaat ggccctactg 1800
taaaggaaga aaaaagtttc ttggtcctcc atttataact caaagcagag tagtattttt 1860
atatttaaat gttaaaaaca aaaaagttat atatatgggt gtgtggatat atatgtcttt 1920
tctaattgag aaaaccatcc tattccctgg gtgccaagtt tgagtgagga gctcggtgta 1980
gaagtgaggc actcttgagg taggggtggg gatgcagtac tgggaaagtt ggttatcttg 2040
ggggttcagc ttcattacta cttagggttt ccctgcccac tctgcaggag cagatgttgg 2100
acaggtagcc agtgggatgc cactgcttgc cgccactgtc cctggcttta gtttaagggg 2160
acgtgtatac ctaatccaca cacgagttag aagtatgagt tggctggtca acttgaacat 2220
tgttacaggc gggtgggtgt tagtgggggg ttattttttg gtgggactag catgtcacta 2280
aagcgggcct tttgatatat taaatttttt aaagcaaaac aagtttagat tttaatcaag 2340
ttcgtagggt ttctaacttt acagaattgc ctgtttgttt caatgactcc ttccacttgg 2400
ctcttagggg aactgaggac aggcctggag ttaatacact tgtcattctg tgtcctagtg 2460
tcctcttcct ccggcagact gtccccttcc ttctgaaaaa gccgatagag tcttgtttta 2520
tttttctttt ataataaaca caccccacct ccatcccagc ttgttgcctt gcagttttct 2580
ggatgtttgt gtcggcagca ggcagctgtg gtttttttct cttgccacga tgactctaat 2640
taccatgtat agtatgttca gttagataac tcactgtaaa cagactgtaa ctgagagcag 2700
agcttgtaaa tcaacctaac gtttataaga tttcctctga cttgtttctt tgtggttcca 2760
aaaaaaaaaa aaaaaaaaaa aacctcaaaa act 2793

<210> 24
<211> 514
<212> PRT
<213> Homo sapiens

<220>
<223> Seryl t-RNA synthetase

<400> 24
Met Val Leu Asp Leu Asp Leu Phe Arg Val Asp Lys Gly Gly Asp Pro
1               5                   10                  15

Ala Leu Ile Arg Glu Thr Gln Glu Lys Arg Phe Lys Asp Pro Gly Leu
            20                  25                  30

Val Asp Gln Leu Val Lys Ala Asp Ser Glu Trp Arg Arg Cys Arg Phe
        35                  40                  45

Arg Ala Asp Asn Leu Ser Lys Leu Lys Asn Leu Cys Ser Lys Thr Ile
    50                  55                  60

Gly Glu Lys Met Lys Lys Lys Glu Pro Val Gly Asp Asp Glu Ser Val
65                  70                  75                  80

Pro Glu Asn Val Leu Ser Phe Asp Asp Leu Thr Ala Asp Ala Leu Ala
                85                  90                  95

Asn Leu Lys Val Ser Gln Ile Lys Lys Val Arg Leu Leu Ile Asp Glu
            100                 105                 110

Ala Ile Leu Lys Cys Asp Ala Glu Arg Ile Lys Leu Glu Ala Glu Arg
        115                 120                 125

Phe Glu Asn Leu Arg Glu Ile Gly Asn Leu Leu His Pro Ser Val Pro

130                 135                 140

Ile Ser Asn Asp Glu Asp Val Asp Asn Lys Val Glu Arg Ile Trp Gly
145                 150                 155                 160

Asp Cys Thr Val Arg Lys Lys Tyr Ser His Val Asp Leu Val Val Met
                165                 170                 175

Val Asp Gly Phe Glu Gly Glu Lys Gly Ala Val Val Ala Gly Ser Arg
            180                 185                 190

Gly Tyr Phe Leu Lys Gly Val Leu Val Phe Leu Glu Gln Ala Leu Ile
        195                 200                 205

Gln Tyr Ala Leu Arg Thr Leu Gly Ser Arg Gly Tyr Ile Pro Ile Tyr
    210                 215                 220

Thr Pro Phe Phe Met Arg Lys Glu Val Met Gln Glu Val Ala Gln Leu
225                 230                 235                 240

Ser Gln Phe Asp Glu Glu Leu Tyr Lys Val Ile Gly Lys Gly Ser Glu
                245                 250                 255

Lys Ser Asp Asp Asn Ser Tyr Asp Glu Lys Tyr Leu Ile Ala Thr Ser
            260                 265                 270

Glu Gln Pro Ile Ala Ala Leu His Arg Asp Glu Trp Leu Arg Pro Glu
        275                 280                 285

Asp Leu Pro Ile Lys Tyr Ala Gly Leu Ser Thr Cys Phe Arg Gln Glu
    290                 295                 300

Val Gly Ser His Gly Arg Asp Thr Arg Gly Ile Phe Arg Val His Gln
305                 310                 315                 320

Phe Glu Lys Ile Glu Gln Phe Val Tyr Ser Ser Pro His Asp Asn Lys
                325                 330                 335

Ser Trp Glu Met Phe Glu Glu Met Ile Thr Thr Ala Glu Glu Phe Tyr
            340                 345                 350

Gln Ser Leu Gly Ile Pro Tyr His Ile Val Asn Ile Val Ser Gly Ser
        355                 360                 365

Leu Asn His Ala Ala Ser Lys Lys Leu Asp Leu Glu Ala Trp Phe Pro
    370                 375                 380

Gly Ser Gly Ala Phe Arg Glu Leu Val Ser Cys Ser Asn Cys Thr Asp
385                 390                 395                 400

Tyr Gln Ala Arg Arg Leu Arg Ile Arg Tyr Gly Gln Thr Lys Lys Met
                405                 410                 415

Met Asp Lys Val Glu Phe Val His Met Leu Asn Ala Thr Met Cys Ala
            420                 425                 430

Thr Thr Arg Thr Ile Cys Ala Ile Leu Glu Asn Tyr Gln Thr Glu Lys
        435                 440                 445

Gly Ile Thr Val Pro Glu Lys Leu Lys Glu Phe Met Pro Pro Gly Leu
    450                 455                 460

Gln Glu Leu Ile Pro Phe Val Lys Pro Ala Pro Ile Glu Gln Glu Pro
465                 470                 475                 480

Ser Lys Lys Gln Lys Lys Gln His Glu Gly Ser Lys Lys Lys Ala Ala
                485                 490                 495

Ala Arg Asp Val Thr Leu Glu Asn Arg Leu Gln Asn Met Glu Val Thr
            500                 505                 510

Asp Ala

<210> 25
<211> 1846
<212> DNA
<213> Homo sapiens

<220>
<223> Seryl t-RNA synthetase

<400> 25
gcagtgcggc ggtcacaggc tgagtgctgc ggcgcgatcc ttgcttccct gagcgttggc 60
ccgggaggaa agaagatggt gctggatctg gatttgtttc gggtggataa aggaggggac 120
ccagccctca tccgagagac gcaggagaag cgcttcaagg acccgggact agtggaccag 180
ctggtgaagg cagacagcga gtggcgacga tgtagatttc gggcagacaa cttgagcaag 240
ctgaagaacc tatgcagcaa gacaatcgga gagaaaatga agaaaaaaga gccagtggga 300
gatgatgagt ctgtcccaga gaatgtgctg agtttcgatg accttactgc agacgcttta 360
gctaacctga aagtctcaca aatcaaaaaa gtccgactcc tcattgatga agccatcctg 420
aagtgtgacg cggagcggat aaagttggaa gcagagcggt ttgagaacct ccgagagatt 480
gggaaccttc tgcacccttc tgtacccatc agtaacgatg aggatgtgga caacaaagta 540
gagaggattt ggggcgattg tacagtcagg aagaagtact ctcatgtgga cctggtggtg 600
atggtagatg gctttgaagg cgaaaagggg gccgtggtgg ctgggagtcg agggtacttc 660
ttgaaggggg tcctggtgtt cctggaacag gctctcatcc agtatgccct tcgcaccttg 720
ggaagtcggg gctacattcc catttatacc ccctttttca tgaggaagga ggtcatgcag 780
gaggtggcac agctcagcca gtttgatgaa gaactttata aggtgattgg caaaggcagt 840
gaaaagtctg atgacaactc ctatgatgag aagtacctga ttgccacctc agagcagccc 900
attgctgccc tgcaccggga tgagtggctc cggccggagg acctgcccat caagtatgct 960
ggcctgtcta cctgcttccg tcaggaggtg ggctcccatg gccgtgacac ccgtggcatc 1020
ttccgagtcc atcagtttga gaagattgaa cagtttgtgt actcatcacc ccatgacaac 1080
aagtcatggg agatgtttga agagatgatt accaccgcag aggagttcta ccagtccctg 1140
gggattcctt accacattgt gaatattgtc tcaggttctt tgaatcatgc tgccagtaag 1200
aagcttgacc tggaggcctg gtttccgggc tcaggagcct tccgtgagtt ggtctcctgt 1260
tctaattgca cggattacca ggctcgccgg cttcgaatcc gatatggcca aaccaagaag 1320
atgatggaca aggtggagtt tgtccatatg ctcaatgcta ccatgtgcgc cactacccgt 1380
accatctgcg ccatcctgga gaactaccag acagagaagg gcatcactgt gcctgagaaa 1440
ttgaaggagt tcatgccgcc aggactgcaa gaactgatcc cctttgtgaa gcctgcgccc 1500
attgagcagg agccatcaaa gaagcagaag aagcaacatg agggcagcaa aaagaaagca 1560
gcagcaagag acgtcaccct agaaaacagg ctgcagaaca tggaggtcac cgatgcttga 1620
acattcctgc ctccctattt gccaggcttt catttctgtc tgctgagatc tcagagcctg 1680
cccaacagca gggaagccaa gcacccattc atccccctgc cccatctgac ctgcgtagct 1740
gagaggggaa cagtgccatg taccacacag atgttcctgt ctcctcgcat gggcataggg 1800
acccatcatt gatgactgat gaaaccatgt aataaagcat ctctgg 1846

<210> 26
<211> 434
<212> PRT
<213> Homo sapiens

<220>

<223> Alpha-enolase

<400> 26
Met Ser Ile Leu Lys Ile His Ala Arg Glu Ile Phe Asp Ser Arg Gly
1               5                   10                  15

Asn Pro Thr Val Glu Val Asp Leu Phe Thr Ser Lys Gly Leu Phe Arg
            20                  25                  30

Ala Ala Val Pro Ser Gly Ala Ser Thr Gly Ile Tyr Glu Ala Leu Glu
        35                  40                  45

Leu Arg Asp Asn Asp Lys Thr Arg Tyr Met Gly Lys Gly Val Ser Lys
    50                  55                  60

Ala Val Glu His Ile Asn Lys Thr Ile Ala Pro Ala Leu Val Ser Lys
65                  70                  75                  80

Lys Leu Asn Val Thr Glu Gln Glu Lys Ile Asp Lys Leu Met Ile Glu
                85                  90                  95

Met Asp Gly Thr Glu Asn Lys Ser Lys Phe Gly Ala Asn Ala Ile Leu
            100                 105                 110

Gly Val Ser Leu Ala Val Cys Lys Ala Gly Ala Val Glu Lys Gly Val
        115                 120                 125

Pro Leu Tyr Arg His Ile Ala Asp Leu Ala Gly Asn Ser Glu Val Ile
    130                 135                 140

Leu Pro Val Pro Ala Phe Asn Val Ile Asn Gly Gly Ser His Ala Gly
145                 150                 155                 160

Asn Lys Leu Ala Met Gln Glu Phe Met Ile Leu Pro Val Gly Ala Ala
                165                 170                 175

Asn Phe Arg Glu Ala Met Arg Ile Gly Ala Glu Val Tyr His Asn Leu
            180                 185                 190

Lys Asn Val Ile Lys Glu Lys Tyr Gly Lys Asp Ala Thr Asn Val Gly
        195                 200                 205

Asp Glu Gly Gly Phe Ala Pro Asn Ile Leu Glu Asn Lys Glu Gly Leu
    210                 215                 220

Glu Leu Leu Lys Thr Ala Ile Gly Lys Ala Gly Tyr Thr Asp Lys Val
225                 230                 235                 240

Val Ile Gly Met Asp Val Ala Ala Ser Glu Phe Phe Arg Ser Gly Lys
                245                 250                 255

Tyr Asp Leu Asp Phe Lys Ser Pro Asp Asp Pro Ser Arg Tyr Ile Ser
            260                 265                 270

Pro Asp Gln Leu Ala Asp Leu Tyr Lys Ser Phe Ile Lys Asp Tyr Pro
        275                 280                 285

Val Val Ser Ile Glu Asp Pro Phe Asp Gln Asp Asp Trp Gly Ala Trp
    290                 295                 300

Gln Lys Phe Thr Ala Ser Ala Gly Ile Gln Val Val Gly Asp Asp Leu

305                 310                 315                 320

Thr Val Thr Asn Pro Lys Arg Ile Ala Lys Ala Val Asn Glu Lys Ser
                325                 330                 335

Cys Asn Cys Leu Leu Leu Lys Val Asn Gln Ile Gly Ser Val Thr Glu
            340                 345                 350

Ser Leu Gln Ala Cys Lys Leu Ala Gln Ala Asn Gly Trp Gly Val Met
        355                 360                 365

Val Ser His Arg Ser Gly Glu Thr Glu Asp Thr Phe Ile Ala Asp Leu
    370                 375                 380

Val Val Gly Leu Cys Thr Gly Gln Ile Lys Thr Gly Ala Pro Cys Arg
385                 390                 395                 400

Ser Glu Arg Leu Ala Lys Tyr Asn Gln Leu Leu Arg Ile Glu Glu Glu
                405                 410                 415

Leu Gly Ser Lys Ala Lys Phe Ala Gly Arg Asn Phe Arg Asn Pro Leu
            420                 425                 430

Ala Lys



<210> 27
<211> 1755
<212> DNA
<213> Homo sapiens

<220>
<223> Alpha-enolase


<400> 27
acggagatct cgccggcttt acgttcacct cggtgtctgc agcaccctcc gcttcctctc 60
ctaggcgacg agacccagtg gctagaagtt caccatgtct attctcaaga tccatgccag 120
ggagatcttt gactctcgcg ggaatcccac tgttgaggtt gatctcttca cctcaaaagg 180
tctcttcaga gctgctgtgc ccagtggtgc ttcaactggt atctatgagg ccctagagct 240
ccgggacaat gataagactc gctatatggg gaagggtgtc tcaaaggctg ttgagcacat 300
caataaaact attgcgcctg ccctggttag caagaaactg aacgtcacag aacaagagaa 360
gattgacaaa ctgatgatcg agatggatgg aacagaaaat aaatctaagt ttggtgcgaa 420
cgccattctg ggggtgtccc ttgccgtctg caaagctggt gccgttgaga agggggtccc 480
cctgtaccgc cacatcgctg acttggctgg caactctgaa gtcatcctgc cagtcccggc 540
gttcaatgtc atcaatggcg gttctcatgc tggcaacaag ctggccatgc aggagttcat 600
gatcctccca gtcggtgcag caaacttcag ggaagccatg cgcattggag cagaggttta 660
ccacaacctg aagaatgtca tcaaggagaa atatgggaaa gatgccacca atgtggggga 720
tgaaggcggg tttgctccca acatcctgga gaataaagaa ggcctggagc tgctgaagac 780
tgctattggg aaagctggct acactgataa ggtggtcatc ggcatggacg tagcggcctc 840
cgagttcttc aggtctggga agtatgacct ggacttcaag tctcccgatg accccagcag 900
gtacatctcg cctgaccagc tggctgacct gtacaagtcc ttcatcaagg actacccagt 960
ggtgtctatc gaagatccct ttgaccagga tgactgggga gcttggcaga agttcacagc 1020
cagtgcagga atccaggtag tgggggatga tctcacagtg accaacccaa agaggatcgc 1080
caaggccgtg aacgagaagt cctgcaactg cctcctgctc aaagtcaacc agattggctc 1140
cgtgaccgag tctcttcagg cgtgcaagct ggcccaggcc aatggttggg gcgtcatggt 1200
gtctcatcgt tcgggggaga ctgaagatac cttcatcgct gacctggttg tggggctgtg 1260
cactgggcag atcaagactg gtgccccttg ccgatctgag cgcttggcca agtacaacca 1320
gctcctcaga attgaagagg agctgggcag caaggctaag tttgccggca ggaacttcag 1380
aaaccccttg gccaagtaag ctgtgggcag gcaagccttc ggtcacctgt tggctacaca 1440
gacccctccc ctcgtgtcag ctcaggcagc tcgaggcccc cgaccaacac ttgcaggggt 1500

ccctgctagt tagcgcccca ccgccgtgga gttcgtaccg cttccttaga acttctacag 1560
aagccaagct ccctggagcc ctgttggcag ctctagcttt tgcagtcgtg taatgggccc 1620
aagtcattgt ttttctcgcc tcactttcca ccaagtgtct agagtcatgt gagcctcgtg 1680
tcatctccgg ggtggccaca ggctagatcc ccggtggttt tgtgctcaaa ataaaaagcc 1740
tcagtgaccc atgag 1755

<210> 28
<211> 316
<212> PRT
<213> Rattus norvegicus

<220>
<223> Aldose reductase

<400> 28
Met Ala Ser His Leu Glu Leu Asn Asn Gly Thr Lys Met Pro Thr Leu
1               5                   10                  15

Gly Leu Gly Thr Trp Lys Ser Pro Pro Gly Gln Val Thr Glu Ala Val
            20                  25                  30

Lys Val Ala Ile Asp Met Gly Tyr Arg His Ile Asp Cys Ala Gln Val
        35                  40                  45

Tyr Gln Asn Glu Lys Glu Val Gly Val Ala Leu Gln Glu Lys Leu Lys
    50                  55                  60

Glu Gln Val Val Lys Arg Gln Asp Leu Phe Ile Val Ser Lys Leu Trp
65                  70                  75                  80

Cys Thr Phe His Asp Gln Ser Met Val Lys Gly Ala Cys Gln Lys Thr
                85                  90                  95

Leu Ser Asp Leu Gln Leu Asp Tyr Leu Asp Leu Tyr Leu Ile His Trp
            100                 105                 110

Pro Thr Gly Phe Lys Pro Gly Pro Asp Tyr Phe Pro Leu Asp Ala Ser
        115                 120                 125

Gly Asn Val Ile Pro Ser Asp Thr Asp Phe Val Asp Thr Trp Thr Ala
    130                 135                 140

Met Glu Gln Leu Val Asp Glu Gly Leu Val Lys Ala Ile Gly Val Ser
145                 150                 155                 160

Asn Phe Asn Pro Leu Gln Ile Glu Arg Ile Leu Asn Lys Pro Gly Leu
                165                 170                 175

Lys Tyr Lys Pro Ala Val Asn Gln Ile Glu Cys His Pro Tyr Leu Thr
            180                 185                 190

Gln Glu Lys Leu Ile Glu Tyr Cys His Cys Lys Gly Ile Val Val Thr
        195                 200                 205

Ala Tyr Ser Pro Leu Gly Ser Pro Asp Arg Pro Trp Ala Lys Pro Glu
    210                 215                 220

Asp Pro Ser Leu Leu Glu Asp Pro Arg Ile Lys Glu Ile Ala Ala Lys
225                 230                 235                 240

Tyr Asn Lys Thr Thr Ala Gln Val Leu Ile Arg Phe Pro Ile Gln Arg
                245                 250                 255

Asn Leu Val Val Ile Pro Lys Ser Val Thr Pro Ala Arg Ile Ala Glu
            260                 265                 270

Asn Phe Lys Val Phe Asp Phe Glu Leu Ser Asn Glu Asp Met Ala Thr
        275                 280                 285

Leu Leu Ser Tyr Asn Arg Asn Trp Arg Val Cys Ala Leu Met Ser Cys
    290                 295                 300

Ala Lys His Lys Asp Tyr Pro Phe His Ala Glu Val
305                 310                 315


<210> 29
<211> 1337
<212> DNA
<213> Rattus norvegicus

<220>
<223> Aldose reductase

<400> 29
ctcttgcggg tcgttgtgcg taacttgcag caatcatggc tagccatctg gaactcaaca 60
acggcaccaa gatgcccacc ctgggtctgg gcacctggaa gtctcctcct ggccaggtga 120
ccgaggctgt gaaggttgct atcgacatgg ggtatcgcca cattgactgc gcccaggtgt 180
accagaatga gaaggaggtg ggggtggccc tccaggagaa gctcaaggag caggtggtga 240
agcgccagga tctcttcatt gtcagcaagc tgtggtgcac gttccacgac cagagcatgg 300
tgaaaggggc ctgccagaag acgctgagcg acctgcagct ggactacctg gacctctacc 360
ttattcactg gccaactggc ttcaagcctg ggcctgacta tttccccctg gatgcatcgg 420
gaaacgtgat tcctagtgac accgattttg tggacacttg gacggctatg gagcaactag 480
tggatgaagg tttggtaaaa gcaatcggag tctccaactt caaccctctt cagattgaga 540
ggatcttgaa caaacctggc ttaaagtata agcctgctgt taaccagatc gagtgccacc 600
catacctaac tcaggagaag ctgattgagt actgccattg caaaggcatc gtggtgactg 660
catacagtcc ccttggttct cctgacaggc cctgggccaa gcctgaggac ccctctctcc 720
tggaggatcc caggatcaag gaaattgcag ccaagtacaa taaaactaca gcccaggtgc 780
tgatccggtt ccccatccaa aggaacctgg tcgtgatccc caagtctgtg acaccagcac 840
gtattgctga gaactttaag gtctttgact ttgagctgag caatgaggac atggccactc 900
tactcagcta caacaggaac tggagggtgt gcgccttgat gagctgtgcc aaacacaagg 960
attacccctt ccacgcagaa gtctgaagct gtggtggacg aatcctgctc ctccccaagc 1020
gacttaacac atgttctttc tgcctcatct gcccttgcaa gtgtccctct gcactgggtg 1080
gcaccttgca gaccagatgg tgagagtttg ttagtttgac gtagaatgtg gagggcagta 1140
ccagtagctg aggagtttct tcggcctttc ttggtcttct tcccacctgg aggactttaa 1200
cacgagtacc ttttccaacc aaagagaaag caagatttat agcccaagtc atgccactaa 1260
cacttaaatt tgagtgctta gaactccagt cctatggggg tcagactttt tgcctcaaat 1320
aaaaactgct tttgtcg 1337


<210> 30
<211> 299
<212> PRT
<213> Rattus norvegicus

<220>
<223> Cytochrome C oxidase, subunit 1

<400> 30
Glu Phe Pro Phe Phe Asp Pro Ala Gly Gly Gly Asp Pro Ile Leu Tyr
1               5                   10                  15

Gln His Leu Phe Trp Phe Phe Gly His Pro Glu Val Tyr Ile Leu Ile
            20                  25                  30

Leu Pro Gly Phe Gly Ile Ile Ser His Val Val Thr Tyr Tyr Ser Gly
        35                  40                  45

Lys Lys Glu Pro Phe Gly Tyr Met Gly Met Val Trp Ala Met Met Ser
    50                  55                  60

Ile Gly Phe Leu Gly Phe Ile Val Trp Ala His His Met Phe Thr Val
65                  70                  75                  80

Gly Leu Asp Val Asp Thr Arg Ala Tyr Phe Thr Ser Ala Thr Met Ile
                85                  90                  95

Ile Ala Ile Pro Thr Gly Val Lys Val Phe Ser Trp Leu Ala Thr Leu
            100                 105                 110

His Gly Gly Asn Ile Lys Trp Ser Pro Ala Met Leu Trp Ala Leu Gly
        115                 120                 125

Phe Ile Phe Leu Phe Thr Val Gly Gly Leu Thr Gly Ile Val Leu Ser
    130                 135                 140

Asn Ser Ser Leu Asp Ile Val Leu His Asp Thr Tyr Tyr Val Val Ala
145                 150                 155                 160

His Phe His Tyr Val Leu Ser Met Gly Ala Val Phe Ala Ile Met Ala
                165                 170                 175

Cys Phe Val His Trp Phe Pro Leu Phe Ser Gly Tyr Thr Leu Asn Asp
            180                 185                 190

Thr Trp Ala Lys Ala His Phe Ala Ile Met Phe Val Gly Val Asn Met
        195                 200                 205

Thr Phe Phe Pro Gln His Phe Leu Gly Leu Ala Gly Met Pro Arg Arg
    210                 215                 220

Tyr Ser Asp Tyr Pro Asp Ala Tyr Thr Thr Trp Asn Thr Val Ser Ser
225                 230                 235                 240

Met Gly Ser Phe Ile Ser Leu Thr Ala Val Leu Val Met Ile Phe Met
                245                 250                 255

Ile Trp Glu Ala Phe Ala Ser Lys Arg Glu Val Leu Ser Ile Ser Tyr
            260                 265                 270

Ser Ser Thr Asn Leu Glu Trp Leu His Gly Cys Pro Pro Pro Tyr His
        275                 280                 285

Thr Phe Glu Glu Pro Ser Tyr Val Lys Val Lys
    290                 295

<210> 31
<211> 987
<212> DNA
<213> Rattus norvegicus

<220>
<223> Cytochrome C oxidase, subunit 1

<400> 31
gaattccctt tcttcgaccc cgctggaggt ggagacccaa ttctttatca acacctattc 60
tgattcttcg gccacccaga agtgtacatc ttaattcttc cagggtttgg aattatttca 120
catgtagtta cctattactc tggaaaaaaa gaacccttcg gatatatagg tatggtatga 180
gccataatat ctattggctt cctaggattt attgtatgag cacatcacat attcacagta 240
ggcctagatg tagacacccg agcctacttt acatctgcca ctataattat cgcaattcct 300
acaggcgtaa aagtattcag ctgactcgct acactacatg gaggaaatat caaatgatcc 360
cccgccatat tatgagcctt agggtttatc ttcttattca cagtaggggg cctaacaggg 420
atcgtactat ctaactcatc ccttgacatt gtacttcatg atacatacta cgtagtagct 480
cacttccact atgtcttatc tataggagca gtattcgcca tcatagcttg cttcgtccac 540
tgattcccac tattctcagg ctatacccta aatgacacat gagcaaaagc ccactttgcc 600
attatatttg taggtgtaaa cataacattt tttcctcaac acttcctagg attagcaggg 660
atacctcgtc gttactctga ttatccagat gcttatacca catgaaatac agtctcctct 720
ataggctcat tcatctcact tacggccgtc cttgtaatga tcttcatgat ttgagaagcc 780
ttcgcatcaa aacgagaagt gctctcaatt tcctactctt caactaacct agaatgactg 840
catggatgcc ccccacctta ccacacattc gaagaacctt cctatgtaaa agttaaataa 900
gaaaggaagg attcgaaccc cctacaactg gtttcaagcc aatttcataa ccattatgtc 960
tttctcaata aaaaaaaaaa ggaattc 987

<210> 32
<211> 334
<212> PRT
<213> Rattus norvegicus

<220>
<223> LDH-B

<400> 32
Met Ala Thr Leu Lys Glu Lys Leu Ile Ala Pro Val Ala Asp Asp Glu
1               5                   10                  15

Thr Ala Val Pro Asn Asn Lys Ile Thr Val Val Gly Val Gly Gln Val
            20                  25                  30

Gly Met Ala Cys Ala Ile Ser Ile Leu Gly Lys Ser Leu Ala Asp Glu
        35                  40                  45

Leu Ala Leu Val Asp Val Leu Glu Asp Lys Leu Lys Gly Glu Met Met
    50                  55                  60

Asp Leu Gln His Gly Ser Leu Phe Leu Gln Thr Pro Lys Ile Val Ala
65                  70                  75                  80

Asp Lys Asp Tyr Ser Val Thr Ala Asn Ser Lys Ile Val Val Val Thr
                85                  90                  95

Ala Gly Val Arg Gln Gln Glu Gly Glu Ser Arg Leu Asn Leu Val Gln
            100                 105                 110

Arg Asn Val Asn Val Phe Lys Phe Ile Ile Pro Gln Ile Val Lys Tyr
        115                 120                 125

Ser Pro Asp Cys Thr Ile Ile Val Val Ser Asn Pro Val Asp Ile Leu
    130                 135                 140

Thr Tyr Val Thr Trp Lys Leu Ser Gly Leu Pro Lys His Arg Val Ile
145                 150                 155                 160

Gly Ser Gly Cys Asn Leu Asp Ser Ala Arg Phe Arg Tyr Leu Met Ala

                165                 170                 175

Glu Lys Leu Gly Ile His Pro Ser Ser Cys His Gly Trp Ile Leu Gly
            180                 185                 190

Glu His Gly Asp Ser Ser Val Ala Val Trp Ser Gly Val Asn Val Ala
        195                 200                 205

Gly Val Ser Leu Gln Glu Leu Asn Pro Glu Met Gly Thr Asp Asn Asp
    210                 215                 220

Ser Glu Asn Trp Lys Glu Val His Lys Met Val Val Asp Ser Ala Tyr
225                 230                 235                 240

Glu Val Ile Lys Leu Lys Gly Tyr Thr Asn Trp Ala Ile Gly Leu Ser
                245                 250                 255

Val Ala Asp Leu Ile Glu Ser Met Leu Lys Asn Leu Ser Arg Ile His
            260                 265                 270

Pro Val Ser Thr Met Val Lys Gly Met Tyr Gly Ile Glu Asn Glu Val
        275                 280                 285

Phe Leu Ser Leu Pro Cys Ile Leu Asn Ala Arg Gly Leu Thr Ser Val
    290                 295                 300

Ile Asn Gln Lys Leu Lys Asp Asp Glu Val Ala Gln Leu Arg Lys Ser
305                 310                 315                 320

Ala Asp Thr Leu Trp Asp Ile Gln Lys Asp Leu Lys Asp Leu
                325                 330

<210> 33
<211> 1217
<212> DNA
<213> Rattus norvegicus

<220>
<223> LDH-B

<400> 33
cagagcctcc tcttgtctgg acaagatggc aacccttaag gaaaagctca ttgcgccagt 60
cgcagacgac gagactgccg tcccgaacaa caagattact gtagtaggcg ttggacaagt 120
tggaatggct tgtgctatca gcattctggg gaagtctctg gctgatgagc ttgccctggt 180
ggatgtcttg gaagacaagc tcaaaggaga aatgatggat ctgcagcacg ggagcttatt 240
tctccagact ccgaaaatcg tggctgataa agattactcc gtgacagcca attctaagat 300
tgtggtggtg accgcgggag tccgccagca ggagggggag agtcggctca acctggtgca 360
gagaaacgtc aatgtattca agttcattat tcctcagatc gtcaagtaca gccccgactg 420
caccatcatc gtggtttcca acccagtgga tattcttacc tatgtcacct ggaagctgag 480
cgggctacct aagcaccgcg tgattggaag tggatgcaat ctggattctg ctcggtttcg 540
ttacctcatg gccgaaaagc ttggtattca tcccagcagc tgccacggct ggatcctggg 600
cgagcacggg gactccagtg tggcagtgtg gagcggggtg aatgtggcag gagtctccct 660
ccaggaactg aacccagaga tgggaacgga caatgacagc gagaactgga aggaggtgca 720
taagatggtg gtggacagtg cctatgaagt catcaagcta aaaggctaca ccaactgggc 780
catcggccta agtgtggctg acctcatcga atccatgctg aaaaacctct ctcggattca 840
ccccgtgtct acaatggtga agggaatgta cggcatcgag aacgaagtct tcctcagtct 900
cccgtgcatc cttaatgctc ggggactgac cagcgtcatc aaccagaagc tgaaggacga 960
tgaggtcgct cagctcagga agagtgcgga caccctgtgg gatatccaga aagacctcaa 1020
ggacctgtga ctgccaggcg ccaggctgta gaaatccaaa cctccaatgt gactaagtga 1080
acctttagtc ttcggccttg tacgtaggtc acagtttgct tcttccctaa catgtgataa 1140
tgagctcaca gatcaaaacc aggagtgttt gatgtttgca ctaggagctc ctgaacaaat 1200

aaagtttagc aattgca                                                    1217

<210> 34
<211> 86
<212> PRT
<213> Homo sapiens

<220>
<223> Cytochrome C oxidase subunit VIb

<400> 34
Met Ala Glu Asp Met Glu Thr Lys Ile Lys Asn Tyr Lys Thr Ala Pro
1               5                   10                  15

Phe Asp Ser Arg Phe Pro Asn Gln Asn Gln Thr Arg Asn Cys Trp Gln
            20                  25                  30

Asn Tyr Leu Asp Phe His Arg Cys Gln Lys Ala Met Thr Ala Lys Gly
        35                  40                  45

Gly Asp Ile Ser Val Cys Glu Trp Tyr Gln Arg Val Tyr Gln Ser Leu
    50                  55                  60

Cys Pro Thr Ser Trp Val Thr Asp Trp Asp Glu Gln Arg Ala Glu Gly
65                  70                  75                  80

Thr Phe Pro Gly Lys Ile
                85


<210> 35
<211> 439
<212> DNA
<213> Homo sapiens

<220>
<223> Cytochrome C oxidase subunit VIb


<400> 35
ttgagctgca ggttgaatcc ggggtgcctt taggattcag caccatggcg gaagacatgg 60
agaccaaaat caagaactac aagaccgccc cttttgacag ccgcttcccc aaccagaacc 120
agactagaaa ctgctggcag aactacctgg acttccaccg ctgtcagaag gcaatgaccg 180
ctaaaggagg cgatatctct gtgtgcgaat ggtaccagcg tgtgtaccag tccctctgcc 240
ccacatcctg ggtcacagac tgggatgagc aacgggctga aggcacgttt cccgggaaga 300
tctgaactgg ctgcatctcc ctttcctctg tcctccatcc ttctcccagg atggtgaagg 360
gggacctggt acccagtgat ccccacccca ggatcctaaa tcatgactta cctgctaata 420
aaaactcatt ggaaaagtg                                                439


<210> 36
<211> 172
<212> PRT
<213> Homo sapiens

<220>
<223> NADH: ubiquinone oxidoreductase PGIV subunit

<400> 36
Met Pro Gly Ile Val Glu Leu Pro Thr Leu Glu Glu Leu Lys Val Asp
1               5                   10                  15

Glu Val Lys Ile Ser Ser Ala Val Leu Lys Ala Ala Ala His His Tyr
            20                  25                  30

Gly Ala Gln Cys Asp Lys Pro Asn Lys Glu Phe Met Leu Cys Arg Trp
        35                  40                  45

Glu Glu Lys Asp Pro Arg Arg Cys Leu Glu Glu Gly Lys Leu Val Asn
    50                  55                  60

Lys Cys Ala Leu Asp Phe Phe Arg Gln Ile Lys Arg His Cys Ala Glu
65                  70                  75                  80

Pro Phe Thr Glu Tyr Trp Thr Cys Ile Asp Tyr Thr Gly Gln Gln Leu
                85                  90                  95

Phe Arg His Cys Arg Lys Gln Gln Ala Lys Phe Asp Glu Cys Val Leu
            100                 105                 110

Asp Lys Leu Gly Trp Val Arg Pro Asp Leu Gly Glu Leu Ser Lys Val
        115                 120                 125

Thr Lys Val Lys Thr Asp Arg Pro Leu Pro Glu Asn Pro Tyr His Ser
    130                 135                 140

Arg Pro Arg Pro Asp Pro Ser Pro Glu Ile Glu Gly Asp Leu Gln Pro
145                 150                 155                 160

Ala Thr His Gly Ser Arg Phe Tyr Phe Trp Thr Lys
                165                 170


<210> 37
<211> 700
<212> DNA
<213> Homo sapiens

<220>
<223> NADH: ubiquinone oxidoreductase PGIV subunit

<400> 37
ggggagttca aggagacggg ggcgacgcgg ctgagggctt ctcgtcgggg tcggggctgc 60
agccgtcatg ccggggatag tggagctgcc cactctagag gagctgaaag tagatgaggt 120
gaaaattagt tctgctgtgc ttaaagctgc ggcccatcac tatggagctc aatgtgataa 180
gcccaacaag gaatttatgc tctgccgctg ggaagagaaa gatccgaggc ggtgtttaga 240
ggaaggcaaa ctggtcaaca agtgtgcttt ggacttcttt aggcagataa aacgtcactg 300
tgcagagcct tttacagaat attggacttg cattgattat actggccagc agttatttcg 360
tcactgtcgc aaacagcagg caaagtttga cgagtgtgtg ctggacaaac tgggctgggt 420
gcggcctgac ctgggagaac tgtcaaaggt caccaaagtg aaaacagatc gacctttacc 480
ggagaatccc tatcactcaa gaccaagacc ggatcccagc cctgagatcg agggagatct 540
gcagcctgcc acacatggca gccgctttta tttctggacc aagtaaagat gggtccgtgg 600
cccacactcg gtcatgtgct cagacaacga ctgatgaaaa cgcccatgcg gtttgcatcg 660
actgatagtg tgttctttcc gggatcacaa acattaacaa                       700


<210> 38
<211> 532
<212> PRT
<213> Mus musculus

<220>
<223> succinate dehydrogenase Fp subunit

<400> 38
Leu Arg Ala Ala Phe Gly Leu Ser Glu Ala Gly Phe Asn Thr Ala Cys
1               5                   10                  15

Leu Thr Lys Leu Phe Pro Thr Arg Ser His Thr Val Ala Ala Gln Gly
            20                  25                  30

Gly Ile Asn Ala Ala Leu Gly Asn Met Glu Glu Asp Asn Trp Arg Trp
        35                  40                  45

His Phe Tyr Asp Thr Val Lys Gly Ser Asp Trp Leu Gly Asp Gln Asp
    50                  55                  60

Ala Ile His Tyr Met Thr Glu Gln Ala Pro Ala Ser Val Val Glu Leu
65                  70                  75                  80

Glu Asn Tyr Gly Met Pro Phe Ser Arg Thr Glu Asp Gly Lys Ile Tyr
                85                  90                  95

Gln Arg Ala Phe Gly Gly Gln Ser Leu Lys Phe Gly Lys Gly Gly Gln
            100                 105                 110

Ala His Arg Cys Cys Cys Val Ala Asp Arg Thr Gly His Ser Leu Leu
        115                 120                 125

His Thr Leu Tyr Gly Arg Ser Leu Arg Tyr Asp Thr Ser Tyr Phe Val
    130                 135                 140

Glu Tyr Phe Ala Leu Asp Leu Leu Met Glu Asn Gly Glu Cys Arg Gly
145                 150                 155                 160

Val Ile Ala Leu Cys Ile Glu Asp Gly Ser Ile His Arg Ile Arg Ala
                165                 170                 175

Lys Asn Thr Val Ile Ala Thr Gly Gly Tyr Gly Arg Thr Tyr Phe Ser
            180                 185                 190

Cys Thr Ser Ala His Thr Ser Thr Gly Asp Gly Thr Ala Met Val Thr
        195                 200                 205

Arg Ala Gly Leu Pro Cys Gln Asp Leu Glu Phe Val Gln Phe His Pro
    210                 215                 220

Thr Gly Ile Tyr Gly Ala Gly Cys Leu Ile Thr Glu Gly Cys Arg Gly
225                 230                 235                 240

Glu Gly Gly Ile Leu Ile Asn Ser Gln Gly Glu Arg Phe Met Glu Arg
                245                 250                 255

Tyr Ala Pro Val Ala Lys Asp Leu Ala Ser Arg Asp Val Val Ser Arg
            260                 265                 270

Ser Met Thr Leu Glu Ile Arg Glu Gly Arg Gly Cys Gly Pro Glu Lys
        275                 280                 285

Asp His Val Tyr Leu Gln Leu His His Leu Pro Pro Glu Gln Leu Ala
    290                 295                 300

Thr Arg Leu Pro Gly Ile Ser Glu Thr Ala Met Ile Phe Ala Gly Val
305                 310                 315                 320

Asp Val Thr Lys Glu Pro Ile Pro Val Leu Pro Thr Val His Tyr Asn
                325                 330                 335

Met Gly Gly Ile Pro Thr Asn Tyr Lys Gly Gln Val Leu Lys His Val
            340                 345                 350

Asn Gly Gln Asp Gln Ile Val Pro Gly Leu Tyr Ala Cys Gly Glu Ala
        355                 360                 365

Ala Cys Ala Ser Val His Gly Ala Asn Arg Leu Gly Ala Asn Ser Leu
    370                 375                 380

Leu Asp Leu Val Val Phe Gly Arg Ala Cys Ala Leu Ser Ile Ala Glu
385                 390                 395                 400

Ser Cys Arg Pro Gly Asp Lys Val Pro Ser Ile Lys Ala Asn Ala Gly
                405                 410                 415

Glu Glu Ser Val Met Asn Leu Asp Lys Leu Arg Phe Ala Asp Gly Ser
            420                 425                 430

Ile Arg Thr Ser Glu Leu Arg Leu Asn Met Gln Lys Ser Met Gln Asn
        435                 440                 445

His Ala Ala Val Phe Arg Val Gly Ser Val Leu Gln Glu Gly Cys Glu
    450                 455                 460

Lys Ile Ser Gln Leu Tyr Gly Asp Leu Lys His Leu Lys Thr Phe Asp
465                 470                 475                 480

Arg Gly Met Val Trp Asn Thr Asp Leu Val Glu Thr Leu Glu Leu Gln
                485                 490                 495

Asn Leu Met Leu Cys Ala Leu Gln Thr Ile Tyr Gly Ala Glu Ala Arg
            500                 505                 510

Lys Glu Ser Arg Gly Ala His Ala Arg Glu Asp Tyr Lys Val Arg Val
        515                 520                 525

Asp Glu Tyr Asp
    530


<210> 39
<211> 1596
<212> DNA
<213> Mus musculus

<220>
<223> succinate dehydrogenase Fp subunit

<400> 39
ttgcgagctg catttggcct ttctgaggca gggtttaata ctgcatgcct tacaaagctc 60
tttcctaccc gatcacatac tgttgcagca cagggaggta tcaatgctgc tctggggaac 120
atggaagagg acaactggag atggcatttc tatgacactg tgaaaggctc cgactggctg 180
ggggatcagg atgccatcca ttacatgaca gagcaagctc ctgcctctgt ggttgagcta 240
gaaaattatg gtatgccatt tagcagaact gaagatggga agatttatca gcgtgcattt 300
ggtggacaga gcctcaagtt tgggaaaggc gggcaggctc atcggtgttg ctgtgtggct 360
gatcggacag gccactcact cttacacacc ttgtatggaa gatctctgcg atatgacacc 420
agttattttg tggaatattt tgcactggat cttctgatgg aaaatgggga gtgccgtggt 480
gtcattgcac tgtgcataga agatgggtcc atacaccgaa taagagcaaa aaacactgtt 540
attgctactg gggctacggg gcgaacctac ttcagctgta catctgccca taccagcaca 600

ggggacggca cagccatggt cactagggct ggtttgcctt gccaggactt agaatttgtt 660
cagttccacc ccacaggtat atacggtgct ggctgcctca tcacagaagg gtgtcgtgga 720
gaggggggga ttctcatcaa cagtcaaggt gaaaggttca tggagagata cgcccctgtt 780
gccaaggacc tggcatcaag agatgttgtg tctcgatcca tgactcttga gatccgtgaa 840
ggaagaggct gtggccctga gaaagatcac gtctacctgc agttgcatca tctgcctcct 900
gagcaactgg ccacacgtct gcccggaatt tcagagacag ccatgatctt cgctggtgtg 960
gatgtcacta aggagcccat tccagtcctc cccactgtgc attacaacat gggtgggatt 1020
cccactaact acaagggaca ggtgctgaag catgtgaatg gccaggatca gattgtgcct 1080
ggactgtatg cctgtgggga ggctgcctgc gcctcagtgc atggtgccaa ccggcttgga 1140
gcaaattctc tcttggacct tgtagtcttt ggcagagcct gtgccctgag cattgcagaa 1200
tcttgcaggc ctggagataa agttccttca attaaggcaa atgctggaga agaatcggtt 1260
atgaatcttg acaagttgag atttgccgat ggaagtataa gaacatcaga actacgccta 1320
aacatgcaga agtcgatgca gaaccatgct gcagtgttcc gtgtggggag tgtattgcaa 1380
gaaggctgtg aaaaaatcag tcagctctat ggagacctaa agcatctaaa gacattcgac 1440
aggggaatgg tttggaacac agacctggtg gagaccctgg agctgcagaa tctgatgctg 1500
tgcgcactgc agaccatata tggtgcagaa gctcggaagg agtcccgggg agcccatgcc 1560
agggaagatt acaaagtgcg ggtcgatgag tatgat 1596

<210> 40
<211> 453
<212> PRT
<213> Homo sapiens

<220>
<223> Core protein II of human mitochondrial cytochrome bc-1 complex

<400> 40
Met Lys Leu Leu Thr Arg Ala Gly Ser Phe Ser Arg Phe Tyr Ser Leu
1               5                   10                  15

Lys Val Ala Pro Lys Val Lys Ala Thr Ala Ala Pro Ala Gly Ala Pro
            20                  25                  30

Pro Gln Pro Gln Asp Leu Glu Phe Thr Lys Leu Pro Asn Gly Leu Val
        35                  40                  45

Ile Ala Ser Leu Glu Asn Tyr Ser Pro Val Ser Arg Ile Gly Leu Phe
    50                  55                  60

Ile Lys Ala Gly Ser Arg Tyr Glu Asp Phe Ser Asn Leu Gly Thr Thr
65                  70                  75                  80

His Leu Leu Arg Leu Thr Ser Ser Leu Thr Thr Lys Gly Ala Ser Ser
                85                  90                  95

Phe Lys Ile Thr Arg Gly Ile Glu Ala Val Gly Gly Lys Leu Ser Val
            100                 105                 110

Thr Ala Thr Arg Glu Asn Met Ala Tyr Thr Val Glu Cys Leu Arg Gly
        115                 120                 125

Asp Val Asp Ile Leu Met Glu Phe Leu Leu Asn Val Thr Thr Ala Pro
    130                 135                 140

Glu Phe Arg Arg Trp Glu Val Ala Asp Leu Gln Pro Gln Leu Lys Ile
145                 150                 155                 160

Asp Lys Ala Val Ala Phe Gln Asn Pro Gln Thr His Val Ile Glu Asn
                165                 170                 175

Leu His Ala Ala Ala Tyr Gln Asn Ala Leu Ala Asn Pro Leu Tyr Cys
            180                 185                 190

Pro Asp Tyr Arg Ile Gly Lys Val Thr Ser Glu Glu Leu His Tyr Phe
        195                 200                 205

Val Gln Asn His Phe Thr Ser Ala Arg Met Ala Leu Ile Gly Leu Gly
    210                 215                 220

Val Ser His Pro Val Leu Lys Gln Val Ala Glu Gln Phe Leu Asn Met
225                 230                 235                 240

Arg Gly Gly Leu Gly Leu Ser Gly Ala Lys Ala Asn Tyr Arg Gly Gly
                245                 250                 255

Glu Ile Arg Glu Gln Asn Gly Asp Ser Leu Val His Ala Ala Phe Val
            260                 265                 270

Ala Glu Ser Ala Val Ala Gly Ser Ala Glu Ala Asn Ala Phe Ser Val
        275                 280                 285

Leu Gln His Val Leu Gly Ala Gly Pro His Val Lys Arg Gly Ser Asn
    290                 295                 300

Thr Thr Ser His Leu His Gln Ala Val Ala Lys Ala Thr Gln Gln Pro
305                 310                 315                 320

Phe Asp Val Ser Ala Phe Asn Ala Ser Tyr Ser Asp Ser Gly Leu Phe
                325                 330                 335

Gly Ile Tyr Thr Ile Ser Gln Ala Thr Ala Ala Gly Asp Val Ile Lys
            340                 345                 350

Ala Ala Tyr Asn Gln Val Lys Arg Ile Ala Gln Gly Asn Leu Ser Asn
        355                 360                 365

Thr Asp Val Gln Ala Ala Lys Asn Lys Leu Lys Ala Gly Tyr Leu Met
    370                 375                 380

Ser Val Glu Ser Ser Glu Cys Phe Leu Glu Glu Val Gly Ser Gln Ala
385                 390                 395                 400

Leu Val Ala Gly Ser Tyr Met Pro Pro Ser Thr Val Leu Gln Gln Ile
                405                 410                 415

Asp Ser Val Ala Asn Ala Asp Ile Ile Asn Ala Ala Lys Lys Phe Val
            420                 425                 430

Ser Gly Gln Lys Ser Met Ala Ala Ser Gly Asn Leu Gly His Thr Pro
        435                 440                 445

Phe Val Asp Glu Leu
    450


<210> 41
<211> 1588
<212> DNA
<213> Homo sapiens

<220>
<223> Core protein II of human mitochondrial cytochrome bc-1 complex

<400> 41
atcttgcttt cctttaatcc ggcagtgacc gtgtgtcaga acaatcttga atcatgaagc 60
tactaaccag agccggctct ttctcgagat tttattccct caaagttgcc cccaaagtta 120
aagccacagc tgcgcctgca ggagcaccgc cacaacctca ggaccttgag tttaccaagt 180
taccaaatgg cttggtgatt gcttctttgg aaaactattc tcctgtatca agaattggtt 240
tgttcattaa agcaggcagt agatatgagg acttcagcaa tttaggaacc acccatttgc 300
tgcgtcttac atccagtctg acgacaaaag gagcttcatc tttcaagata acccgtggaa 360
ttgaagcagt tggtggcaaa ttaagtgtga ccgcaacaag ggaaaacatg gcttatactg 420
tggaatgcct gcggggtgat gttgatattc taatggagtt cctgctcaat gtcaccacag 480
caccagaatt tcgtcgttgg gaagtagctg accttcagcc tcagctaaag attgacaaag 540
ctgtggcctt tcagaatccg cagactcatg tcattgaaaa tttgcatgca gcagcttacc 600
agaatgcctt ggctaatccc ttgtattgtc ctgactatag gattggaaaa gtgacatcag 660
aggagttaca ttacttcgtt cagaaccatt tcacaagtgc aagaatggct ttgattggac 720
ttggtgtgag tcatcctgtt ctaaagcaag ttgctgaaca gtttctcaac atgaggggtg 780
ggcttggttt atctggtgca aaggccaact accgtggagg tgaaatccga gaacagaatg 840
gagacagtct tgtccatgct gcttttgtag cagaaagtgc tgtcgcggga agtgcagagg 900
caaatgcatt tagtgttctt cagcatgtcc tcggtgctgg gccacatgtc aagaggggca 960
gcaacaccac cagccatctg caccaggctg ttgccaaggc aactcagcag ccatttgatg 1020
tttctgcatt taatgccagt tactcagatt ctggactctt tgggatttat actatctccc 1080
aggccacagc tgctggagat gttatcaagg ctgcctataa tcaagtaaaa agaatagctc 1140
aaggaaacct ttccaacaca gatgtccaag ctgccaagaa caagctgaaa gctggatacc 1200
taatgtcagt ggagtcttct gagtgtttcc tggaagaagt cgggtcccag gctctagttg 1260
ctggttctta catgccacca tccacagtcc ttcagcagat tgattcagtg gctaatgctg 1320
atatcataaa tgcggcaaag aagtttgttt ctggccagaa gtcaatggca gcaagtggaa 1380
atttgggaca tacacctttt gttgatgagt tgtaatactg atgcacacat tacaggagag 1440
agctgaacgt tctctcaccc agagcagcaa acacatgaaa gtcagaagtc tctaatatat 1500
catttgtctt ttttccagtg aggtaaaata aggcataaat gcaggtaatt attcccagct 1560
gacctaaagt caataaaaca ttctgttt 1588

<210> 42
<211> 358
<212> PRT
<213> Rattus norvegicus

<220>
<223> Stearoyl-CoA desaturase-2

<400> 42
Met Pro Ala His Ile Leu Gln Glu Ile Ser Gly Ser Tyr Ser Ala Thr
1               5                   10                  15

Thr Thr Ile Thr Ala Pro Pro Ser Gly Gly Gln Gln Asn Gly Gly Glu
            20                  25                  30

Lys Phe Glu Lys Asn Pro His His Trp Gly Ala Asp Val Arg Pro Glu
        35                  40                  45

Ile Lys Asp Asp Leu Tyr Asp Pro Ser Tyr Gln Asp Glu Glu Gly Pro
    50                  55                  60

Pro Pro Lys Leu Glu Tyr Val Trp Arg Asn Ile Ile Leu Met Ala Leu
65                  70                  75                  80

Leu His Ile Gly Ala Leu Tyr Gly Ile Thr Leu Val Pro Ser Cys Lys
                85                  90                  95

Val Tyr Thr Cys Leu Phe Ala Tyr Leu Tyr Tyr Val Ile Ser Ala Leu
            100                 105                 110

Gly Ile Thr Ala Gly Ala His Arg Leu Trp Ser His Arg Thr Tyr Lys

115                 120                 125

Ala Arg Leu Pro Leu Arg Leu Phe Leu Ile Ile Ala Asn Thr Met Ala
    130                 135                 140

Phe Gln Asn Asp Val Tyr Glu Trp Ala Arg Asp His Arg Ala His His
145                 150                 155                 160

Lys Phe Ser Glu Thr His Ala Asp Pro His Asn Ser Arg Arg Gly Phe
                165                 170                 175

Phe Phe Ser His Val Gly Trp Leu Leu Val Arg Lys His Pro Ala Val
            180                 185                 190

Lys Glu Lys Gly Gly Lys Leu Asp Met Ser Asp Leu Lys Ala Glu Lys
        195                 200                 205

Leu Val Met Phe Gln Arg Arg Tyr Tyr Lys Pro Gly Leu Leu Leu Met
    210                 215                 220

Cys Phe Ile Leu Pro Thr Leu Val Pro Trp Tyr Cys Trp Gly Glu Thr
225                 230                 235                 240

Phe Val Asn Ser Leu Cys Val Ser Thr Phe Leu Arg Tyr Ala Val Val
                245                 250                 255

Leu Asn Ala Thr Trp Leu Val Asn Ser Ala Ala His Leu Tyr Gly Tyr
            260                 265                 270

Arg Pro Tyr Asp Lys Asn Ile Ser Ser Arg Glu Asn Ile Leu Val Ser
        275                 280                 285

Met Gly Ala Val Gly Glu Gly Phe His Asn Tyr His His Ala Phe Pro
    290                 295                 300

Tyr Asp Tyr Ser Ala Ser Glu Tyr Arg Trp His Ile Asn Phe Thr Thr
305                 310                 315                 320

Phe Phe Ile Asp Cys Met Ala Leu Leu Gly Leu Ala Tyr Asp Arg Lys
                325                 330                 335

Arg Val Ser Lys Ala Ala Val Leu Ala Arg Ile Lys Arg Thr Gly Glu
            340                 345                 350

Glu Ser Cys Lys Ser Gly
        355

<210> 43
<211> 5055
<212> DNA
<213> Rattus norvegicus

<220>
<223> Stearoyl-CoA desaturase-2

<400> 43
ctgggctagg gatgggcaca atgcggtgtt ggtgtctgca gtattctact cctggacacc 60
ggtggcctgc aagtgcgctt ttggcgtcct ctcatttttc tatccttatc tccgtccgcg 120
gctgccttgg ccagccagtt tttgattttt tatctgttcc ttgatcgata caaagaacct 180
aaggacatac aggacattaa tacccaactg ccagctctgg cccagggctt gtactgtgca 240
gcgggctggc tgcagaaact taagtcataa cactctttgt cgctgaggtc tgaagctctc 300

tgcacgttct catccctggg aacgtgaccc cagcatccga cgccaagatg ccggctcaca 360
tactgcaaga gatctctggc tcttactcgg ccaccaccac aatcacagcg ccaccttctg 420
ggggacagca gaatggagga gagaagtttg aaaagaatcc tcaccactgg ggagcagatg 480
ttcgccctga aattaaagat gacctatacg accccagcta ccaggatgag gaggggcccc 540
cgcccaagct ggagtacgtc tggaggaaca tcatcctcat ggccctgctg cacattggag 600
ccctgtacgg gatcacactg gtcccctcct gcaaggtcta cacctgcctc tttgcatatt 660
tgtactacgt aatcagcgcc ctgggcatca cagccggggc tcatcgcctg tggagccaca 720
ggacttacaa ggctcggctg cccctgaggc tctttctcat catcgccaac accatggcat 780
tccagaacga tgtgtacgaa tgggcccggg atcaccgcgc ccaccacaag ttctcagaga 840
cacacgccga ccctcacaac tcccgccgtg gctttttctt ctctcacgtg ggttggctgc 900
ttgtgcgcaa acacccggct gtcaaagaga agggcggaaa gctggacatg tctgacctga 960
aagctgagaa gctggtgatg ttccagagga ggtattacaa gcccggcctc ctgctcatgt 1020
gcttcatcct gcccacgctg gtgccctggt actgctgggg tgagacgttt gtaaacagcc 1080
tgtgcgtcag cactttctta cggtacgctg tggtgctcaa cgccacctgg ctggtgaaca 1140
gcgccgccca cctctatgga tatcgcccct acgacaagaa catcagctct cgggagaaca 1200
tcctggtgtc gatgggagct gtgggcgagg gcttccacaa ctaccatcac gccttcccct 1260
atgactactc tgccagtgag taccgctggc acatcaactt caccacgttc ttcatcgact 1320
gcatggccct cctgggcctg gcttatgacc ggaagagggt gtccaaggcc gctgtcttag 1380
ccaggattaa gagaactggg gaggagagct gcaagagtgg ctgagttcgg ggtcttccag 1440
ttcctgttcc aagagccggc ttggcagagg cttactgttc tgttaattaa ctactgagta 1500
ttgctaccca gatgctaaaa tgatgaagtt aacccattct ggtacagtat tgtaaaacgg 1560
acaaatatcg gaagtcaaca gctctacctt ttgatgctaa gctgttctcc ccttctatct 1620
tcttttcccg ttgtcttttt cttcgctttg tccccaatga cctcacccct catcgcctcc 1680
cctaagccag cagccactcc gctgtagttc agagttcacc ttccagagcg taccagcttt 1740
tcaagggtct acagtaaagg ttcacgcccc agccccaact taacgttgct ccaagttaca 1800
gatagaagaa aaatcttgag aaatttctcc tgttatttta gtccagactt tgccagatgg 1860
aatgggagaa aaaatagttt tatttggcag agagcttgcg aagtgggtaa atcttaaggg 1920
gccaaattca acactcgggc tgtgattcat caaaattgtg ggagaggggg atctcctgtg 1980
atcaggctcc gctccgtctg gccgaggtgc tggcgtctct tgacacagcg tgcctcctct 2040
cctaattctg attgggtgac agccgtggaa ctttgtgaca ctaaaatatg gcaacacatc 2100
ggtatctcag tgtactttag gctaaagatt taaaaaaata gagaagtgaa aaacaatttg 2160
taggaaaagt ggtctttcct aaaataagat tttcttttcc tttcataaca aggacctggg 2220
gccaggcgtc gtggcacccg cctcaaaaca gttgagagga gaggggcagg tgggtctctg 2280
agtttgaagc cagccagggc tagatagtga ggccttgtct caaatagaaa agccaacaat 2340
tagcaggaca cacggagccg ggggtcctct tgcgaggcgt gtctggaatc catgagggcg 2400
gtcgctcgtg gtttttgagc ttcctgtgag ctgcaccccc agacacttgt aattcctgtt 2460
cttccgtctt tcggatgcag gttgctccca caaattcagg atagctatgg cgtttccatg 2520
catcccacaa tggaaggagt gtaagacttg agcttgagtc acagctaaaa cccagaagac 2580
acaaagtcag tctgtcctgg atcctacaaa cactcaacga gtgcctccct ctggaaaaga 2640
tggtgaacac cttccttcca ctgttgggca gtggacaggg aagttggagg tcgctgtagg 2700
cagctccaag gtttctaggg gcaccttgcc aggggacctc acaactttgg cggtctctag 2760
tggagcaggt taggttttag ctccctttgg ttcccgctgt gctttgtggt aaggcttttc 2820
cctgggctgt tgcctcattc ctcactagct ctgtgaccag gtaggactct gggatacatg 2880
gctgaaagct cttttgctct accacatctg cctctctttc cctacccatt tttttgtgtt 2940
tcggggtggt atttttgtgg tggttgtttt gttttatttc ttctttcctc tttgacgttt 3000
agtcttgctc taggcagaaa cgtcctggat atttacggtc agtaaagatg tccggtccca 3060
gcagagatac aacctctatc tgcagtaggt agagaaggct gtccctttcc ggtggctttc 3120
agattctcca cctctatccc tcccaggcct ctgatattct ttaaacttta aaggaaaagt 3180
ttggccgact ttctagaaac atccctggct cagagaggct ctctgtctcc tcggttgttc 3240
taaaacaaat ggtccgggtg gtgtgggtgc tggcccaaga ctcgattcct ctgagttagg 3300
gcagagcatg ctccctgcac tcagcaaagc ctctcgggct gatcagtgct cctgggtgat 3360
gctgtggtgc ttttaggagg aagacagtgt tggctggctg gaaagcatga cagttaaggt 3420
tgtgttctta agtgcctcag tttgaaagct ttggctaggg taggagatct gagatgccgt 3480
ggactttgca gaagagattt tgtgcagcaa cgcagaagcc tggctgtgtt aagattggct 3540
gtacaggata gcgaatgctg tggttggagg gcacagtctt cccgtgttag ttaaatagag 3600
aggctttagg acgtctctgc gcccacttga aggtagcctt atcctttagc tgttcgctaa 3660
aatagaatcc ttgagagatt gacaatatat ttgctggcct gtcgccctat tccttggaga 3720
ggccttttga atggttttaa ttccactggt tatccggtgc ccacttaatc aaatgtgaaa 3780
aataaaaagc agctctcttc ttagcctgcc cccgtggatc tgaggaccct gttagataca 3840
gctgtcggta agggtgtacc agctcactaa gaaacactac tgtcaagatt ttaaagctca 3900
gaccaagtga cacgttaata agaaactaag atctgattga attttctaac agtccttgcc 3960
tcgtggacac gctaaaattc gacttgggtg ccttacctct tttctaacta gtgttactca 4020

tgggcctgcg ctcccttcct ctgcggattc cttagaaccg gagagcctgt aagtgtgaca 4080
ggctcaaaac tggcctggct ggagggttag gtggactttg atctgcagtg ttccctctgg 4140
ggtcttcgtt tggggaatat ttcttggggc tgttttgctg aagtgcccgt agctgatgga 4200
tggtggaagt aatttgaacg tacgcgattt gtaggttttg ttatttgttt taagatttaa 4260
aggctggttg tagcatttaa aagtgaaagc tttttcttcc tggtttgcta gtgttctgcg 4320
tgtcttcttt agttgaaata ggttagtgga gaagttaaga aagcggtggc cgtgttacgc 4380
tggtggtgaa ggccagggcc gcctacaaac tctcagtttt gttttgtttt gtttttttat 4440
aacagtccca ctctgacaat aatgagttca ctccttggat aaggttgtta atgagggaag 4500
agcccttcag acctaatcag ctcttactgg acccagctgt caacactgtt gcattgggga 4560
ttaagtttcc aaccccgtga atcatgggac agactcaaac cgtagcactg ggcatgtcgc 4620
ctaacaatat aatgtgactc agtattctgt gagacatggg ataatattcc tgacctacct 4680
ccacgggtag gtgcgagcat gagcaaatgc tctttataaa acatttgggg atcctcagca 4740
gaggagtatt taagtcctgc acatttttat agtaaccgtc caccatggac ttgacacgtg 4800
cctatgtccc agacgacatt gttaatctac atcattcttc tcagaggttg aatggatgca 4860
ttagataatg tatactcatt gctatggaaa cctgcaggac tccttttcct tccttgggtg 4920
ggattttttt ttcctttatt atgtgcaaca gtagctattt gtaatctaat aattttcatt 4980
aataccaact ctgaaagtat ttctactcat ctgaggttgt gtttgttcat aataaaatga 5040
aatggatctg actgc 5055

<210> 44
<211> 605
<212> PRT
<213> Rattus norvegicus

<220>
<223> Ribophorin 1

<400> 44
Met Glu Ala Pro Ile Val Leu Leu Leu Leu Leu Trp Leu Ala Leu Ala
1               5                   10                  15

Pro Thr Pro Gly Ser Ala Ser Ser Glu Ala Pro Pro Leu Val Asn Glu
            20                  25                  30

Asp Val Lys Arg Thr Val Asp Leu Ser Ser His Leu Ala Lys Val Thr
        35                  40                  45

Ala Glu Val Val Leu Ala His Pro Gly Gly Gly Ser Thr Ala Arg Ala
    50                  55                  60

Ser Ser Phe Val Leu Ala Leu Glu Pro Glu Leu Glu Ser Arg Leu Ala
65                  70                  75                  80

His Leu Gly Val Gln Val Lys Gly Glu Asp Glu Glu Asp Asn Asn Leu
                85                  90                  95

Glu Val Arg Glu Thr Lys Met Lys Gly Lys Ser Gly Arg Phe Phe Thr
            100                 105                 110

Val Lys Leu Pro Val Ala Leu Asp Pro Gly Ser Lys Ile Ser Ile Val
        115                 120                 125

Val Glu Thr Val Tyr Thr His Val Leu His Pro Tyr Pro Thr Gln Ile
    130                 135                 140

Thr Gln Ser Glu Lys Gln Phe Val Val Phe Glu Gly Asn His Tyr Phe
145                 150                 155                 160

Tyr Ser Pro Tyr Pro Thr Lys Thr Gln Thr Met Arg Val Arg Leu Ala
                165                 170                 175

Ser Arg Asn Val Glu Ser His Thr Lys Leu Gly Asn Pro Ser Arg Ser
            180                 185                 190

Glu Asp Ile Leu Asp Tyr Gly Pro Phe Lys Asp Ile Pro Ala Tyr Ser
        195                 200                 205

Gln Asp Thr Phe Lys Val His Tyr Glu Asn Asn Ser Pro Phe Leu Thr
    210                 215                 220

Ile Thr Ser Met Thr Arg Val Ile Glu Val Ser His Trp Gly Asn Ile
225                 230                 235                 240

Ala Val Glu Glu Asn Val Asp Leu Lys His Thr Gly Ala Val Leu Lys
                245                 250                 255

Gly Pro Phe Ser Arg Tyr Asp Tyr Gln Arg Gln Pro Asp Ser Gly Ile
            260                 265                 270

Ser Ser Ile Arg Ser Phe Lys Thr Ile Leu Pro Ala Ala Ala Gln Asp
        275                 280                 285

Val Tyr Tyr Arg Asp Glu Ile Gly Asn Val Ser Thr Ser His Leu Leu
    290                 295                 300

Ile Leu Asp Asp Ser Val Glu Met Glu Ile Arg Pro Arg Phe Gly Leu
305                 310                 315                 320

Phe Gly Gly Trp Lys Thr His Tyr Ile Val Gly Tyr Asn Leu Pro Ser
                325                 330                 335

Tyr Glu Tyr Leu Tyr Asn Leu Gly Asp Gln Tyr Ala Leu Lys Met Arg
            340                 345                 350

Phe Val Asp His Val Phe Asp Glu Gln Val Ile Asp Ser Leu Thr Val
        355                 360                 365

Lys Ile Ile Leu Pro Glu Gly Ala Lys Asn Ile Gln Val Asp Ser Pro
    370                 375                 380

Tyr Asp Ile Ser Arg Ala Pro Asp Glu Leu His Tyr Thr Tyr Leu Asp
385                 390                 395                 400

Thr Phe Gly Arg Pro Val Ile Val Ala Tyr Lys Lys Asn Leu Val Glu
                405                 410                 415

Gln His Ile Gln Asp Ile Val Val His Tyr Thr Phe Asn Lys Val Leu
            420                 425                 430

Met Leu Gln Glu Pro Leu Leu Val Val Ala Ala Phe Tyr Ile Leu Phe
        435                 440                 445

Phe Thr Val Ile Ile Tyr Val Arg Leu Asp Phe Ser Ile Thr Lys Asp
    450                 455                 460

Pro Ala Ala Glu Ala Arg Met Lys Val Ala Cys Ile Thr Glu Gln Val
465                 470                 475                 480

Leu Thr Leu Val Asn Lys Arg Leu Gly Leu Tyr Arg His Phe Asp Glu
                485                 490                 495

Thr Val Asn Arg Tyr Lys Gln Ser Arg Asp Ile Ser Thr Leu Asn Ser
            500                 505                 510

Gly Lys Lys Ser Leu Glu Thr Glu His Lys Ala Val Thr Ser Glu Ile
        515                 520                 525

Ala Val Leu Gln Ser Arg Leu Lys Thr Glu Gly Ser Asp Leu Cys Asp
    530                 535                 540

Arg Val Ser Glu Met Gln Lys Leu Asp Ala Gln Val Lys Glu Leu Val
545                 550                 555                 560

Leu Lys Ser Ala Val Glu Ala Glu Arg Leu Val Ala Gly Lys Leu Lys
                565                 570                 575

Lys Asp Thr Tyr Ile Glu Asn Glu Lys Leu Ser Ser Gly Lys Arg Gln
            580                 585                 590

Glu Leu Val Thr Lys Ile Asp His Ile Leu Asp Ala Leu
        595                 600                 605

<210> 45
<211> 2214
<212> DNA
<213> Rattus norvegicus

<220>
<223> Ribophorin 1

<400> 45
   tcgcggtcat ggaggcgccg atcgtcttgc tgctgctgct atggctcgcc ttggccccga 60
   cgcctggcag cgcctcttcg gaggctccgc cgctggtcaa cgaggacgtg aagcgcacgg 120
   tggacctgag cagccaccta gccaaggtga cggctgaggt ggtcctggcg cacccgggcg 180
   gcggctctac agcacgagcc agctctttcg ttctggccct ggagcccgaa ctggagtcgc 240
   gacttgcaca cctaggcgtg caggtaaagg gagaagatga ggaagacaac aacctagaag 300
   tacgagaaac caaaatgaag gggaaaagtg ggaggttttt caccgtcaag ctcccagttg 360
   ctcttgatcc tgggtccaag atctcaatcg ttgtggaaac tgtctacacc catgtgcttc 420
   atccataccc gactcagata actcagtcag agaaacagtt tgtggtgttt gagggcaacc 480
   attacttcta ctctccctat ccaacaaaga cccagaccat gcgagtgaga cttgcttccc 540
   gaaatgtgga gagccatacc aagctgggga acccctcaag gtctgaggac atcctggact 600
   atgggccttt taaagacatc cctgcctaca gtcaggatac tttcaaagta cattatgaga 660
   acaatagccc tttcctgacc atcaccagta tgacccgggt cattgaggtt tctcactggg 720
   gcaatattgc tgtggaagag aacgtggact tgaagcatac aggtgcggtg ctgaagggac 780
   ctttctcccg ctacgattac cagaggcagc ctgacagtgg gatctcctcc attcgttctt 840
   ttaagaccat ccttcctgct gctgcccagg atgtgtatta ccgggatgaa atcggtaatg 900
   tttccaccag ccacctcctt attttggatg actccgtgga aatggaaatc cggcctcgat 960
   ttggtctctt tggagggtgg aagacacact acatcgttgg ttacaacctc ccaagctatg 1020
   agtacctcta taatctgggt gaccagtatg cactgaagat gcggtttgta gaccacgtgt 1080
   ttgatgagca agtgatagat tctctgacag tgaagatcat ccttcctgag ggagccaaga 1140
   acatccaggt agacagtccc tacgatatta gccgggcccc agatgagctg cattacacct 1200
   acctagacac attcggccgc ccggtgattg ttgcttacaa gaagaatcta gttgaacagc 1260
   acattcagga cattgtggtg cactacacat tcaacaaggt gctcatgctg caggagcctc 1320
   tgctggttgt ggccgccttc tacatcctgt tcttcaccgt catcatctac gtccgtctag 1380
   acttttccat caccaaggac ccagcagcag aagccaggat gaaagtggcc tgtatcacag 1440
   agcaggtctt aaccctggtc aacaagaggt taggcctcta ccgtcacttt gatgagactg 1500
   tcaatagata caagcagtcc cgggacatct ctaccctcaa tagtggcaag aagagcctag 1560
   agacagagca caaagctgtg accagtgaga ttgctgtgct gcagtctagg ctgaagacgg 1620
   agggctctga cctgtgtgac agggtgagcg aaatgcagaa gctggacgcg caggtcaagg 1680
   agctggtact gaagtctgcg gtggaggcag agaggctggt ggctggcaag ctcaagaagg 1740
   acacatacat cgagaatgaa aagctcagct caggaaaacg ccaggagctg gtcaccaaga 1800
   tcgaccacat cctagacgct ctgtagctct tgtctccaga gcaggcctgg gtggcctaca 1860
   cttctggatg gaataggtag agggaggaag ttgtgattag cctaagacct caagaaaaaa 1920
   gctgaggccc atccatggct gtgtaagaag cttgtccctt atttctgaat ggttctcttt 1980
   tttacaaaaa caaaaaccta aacaaaaatc ctgcccaaaa aacaaacttg gttgtgtttt 2040
   gagcaaaagc attttgagta gttgattgct gttttgtttt gatacctggg tattttttct 2100
   gtctgtaagc cctgttttat ggccttctga ttcctaatgt ttggttcttt tgtgagcgta 2160
   tgcatggttt tttaaaagtg tgtatgacca aataaaaata aaggagggac tgtg 2214

<210> 46
<211> 284
<212> PRT
<213> Rattus norvegicus

<220>
<223> Sulfotransferase-like protein

<400> 46
Met Ala Glu Ser Glu Ala Glu Thr Pro Gly Thr Pro Gly Glu Phe Glu
1               5                   10                  15

Ser Lys Tyr Phe Glu Phe His Gly Val Arg Leu Pro Pro Phe Cys Arg
            20                  25                  30

Gly Lys Met Glu Asp Ile Ala Asp Phe Pro Val Arg Pro Ser Asp Val
        35                  40                  45

Trp Ile Val Thr Tyr Pro Lys Ser Gly Thr Ser Leu Leu Gln Glu Val
    50                  55                  60

Val Tyr Leu Val Ser Gln Gly Ala Asp Pro Asp Glu Ile Gly Leu Met
65                  70                  75                  80

Asn Ile Asp Glu Gln Leu Pro Val Leu Glu Tyr Pro Gln Pro Gly Leu
                85                  90                  95

Asp Ile Ile Lys Glu Leu Thr Ser Pro Arg Leu Ile Lys Ser His Leu
            100                 105                 110

Pro Tyr Arg Phe Leu Pro Ser Asp Leu His Asn Gly Asp Ser Lys Val
        115                 120                 125

Ile Tyr Met Ala Arg Asn Pro Lys Asp Leu Val Val Ser Tyr Tyr Gln
    130                 135                 140

Phe His Arg Ser Leu Arg Thr Met Ser Tyr Arg Gly Thr Phe Gln Glu
145                 150                 155                 160

Phe Cys Arg Arg Phe Met Asn Asp Lys Leu Gly Tyr Gly Ser Trp Phe
                165                 170                 175

Glu His Val Gln Glu Phe Trp Glu His Arg Met Asp Ala Asn Val Leu
            180                 185                 190

Phe Leu Lys Tyr Glu Asp Met His Arg Asp Leu Val Thr Met Val Glu
        195                 200                 205

Gln Leu Ala Arg Phe Leu Gly Val Ser Cys Asp Lys Ala Gln Leu Glu
    210                 215                 220

Ser Leu Ile Glu His Cys His Gln Leu Val Asp Gln Cys Cys Asn Ala
225                 230                 235                 240

Glu Ala Leu Pro Val Gly Arg Gly Arg Val Gly Leu Trp Lys Asp Ile
                245                 250                 255

Phe Thr Val Ser Met Asn Glu Lys Phe Asp Leu Val Tyr Lys Gln Lys
            260                 265                 270

Met Gly Lys Cys Asp Leu Thr Phe Asp Phe Tyr Leu
        275                 280

<210> 47
<211> 2239
<212> DNA
<213> Rattus norvegicus

<220>
<223> Sulfotransferase-like protein

<400> 47
ggcacgagcc ctggagctgc gggccgggcc gggcggcagc atggcggaga gcgaagcgga 60
gaccccgggc accccgggcg agttcgagag caagtacttc gagttccatg gcgtgcggct 120
gcctcccttc tgccgcggga agatggagga catcgccgac ttcccagtgc ggcccagcga 180
tgtgtggatc gtcacctacc ccaagtcagg tacgagcttg ctgcaagagg tggtctactt 240
ggtgagccag ggggctgacc ctgatgagat tggcctcatg aacattgacg agcagctgcc 300
agtactggag tacccacagc ccgggctgga catcatcaag gaactgacgt ctccccgcct 360
catcaagagc caccttccct accgcttcct gccctctgac ctccacaacg gggactccaa 420
ggtcatctac atggctcgca acccaaagga cctggtggta tcttactatc agttccaccg 480
ctcgctgcgg accatgagtt accgaggaac cttccaggag ttctgtcgga ggttcatgaa 540
cgacaaattg ggctatggct cctggtttga gcacgttcag gaattctggg aacatcgaat 600
ggacgcaaat gtgcttttcc tcaagtatga agatatgcac cgggacctgg tgaccatggt 660
ggagcagctg gccagattcc tgggtgtgtc ctgtgataag gcccagctgg agtccttgat 720
cgagcactgc caccagctgg tggaccagtg ctgcaatgct gaggccctgc ctgtgggccg 780
gggaagagtc gggctgtgga aggacatctt cactgtctcc atgaacgaga agtttgatct 840
agtgtataaa cagaagatgg ggaagtgtga cctcacgttt gacttttatt tataatacca 900
gaatccgcaa acttgcatgc tcacggtccc cagacgctct actagctaaa agtcctgttt 960
gcgttcattt attccttgct ggacactccg gaagttgctt ggaaacagca gggggcgagc 1020
ctggtggaga gagccgagga gtgagatgat gctgtctaca gcagccgcct ggccgtagcg 1080
tttgagtctc cacgtgctgg ccataccaca cgcacgggca ggcaaatgtc tgcccctgta 1140
ccccatgtta ttcactgtat tttatagcgc ttttcactga aaatcgaaat aaatatgtca 1200
accaaatgca agtccatttc caagggggag caggagcgag ctgtgccggg atgctaggaa 1260
gctctcaggg gtcactgtaa tttttatagt cttctctcta ggcacagcca tcccaccttg 1320
tcccgagatc aaaggacagg ttggggggag ctggagaggt gggtgtagcc tgtggagttc 1380
agttttctgg aaccatcctg acaccaagcc aggtcctgtc ccaggtctct gagcccagta 1440
cagaatgttc tggaagggaa gctggcctga ggctgcggat ggcatctgaa ggcatcagtg 1500
gtgtgtgaag cgtacccaca gacaccagac tgcaccaaga actagtatat ctcatagcct 1560
tcacttgctc cccaaggccg tgactggcac actcccaatg gcaagagaca tgacatacca 1620
tggttctgaa gtccctagtc atcgtcacgt ctgtgccacc tgtgccaagg ctctcgctcg 1680
ggagacccag tctgcaggtt gactgtcctc catgcagata ggattggcaa gcccacctgt 1740
ctgagggggc acttagccct caaggatgtc tgagggagca atggccccat gtactgtcca 1800
ccctgacacc taccggctct gtcttccagc tccaatttgg ctcaagtgat gaccagatga 1860
tgggtggacc aggagattgc tgcttgtggg gaccaactta ttctgttcct taccaggatg 1920
ggaagagtct taacgtggtg gggggaggct ctggctgtgg aaggtgcctg ctgtgcgcca 1980
tctctgaagc aggtttggtt gtagcagcgt tacctctgtc tagaggaaag cttgtgtggg 2040
tctggtgcat ggttctctga ggtgggcatg gcagtgcctt tcctgtgtgt ctggggagga 2100
aggctgcttt ctgtgaaatt ttctttctat ttttctattt ttagtactgt acggatgttc 2160
tggaaccaca cacggtactc tgtgcttgct tgcatcttta ataaagacac cctcccctgt 2220
caaaaaaaaa aaaaaaaaa                                              2239

<210> 48
<211> 235
<212> PRT
<213> Rattus norvegicus

<220>
<223> F1-ATPase alpha subunit

<400> 48
Val Ala Asp Arg Gln Met Ser Leu Leu Leu Arg Arg Pro Pro Gly Arg
1               5                   10                  15

Glu Ala Tyr Pro Gly Asp Val Phe Tyr Leu His Ser Arg Leu Leu Glu
            20                  25                  30

Arg Ala Ala Lys Met Asn Asp Ser Phe Gly Gly Gly Ser Leu Thr Ala
        35                  40                  45

Leu Pro Val Ile Glu Thr Gln Ala Gly Asp Val Ser Ala Tyr Ile Pro
    50                  55                  60

Thr Asn Val Ile Ser Ile Thr Asp Gly Gln Ile Phe Leu Glu Thr Glu
65                  70                  75                  80

Leu Phe Tyr Lys Gly Ile Arg Pro Ala Ile Asn Val Gly Leu Ser Val
                85                  90                  95

Ser Arg Val Gly Ser Ala Ala Gln Thr Arg Ala Met Lys Gln Val Ala
            100                 105                 110

Gly Thr Met Lys Leu Glu Leu Ala Gln Tyr Arg Glu Val Ala Ala Phe
        115                 120                 125

Ala Gln Phe Gly Ser Asp Leu Asp Ala Ala Thr Gln Gln Leu Leu Ser
    130                 135                 140

Arg Gly Val Arg Leu Thr Glu Leu Leu Lys Gln Gly Gln Tyr Ser Pro
145                 150                 155                 160

Met Ala Ile Glu Glu Gln Val Ala Val Ile Tyr Ala Gly Val Arg Gly
                165                 170                 175

Tyr Leu Asp Lys Leu Glu Pro Ser Lys Ile Thr Lys Phe Glu Ser Ala
            180                 185                 190

Phe Leu Ser His Val Val Ser Gln His Gln Ser Leu Leu Gly Asn Ile
        195                 200                 205

Arg Ser Asp Gly Lys Ile Ser Glu Gln Ser Asp Ala Lys Leu Lys Glu
    210                 215                 220

Ile Val Thr Asn Phe Leu Ala Gly Phe Glu Pro
225                 230                 235


<210> 49
<211> 1066
<212> DNA
<213> Rattus norvegicus

<220>
<223> F1-ATPase alpha subunit

<400> 49
gtggctgacc gccagatgtc tctgctgctc cgccgacccc cgggtcgaga ggcctatccc 60
ggtgatgtgt tttacctaca ctctcgcctg ctggagagag cagccaagat gaacgattcc 120
tttggtggtg gctctttgac tgccttacca gtcattgaaa cacaggctgg tgatgtgtcc 180

gcctacattc caacaaacgt tatttccatc accgatggac agatcttctt ggaaacagaa 240
ttgttctata aaggcatccg ccctgccatt aatgtgggct tgtctgtgtc ccgtgtcgga 300
tctgccgccc agaccagagc catgaagcag gtggcaggca ccatgaagct ggagttggcc 360
cagtaccggg aggtcgctgc ttttgcccag tttggttctg atctggatgc tgccactcag 420
cagctcttga gccgtggcgt gcgcctgacc gagctgctaa agcaaggaca gtactctccc 480
atggctattg aagaacaggt ggctgtcatc tatgcaggcg tccggggtta tcttgataaa 540
ctggagccca gtaagatcac aaagttcgag agtgctttct tgtctcatgt tgtgagccag 600
caccagagcc tcttgggcaa tatcaggtct gatgggaaaa tctcagaaca gtcggatgca 660
aagctgaagg aaatcgtaac aaacttcttg gctgggtttg aaccttaaag ccctgccact 720
gtcaccagac actgctttgg ttttgtcatt tattgtggta aaatcagcac catttgtaaa 780
ggtttactct tgtactccct gatgtacaga aatcacatga ataaaagttc catattgagt 840
gctagtttgt gtgggacggg ctcagatgta tcctgggact tgtggaggtc agagagcagc 900
ctgtaggagt cagtgccctc ctttccctgg gaatttaact caggttatta tttaaagctc 960
gacttcaggg gttggggatt tagctcagtg gtagagcgct tgcctaggaa gcgcaaggcc 1020
ctgggttcgg tccccagctc cggaaaaaaa agaaccaacc cgaatt 1066

<210> 50
<211> 168
<212> PRT
<213> Rattus norvegicus

<220>
<223> F1F0 ATPase delta subunit

<400> 50
Met Leu Pro Ala Ala Leu Leu Arg His Pro Gly Leu Arg Arg Leu Val
1               5                   10                  15

Leu Gln Ala Arg Thr Tyr Ala Gln Ala Ala Ala Ser Pro Ala Pro Ala
            20                  25                  30

Ala Gly Pro Gly Gln Met Ser Phe Thr Phe Ala Ser Pro Thr Gln Val
        35                  40                  45

Phe Phe Asp Gly Ala Asn Val Arg Gln Val Asp Val Pro Thr Leu Thr
    50                  55                  60

Gly Ala Phe Gly Ile Leu Ala Ser His Val Pro Thr Leu Gln Val Leu
65                  70                  75                  80

Arg Pro Gly Leu Val Met Val His Ala Glu Asp Gly Thr Thr Thr Lys
                85                  90                  95

Tyr Phe Val Ser Ser Gly Ser Val Thr Val Asn Ala Asp Ser Ser Val
            100                 105                 110

Gln Leu Leu Ala Glu Glu Val Val Thr Leu Asp Met Leu Asp Leu Gly
        115                 120                 125

Ala Ala Arg Ala Asn Leu Glu Lys Ala Gln Ser Glu Leu Ser Gly Ala
    130                 135                 140

Ala Asp Glu Ala Ala Arg Ala Glu Ile Gln Ile Arg Ile Glu Ala Asn
145                 150                 155                 160

Glu Ala Leu Val Lys Ala Leu Glu
                165

<210> 51
<211> 811

<212> DNA
<213> Rattus norvegicus

<220>
<223> F1F0 ATPase delta subunit

<400> 51
ggaattccgc tgctgtcgct aagctaaagt cactgacgtt tccgccacca tgctgcccgc 60
cgcattgctt cgtcacccag gtctgcgccg tctggtgctc caggcgcgta cgtacgccca 120
ggccgccgcc tcacctgccc ccgccgctgg gcctggacag atgtccttca ccttcgcctc 180
cccgacgcag gtgttctttg atggtgccaa tgtccggcaa gtggatgtgc ctacgctgac 240
tggagccttt ggcatcctgg catcccatgt ccccacacta caggtcctac ggcctgggct 300
ggtaatggtt catgcggaag atggcaccac aactaagtat tttgtgagca gcggctccgt 360
cactgtgaat gcggactcct ctgtgcaatt actagctgaa gaagtcgtga cactggacat 420
gctggacctc ggggcagccc gggcaaacct ggagaaggcg cagtcagagc tgtcaggggc 480
agcagatgag gcagcacggg ctgagatcca aatccggatc gaggccaatg aagccctggt 540
gaaggccctg gagtaggcgt actttgtctg tcacccacag ggtaacagag gcaggtcctg 600
gggggctgaa gtcgccacca gggggcagca gtgctccggt tactggctta aagcttccct 660
ggtgctgcct gccaggtcat ggagggcttc cccaggcttc cagagtctgg gatccccagg 720
atgcctctgg agagctggcc ttgattgccc ctcaaagcca cccggacagt cagctggccc 780
agcctatctg cattaaatac cacggaattc c 811

<210> 52
<211> 572
<212> PRT
<213> Homo sapiens

<220>
<223> Dihydropyrimidinase-related protein

<400> 52

<210> 53
<211> 4459
<212> DNA
<213> Homo sapiens

<220>
<223> Dihydropyrimidinase-related protein

<400> 53
gtttctctct ctccttctct ctctctctct ctctctcttt tttttccgcc ctagctgggg 60
ctgtgttgga ggagaggaag aaagagagac agaggattgc attcatccgt tacgttcttg 120
aaatttccta atagcaagac cagcgaagcg gttgcaccct tttcaatctt gcaaaggaaa 180
aaaacaaaac aaaacaaaaa aaacccaagt ccccttcccg gcagtttttg ccttaaagct 240
gccctcttga aattaatttt ttcccaggag agagatgtct tatcagggga agaaaaatat 300
tccacgcatc acgagcgatc gtcttctgat caaaggaggt aaaattgtta atgatgacca 360
gtcgttctat gcagacatat acatggaaga tgggttgatc aagcaaatag gagaaaatct 420
gattgtgcca ggaggagtga agaccatcga ggcccactcc cggatggtga tccccggagg 480
aattgacgtc cacactcgtt tccagatgcc tgatcaggga atgacgtctg ctgatgattt 540
cttccaagga accaaggcgg ccctggctgg gggaaccact atgatcattg accacgttgt 600
tcctgagcct gggacaagcc tgctcgctgc ctttgaccag tggagggaat gggccgacag 660
caagtcctgc tgtgactact ctctgcatgt ggacatcagc gagtggcata agggcatcca 720
ggaggagatg gaagcgcttg tgaaggatca cggggtaaat tccttcctcg tgtacatggc 780
tttcaaagat cgcttccagc taacggattg ccagatttat gaagtactga gtgtgatccg 840
ggatattggc gccatagccc aagtccacgc agaaaatggc gacatcattg cagaggagca 900
gcagaggatc ctggatctgg gcatcacggg ccccgaggga catgtgctga gccgacctga 960
ggaggtcgag gccgaagccg tgaatcgtgc catcaccatc gccaaccaga ccaactgccc 1020
gctgtatatc accaaggtga tgagcaaaag ctctgctgag gtcatcgccc aggcacggaa 1080
gaagggaact gtggtgtatg gcgagcccat cactgccagc ttgggaacgg acggctccca 1140
ttactggagc aagaactggg ccaaggctgc tgcctttgtc acctccccac ccttgagccc 1200
tgatccaacc actccagact ttctcaactc cttgctgtcc tgtggagacc tccaggtcac 1260
gggcagtgcc cattgcacgt ttaacactgc ccagaaggct gtaggaaagg acaacttcac 1320
cctgattccg gagggcacca atggcactga ggagcggatg tccgtcatct gggacaaggc 1380
tgtggtcact gggaagatgg atgagaacca gtttgtggct gtgaccagca ccaatgcagc 1440
caaagtcttc aacctttacc cccggaaagg ccgcattgct gtgggatccg atgccgacct 1500
ggtcatctgg gaccccgaca gcgttaaaac catctctgcc aagacacaca acagctctct 1560
cgagtacaac atctttgaag gcatggagtg ccgcggctcc ccactggtgg tcatcagcca 1620
ggggaagatt gtcctggagg acggcaccct gcatgtcacc gaaggctctg gacgctacat 1680
tccccggaag cccttccctg attttgttta caagcgtatc aaggcaagga gcaggctggc 1740
tgagctgaga ggggttcctc gtggcctgta tgacggacct gtgtgtgaag tgtctgtgac 1800
gcccaagaca gtcactccag cctcctcggc caagacgtct cctgccaagc agcaggcccc 1860
acctgtccgg aacctgcacc agtctggatt cagtttgtct ggtgctcaga ttgatgacaa 1920
cattccccgc cgcaccaccc agcgtatcgt ggcgcccccc ggtggccgtg ccaacatcac 1980
cagcctgggc tagagctcct gggctgtgcg tccactgggg actggggatg ggacacctga 2040
ggacattctg agacttcttt cttccttcct tttttttttt ttgttttttt ttttaagagc 2100
ctgtgatagt tactgtggag cagccagttc atggggtccc ccttggggcc ccacaccccg 2160

tctctcacca agagttactg attttgctca tccacttccc tacacatcta tgggtatcac 2220
acccaagact acccaccaag ctcatacagg gaaccacacc caacacttag acatgcgaac 2280
aagcagcccc cagcgagggt ctccttcgcc ttcaacctcc tagtgtctgt tagcatcttc 2340
cttttcatgg ggggagggaa gataaagtga attgcccaga gctgcctttt tcttttcttt 2400
ttaaaaattt taagaagttt tccttgtggg gctggggagg ggccggggtc agggagagtc 2460
tttttttttt tttttttaaa tactaaattg gaacatttaa ttccatatta atacaagggg 2520
tttgaactgg acatcctaat gatgcaatta cgtcatcacc cagctgattc cgggtggttg 2580
gcaaactcat cgtgtctgtc ctgagaggct ccacaatgcc cacccgcatc gccattctgt 2640
agtcttcagg gtcagctgtt gataaagggg caggcttgcg ttattggcct agattttgct 2700
gcagattaaa tcctttgagg attctcttct cttttaccat ttttctgcgt gctctcactc 2760
tctctttctc tctctagctt tttaattcat gaatattttc gtgtctgtct ctctctctct 2820
ctgtgtttcc tccagccctt gtctcggaga cggtgttttc ctcccttgcc ccattatctt 2880
ttcacctccc aggtctacca tttcatggtg gtcgttgggt ccgcctaaag gatttgagcg 2940
tttgccattg caagcatagt gctgtgtcat cctggtccat gtaggactgg tgctaaccac 3000
ctgccatcat gaggatgtgt gctagagtgt gggaccctgg ccaagtgcag gaatggccca 3060
tgccgtctca cccacagtat cacacgtgga accgcagaca gggcccagaa gctttagagg 3120
tatgaggctg cagaaccgga gagattttcc tctgtgcagt gctctctggc taaagtcacg 3180
gtcaaaccta aacaccgagc ctcattaacc caagtgaacc aaccaaagtc accagttcag 3240
aagtgctaag ctaataggag tctgacccga gggcctgctg cttcctggtt aagtatcttt 3300
tgagattcta gaacacatgg gagcttttta ttttcgggga aaaaccgtat ttttttcttg 3360
tccaattatt tctaaagaca cactacatag aaagaggccc tataaactca aaaagtcatt 3420
gggaaactta aagtctattc tactttgcaa gaggagaaat gtgttttatg aacgatagat 3480
cacatcagaa ctcctgtggg gaggaaacct tataaattaa acacatggcc cccttagaga 3540
ccacaggtga tgtctgtctc catccttccc tctccttttc tgtcaccttt ccccctagct 3600
ggctcctttg gacctacccc tgtccttgct gacttgtgtt gcattgtatt ccaaacgtgt 3660
ttacaggttc tcttaagcaa tgttgtattt gcaggctttt ctgaatacca aatctgcttt 3720
ttgtaaagcg taaaaacatc acaaagtagg tcattccatc accacccttg tctctctaca 3780
cattttgcct ttggggatct ggttggggtt ttgggttttt tgttgttgtt gtttatttgt 3840
tattttaaag gtaaattgca cttttaaaaa aataattggt tgacttaata tatttgcttt 3900
ttttctcacc tgcacttaga ggaaatttga acaagttgga aaaaaacaat ttttgtttca 3960
attctaagaa acacttgcag ctctagtatt cacttgagtc ttcctgtttt tcctgtaccg 4020
ggtcatggta atttttggtt gttttggttg ttttcttaaa aaacaagtta aaacctgacg 4080
atttctgcag gctgtgtaag catgtttacc tgttggcttg ctttgtgtgt ctgttaaatg 4140
aatgtcatat gtaaatgcta aaataaatcg acagtgtctc agaactgaat aactgcagtg 4200
acttgatgct ctaaaacagt gtaggattta agaatagatg gtttttaatc ctggaaattg 4260
tgattgtgac ccatgagtgg aggaactttc agttctaaag ctgataaagt gtgtagccag 4320
aagagtactt ttttttttgt aaccactgtc ttgatggcaa aataattatg gtaaaaaaca 4380
agtctcgtgt ttattattcc ttaagaactc tgtgttatat taccatggaa cgcctaataa 4440
agcaaaatgt ggttgtttc 4459

<210> 54
<211> 1410
<212> DNA
<213> Rattus norvegicus

<220>
<223> Myelin basic protein S

<400> 54
ttcggcttcc gaaggcctga tgtgatggca tcacagaaga gaccctcaca gcgacacgga 60
tccaagtact tggccacagc aagtaccatg gaccatgccc ggcatggctt cctcccaagg 120
cacagagaca cgggcatcct tgactccatc gggcgcttct ttagcggtga caggggtgcg 180
cccaagcggg gctctggcaa ggactcacac acaagaacta cccactacgg ctccctgccc 240
cagaagtcgc agaggaccca agatgaaaac ccagtagtcc acttcttcaa gaacattgtg 300
acacctcgta caccccctcc atcccaagga aaggggagag gcctgtccct cagcagattt 360
agctggggag gaagagacag ccgctctgga tctcccatag caagacgctg agagcctccc 420
tgctcagcct tcccgaatcc tgccctcggc ttcttaatat aactgcctta aacgtttaat 480
tctacttgca ccaaatagct agttagagca gaccctctct taatcccgtg gggctgtgaa 540
cgcggcgggc cagcccacgg caccctgact ggctaaaact gtttgtccct ttttatttga 600
agattgagtt tcctcggggt cttctctgcc ccgacttgct ccccgtgtac cttggtcgac 660

tccggaggtt caggtgcacg gacacccttt caagttcacc cctactccat cctcagactt 720
tcttttcacg gcgaggcgca cccctccagc ttccgtgggc actgcggata gacaggcaca 780
ccgccaagga gccagagagc atggcgcagg ggactatgtg gtccaggctt cctttgtttt 840
ctttccccta aagagctttg tttttcctga caggatcaga cagtcttgga gtggcttaca 900
caacgggggc ttgtggtatg tgagcacagg tcgggcagct gtgagagtcc agagtggggt 960
ggccctgggg acgcttccag gccagcggtt ccctgcaccc caccagctga tttcgagcgt 1020
ggcagaggga aggaaagggg cgagcgggct gggcaatgga cccgacagga aacggggact 1080
taggggaaca cgctggagat gccatgtgtg gctgccgaag gtcaccatct ctcctcagtg 1140
ctccccagag caggtgcttt taagaaccct gtttcctctc agagcctagg gagagtccaa 1200
ggacatggcg catcaggaag tgggactgca ggagttctct ggtggcctcg tgctgtccct 1260
ctggccactt ctcactttag ggtggtcacg gcagctcgcc atggcagtgc cattggtgca 1320
cactaacctc atggaaaagt aaccattccc tgcctcttag aaagaactca ttcttagttt 1380
taggagggtt cctgtcgctg aatcaagtcg 1410

<210> 55
<211> 698
<212> PRT
<213> Rattus norvegicus

<220>
<223> Transferrin

<400> 55
Met Arg Phe Ala Val Gly Ala Leu Leu Ala Cys Ala Ala Leu Gly Leu
1               5                   10                  15

Cys Leu Ala Val Pro Asp Lys Thr Val Lys Trp Cys Ala Val Ser Glu
            20                  25                  30

His Glu Asn Thr Lys Cys Ile Ser Phe Arg Asp His Met Lys Thr Val
        35                  40                  45

Leu Pro Ala Asp Gly Pro Arg Leu Ala Cys Val Lys Lys Thr Ser Tyr
    50                  55                  60

Gln Asp Cys Ile Lys Ala Ile Ser Gly Gly Glu Ala Asp Ala Ile Thr
65                  70                  75                  80

Leu Asp Gly Gly Trp Val Tyr Asp Ala Gly Leu Thr Pro Asn Asn Leu
                85                  90                  95

Lys Pro Val Ala Ala Glu Phe Tyr Gly Ser Leu Glu His Pro Gln Thr
            100                 105                 110

His Tyr Leu Ala Val Ala Val Val Lys Lys Gly Thr Asp Phe Gln Leu
        115                 120                 125

Asn Gln Leu Gln Gly Lys Lys Ser Cys His Thr Gly Leu Gly Arg Ser
    130                 135                 140

Ala Gly Trp Ile Ile Pro Ile Gly Leu Leu Phe Cys Asn Leu Pro Glu
145                 150                 155                 160

Pro Arg Lys Pro Leu Glu Lys Ala Val Ala Ser Phe Phe Ser Gly Ser
                165                 170                 175

Cys Val Pro Cys Ala Asp Pro Val Ala Phe Pro Gln Leu Cys Gln Leu
            180                 185                 190

Cys Pro Gly Cys Gly Cys Ser Pro Thr Gln Pro Phe Phe Gly Tyr Val
        195                 200                 205

Gly Ala Phe Lys Cys Leu Arg Asp Gly Gly Gly Asp Val Ala Phe Val
    210                 215                 220

Lys His Thr Thr Ile Phe Glu Val Leu Pro Gln Lys Ala Asp Arg Asp
225                 230                 235                 240

Gln Tyr Glu Leu Leu Cys Leu Asp Asn Thr Arg Lys Pro Val Asp Gln
                245                 250                 255

Tyr Glu Asp Cys Tyr Leu Ala Arg Ile Pro Ser His Ala Val Val Ala
            260                 265                 270

Arg Asn Gly Asp Gly Lys Glu Asp Leu Ile Trp Glu Ile Leu Lys Val
        275                 280                 285

Ala Gln Glu His Phe Gly Lys Gly Lys Ser Lys Asp Phe Gln Leu Phe
    290                 295                 300

Gly Ser Pro Leu Gly Lys Asp Leu Leu Phe Lys Asp Ser Ala Phe Gly
305                 310                 315                 320

Cys Tyr Gly Val Pro Pro Arg Met Asp Tyr Arg Leu Tyr Leu Gly His
                325                 330                 335

Ser Tyr Val Thr Ala Ile Arg Asn Gln Arg Glu Gly Val Cys Pro Glu
            340                 345                 350

Ala Ser Ile Asp Ser Ala Pro Val Lys Trp Cys Ala Leu Ser His Gln
        355                 360                 365

Glu Arg Ala Lys Cys Asp Glu Trp Ser Val Thr Ser Asn Gly Gln Ile
    370                 375                 380

Glu Cys Glu Ser Ala Glu Ser Thr Glu Asp Cys Ile Asp Lys Ile Val
385                 390                 395                 400

Asn Gly Glu Ala Asp Ala Met Ser Leu Asp Gly Gly His Ala Tyr Ile
                405                 410                 415

Ala Gly Gln Cys Gly Leu Val Pro Val Met Ala Glu Asn Tyr Asp Ile
            420                 425                 430

Ser Ser Cys Thr Asn Pro Gln Ser Asp Val Phe Pro Lys Gly Tyr Tyr
        435                 440                 445

Ala Val Ala Val Val Lys Ala Ser Asp Ser Ser Ile Asn Trp Asn Asn
    450                 455                 460

Leu Lys Gly Lys Lys Ser Cys His Thr Gly Val Asp Arg Thr Ala Gly
465                 470                 475                 480

Trp Asn Ile Pro Met Gly Leu Leu Phe Ser Arg Ile Asn His Cys Lys
                485                 490                 495

Phe Asp Glu Phe Phe Ser Gln Gly Cys Ala Pro Gly Tyr Lys Lys Asn
            500                 505                 510

Ser Thr Leu Cys Asp Leu Cys Ile Gly Pro Ala Lys Cys Ala Pro Asn
        515                 520                 525

Asn Arg Glu Gly Tyr Asn Gly Tyr Thr Gly Ala Phe Gln Cys Leu Val

530                 535                 540

Glu Lys Gly Asp Val Ala Phe Val Lys His Gln Thr Val Leu Glu Asn
545                 550                 555                 560

Thr Asn Gly Lys Asn Thr Ala Ala Trp Ala Lys Asp Leu Lys Gln Glu
                565                 570                 575

Asp Phe Gln Leu Leu Cys Pro Asp Gly Thr Lys Lys Pro Val Thr Glu
            580                 585                 590

Phe Ala Thr Cys His Leu Ala Gln Ala Pro Asn His Val Val Val Ser
        595                 600                 605

Arg Lys Glu Lys Ala Ala Arg Val Ser Thr Val Leu Thr Ala Gln Lys
    610                 615                 620

Asp Leu Phe Trp Lys Gly Asp Lys Asp Cys Thr Gly Asn Phe Cys Leu
625                 630                 635                 640

Phe Arg Ser Ser Thr Lys Asp Leu Leu Phe Arg Asp Asp Thr Lys Cys
                645                 650                 655

Leu Thr Lys Leu Pro Glu Gly Thr Thr Tyr Glu Glu Tyr Leu Gly Ala
            660                 665                 670

Glu Tyr Leu Gln Ala Val Gly Asn Ile Arg Lys Cys Ser Thr Ser Arg
        675                 680                 685

Leu Leu Glu Ala Cys Thr Phe His Lys Ser
    690                 695


<210> 56
<211> 2293
<212> DNA
<213> Rattus norvegicus

<220>
<223> Transferrin

<400> 56
cgtccgctcc tcgccacaca caccgagagg atgaggttcg ctgtgggtgc cctgctggct 60
tgtgccgccc tgggactgtg tctggctgtc cctgacaaaa cggtcaaatg gtgcgcagtg 120
tctgagcatg agaacaccaa gtgtatcagt ttccgtgacc acatgaaaac cgtccttcca 180
gctgatggcc cccggcttgc ctgtgtgaag aaaacctcct atcaagattg catcaaggcc 240
atttctggag gtgaagctga tgccattacc ttggatgggg gttgggtgta cgatgcaggc 300
ctgactccca acaacctgaa gcctgtggca gcagagtttt atggatcact tgaacatcca 360
cagacccact acttggctgt ggccgtggtg aagaagggaa cagacttcca gctgaaccag 420
ctccagggca agaagtcctg ccacactggc ctgggcaggt ctgcaggctg gattatcccc 480
attggcttac ttttctgtaa cttgccagag ccccgcaagc ctcttgagaa agctgtggcc 540
agtttcttct cgggcagttg tgtcccctgt gcagatccag tggccttccc ccagctgtgt 600
caactgtgtc caggctgtgg ctgctccccg actcaaccgt tctttggcta cgtaggcgcc 660
ttcaagtgtc tgagagatgg aggtggagat gtggcctttg tcaagcatac aaccatattt 720
gaggtcttgc cacagaaggc tgacagggat caatatgagc tgctctgcct tgacaatacc 780
cgcaagccag tggatcagta tgaggactgc tacctagccc ggatcccttc tcatgctgtt 840
gtggctcgaa atggagatgg caaagaggac ttgatctggg agatcctcaa agtggctcag 900
gaacactttg gcaaaggcaa atcaaaagac ttccaactgt tcggctctcc tcttgggaaa 960
gacctgctgt ttaaggattc tgcctttggc tgttacgggg tgcccccaag gatggactac 1020
aggctgtacc tcggccacag ctatgtcact gccattcgaa atcagcggga aggcgtgtgc 1080
ccggaggcct ccatcgacag cgcgccagtg aaatggtgtg cactgagtca ccaagagaga 1140
gccaagtgtg atgagtggag cgtcacgagc aatgggcaga tagagtgtga gtcagcagag 1200

agcactgagg actgcattga caagattgtg aatggagaag cagatgccat gagcttggat 1260
ggaggtcatg cctacatagc aggccagtgt ggactagtgc ccgtcatggc agagaactat 1320
gatatctctt cgtgtacaaa cccacaatca gatgtctttc ctaaagggta ttatgccgtg 1380
gctgtggtga aggcatcaga ctccagcatc aactggaaca acctgaaagg caagaagtcc 1440
tgccatactg gagtagacag aaccgccggc tggaacatcc ctatgggcct gctgttcagc 1500
aggatcaacc actgcaagtt cgatgaattt ttcagtcaag gctgtgctcc tggctataag 1560
aagaattcca ccctctgtga cctgtgtatt ggcccagcaa aatgtgctcc gaacaacaga 1620
gagggatata atggttatac aggggctttc cagtgcctcg ttgagaaggg agacgtagcc 1680
tttgtgaagc accagactgt cctggaaaac acgaacggaa agaacactgc tgcatgggct 1740
aaggatctga agcaggaaga cttccagctg ctgtgccctg atggtaccaa gaagcctgta 1800
accgagttcg ccacctgcca cctggcccaa gctccaaacc atgttgtggt ctcacgaaaa 1860
gagaaggcag cccgggttag cactgtgctg actgcccaga aggatttatt ttggaaaggt 1920
gacaaggact gcactggcaa tttctgtttg ttccggtctt ccaccaagga ccttctgttc 1980
agagatgaca ccaagtgttt gactaaactt ccagaaggta ccacatatga agagtactta 2040
ggagcagagt acttgcaagc tgttggaaac ataaggaagt gttcaacctc acgactccta 2100
gaagcctgca ctttccacaa aagttaaaat ccaagaggtg ggtgccactg tggtggagga 2160
ggatgccccc gtgatccatg ggcttctcct ggcctccata gccctgagcg gctggggcta 2220
actgtgtccg tcttactgct tggtgttgtt accacataca cagagcacaa aataaaaaat 2280
gactgttgac ttt 2293

<210> 57
<211> 542
<212> PRT
<213> Rattus norvegicus

<220>
<223> NF-L

<400> 57
Met Ser Ser Phe Ser Tyr Glu Pro Tyr Phe Ser Thr Ser Tyr Lys Arg
1               5                   10                  15

Arg Tyr Val Glu Thr Pro Arg Val His Ile Ser Ser Val Arg Ser Gly
            20                  25                  30

Tyr Ser Thr Ala Arg Ser Ala Tyr Ser Ser Tyr Ser Ala Pro Val Ser
        35                  40                  45

Ser Ser Leu Ser Val Arg Arg Ser Tyr Ser Ser Ser Ser Gly Ser Leu
    50                  55                  60

Met Pro Ser Leu Glu Asn Leu Asp Leu Ser Gln Val Ala Ala Ile Ser
65                  70                  75                  80

Asn Asp Leu Lys Ser Ile Arg Thr Gln Glu Lys Ala Gln Leu Gln Asp
                85                  90                  95

Leu Asn Asp Arg Phe Ala Ser Phe Ile Glu Arg Val His Glu Leu Glu
            100                 105                 110

Gln Gln Asn Lys Val Leu Glu Ala Glu Leu Leu Val Leu Arg Gln Lys
        115                 120                 125

His Ser Glu Pro Ser Arg Phe Arg Ala Leu Tyr Glu Gln Glu Ile Arg
    130                 135                 140

Asp Leu Arg Leu Ala Ala Glu Asp Ala Thr Asn Glu Lys Gln Ala Leu
145                 150                 155                 160

Gln Gly Glu Arg Glu Gly Leu Glu Glu Thr Leu Arg Asn Leu Gln Ala
                165                 170                 175

Arg Tyr Glu Glu Glu Val Leu Ser Arg Glu Asp Ala Glu Gly Arg Leu
            180                 185                 190

Met Glu Ala Arg Lys Gly Ala Asp Glu Ala Ala Leu Ala Arg Ala Glu
        195                 200                 205

Leu Glu Lys Arg Ile Asp Ser Leu Met Asp Glu Ile Ala Phe Leu Lys
    210                 215                 220

Lys Val His Glu Glu Glu Ile Ala Glu Leu Gln Ala Gln Ile Gln Tyr
225                 230                 235                 240

Ala Gln Ile Ser Val Glu Met Asp Val Ser Ser Lys Pro Asp Leu Ser
                245                 250                 255

Ala Ala Leu Lys Asp Ile Arg Ala Gln Tyr Glu Lys Leu Ala Ala Lys
            260                 265                 270

Asn Met Gln Asn Ala Glu Glu Trp Phe Lys Ser Arg Phe Thr Val Leu
        275                 280                 285

Thr Glu Ser Ala Ala Lys Asn Thr Asp Ala Val Arg Ala Ala Lys Asp
    290                 295                 300

Glu Val Ser Glu Ser Arg Arg Leu Leu Lys Ala Lys Thr Leu Glu Ile
305                 310                 315                 320

Glu Ala Cys Arg Gly Met Asn Glu Ala Leu Glu Lys Gln Leu Gln Glu
                325                 330                 335

Leu Glu Asp Lys Gln Asn Ala Asp Ile Ser Ala Met Gln Asp Thr Ile
            340                 345                 350

Asn Lys Leu Glu Asn Glu Leu Arg Ser Thr Lys Ser Glu Met Ala Arg
        355                 360                 365

Tyr Leu Lys Glu Tyr Gln Asp Leu Leu Asn Val Lys Met Ala Leu Asp
    370                 375                 380

Ile Glu Ile Ala Ala Tyr Arg Lys Leu Leu Glu Gly Glu Glu Thr Arg
385                 390                 395                 400

Leu Ser Phe Thr Ser Val Gly Ser Ile Thr Ser Gly Tyr Ser Gln Ser
                405                 410                 415

Ser Gln Val Phe Gly Arg Ser Ala Tyr Ser Gly Leu Gln Ser Ser Ser
            420                 425                 430

Tyr Leu Met Ser Ala Arg Ala Phe Pro Ala Tyr Tyr Thr Ser His Val
        435                 440                 445

Gln Glu Glu Gln Ser Glu Val Glu Glu Thr Ile Glu Ala Thr Lys Ala
    450                 455                 460

Glu Glu Ala Lys Asp Glu Pro Pro Ser Glu Gly Glu Ala Glu Glu Glu
465                 470                 475                 480

Glu Lys Glu Lys Glu Glu Gly Glu Glu Glu Glu Gly Ala Glu Glu Glu
                485                 490                 495

Glu Ala Ala Lys Asp Glu Ser Glu Asp Ala Lys Glu Glu Glu Gly Gly

                500                 505                 510

Glu Gly Glu Glu Glu Asp Thr Lys Glu Ser Glu Glu Glu Glu Lys Lys
        515                 520                 525

Glu Glu Ser Ala Gly Glu Glu Gln Ala Ala Lys Lys Lys Asp
    530                 535                 540

<210> 58
<211> 2032
<212> DNA
<213> Rattus norvegicus

<220>
<223> NF-L

<400> 58
gaattccccc ggttcttctc tctaggtccc ctatctccgc cccgggcctg aggggcgcac 60
cgaccgccac catgagttcg ttcagctacg agccgtactt ttcgacctcc tacaagcggc 120
gctacgtgga gacgccccgg gtgcacatct ccagcgtgcg cagcggctac agcacggcgc 180
gctctgcgta ctccagctac tccgcgcccg tctcctcctc tctgtcggtg cgccgcagct 240
actcatccag ctccggctct ttgatgccca gcctggagaa cctcgatctg agccaggtag 300
ccgccatcag caacgacctc aagtctatcc gcacacagga gaaggcacag ctgcaggacc 360
tcaacgatcg cttcgccagc ttcatcgagc gcgtgcacga gctggagcag cagaacaagg 420
tcctggaagc cgagctgttg gtgctgcgcc agaagcactc agagccttcc cgcttccgcg 480
ccctgtatga gcaggagatc cgtgatctgc gactggcggc cgaagacgcc actaacgaga 540
agcaggcgct gcagggcgag cgcgaggggc tggaggagac tctgcgcaac ctgcaggctc 600
gctacgagga ggaggtgctg agccgcgagg acgccgaggg ccggctgatg gaagcccgca 660
aaggcgcgga tgaggctgcg ctcgcccgcg ccgagctgga gaagcgcatc gacagcctga 720
tggacgagat agccttcctg aaaaaggtgc acgaggaaga gatcgccgag ctgcaggctc 780
agatccagta tgctcagatc tccgtggaga tggacgtgtc ctccaagccc gacctctccg 840
ccgctctcaa ggacatccgc gctcagtacg agaagctggc cgccaagaat atgcagaatg 900
ccgaagagtg gttcaagagc cgcttcacgg tgctaaccga gagcgccgcc aagaacaccg 960
acgcagtgcg cgctgccaag gacgaggtgt cggaaagccg ccgcctactt aaggctaaga 1020
ccctggagat cgaagcctgc cggggtatga acgaagctct agagaagcag ctgcaggagc 1080
tggaggacaa gcagaatgca gacatcagcg ccatgcagga cacaatcaac aaactggaga 1140
atgagctgcg aagcacgaag agcgagatgg ccaggtacct gaaggagtac caggacctcc 1200
tcaatgtcaa gatggcattg gacattgaga ttgcagctta caggaaactc ttggaaggcg 1260
aagaaaccag gctcagtttc accagcgtgg gtagcataac cagcggctac tctcagagct 1320
cgcaggtctt tggccgttct gcttacagtg gcttgcagag cagctcctac ttgatgtctg 1380
ctcgagcatt cccagcctac tataccagcc acgtccagga agagcagtca gaggtggagg 1440
agaccattga ggctacgaaa gctgaggagg ccaaggatga gcccccctct gaaggagaag 1500
cagaagagga ggagaaggag aaagaggagg gggaggaaga ggaaggtgct gaggaggaag 1560
aagctgccaa ggatgagtct gaagatgcca aagaagaaga aggtggtgag ggtgaagagg 1620
aagacaccaa agagtcagaa gaggaagaga agaaagagga gagtgctggg gaggagcaag 1680
ctgctaagaa gaaagattga gccccattcc caactatccc aggaaaaaag tctccccaaa 1740
tcaggtcaac ctcatcacca aaccaaccag ttgagttcca gatcctatac agattaagaa 1800
gtcaatatat gtataattct gagatgactt aggttggaca ttcaatgttg tgctatgact 1860
ttcctcctta tgcagagtat ctgtttgctt gcagagtggc tttctggctt gctgccaacc 1920
tgtgcatggt ccatgcttat gagttcagga tctacggcaa tgtgaatcac acagatgttt 1980
acaataataa taataaaaaa accacacata cacaacatga ataaatgaat tc 2032

<210> 59
<211> 626
<212> PRT
<213> Rattus norvegicus

<220>
<223> MAG

<400> 59
Met Ile Phe Leu Thr Thr Leu Pro Leu Phe Trp Ile Met Ile Ser Ala
1               5                   10                  15

Ser Arg Gly Gly His Trp Gly Ala Trp Met Pro Ser Ser Ile Ser Ala
            20                  25                  30

Phe Glu Gly Thr Cys Val Ser Ile Pro Cys Arg Phe Asp Phe Pro Asp
        35                  40                  45

Glu Leu Arg Pro Ala Val Val His Gly Val Trp Tyr Phe Asn Ser Pro
    50                  55                  60

Tyr Pro Lys Asn Tyr Pro Pro Val Val Phe Lys Ser Arg Thr Gln Val
65                  70                  75                  80

Val His Glu Ser Phe Gln Gly Arg Ser Arg Leu Leu Gly Asp Leu Gly
                85                  90                  95

Leu Arg Asn Cys Thr Leu Leu Leu Ser Thr Leu Ser Pro Glu Leu Gly
            100                 105                 110

Gly Lys Tyr Tyr Phe Arg Gly Asp Leu Gly Gly Tyr Asn Gln Tyr Thr
        115                 120                 125

Phe Ser Glu His Ser Val Leu Asp Ile Ile Asn Thr Pro Asn Ile Val
    130                 135                 140

Val Pro Pro Glu Val Val Ala Gly Thr Glu Val Glu Val Ser Cys Met
145                 150                 155                 160

Val Pro Asp Asn Cys Pro Glu Leu Arg Pro Glu Leu Ser Trp Leu Gly
                165                 170                 175

His Glu Gly Leu Gly Glu Pro Thr Val Leu Gly Arg Leu Arg Glu Asp
            180                 185                 190

Glu Gly Thr Trp Val Gln Val Ser Leu Leu His Phe Val Pro Thr Arg
        195                 200                 205

Glu Ala Asn Gly His Arg Leu Gly Cys Gln Ala Ala Phe Pro Asn Thr
    210                 215                 220

Thr Leu Gln Phe Glu Gly Tyr Ala Ser Leu Asp Val Lys Tyr Pro Pro
225                 230                 235                 240

Val Ile Val Glu Met Asn Ser Ser Val Glu Ala Ile Glu Gly Ser His
                245                 250                 255

Val Ser Leu Leu Cys Gly Ala Asp Ser Asn Pro Pro Pro Leu Leu Thr
            260                 265                 270

Trp Met Arg Asp Gly Met Val Leu Arg Glu Ala Val Ala Glu Ser Leu
        275                 280                 285

Tyr Leu Asp Leu Glu Glu Val Thr Pro Ala Glu Asp Gly Ile Tyr Ala
    290                 295                 300

Cys Leu Ala Glu Asn Ala Tyr Gly Gln Asp Asn Arg Thr Val Glu Leu
305                 310                 315                 320

Ser Val Met Tyr Ala Pro Trp Lys Pro Thr Val Asn Gly Thr Val Val

                325                 330                 335

Ala Val Glu Gly Glu Thr Val Ser Ile Leu Cys Ser Thr Gln Ser Asn
            340                 345                 350

Pro Asp Pro Ile Leu Thr Ile Phe Lys Glu Lys Gln Ile Leu Ala Thr
        355                 360                 365

Val Ile Tyr Glu Ser Gln Leu Gln Leu Glu Leu Pro Ala Val Thr Pro
    370                 375                 380

Glu Asp Asp Gly Glu Tyr Trp Cys Val Ala Glu Asn Gln Tyr Gly Gln
385                 390                 395                 400

Arg Ala Thr Ala Phe Asn Leu Ser Val Glu Phe Ala Pro Ile Ile Leu
                405                 410                 415

Leu Glu Ser His Cys Ala Ala Ala Arg Asp Thr Val Gln Cys Leu Cys
            420                 425                 430

Val Val Lys Ser Asn Pro Glu Pro Ser Val Ala Phe Glu Leu Pro Ser
        435                 440                 445

Arg Asn Val Thr Val Asn Glu Thr Glu Arg Glu Phe Val Tyr Ser Glu
    450                 455                 460

Arg Ser Gly Leu Leu Leu Thr Ser Ile Leu Thr Leu Arg Gly Gln Ala
465                 470                 475                 480

Gln Ala Pro Pro Arg Val Ile Cys Thr Ser Arg Asn Leu Tyr Gly Thr
                485                 490                 495

Gln Ser Leu Glu Leu Pro Phe Gln Gly Ala His Arg Leu Met Trp Ala
            500                 505                 510

Lys Ile Gly Pro Val Gly Ala Val Val Ala Phe Ala Ile Leu Ile Ala
        515                 520                 525

Ile Val Cys Tyr Ile Thr Gln Thr Arg Arg Lys Lys Asn Val Thr Glu
    530                 535                 540

Ser Pro Ser Phe Ser Ala Gly Asp Asn Pro His Val Leu Tyr Ser Pro
545                 550                 555                 560

Glu Phe Arg Ile Ser Gly Ala Pro Asp Lys Tyr Glu Ser Glu Lys Arg
                565                 570                 575

Leu Gly Ser Glu Arg Arg Leu Leu Gly Leu Arg Gly Glu Pro Pro Glu
            580                 585                 590

Leu Asp Leu Ser Tyr Ser His Ser Asp Leu Gly Lys Arg Pro Thr Lys
        595                 600                 605

Asp Ser Tyr Thr Leu Thr Glu Glu Leu Ala Glu Tyr Ala Glu Ile Arg
    610                 615                 620

Val Lys
625

<210> 60
<211> 2475

<212> DNA
<213> Rattus norvegicus

<220>
<223> MAG

<400> 60
cagaagccag accatccaac cttctgtatc agtgctcctc gtcgcctcac tgtacttcac 60
ggaagagact tggttgactg gccacttgga gcggaatcag gagacattcc caactcaggg 120
agactgaggt gagggcccta gctcgcccac ttgctggaca agatgatatt ccttaccacc 180
ctgcctctgt tttggataat gatttcagct tctcgagggg ggcactgggg tgcctggatg 240
ccctcgtcca tctcagcctt cgagggcacg tgtgtctcca tcccctgccg tttcgacttc 300
ccggatgagc tcagaccggc tgtggtacat ggcgtctggt atttcaacag tccctacccc 360
aagaactacc cgccagtggt cttcaagtcc cgcacacaag tggtccacga gagcttccag 420
ggccgtagcc gcctgttggg agacctgggc ctacgaaact gcaccctgct tctcagcacg 480
ctgagccctg agctgggagg gaaatactat ttccgaggtg acctgggcgg ctacaaccag 540
tacaccttct cggagcacag cgtcctggac atcatcaaca cccccaacat cgtggtgccc 600
ccagaagtgg tggcaggaac ggaagtagag gtcagctgca tggtgccgga caactgccca 660
gagctgcgcc ctgagctgag ctggctgggc cacgaggggc taggggagcc cactgttctg 720
ggtcggctgc gggaggatga aggcacctgg gtgcaggtgt cactgctaca cttcgtgcct 780
actagagagg ccaacggcca ccgtctgggc tgtcaggctg ccttccccaa caccaccttg 840
cagttcgagg gttacgccag tctggacgtc aagtaccccc cggtgattgt ggagatgaat 900
tcctctgtgg aggccattga gggctcccat gtcagcctgc tctgtggggc tgacagcaac 960
ccgccaccgc tgctgacttg gatgcgggat gggatggtgt tgagggaggc agttgctgag 1020
agcctgtacc tggatctgga ggaggtgacc ccagcagagg acggcatcta tgcttgcctg 1080
gcagagaatg cctatggcca ggacaaccgc acggtggagc tgagcgtcat gtatgcacct 1140
tggaagccca cagtgaatgg gacggtggtg gcggtagagg gggagacagt ctccatcctg 1200
tgttccacac agagcaaccc ggaccctatt ctcaccatct tcaaggagaa gcagatcctg 1260
gccacggtca tctatgagag tcagctgcag ctggaactcc ctgcagtgac gcccgaggac 1320
gatggggagt actggtgtgt agctgagaac cagtatggcc agagagccac cgccttcaac 1380
ctgtctgtgg agtttgctcc cataatcctt ctggaatcgc actgtgcagc ggccagagac 1440
accgtgcagt gcctgtgtgt ggtaaaatcc aacccggaac cctccgtggc ctttgagctg 1500
ccttcccgca acgtgactgt gaacgagaca gagagggagt ttgtgtactc agagcgcagc 1560
ggcctcctgc tcaccagcat cctcacgctc cggggtcagg cccaagcccc accccgcgtc 1620
atttgtacct ccaggaacct ctacggcacc cagagcctcg agctgccttt ccagggagca 1680
caccgactga tgtgggccaa aatcggccct gtgggtgctg tggtcgcctt tgccatcctg 1740
attgccattg tctgctacat cacccagaca agaagaaaaa agaacgtcac agagagcccc 1800
agcttctcag cgggagacaa ccctcatgtc ctgtacagcc ccgaattccg aatctctgga 1860
gcacctgata agtatgagag tgagaagcgc ctggggtccg agaggaggct gctgggcctt 1920
aggggggaac ccccagaact ggacctcagt tattcccact cagacctggg gaaacgaccc 1980
accaaggaca gctacaccct gacagaggag ctggctgagt acgcagaaat ccgagtcaag 2040
tgaggaagct gggggctggc cctgtggctc accccccatc aggaccctcg cttggccccc 2100
actggccgtg ggctcccttt ctcttgagag tggtaggggt gggggcggga aggggcgggg 2160
caggaaacag tgaggtctta ggggcccggc ctcccctcct tcccggctgc tcctctctgc 2220
caacatcctg cacctatgtt acagctccct ctcccctcct tttaacctca gctgttgaga 2280
ggggtgctct gtctgtccat gttatttatt gttatcctgg tctcctgtcc ccttacccgg 2340
ccccaggacc tgtacaaaag ggacatgaaa taaatgtcct aatgacaagt gccagtctag 2400
acccatcctt tggaggaaag gggcatatta gtaatacttt tctcgttgct gtaacaaaat 2460
actggacaaa aacac 2475

<210> 61
<211> 845
<212> PRT
<213> Rattus norvegicus

<220>
<223> NF-M protein

<400> 61
Met Ser Tyr Thr Leu Asp Ser Leu Gly Asn Pro Ser Ala Tyr Arg Arg
1               5                   10                  15

Val Pro Thr Glu Thr Arg Ser Ser Phe Ser Arg Val Ser Gly Ser Pro
            20                  25                  30

Ser Ser Gly Phe Arg Ser Gln Ser Trp Ser Arg Gly Ser Pro Ser Thr
        35                  40                  45

Val Ser Ser Ser Tyr Lys Arg Ser Ala Leu Ala Pro Arg Leu Ala Tyr
    50                  55                  60

Ser Ser Ala Met Leu Ser Ser Ala Glu Ser Ser Leu Asp Phe Ser Gln
65                  70                  75                  80

Ser Ser Ser Leu Leu Asn Gly Gly Ser Gly Gly Asp Tyr Lys Leu Ser
                85                  90                  95

Arg Ser Asn Glu Lys Glu Gln Leu Gln Gly Leu Asn Asp Arg Phe Ala
            100                 105                 110

Gly Tyr Ile Glu Lys Val His Tyr Leu Glu Gln Gln Asn Lys Glu Ile
        115                 120                 125

Glu Ala Glu Ile His Ala Leu Arg Gln Lys Gln Ala Ser His Ala Gln
    130                 135                 140

Leu Gly Asp Ala Tyr Asp Gln Glu Ile Arg Glu Leu Arg Ala Thr Leu
145                 150                 155                 160

Glu Met Val Asn His Glu Lys Ala Gln Val Gln Leu Asp Ser Asp His
                165                 170                 175

Leu Glu Glu Asp Ile His Arg Leu Lys Glu Arg Phe Glu Glu Glu Ala
            180                 185                 190

Arg Leu Arg Asp Asp Thr Glu Ala Ala Ile Arg Ala Val Arg Lys Asp
        195                 200                 205

Ile Glu Glu Ser Ser Met Val Lys Val Glu Leu Asp Lys Lys Val Gln
    210                 215                 220

Ser Leu Gln Asp Glu Val Ala Phe Leu Arg Ser Asn His Glu Glu Glu
225                 230                 235                 240

Val Ala Asp Leu Leu Ala Gln Ile Gln Ala Ser His Ile Thr Val Glu
                245                 250                 255

Arg Lys Asp Tyr Leu Lys Thr Asp Ile Ser Thr Ala Leu Lys Glu Ile
            260                 265                 270

Arg Ser Gln Leu Glu Cys His Ser Asp Gln Asn Met His Gln Ala Glu
        275                 280                 285

Glu Trp Phe Lys Cys Arg Tyr Ala Lys Leu Thr Glu Ala Ala Glu Gln
    290                 295                 300

Asn Lys Glu Ala Ile Arg Ser Ala Lys Glu Glu Ile Ala Glu Tyr Arg
305                 310                 315                 320

Arg Gln Leu Gln Ser Lys Ser Ile Glu Leu Glu Ser Val Arg Gly Thr
                325                 330                 335

Lys Glu Ser Leu Glu Arg Gln Leu Ser Asp Ile Glu Glu Arg His Asn

            340                 345                 350

His Asp Leu Ser Ser Tyr Gln Asp Thr Ile Gln Gln Leu Glu Asn Glu
        355                 360                 365

Leu Arg Gly Thr Lys Trp Glu Met Ala Arg His Leu Arg Glu Tyr Gln
    370                 375                 380

Asp Leu Leu Asn Val Lys Met Ala Leu Asp Ile Glu Ile Ala Ala Tyr
385                 390                 395                 400

Arg Lys Leu Leu Glu Gly Glu Glu Thr Arg Phe Ser Thr Phe Ser Gly
                405                 410                 415

Ser Ile Thr Gly Pro Leu Tyr Thr His Arg Gln Pro Ser Val Thr Ile
            420                 425                 430

Ser Ser Lys Ile Gln Lys Thr Lys Val Glu Ala Pro Lys Leu Lys Val
        435                 440                 445

Gln His Lys Phe Val Glu Glu Ile Ile Glu Glu Thr Lys Val Glu Asp
    450                 455                 460

Glu Lys Ser Glu Met Glu Asp Ala Leu Thr Val Ile Ala Glu Glu Leu
465                 470                 475                 480

Ala Ala Ser Ala Lys Glu Glu Lys Glu Glu Ala Glu Glu Lys Glu Glu
                485                 490                 495

Glu Pro Glu Val Lys Ser Pro Val Lys Ser Pro Glu Ala Lys Glu Glu
            500                 505                 510

Glu Glu Gly Glu Lys Glu Glu Glu Glu Glu Gly Gln Glu Glu Glu Glu
        515                 520                 525

Glu Glu Asp Glu Gly Val Lys Ser Asp Gln Ala Glu Glu Gly Gly Ser
    530                 535                 540

Glu Lys Glu Gly Ser Ser Glu Lys Asp Glu Gly Glu Gln Glu Glu Glu
545                 550                 555                 560

Gly Glu Thr Glu Ala Glu Gly Glu Gly Glu Glu Ala Glu Ala Lys Glu
                565                 570                 575

Glu Lys Lys Thr Glu Gly Lys Val Glu Glu Met Ala Ile Lys Glu Glu
            580                 585                 590

Ile Lys Val Glu Lys Pro Glu Lys Ala Lys Ser Pro Val Pro Lys Ser
        595                 600                 605

Pro Val Glu Glu Val Lys Pro Lys Pro Glu Ala Lys Ala Gly Lys Asp
    610                 615                 620

Glu Gln Lys Glu Glu Glu Lys Val Glu Glu Lys Lys Glu Val Ala Lys
625                 630                 635                 640

Glu Ser Pro Lys Glu Glu Lys Val Glu Lys Lys Glu Glu Lys Pro Lys
                645                 650                 655

Asp Val Pro Asp Lys Lys Lys Ala Glu Ser Pro Val Lys Glu Lys Ala
            660                 665                 670

Val Glu Glu Met Ile Thr Ile Thr Lys Ser Val Lys Val Ser Leu Glu
      675                 680                 685

Lys Asp Thr Lys Glu Glu Lys Pro Gln Gln Gln Glu Lys Val Lys Glu
    690                 695                 700

Lys Ala Glu Glu Glu Gly Gly Ser Glu Glu Glu Val Gly Asp Lys Ser
705                 710                 715                 720

Pro Gln Glu Ser Lys Lys Glu Asp Ile Ala Ile Asn Gly Glu Val Glu
                725                 730                 735

Gly Lys Glu Glu Glu Glu Gln Glu Thr Gln Glu Lys Gly Ser Gly Gln
            740                 745                 750

Glu Glu Glu Lys Gly Val Val Thr Asn Gly Leu Asp Val Ser Pro Ala
        755                 760                 765

Glu Glu Lys Lys Gly Glu Asp Arg Ser Asp Asp Lys Val Val Val Thr
    770                 775                 780

Lys Lys Val Glu Lys Ile Thr Ser Glu Gly Gly Asp Gly Ala Thr Lys
785                 790                 795                 800

Tyr Ile Thr Lys Ser Val Thr Val Thr Gln Lys Val Glu Glu His Glu
                805                 810                 815

Glu Thr Phe Glu Glu Lys Leu Val Ser Thr Lys Lys Val Glu Lys Val
            820                 825                 830

Thr Ser His Ala Ile Val Lys Glu Val Thr Gln Gly Asp
        835                 840                 845


<210> 62
<211> 2717
<212> DNA
<213> Rattus norvegicus

<220>
<223> NF-M protein

<400> 62
ggcctccaag atgagctaca cgctggactc gctgggcaac ccgtccgcct accggcgcgt 60
tccaaccgag acccggtcca gcttcagtcg tgtgagcggt tccccgtcca gcggcttccg 120
ctcgcagtcc tggtcccgcg gctcgcccag caccgtgtcc tcctcctaca agcgcagcgc 180
gctcgccccg cgcctcgcct acagctcggc tatgctcagc tcggccgaga gcagcctcga 240
cttcagccag tcctcttcgc tgcttaacgg cggctccggc ggcgactaca agctgtcccg 300
ctcaaacgag aaagagcagc tgcaggggct gaacgaccgt ttcgccggct acatcgagaa 360
agtgcactac ttggaacaac agaacaagga gatcgaggca gagatccacg cgctgcggca 420
gaagcaggcc tcgcacgccc agctgggtga cgcttacgac caggagatcc gagagctgcg 480
cgccaccctg gagatggtga atcacgagaa ggctcaagtg cagctggact ctgatcactt 540
ggaggaagac atccaccggc tcaaggagcg cttcgaggag gaggcgcggc tgcgggacga 600
cacggaggct gccatccggg cggtgcgcaa agacatagag gagtcgtcga tggttaaggt 660
ggagctggac aagaaggttc agtcgctgca ggatgaggtg gccttcctgc ggagcaatca 720
cgaagaggag gtggccgacc tgctggccca gatccaggcg tcgcacatca ccgtagagcg 780
caaagactac ctgaagacag acatctccac ggcgctgaaa gagatccgct cccagctcga 840
gtgtcactcc gaccagaaca tgcaccaggc cgaagagtgg ttcaaatgcc gctacgccaa 900
gctcaccgag gcggccgagc agaacaagga ggccatccgc tccgctaaag aagagatcgc 960
cgagtaccgg cgccagctgc agtccaagag cattgagctc gagtcggtgc gaggcactaa 1020
ggagtccctg gaacggcagc tcagcgacat cgaggagcgc cacaaccacg acctcagcag 1080
ctaccaggac accatccagc agctggaaaa tgagcttcgg ggaacaaagt gggaaatggc 1140

tcgtcatttg cgagaatacc aggatctcct taacgtcaag atggctctgg acatcgagat 1200
cgccgcatat aggaaactac tggagggtga agagaccaga tttagcacat tttcaggaag 1260
catcactggg cctctgtaca cacaccgaca gccctcagtc acaatatcca gtaagattca 1320
gaagaccaaa gtcgaggccc ccaagctcaa ggtccaacac aaatttgtgg aggagatcat 1380
tgaggagact aaagtggaag atgagaagtc agaaatggaa gacgccctca cagtcattgc 1440
agaggaattg gcagcctctg ccaaagagga gaaagaagag gcagaagaaa aggaagagga 1500
accggaagtg aagtctcccg tgaagtctcc tgaggctaag gaagaggagg aaggggaaaa 1560
ggaggaagaa gaggaaggcc aagaggaaga agaggaggaa gatgaaggtg tcaagtcaga 1620
ccaggcagaa gagggaggat ctgagaagga aggctcgagt gaaaaggatg aaggtgagca 1680
agaagaagaa ggggaaactg aggcagaagg tgaaggagag gaagcagaag ctaaggagga 1740
aaagaaaaca gagggaaagg tcgaggaaat ggctatcaag gaggaaatca aggtcgagaa 1800
gcccgagaaa gccaagtccc ctgtgccaaa atcaccggtg gaagaagtaa agccaaaacc 1860
agaagccaaa gccggaaagg atgagcagaa ggaggaagag aaagttgagg agaagaagga 1920
ggtagccaag gaatcaccca aggaagagaa ggtggagaaa aaggaggaga agccaaaaga 1980
tgtcccagat aaaaagaagg ctgagtcccc agtgaaagaa aaggccgtag aggaaatgat 2040
caccattact aagtcggtaa aggtgagcct ggagaaagac accaaagagg agaagcctca 2100
gcagcaggag aaggtgaagg agaaggcaga ggaggagggg ggtagtgagg aggaagtggg 2160
tgacaaaagc ccgcaagaat ccaagaagga agacatagct atcaatgggg aggtggaagg 2220
aaaagaggag gaggagcagg aaactcagga gaagggcagt gggcaagagg aggagaaagg 2280
ggtggtcact aatggcttag atgtgagccc tgcggaggaa aagaaagggg aggatagaag 2340
tgatgacaaa gtggtggtga ccaagaaggt agaaaaaatc accagcgagg gaggcgatgg 2400
tgctaccaaa tacatcacca aatctgttac tgtcactcaa aaggttgaag agcatgagga 2460
gacctttgag gagaagctgg tgtcaactaa aaaggtagaa aaggtcactt cacatgccat 2520
agtcaaggaa gtcacccagg gtgactaaga tcggagtcca ttgcaaaagg ttaagccata 2580
caacaatttc aaaatgcatg tgattgacag cttcaaaaca gaatgggttc tcccatgagg 2640
gctccagaca ttgtattttc ctttgtgcaa tatgagggaa ctgcatgcaa gctcagggtg 2700
cctcctcagt ccttggg 2717

<210> 63
<211> 707
<212> PRT
<213> Rattus norvegicus

<220>
<223> Neurodegeneration associated protein 1

<400> 63
Met Ser Gln Tyr Thr Glu Lys Glu Pro Ser Val Met Asp Gln Asp Ser
1               5                   10                  15

Ser Lys Ala Ala Trp Pro Arg Ala Ala Gly Gly Tyr Gln Thr Ile Thr
            20                  25                  30

Gly Arg Arg Tyr Gly Arg Arg His Ala Tyr Val Ser Phe Lys Pro Cys
        35                  40                  45

Met Thr Arg His Glu Arg Ser Leu Gly Arg Ala Gly Asp Asp Tyr Glu
    50                  55                  60

Val Leu Glu Leu Asp Asp Val Ala Lys Glu Asn Thr Ala Gly Ser Ser
65                  70                  75                  80

Ser Leu Asp Gln Val His Pro Ser Leu Pro Ser Glu Thr Thr Val Glu
                85                  90                  95

Lys Ser Glu Thr Glu Ile Pro Thr Cys Gly Pro Ala Leu Asn Gln Ser
            100                 105                 110

Thr Glu Ser Asn Pro Ser Val Ala Thr Val Cys His Ser Glu Glu Val
        115                 120                 125

Arg Glu Thr Leu Asp Ser Ser Thr Asn Leu Gln Asn His Ala Glu Arg
    130                 135                 140

Glu Cys Thr Pro Ala Val Cys Asn Ala Ser Ser Val Gln Asn Gly Ile
145                 150                 155                 160

Val Leu Val His Thr Asp Ser Tyr Asp Pro Asp Ser Lys His Asp Glu
                165                 170                 175

Asn Asp Ser Leu Gln Leu Cys Ala Gln Ala Val Glu Gly Gly Arg Arg
            180                 185                 190

Gln Lys Val Leu Gly Asn Ala Val Phe Glu Leu Glu Asn Gly Glu Val
        195                 200                 205

Glu Arg Tyr Ala Asp Leu Cys Pro Ser Val Pro Ser Leu Ser Gly Glu
    210                 215                 220

Ile Arg Glu Glu Ser Glu Glu Leu Gly Ser Ala Leu Leu Glu Lys Asn
225                 230                 235                 240

Ser Ala Gly Asp Ala Glu Ala Val His Gln Asp Gly Gln Glu Phe Gln
                245                 250                 255

Arg Ser Ser Glu Asp Gly Ile Val Arg Lys Arg Arg Gln Asp Asp Thr
            260                 265                 270

Asp Gln Gly Arg Gln Thr Glu Asn Ser Thr Glu Asp Ala Asp Cys Val
        275                 280                 285

Pro Gly His Val Glu Gln Asn Thr Ser Glu Arg Ala Asn His His Gly
    290                 295                 300

Ser Ser Pro Glu Gln Val Val Arg Pro Lys Val Arg Lys Val Ile Ser
305                 310                 315                 320

Ser Ser Gln Val Asp Gln Glu Ser Gly Phe Asn Arg His Glu Ala Lys
                325                 330                 335

Gln Arg Ser Val Gln Arg Trp Arg Glu Ala Leu Glu Val Glu Glu Cys
            340                 345                 350

Ser Ser Asp Asp Pro Ile Ile Lys Cys Asp Asp Tyr Asp Gly Asp His
        355                 360                 365

Asp Cys Met Phe Leu Thr Pro Ser Tyr Ser Arg Val Thr Pro Arg Glu
    370                 375                 380

Ala Glu Arg His Arg Ala Thr Ala Glu Asn Gly Ala Thr Ala Ser Gly
385                 390                 395                 400

Arg Gln Glu Ala Arg Glu Asn Ala Phe Trp Asn Ala Cys Gly Glu Tyr
                405                 410                 415

Tyr Gln Leu Phe Asp Lys Asp Glu Asp Ser Ser Glu Cys Ser Asp Gly
            420                 425                 430

Glu Trp Ser Ala Ser Leu Pro His Arg Phe Ser Gly Thr Glu Lys Asp
        435                 440                 445

Gln Ser Ser Ser Asp Glu Ser Trp Glu Thr Leu Pro Gly Lys Asp Glu
    450                 455                 460

Asn Glu Pro Glu Leu Gln Ser Asp Ser Ser Gly Pro Glu Glu Glu Asn
465                 470                 475                 480

Gln Glu Leu Ser Leu Gln Glu Gly Glu Gln Thr Ser Leu Glu Glu Gly
                485                 490                 495

Glu Ile Pro Trp Leu Gln Tyr Asn Glu Val Asn Glu Ser Ser Ser Asp
            500                 505                 510

Glu Gly Asn Glu Pro Ala Asn Glu Phe Ala Gln Pro Glu Ala Phe Met
        515                 520                 525

Leu Asp Gly Asn Asn Asn Leu Glu Asp Asp Ser Ser Val Ser Glu Asp
    530                 535                 540

Leu Asp Val Asp Trp Ser Leu Phe Asp Gly Phe Ala Asp Gly Leu Gly
545                 550                 555                 560

Val Ala Glu Ala Ile Ser Tyr Val Asp Pro Gln Phe Leu Thr Tyr Met
                565                 570                 575

Ala Leu Glu Glu Arg Leu Ala Gln Ala Met Glu Thr Ala Leu Ala His
            580                 585                 590

Leu Glu Ser Leu Ala Val Asp Val Glu Val Ala Asn Pro Pro Ala Ser
        595                 600                 605

Lys Glu Ser Ile Asp Gly Leu Pro Glu Thr Leu Val Leu Glu Asp His
    610                 615                 620

Thr Ala Ile Gly Gln Glu Gln Cys Cys Pro Ile Cys Cys Ser Glu Tyr
625                 630                 635                 640

Ile Lys Asp Asp Ile Ala Thr Glu Leu Pro Cys His His Phe Phe His
                645                 650                 655

Lys Pro Cys Val Ser Ile Trp Leu Gln Lys Ser Gly Thr Cys Pro Val
            660                 665                 670

Cys Arg Arg His Phe Pro Pro Ala Val Ile Asp Ala Ser Ala Ala Ala
        675                 680                 685

Ser Ser Glu Pro Asp Leu Asp Ala Ser Pro Ala Asn Asp Asn Ala Glu
    690                 695                 700

Glu Ala Pro
705

<210> 64
<211> 4758
<212> DNA
<213> Rattus norvegicus

<220>
<223> Neurodegeneration associated protein 1

<400> 64
tggcgggtgg aggatcgagc gctgttctcg ctctggagcc gctgcacatc tcggaatcat 60
ttgcagctta ttcactgtgt aaggaaaaac caaattacat ccataattct attgaactgg 120
aagcatagac gctgccatat atgtcacagt atacagaaaa agaaccatca gtaatggatc 180

aagactccag caaggctgcg tggcctagag cagcaggagg ataccagacg attacaggca 240
ggaggtacgg aaggagacat gcgtatgtca gttttaagcc atgtatgacc aggcatgaac 300
ggagcttggg tcgggctggc gatgactatg aagttctgga actggatgac gttgccaagg 360
aaaacaccgc aggttccagt tcattggatc aagtccatcc ttctttaccc agtgaaacta 420
cagttgaaaa aagtgaaaca gaaattccta cttgtggtcc agcactgaat caaagcacgg 480
agagcaaccc atccgttgcc acagtgtgtc atagtgagga agtcagggag accttagaca 540
gcagtacgaa tcttcagaat cacgctgaga gagagtgtac gccagcagtt tgtaatgcct 600
caagtgtcca gaatggaatt gtgttggttc atactgactc ttatgatcca gacagcaagc 660
atgatgagaa tgactctctt caactttgtg cccaagctgt ggaaggtggt agacgtcaga 720
aggtattagg caatgcagtc tttgagctgg aaaatggaga ggtagagaga tatgctgatc 780
tgtgtccctc agttccctct ctcagtggtg aaataaggga ggagtctgaa gagctaggtt 840
cagcactctt agagaaaaat tctgctggcg atgcggaggc tgtccatcag gatgggcagg 900
aatttcagag gtcttctgaa gacggcattg ttagaaagag gcgacaagat gataccgatc 960
agggaagaca gacagaaaat tcaactgaag atgcagactg tgttccaggc catgttgaac 1020
aaaatactag tgagagagcc aatcaccatg gaagttctcc tgaacaggta gtgaggccca 1080
aagttagaaa agtgatcagt tcaagccagg tggaccaaga gagcggtttt aataggcacg 1140
aggctaagca aagaagtgtt cagaggtgga gagaggctct ggaagttgag gaatgcagtt 1200
cagatgaccc tataatcaag tgtgacgatt atgatggaga ccatgactgc atgttcctaa 1260
ccccatccta ctcaagagtt acgccaaggg aagcagaacg tcaccgtgcg acagcagaaa 1320
atggagccac agcttcagga aggcaagagg ctcgggaaaa tgccttttgg aatgcctgtg 1380
gagagtatta ccagctcttt gacaaagacg aagacagttc agagtgcagt gatggggaat 1440
ggtctgcttc tctgcctcac cgattttctg gcacagaaaa agaccagtcc tcaagcgatg 1500
aaagctggga aactctgcca ggaaaagatg agaatgaacc tgagctacag agtgatagta 1560
gtggccctga ggaagaaaac caagaattgt ctcttcagga gggagaacag acgtccttgg 1620
aggaggggga gattccctgg ttacagtaca atgaggtcaa tgagagcagc agcgatgaag 1680
ggaacgagcc tgccaatgag tttgcacaac cagaagcttt catgttggat gggaacaaca 1740
acctggagga cgactcgagc gtgagtgaag acctggatgt ggactggagc ctatttgatg 1800
gttttgcgga tggacttggt gttgctgaag ctatttccta cgtggatcct cagttcctca 1860
cctacatggc actagaagaa cgcttagccc aggctatgga gactgcactg gcacacttag 1920
agtctcttgc tgtagacgtt gaggtggcta acccacctgc cagtaaggaa agcatcgacg 1980
gacttccaga aacccttgtc ctagaagatc acaccgctat tggtcaggag cagtgctgtc 2040
ccatctgctg cagtgagtac attaaggatg acatcgcaac agagctgccc tgccatcact 2100
tctttcataa gccttgtgtc tccatttggc tacagaagtc cggaacgtgc cctgtgtgcc 2160
gccgccactt cccacctgca gtgattgacg catctgcagc tgcttcctct gaaccagacc 2220
ttgatgcctc gcctgcaaac gacaatgctg aggaagcccc gtaagccgtc acggggaaat 2280
gagatcgaaa ttctatcaca gtaaatctgc aaattccttc taaatctgat gtgcaaataa 2340
ttatatataa atatatttaa aatgctctat atagtatatg ccgtagttta gaaagagaat 2400
attaaccttt ctaaactgaa tttaggtttg cagaagatac taaacatttt caagctgaat 2460
gttgaagcag tgcatccatt ttctttagtt gaacatggtg tccattgtaa cgtcagtagt 2520
catctctgtg gcatacgttt ctgttttgtc tgaagtgctg ccactaagag atcagaatta 2580
agctctcctt tccacatcag atgtgaaagg agagcttaat tctgatctct gtggcatacg 2640
tttctgtttt gtctgaagtg ctgccactaa gagatcagaa ttaagctctc ctttccacat 2700
cagatgtgaa aactgtgatc acaacagtag tacagtttgg tttcattgaa aataaactga 2760
attctaaagc atgctttttc actggtccct ttgcttttgc tacttcgaga cctcttggtt 2820
tatataacac tgaggttaag attaaagctt ttcagaatgc caggcaaaga ctagagtttt 2880
gacccgaaca cacacacaaa ataaaaaaat aaaaaataaa ttacatctta gatgtaaact 2940
tcctgaaact gtcatgtaat aaactattca gtgaacgttg gatgtttaaa ggctagtaaa 3000
gcatgtgaat tatctgatgt gcttcatgct cggcacagtc tcatgagctt ggcctatgaa 3060
acactgtcta agccctcatg cagtctgctt ctacttgtta aacactgaag gtccagacca 3120
gtcttggcat ctgattaaga aattggatgt agttagctaa ggtagtttgg gttctacttt 3180
aggagatctg aagatttgca catggccatg tctgttacag catcacccca gattagaaag 3240
gcacagcttt taggtggtca cacgagtagg caagtctgtg cagacgctgg tgtgataggt 3300
agcttacagg tgagccctga catcagcaca gggacaacgg ctcagcaggc ccagcactgg 3360
agcccatccg cgtgcacatc tacctgccct gctcatccag ttcgggaata tttctatagc 3420
ttcccaaatc agcttcatct gatttgtgta atatatctga gtatttatag tctttctttt 3480
ttctccagtc ttaaaaataa atgaattttc actgttggca catttgaggc ttaaatataa 3540
gaaacctaac acttgcattc tgatttttgc atatattgta aatgtggctg gtatttacag 3600
cgaatcctgt gtgtcctttt atgggtaaaa caaaagtgaa cattgcatgc aagtaatgtg 3660
gtgaatttgt aatttagggg ttttctgggg cttctgtgac tgtggatgct taaattcagg 3720
ccttaatgtt gctctagcta gcatgtttcc ttctaatgta catagtcagc ctgtatccac 3780
taatctctgc ttgtcttctc ctccgtggtc ttcacaccgt atccactgat ggtcagttag 3840
tgtcagaggt cacccaggtt agcatcagtt cccatgtgtg ccgtgcggat tgtgagccgt 3900

gtggttgctt ggttggtttt cttttatgta ctttgattag ctgtggcact tactgagtaa 3960
acttacgttg ctgcagactg ctgtgtaaca agccacgttt tgtgtttagt cagtattaca 4020
cacgggacct tgttttattc ttctagatag cagctgtccc aaagaaaata tttcttcttt 4080
gcctattaag atttagctat tatctgccag ttgttaaggg gtttgattcc aaactcaacc 4140
agagttgaac gtaagaaagc tgctgtactc ttgctgtcca tctgttacgt cctccgttgt 4200
tggctcctga ctacgtaccc acggctgctg aaggagactt gttttaattt gttttaaaaa 4260
ggaaaatgaa gttttaggac tttgatggtg gggaagctga caagttcaga ccccagtctg 4320
tcatttgctc ttagcttgga tccttttgaa aagttaagaa tatatgaagg taaattagaa 4380
aaagtaaaaa tattgataaa atgtcattta tcttatgtct attttatgtt gttatttggc 4440
ctattaattt taattttttg aacattttct tatttcttgt aatatgaatg ccgatattta 4500
aagtttgact cacttgggtt tcttttgtgt ttcttagttg ttatctctaa gcctaactaa 4560
cttttaagat tatttgtgat atggatttat ataagctgtt aaatatatat gagctgttaa 4620
aatggaatgc aaggttttcc aaagaccagg tctaactgta atgattggtt tattgttcta 4680
caatcccagc ccggcatttt ccgtgtaaat cataaacaat aaacaggata tactcagtgt 4740
tcatttctaa aaaaaaaa 4758

<210> 65
<211> 1488
<212> DNA
<213> Rattus norvegicus

<220>
<223> Brain S-100 protein beta-subunit

<400> 65
aagtccacac ccagtcctct ctggaggaag aaaagggagc ttctctgtct accctcctag 60
tccttggaca ccgaagccag agaggactcc ggcggcaaaa ggtgaccagg agcctccggg 120
atgtctgagc tggagaaggc catggttgcc ctcattgatg tcttccatca gtattcaggg 180
agagagggtg acaagcacaa gctgaagaag tcagaactga aggagctcat caacaacgag 240
ctctctcact tcctggagga aatcaaagag caggaagtgg tggacaaagt gatggagacg 300
ctggacgaag atggggatgg ggagtgtgac ttccaggagt ttatggcctt cgtctccatg 360
gtgaccacag cctgtcatga gttctttgaa catgagtgag acaaaaaaaa aaaaaaaaaa 420
gtggctgagc cgtttccccg tggcagacat gagggccacg agaggaggca cggcagaagg 480
ctcgtgggct ggaaggagct gcgctctcta gacgcatata actaattagg aagcttgatt 540
tgcttcaggg atgaaactct gaccccgttc ccaagggctg ctttaagtta gcacttcgtt 600
tctgctacac taggtattcc tgtgagctga atggtcccgg gaactattga taagagtcac 660
tgagggacga aatcaacact ctgtgggtat agcactggtt gtagaccacc atgctcctgt 720
ggaagggtca cctgtaagaa tcaaggcaga ctaccaatag cacctccgtt ggacagcttt 780
cttaggtgta atgtatgctg tccatgcatc tacagaccca cagctggatc cactgccacc 840
cgaagaggtt ggctcgccct tacaactgct tgtcctctgt gcaaacgatg ccccggaaag 900
ttagacctat cacccacacc ctcccaccac ccccaggcca aaaggacagc ccacccaagt 960
cccctccccc acagcgaatc gcggtttgtt accaagtacg tatttgacat caacagttcc 1020
aactggtgga acgattagat ctcgcacact aagtattagc accctaactc acgaccgaga 1080
atcaaaattc tgctcagtag acgtctcctt tcaggatgac accattgtcc ccataggaca 1140
cggacagagg agggcactgg agagagtgtc aggtcttttt ctagctgtat cttcctctct 1200
ccctctgctg cccataatgt gagtgaccct ctagggtgag acttgcaggg tgagctgctg 1260
aggaatgaag ggccactgag atgtgtcctt tagctgctgg gtgtcatgtc tgacctgctg 1320
gtgcctaggg cctgcttaac actcggcaag gctgcgagcc gaggactgtg ggaagccgga 1380
cttgatgctt tctaacctgc atatttgaat gccgaaggtc aaacaatcca agttacagat 1440
aaataaaaac cgcattgcaa gtattaaaaa gccattctag gaaaattc 1488

<210> 66
<211> 2364
<212> PRT
<213> Rattus norvegicus

<220>
<223> Microtubule associated protein IB

<400> 66

Gly Gln Lys Ile Leu His His Arg Ser Asp Val Leu Glu Thr Val Val
1               5                   10                  15

Leu Ile Asn Pro Ser Asp Glu Ala Val Ser Thr Glu Val Arg Leu Met
            20                  25                  30

Ile Thr Asp Ala Ala Arg His Lys Leu Leu Val Leu Thr Gly Gln Cys
        35                  40                  45

Phe Glu Asn Thr Gly Glu Leu Ile Leu Gln Ser Gly Ser Phe Ser Phe
    50                  55                  60

Gln Asn Phe Ile Glu Ile Phe Thr Asp Gln Glu Ile Gly Glu Leu Leu
65                  70                  75                  80

Ser Thr Thr His Pro Ala Asn Lys Ala Ser Leu Thr Leu Phe Cys Pro
                85                  90                  95

Glu Glu Gly Asp Trp Lys Asn Ser Asn Leu Asp Arg His Asn Leu Gln
            100                 105                 110

Asp Phe Ile Asn Ile Lys Leu Asn Ser Ala Ser Ile Leu Pro Glu Met
        115                 120                 125

Glu Gly Leu Ser Glu Phe Thr Glu Tyr Leu Ser Glu Ser Val Glu Val
    130                 135                 140

Pro Ser Pro Phe Asp Ile Leu Glu Pro Pro Thr Ser Gly Gly Phe Leu
145                 150                 155                 160

Lys Leu Ser Lys Pro Cys Cys Tyr Ile Phe Pro Gly Gly Arg Gly Asp
                165                 170                 175

Ser Ala Leu Phe Ala Val Asn Gly Phe Asn Met Leu Ile Asn Gly Gly
            180                 185                 190

Ser Glu Arg Lys Ser Cys Phe Trp Lys Leu Ile Arg His Leu Asp Arg
        195                 200                 205

Val Asp Ser Ile Leu Leu Thr His Ile Gly Asp Asp Asn Leu Pro Gly
    210                 215                 220

Ile Asn Ser Met Leu Gln Arg Lys Ile Ala Glu Leu Glu Glu Glu Ser
225                 230                 235                 240

Gln Gly Ser Thr Ser Asn Ser Asp Trp Met Lys Asn Leu Ile Ser Pro
                245                 250                 255

Asp Leu Gly Val Val Phe Leu Asn Val Pro Glu Asn Leu Lys Asn Pro
            260                 265                 270

Glu Pro Asn Ile Lys Met Lys Arg Ser Thr Glu Glu Ala Cys Phe Thr
        275                 280                 285

Leu Gln Tyr Leu Asn Lys Leu Ser Met Lys Pro Glu Pro Leu Phe Arg
    290                 295                 300

Ser Val Gly Asn Ala Ile Glu Pro Val Ile Leu Phe Gln Lys Met Gly
305                 310                 315                 320

Val Gly Lys Leu Lys Met Tyr Val Leu Asn Pro Val Lys Ser Ser Lys
                325                 330                 335

Glu Met Gln Tyr Phe Met Gln Gln Trp Thr Gly Thr Asn Lys Asp Lys
            340                 345                 350

Ala Glu Leu Ile Leu Pro Asn Gly Gln Glu Val Asp Ile Pro Ile Ser
        355                 360                 365

Tyr Leu Ala Ser Val Ser Ser Leu Ile Val Trp His Pro Ala Asn Pro
    370                 375                 380

Ala Glu Lys Ile Ile Arg Val Leu Phe Pro Gly Asn Ser Thr Gln Tyr
385                 390                 395                 400

Asn Ile Leu Glu Gly Leu Glu Lys Leu Lys His Leu Asp Phe Leu Lys
                405                 410                 415

Gln Pro Leu Ala Thr Gln Lys Asp Leu Thr Gly Gln Val Ser Thr Pro
            420                 425                 430

Pro Val Lys Gln Val Lys Leu Lys Gln Arg Ala Asp Ser Arg Glu Ser
        435                 440                 445

Leu Lys Pro Ala Thr Lys Pro Leu Ser Ser Lys Ser Val Arg Lys Glu
    450                 455                 460

Ser Lys Glu Glu Ala Pro Glu Ala Thr Lys Ala Ser Gln Val Glu Lys
465                 470                 475                 480

Thr Pro Lys Val Glu Ser Lys Glu Lys Val Ile Val Lys Lys Asp Lys
                485                 490                 495

Pro Gly Lys Val Glu Ser Lys Pro Ser Val Thr Glu Lys Glu Val Pro
            500                 505                 510

Ser Lys Glu Glu Gln Ser Pro Val Lys Ala Glu Val Ala Glu Lys Ala
        515                 520                 525

Ala Thr Glu Ser Lys Pro Lys Val Thr Lys Asp Lys Val Val Lys Lys
    530                 535                 540

Glu Ile Lys Thr Lys Pro Glu Glu Lys Lys Glu Glu Lys Pro Lys Lys
545                 550                 555                 560

Glu Val Ala Lys Lys Glu Asp Lys Thr Pro Leu Lys Lys Asp Glu Lys
                565                 570                 575

Pro Lys Lys Glu Glu Ala Lys Lys Glu Ile Lys Lys Glu Ile Lys Lys
            580                 585                 590

Glu Glu Lys Lys Glu Leu Lys Lys Glu Val Lys Lys Glu Thr Pro Leu
        595                 600                 605

Lys Asp Ala Lys Lys Glu Val Lys Lys Asp Glu Lys Lys Glu Val Lys
    610                 615                 620

Lys Glu Glu Lys Glu Pro Lys Lys Glu Ile Lys Lys Ile Ser Lys Asp
625                 630                 635                 640

Ile Lys Lys Ser Thr Pro Leu Ser Asp Thr Lys Lys Pro Ala Ala Leu
                645                 650                 655

Lys Pro Lys Val Ala Lys Lys Glu Glu Pro Thr Lys Lys Glu Pro Ile

            660                 665                 670

Ala Ala Gly Lys Leu Lys Asp Lys Gly Lys Val Lys Val Ile Lys Lys
        675                 680                 685

Glu Gly Lys Thr Thr Glu Ala Ala Ala Thr Ala Val Gly Thr Ala Ala
    690                 695                 700

Val Ala Ala Ala Ala Gly Val Ala Ala Ser Gly Pro Ala Lys Glu Leu
705                 710                 715                 720

Glu Ala Glu Arg Ser Leu Met Ser Ser Pro Glu Asp Leu Thr Lys Asp
                725                 730                 735

Phe Glu Glu Leu Lys Ala Glu Glu Ile Asp Val Ala Lys Asp Ile Lys
            740                 745                 750

Pro Gln Leu Glu Leu Ile Glu Asp Glu Glu Lys Leu Lys Glu Thr Glu
        755                 760                 765

Pro Gly Glu Ala Tyr Val Ile Gln Lys Glu Thr Glu Val Ser Lys Gly
    770                 775                 780

Ser Ala Glu Ser Pro Asp Glu Gly Ile Thr Thr Thr Glu Gly Glu Gly
785                 790                 795                 800

Glu Cys Glu Gln Thr Pro Glu Glu Leu Glu Pro Val Glu Lys Gln Gly
                805                 810                 815

Val Asp Asp Ile Glu Lys Phe Glu Asp Glu Gly Ala Gly Phe Glu Glu
            820                 825                 830

Ser Ser Glu Ala Gly Asp Tyr Glu Glu Lys Ala Glu Thr Glu Glu Ala
        835                 840                 845

Glu Glu Pro Glu Glu Asp Gly Glu Asp Asn Val Ser Gly Ser Ala Ser
    850                 855                 860

Lys His Ser Pro Thr Glu Asp Glu Glu Ile Ala Lys Ala Glu Ala Asp
865                 870                 875                 880

Val His Ile Lys Glu Lys Arg Glu Ser Val Ala Ser Gly Asp Asp Arg
                885                 890                 895

Ala Glu Glu Asp Met Asp Glu Ala Leu Glu Lys Gly Glu Ala Glu Gln
            900                 905                 910

Ser Glu Glu Glu Gly Glu Glu Glu Glu Asp Lys Ala Glu Asp Ala Arg
        915                 920                 925

Glu Glu Asp His Glu Pro Asp Lys Thr Glu Ala Glu Asp Tyr Val Met
    930                 935                 940

Ala Val Val Asp Lys Ala Ala Glu Ala Gly Val Thr Glu Asp Gln Tyr
945                 950                 955                 960

Asp Phe Leu Gly Thr Pro Ala Lys Gln Pro Gly Val Gln Ser Pro Ser
                965                 970                 975

Arg Glu Pro Ala Ser Ser Ile His Asp Glu Thr Leu Pro Gly Gly Ser
            980                 985                 990

Glu Ser Glu Ala Thr Ala Ser Asp Glu Glu Asn Arg Glu Asp Gln Pro
        995                 1000                1005

Glu Glu Phe Thr Ala Thr Ser Gly Tyr Thr Gln Ser Thr Ile Glu Ile
    1010                1015                1020

Ser Ser Glu Pro Thr Pro Met Asp Glu Met Ser Thr Pro Arg Asp Val
1025                1030                1035                1040

Met Thr Asp Glu Thr Asn Asn Glu Glu Thr Glu Ser Pro Ser Gln Glu
                1045                1050                1055

Phe Val Asn Ile Thr Lys Tyr Glu Ser Ser Leu Tyr Ser Gln Glu Tyr
            1060                1065                1070

Ser Lys Pro Val Val Ala Ser Phe Asn Gly Leu Ser Asp Gly Ser Lys
        1075                1080                1085

Thr Asp Ala Thr Asp Gly Arg Asp Tyr Asn Ala Ser Ala Ser Thr Ile
    1090                1095                1100

Ser Pro Pro Ser Ser Met Glu Glu Asp Lys Phe Ser Lys Ser Ala Leu
1105                1110                1115                1120

Arg Asp Ala Tyr Arg Pro Glu Glu Thr Asp Val Lys Thr Gly Ala Glu
                1125                1130                1135

Leu Asp Ile Lys Asp Val Ser Asp Glu Arg Leu Ser Pro Ala Lys Ser
            1140                1145                1150

Pro Ser Leu Ser Pro Ser Pro Pro Ser Pro Ile Glu Lys Thr Pro Leu
        1155                1160                1165

Gly Glu Arg Ser Val Asn Phe Ser Leu Thr Pro Asn Glu Ile Lys Ala
    1170                1175                1180

Ser Ala Glu Gly Glu Ala Thr Ala Val Val Ser Pro Gly Val Thr Gln
1185                1190                1195                1200

Ala Val Val Glu Glu His Cys Ala Ser Pro Glu Glu Lys Thr Leu Glu
                1205                1210                1215

Val Val Ser Pro Ser Gln Ser Val Thr Gly Ser Ala Gly His Thr Pro
            1220                1225                1230

Tyr Tyr Gln Ser Pro Thr Asp Glu Lys Ser Ser His Leu Pro Thr Glu
        1235                1240                1245

Val Thr Glu Asn Ala Gln Ala Val Pro Val Ser Phe Glu Phe Thr Glu
    1250                1255                1260

Ala Lys Asp Glu Asn Glu Arg Ser Ser Ile Ser Pro Met Asp Glu Pro
1265                1270                1275                1280

Val Pro Asp Ser Glu Ser Pro Ile Glu Lys Val Leu Ser Pro Leu Arg
                1285                1290                1295

Ser Pro Pro Leu Ile Gly Ser Glu Ser Ala Tyr Glu Asp Phe Leu Ser
            1300                1305                1310

Ala Asp Asp Lys Ala Leu Gly Arg Arg Ser Glu Ser Pro Phe Glu Gly
        1315                1320                1325

Lys Asn Gly Lys Gln Gly Phe Ser Asp Lys Glu Ser Pro Val Ser Asp
    1330                1335                1340

Leu Thr Ser Asp Leu Tyr Gln Asp Lys Gln Glu Glu Lys Arg Ala Gly
1345                1350                1355                1360

Phe Ile Pro Ile Lys Glu Asp Phe Ser Pro Glu Lys Lys Ala Ser Asp
                1365                1370                1375

Ala Glu Ile Met Ser Ser Gln Ser Ala Leu Ala Leu Asp Glu Arg Lys
            1380                1385                1390

Leu Gly Gly Asp Gly Ser Pro Thr Gln Val Asp Val Ser Gln Phe Gly
        1395                1400                1405

Ser Phe Lys Glu Asp Thr Lys Met Ser Ile Ser Glu Gly Thr Val Ser
    1410                1415                1420

Asp Lys Ser Ala Thr Pro Val Asp Glu Gly Ala Glu Asp Thr Tyr Ser
1425                1430                1435                1440

His Met Glu Gly Val Ala Ser Val Ser Thr Ala Ser Val Ala Thr Ser
                1445                1450                1455

Ser Phe Pro Glu Pro Thr Thr Asp Asp Val Ser Pro Ser Leu His Ala
            1460                1465                1470

Glu Val Gly Ser Pro His Ser Thr Glu Val Asp Asp Ser Leu Ser Val
        1475                1480                1485

Ser Val Val Gln Thr Pro Thr Thr Phe Gln Glu Thr Glu Met Ser Pro
    1490                1495                1500

Ser Lys Glu Glu Cys Pro Arg Pro Met Ser Ile Ser Pro Pro Asp Phe
1505                1510                1515                1520

Ser Pro Lys Thr Ala Lys Ser Arg Thr Pro Val Gln Asp His Arg Ser
                1525                1530                1535

Glu Gln Ser Ser Met Ser Ile Glu Phe Gly Gln Glu Ser Pro Glu His
            1540                1545                1550

Ser Leu Ala Met Asp Phe Ser Arg Gln Ser Pro Asp His Pro Thr Val
        1555                1560                1565

Gly Ala Gly Met Leu His Ile Thr Glu Asn Gly Pro Thr Glu Val Asp
    1570                1575                1580

Tyr Ser Pro Ser Asp Ile Gln Asp Ser Ser Leu Ser His Lys Ile Pro
1585                1590                1595                1600

Pro Thr Glu Glu Pro Ser Tyr Thr Gln Asp Asn Asp Leu Ser Glu Leu
                1605                1610                1615

Ile Ser Val Ser Gln Val Glu Ala Ser Pro Ser Thr Ser Ser Ala His
            1620                1625                1630

Thr Pro Ser Gln Ile Ala Ser Pro Leu Gln Glu Asp Thr Leu Ser Asp
        1635                1640                1645

Val Val Pro Pro Arg Asp Met Ser Leu Tyr Ala Ser Leu Ala Ser Glu

1650                1655                1660

Lys Val Gln Ser Leu Glu Gly Glu Lys Leu Ser Pro Lys Ser Asp Ile
1665                1670                1675                1680

Ser Pro Leu Thr Pro Arg Glu Ser Ser Pro Thr Tyr Ser Pro Gly Phe
                1685                1690                1695

Ser Asp Ser Thr Ser Gly Ala Lys Glu Ser Thr Ala Ala Tyr Gln Thr
            1700                1705                1710

Ser Ser Ser Pro Pro Ile Asp Ala Ala Ala Ala Glu Pro Tyr Gly Phe
        1715                1720                1725

Arg Ser Ser Met Leu Phe Asp Thr Met Gln His His Leu Ala Leu Ser
    1730                1735                1740

Arg Asp Leu Thr Thr Ser Ser Val Glu Lys Asp Asn Gly Gly Lys Thr
1745                1750                1755                1760

Pro Gly Asp Phe Asn Tyr Ala Tyr Gln Lys Pro Glu Ser Thr Thr Glu
                1765                1770                1775

Ser Pro Asp Glu Glu Asp Tyr Asp Tyr Glu Ser His Glu Lys Thr Ile
            1780                1785                1790

Gln Ala His Asp Val Gly Gly Tyr Tyr Tyr Glu Lys Thr Glu Arg Thr
        1795                1800                1805

Ile Lys Ser Pro Cys Asp Ser Gly Tyr Ser Tyr Glu Thr Ile Glu Lys
    1810                1815                1820

Thr Thr Lys Thr Pro Glu Asp Gly Gly Tyr Ser Cys Glu Ile Thr Glu
1825                1830                1835                1840

Lys Thr Thr Arg Thr Pro Glu Glu Gly Gly Tyr Ser Tyr Glu Ile Ser
                1845                1850                1855

Glu Lys Thr Thr Arg Thr Pro Glu Val Ser Gly Tyr Thr Tyr Glu Lys
            1860                1865                1870

Thr Glu Arg Ser Arg Arg Leu Leu Asp Asp Ile Ser Asn Gly Tyr Asp
        1875                1880                1885

Asp Thr Glu Asp Gly Gly His Thr Leu Gly Asp Cys Ser Tyr Ser Tyr
    1890                1895                1900

Glu Thr Thr Glu Lys Ile Thr Ser Phe Pro Glu Ser Glu Ser Tyr Ser
1905                1910                1915                1920

Tyr Glu Thr Thr Thr Lys Thr Thr Arg Ser Pro Asp Thr Ser Ala Tyr
                1925                1930                1935

Cys Tyr Glu Thr Met Glu Lys Ile Thr Lys Thr Pro Gln Ala Ser Thr
            1940                1945                1950

Tyr Ser Tyr Glu Thr Ser Asp Arg Cys Tyr Thr Pro Glu Arg Lys Ser
        1955                1960                1965

Pro Ser Glu Ala Arg Gln Asp Val Asp Leu Cys Leu Val Ser Ser Cys
    1970                1975                1980

Glu Phe Lys His Pro Lys Thr Glu Leu Ser Pro Ser Phe Ile Asn Pro
1985                1990                1995                2000

Asn Pro Leu Glu Trp Phe Ala Gly Glu Glu Pro Thr Glu Glu Ser Glu
            2005                2010                2015

Arg Pro Leu Thr Gln Ser Gly Gly Ala Pro Pro Pro Ser Gly Gly Lys
        2020                2025                2030

Gln Gln Gly Arg Gln Cys Asp Glu Thr Pro Pro Thr Ser Val Ser Glu
    2035                2040                2045

Ser Ala Pro Ser Gln Thr Asp Ser Asp Val Pro Pro Glu Thr Glu Glu
    2050                2055                2060

Cys Pro Ser Ile Thr Ala Asp Ala Asn Leu Asp Ser Glu Asp Glu Ser
2065                2070                2075                2080

Glu Thr Ile Pro Thr Asp Lys Thr Val Thr Tyr Lys His Met Asp Pro
            2085                2090                2095

Pro Pro Ala Pro Met Gln Asp Arg Ser Pro Ser Pro Arg His Pro Asp
        2100                2105                2110

Val Ser Met Val Asp Pro Glu Ala Leu Ala Ile Glu Gln Asn Leu Gly
    2115                2120                2125

Lys Ala Leu Lys Lys Asp Leu Lys Glu Lys Ala Lys Thr Lys Lys Pro
    2130                2135                2140

Gly Thr Lys Thr Lys Ser Ser Ser Pro Val Lys Lys Gly Asp Gly Lys
2145                2150                2155                2160

Ser Lys Pro Ser Ala Ala Ser Pro Lys Pro Gly Ala Leu Lys Glu Ser
            2165                2170                2175

Ser Asp Lys Val Ser Arg Val Ala Ser Pro Lys Lys Lys Glu Ser Val
        2180                2185                2190

Glu Lys Ala Met Lys Thr Thr Thr Thr Pro Glu Val Lys Ala Thr Arg
    2195                2200                2205

Gly Glu Glu Lys Asp Lys Glu Thr Lys Asn Ala Ala Asn Ala Ser Ala
    2210                2215                2220

Ser Lys Ser Val Lys Thr Ala Thr Ala Gly Pro Gly Thr Thr Lys Thr
2225                2230                2235                2240

Ala Lys Ser Ser Thr Val Pro Pro Gly Leu Pro Val Tyr Leu Asp Leu
            2245                2250                2255

Cys Tyr Ile Pro Asn His Ser Asn Ser Lys Asn Val Asp Val Glu Phe
        2260                2265                2270

Phe Lys Arg Val Arg Ser Ser Tyr Tyr Val Val Ser Gly Asn Asp Pro
    2275                2280                2285

Ala Ala Glu Glu Pro Ser Arg Ala Val Leu Asp Ala Leu Leu Glu Gly
    2290                2295                2300

Lys Ala Gln Trp Gly Ser Asn Met Gln Val Thr Leu Ile Pro Thr His
2305                2310                2315                2320

Asp Ser Glu Val Met Arg Glu Trp Tyr Gln Glu Thr His Glu Lys Gln
            2325                2330                2335

Gln Asp Leu Asn Ile Met Val Leu Ala Ser Ser Ser Thr Val Val Met
        2340                2345                2350

Gln Asp Glu Ser Phe Pro Ala Cys Lys Ile Glu Leu
    2355                2360

<210> 67
<211> 7095
<212> DNA
<213> Rattus norvegicus

<220>
<223> Microtubule associated protein IB

<400> 67
ggacaaaaga tcctccatca ccgaagtgac gtcttagaaa ctgtagttct gatcaaccct 60
tcggatgaag cagtcagcac cgaggtgcgt ttgatgatca ctgacgccgc ccgccataaa 120
ctgctggtgc tcaccggaca gtgctttgag aacactggag agctcatcct ccagtcaggc 180
tctttctcct tccagaactt catagagatt ttcaccgacc aagagattgg ggagctccta 240
agcaccaccc atcctgccaa caaagccagc ctcaccctct tctgccctga ggaaggagac 300
tggaagaact ccaaccttga cagacacaat ctccaagact tcatcaacat caagctcaac 360
tcagcttcta tcttgccaga aatggaggga ctttctgagt tcaccgagta cctctcggag 420
tctgtcgaag tcccctcccc ctttgacatc ctggagcccc cgacctcggg cggatttctg 480
aagctctcca agccttgttg ttacattttt ccggggggcc gcggggactc tgccctgttc 540
gcagtgaacg gattcaacat gctcattaac ggaggatcag aaagaaagtc ctgcttctgg 600
aagctcattc ggcacttgga ccgggtggac tccatcctgc tcacccacat tggggatgac 660
aacttgcccg ggatcaacag catgttgcaa cgcaagattg cagagctgga agaggagtcc 720
cagggctcca ccagcaacag tgactggatg aaaaacctca tctcccctga cttgggggtt 780
gtgtttctca atgtacctga aaatctgaaa aacccagaac ccaacatcaa gatgaagaga 840
agtacagaag aagcatgctt caccctccag tacctaaaca aactgtccat gaaaccagag 900
cctttattta gaagtgtagg caatgccatt gagcctgtca tcctgttcca aaaaatggga 960
gtgggtaaac tgaagatgta tgtgcttaac ccagtcaaaa gcagcaagga aatgcagtat 1020
ttcatgcagc agtggactgg aaccaacaaa gacaaggctg aacttatcct gcccaatggt 1080
caagaagtag acatcccgat ttcctaccta gcttccgtct cgtctttgat tgtgtggcac 1140
ccagccaacc ctgctgagaa aatcatccgg gttctgtttc ctggaaacag cacccagtac 1200
aacatcctag aagggctgga aaaactcaaa catctagact tcctaaagca gccactggcc 1260
acccaaaaag atctcactgg ccaggtgtcc acccccccag tgaaacaggt caagttgaaa 1320
cagcgggctg acagccgaga gagtctgaag ccagccacaa aaccactttc cagtaaatca 1380
gtgaggaagg agtccaaaga ggaggcccct gaagccacaa aagccagcca agtggaaaaa 1440
acacccaaag ttgaaagcaa agagaaagtg atagtgaaaa aagacaagcc aggaaaggta 1500
gaaagtaagc catcggtgac ggaaaaggag gtgcccagca aagaggagca gtcgcccgtc 1560
aaagctgagg tggctgagaa ggcggccacg gagagcaaac ccaaagtcac caaagacaaa 1620
gtggtaaaaa aggaaataaa gacaaaaccc gaagaaaaga aagaggagaa gcccaagaag 1680
gaagtggcta aaaaggaaga caaaactccc ctcaagaaag acgagaagcc caaaaaggaa 1740
gaggcgaaga aggagatcaa gaaagaaatc aaaaaggaag agaaaaagga gctgaagaaa 1800
gaggtgaaga aggaaacgcc cctgaaggac gccaagaagg aggtgaagaa agacgagaag 1860
aaagaagtta aaaaggaaga gaaggaaccc aaaaaggaga ttaagaagat ctccaaggac 1920
ataaagaaat ccactcctct gtcagacaca aagaaaccgg ctgcattgaa accaaaagta 1980
gcaaagaaag aagagcccac caagaaggag cctattgctg ctgggaaact caaggacaag 2040
gggaaggtca aagtcattaa aaaggaaggc aagaccacag aggccgctgc cacagctgtt 2100
ggcactgctg ccgtggctgc agcagccgga gtagcggcca gcggtcctgc caaagaactt 2160
gaagctgagc ggtccctcat gtcgtcccct gaggatctaa ccaaggactt tgaggagcta 2220
aaggctgagg agatcgatgt agcgaaggac atcaagcctc agctggagct cattgaagat 2280
gaagagaaac tgaaggaaac cgagccggga gaagcctacg tcattcagaa agagacggaa 2340
gtcagcaaag gttctgctga gtcacctgat gaagggatca ccaccactga gggggaaggg 2400
gagtgcgagc aaacccccga ggagctggag ccagttgaga agcagggcgt ggatgacatc 2460
gagaagttcg aggatgaagg cgctggtttt gaagaatcct cagaggccgg agactacgaa 2520

gagaaggcag aaactgagga ggccgaggag ccggaagaag acggggaaga caatgtgagc 2580
gggagcgcct cgaagcacag ccccacagaa gacgaagaaa tcgctaaggc tgaggcggac 2640
gtacacatca aggagaagag ggagtctgtg gccagcggcg atgaccgggc cgaagaagac 2700
atggatgaag cgcttgagaa aggagaagct gaacagtctg aggaggaggg tgaggaggag 2760
gaggacaaag cagaggacgc cagagaggaa gaccatgagc ccgacaaaac tgaggctgaa 2820
gattatgtga tggctgtggt tgacaaggcc gcggaggccg gagtcaccga ggatcagtat 2880
gatttcctgg ggacaccggc caagcaacct ggagtccagt ctcctagccg agaacccgcg 2940
tcttcaattc atgatgagac cctacccgga ggctccgaga gcgaggccac tgcttcagat 3000
gaggagaatc gagaagacca gcctgaggaa ttcactgcta cctccggata tactcagtcc 3060
accatcgaga tatctagtga gccgactcca atggatgaga tgtccactcc tcgagatgtg 3120
atgaccgacg agaccaacaa tgaggagaca gagtccccgt ctcaggagtt cgtgaacatt 3180
accaaatacg agtcttcgct gtactctcag gagtactcca aacctgtggt tgcatcattc 3240
aatggattgt cagacgggtc aaagacagac gccactgacg gtagggatta caacgcttcc 3300
gcctccacca tatcaccacc ttcgtccatg gaagaagaca aattcagcaa gtctgctctt 3360
cgtgacgctt accgcccaga agagacggac gtgaaaaccg gtgccgagtt ggacatcaaa 3420
gatgtttcgg atgagagact tagcccagcc aagagtccat ccctgagtcc ttctccacca 3480
tcacccatag agaagactcc cctgggtgaa cgtagcgtga atttctctct gacacccaac 3540
gagatcaaag cctctgcaga gggagaggca acagcagtag tgtcccccgg agtgacccaa 3600
gcagtagttg aagaacactg tgccagtcct gaggagaaga ccttggaggt agtgtcaccg 3660
tctcagtctg tgacaggcag tgcgggccac acaccttact accaatctcc caccgacgaa 3720
aagtccagtc acctacctac agaagtcact gagaacgcgc aggcagtccc ggtgagcttt 3780
gaattcactg aggccaaaga tgagaacgag aggtcgtcca tcagccccat ggatgaacct 3840
gtgcctgact cagagtctcc tatcgagaaa gttctgtctc cgttacgcag ccctcccctt 3900
attggatccg agtccgcata tgaagacttc ctgagtgcgg atgacaaggc tcttggcaga 3960
cgttcagaaa gcccctttga agggaagaat ggaaagcaag gcttctcaga caaagaaagc 4020
ccagtttctg acctgacttc cgatctttac caagacaagc aggaagagaa aagggcgggc 4080
ttcataccga taaaggaaga ctttagtcca gaaaagaaag ccagcgatgc tgaaatcatg 4140
agttctcaat cagctctggc tttggatgaa aggaaactgg gaggagatgg atctccaacg 4200
caagtagatg tcagtcagtt tggctctttc aaagaagaca ccaagatgtc catttcggaa 4260
ggcaccgttt cagacaagtc cgccacgcct gtggatgagg gcgcggaaga cacctattca 4320
cacatggaag gtgtggcctc agtgtcaacg gcctctgtgg ctaccagctc gtttccagag 4380
ccaaccacag atgacgtgtc tccttctctc cacgctgaag tgggctctcc acattccaca 4440
gaggtggatg actccctgtc ggtgtcggtg gtgcaaacac caactacttt ccaggaaaca 4500
gaaatgtctc cgtctaaaga agagtgccca agaccaatgt cgatttctcc tcctgacttc 4560
tcccctaaga cagccaaatc caggacacca gttcaagatc accgatccga acagtcttca 4620
atgtctattg aattcggtca ggaatccccc gagcattctc ttgctatgga ctttagtcgg 4680
cagtctccag accaccctac tgtgggtgct ggtatgcttc acatcaccga aaatgggcca 4740
actgaggtgg actacagtcc ctccgatatc caggactcta gtttgtcaca taagattcct 4800
cctacagaag agccatccta cacccaggat aatgatctgt ccgagctcat ctctgtgtct 4860
caggtggagg cttccccatc cacctcttct gctcacactc cttctcagat agcctctcct 4920
cttcaggaag acactctctc tgatgtcgtt cctcccagag atatgtcctt atatgcctcg 4980
cttgcgtctg agaaagtgca gagcctggaa ggagagaaac tctctccaaa atccgatatt 5040
tctccgctca cccctcgaga gtcctcacct acatattcac ctggcttttc agattctacc 5100
tctggagcta aagagagtac agcggcttac caaacctcct cttccccacc aatagatgca 5160
gcagccgcag agccctacgg cttccgctcc tcaatgttat ttgatacaat gcagcatcac 5220
ctggccttga gtagagattt gaccacatct agtgtggaga aggacaatgg agggaagaca 5280
cccggtgact ttaactatgc ctatcaaaag cccgagagca ccaccgaatc cccagatgaa 5340
gaagattatg actatgaatc tcacgagaaa accatccagg cccacgatgt gggtggttac 5400
tactatgaga agacagagag aaccataaaa tccccatgtg acagtggata ctcctatgag 5460
accattgaga agaccaccaa gaccccagaa gatggtggct actcctgtga aattaccgag 5520
aaaaccactc ggacccctga agagggcggg tactcgtatg agatcagcga gaagacaaca 5580
cgaacccctg aagtaagtgg ctacacctat gagaagaccg agaggtccag aaggctcctc 5640
gatgacatta gcaatggcta cgatgacact gaggatggtg gccacacact tggcgactgt 5700
agctattcct acgaaaccac tgagaaaatt accagctttc ctgaatctga aagctattcc 5760
tatgagacaa ctacaaaaac aacacggagt ccagacacct ctgcatactg ttacgagacc 5820
atggagaaga tcaccaagac cccacaggca tccacatact cctatgagac ctcagaccga 5880
tgctacactc cagaaaggaa gtccccctcg gaggcacgcc aggatgttga cttgtgtctg 5940
gtgtcctcct gtgaattcaa gcatcccaag accgagctct caccttcctt cattaatcca 6000
aaccctctcg agtggtttgc tggggaagag cccactgaag aatctgagag gcctctcact 6060
cagtctggag gagccccccc accttcagga ggaaaacaac agggcagaca atgcgatgaa 6120
actccaccca cctcagtcag tgagtcagct ccatcccaga cggactctga tgttccccca 6180
gagacagaag agtgcccctc catcacagct gatgccaacc ttgactctga agatgagtca 6240

gaaaccatcc ccacagacaa aacggttacg tacaaacaca tggacccgcc tccagccccc 6300
atgcaagacc gaagcccttc tcctcgccac cctgatgtgt ccatggtgga tccagaggcc 6360
ttggctattg agcagaacct aggcaaggct ctgaaaaagg atctgaagga gaaggccaag 6420
accaagaaac caggcacaaa gaccaagtcc tcttcacctg tcaaaaaggg tgatggaaag 6480
tccaagcctt cagcagcttc ccccaaacca ggagccttga aggaatcctc tgacaaggtg 6540
tccagagtgg cttctcccaa gaagaaagag tctgtggaga aagctatgaa gaccaccacc 6600
actcctgagg tcaaagccac acgaggggaa gagaaggaca aggaaactaa gaatgcagcc 6660
aatgcttctg catccaagtc agtgaagact gcaacagcag gaccaggaac cactaagacg 6720
gccaagtcgt ccaccgtgcc tcccggcctc cctgtgtatt tggacctctg ctatattccc 6780
aaccacagca acagtaagaa tgtcgatgtt gagtttttca agagagtgag gtcatcttac 6840
tacgtggtga gtgggaacga ccctgccgcg gaggagccca gccgggctgt cctggatgcc 6900
ttgttggaag ggaaggctca gtggggaagc aacatgcagg tgactctgat cccaacacat 6960
gactctgagg tgatgaggga gtggtaccag gagacccacg agaagcagca agacctcaac 7020
atcatggtcc tagcaagcag tagtacagtg gtcatgcaag acgagtcctt ccctgcatgc 7080
aagatagaac tgtag 7095

<210> 68
<211> 379
<212> PRT
<213> Rattus norvegicus

<220>
<223> cdc 37 homolog

<400> 68
Met Val Asp Tyr Ser Val Trp Asp His Ile Glu Val Ser Asp Asp Glu
1               5                   10                  15

Asp Glu Thr His Pro Asn Ile Asp Thr Ala Ser Leu Phe Arg Trp Arg
            20                  25                  30

His Gln Ala Arg Val Glu Arg Met Glu Gln Phe Gln Lys Glu Lys Glu
        35                  40                  45

Glu Leu Asp Arg Gly Cys Arg Glu Cys Lys Arg Lys Val Ala Glu Phe
    50                  55                  60

Gln Arg Lys Leu Lys Glu Leu Glu Val Ala Glu Gly Gly Gly Gln Val
65                  70                  75                  80

Glu Leu Glu Arg Leu Arg Ala Glu Ala Gln Gln Leu Arg Lys Glu Glu
                85                  90                  95

Arg Thr Gly Ser Arg Ser Trp Arg Thr Cys Gly Lys Lys Glu Lys Asn
            100                 105                 110

Met Pro Trp Asn Val Asp Thr Leu Ser Lys Asp Gly Phe Ser Lys Ser
        115                 120                 125

Met Val Asn Thr Lys Pro Glu Lys Ala Glu Glu Asp Ser Glu Glu Ala
    130                 135                 140

Arg Glu Gln Lys His Lys Thr Phe Val Glu Lys Tyr Glu Lys Gln Ile
145                 150                 155                 160

Lys His Phe Gly Met Leu His Arg Trp Asp Asp Ser Gln Lys Tyr Leu
                165                 170                 175

Ser Asp Asn Val His Leu Val Cys Glu Glu Thr Ala Asn Tyr Leu Val
            180                 185                 190

Ile Trp Cys Ile Asp Leu Glu Val Glu Glu Lys Cys Ala Leu Met Glu
        195                 200                 205

Gln Val Ala His Gln Thr Met Val Met Gln Phe Ile Leu Glu Leu Ala
    210                 215                 220

Lys Ser Leu Lys Val Asp Pro Arg Ala Cys Phe Arg Gln Phe Phe Thr
225                 230                 235                 240

Lys Ile Lys Thr Ala Asp Gln Gln Tyr Met Glu Gly Phe Lys Tyr Glu
                245                 250                 255

Leu Glu Ala Phe Lys Glu Arg Val Arg Gly Arg Ala Lys Leu Arg Ile
            260                 265                 270

Glu Lys Ala Met Lys Glu Tyr Glu Glu Glu Glu Arg Lys Lys Arg Leu
        275                 280                 285

Gly Pro Gly Gly Leu Asp Pro Val Glu Val Tyr Glu Ser Leu Pro Glu
    290                 295                 300

Glu Leu Gln Lys Cys Phe Asp Val Lys Asp Val Gln Met Leu Gln Asp
305                 310                 315                 320

Ala Ile Ser Lys Met Asp Pro Thr Asp Ala Lys Tyr His Met Gln Arg
                325                 330                 335

Cys Ile Asp Ser Gly Leu Trp Val Pro Asn Ser Lys Ser Gly Glu Ala
            340                 345                 350

Lys Glu Gly Glu Glu Ala Gly Pro Gly Asp Pro Leu Leu Glu Ala Val
        355                 360                 365

Pro Lys Ala Gly Phe Glu Lys Asp Ile Ser Ala
    370                 375

<210> 69
<211> 1604
<212> DNA
<213> Rattus norvegicus

<220>
<223> cdc 37 homolog

<400> 69
cgagcggact ctctagccct tgtgggtccc tgttcagcgc ccggccgtgt ggagacccgg 60
gagccagtcc gggcctccga ggcgaagatg gtggactaca gcgtttggga tcacatcgag 120
gtgtcggacg atgaggacga gacgcacccc aacatagaca cggccagcct gttccgctgg 180
cggcaccagg cccgggtgga acgcatggaa cagtttcaga aggagaaaga agaactggac 240
cggggctgcc gggaatgcaa gcgcaaggta gccgagttcc agcgcaagct gaaggaactg 300
gaagtggctg agggtggtgg ccaggtggag cttgagcggc ttcgagcgga ggcacagcag 360
ctgcgcaagg aggagcgaac tgggagcaga agctggagga catgcggcaa aaaggagaag 420
aacatgccct ggaatgtgga cacactcagc aaagatggct tcagcaagag catggtcaat 480
accaagcctg agaaggcaga ggaagactca gaggaggcaa gggagcagaa acacaagacc 540
ttcgttgaaa aatacgagaa acagatcaaa catttcggca tgctccaccg ctgggatgac 600
agccagaagt acctgtcaga caatgtccac ctggtgtgcg aggagacagc caactacctg 660
gttatctggt gcattgacct ggaagtcgag gagaagtgtg ccctgatgga gcaggtggct 720
caccagacca tggtcatgca gttcatcctg gagttggcca agagtctgaa ggtcgacccc 780
cgggcctgct tccggcagtt tttcaccaag atcaagactg ctgaccagca gtacatggag 840
ggcttcaagt atgaactgga ggcctttaag gagcgtgtgc ggggccgcgc caagctgcga 900
atagagaagg ccatgaagga gtacgaggag gaggagcgca agaagaggct aggccctggt 960

ggcctggacc ctgtggaggt ctacgaatct ctgcctgagg agctgcagaa gtgctttgat 1020
gtgaaggatg tacagatgct gcaagacgcc atcagcaaga tggatcctac tgatgccaag 1080
tatcacatgc agcgttgcat cgattctggc ctctgggtcc ccaactccaa gtctggtgag 1140
gccaaggagg gggaggaggc gggccccggg gacccattgc tggaagccgt ccccaaagcg 1200
ggcttcgaga aagacatcag tgcgtgaccc ttctccagaa aaggaaaccc agccaccatg 1260
tccctagctc ccgacttctt cacttctgct gctcctcacg tcctggccca tggcctgccc 1320
catgtcccct ctcctccggc ctgtccccag cactcatcag attctctgct tcgggttgag 1380
gggctctgct gtctgcagcc cccccacccc catcagcgtt atgccaaagt cctgggggtc 1440
tggggagggg cagcagctgc caggttggtc caccgggttg gggatacaca tgtccccatc 1500
tgggcagggt ctgtccctat gggtctatgg tcactgtttc tcttctgtct agctttctgt 1560
cctgcaactg gcaacaatgt ccaataaaca gttccaggtg ctgg 1604

<210> 70
<211> 216
<212> PRT
<213> Homo sapiens

<220>
<223> Rab5c-like protein

<400> 70
Met Ala Gly Arg Gly Gly Ala Arg Arg Pro Asn Gly Pro Ala Ala Gly
1               5                   10                  15

Asn Lys Ile Cys Gln Phe Lys Leu Val Leu Leu Gly Glu Ser Ala Val
            20                  25                  30

Gly Lys Ser Ser Leu Val Leu Arg Phe Val Lys Gly Gln Phe His Glu
        35                  40                  45

Tyr Gln Glu Ser Thr Ile Gly Ala Ala Phe Leu Thr Gln Thr Val Cys
    50                  55                  60

Leu Asp Asp Thr Thr Val Lys Phe Glu Ile Trp Asp Thr Ala Gly Gln
65                  70                  75                  80

Glu Arg Tyr His Ser Leu Ala Pro Met Tyr Tyr Arg Gly Ala Gln Ala
                85                  90                  95

Ala Ile Val Val Tyr Asp Ile Thr Asn Thr Asp Thr Phe Ala Arg Ala
            100                 105                 110

Lys Asn Trp Val Lys Glu Leu Gln Arg Gln Ala Ser Pro Asn Ile Val
        115                 120                 125

Ile Ala Leu Ala Gly Asn Lys Ala Asp Leu Ala Ser Lys Arg Ala Val
    130                 135                 140

Glu Phe Gln Glu Ala Gln Ala Tyr Ala Asp Asp Asn Ser Leu Leu Phe
145                 150                 155                 160

Met Glu Thr Ser Ala Lys Thr Ala Met Asn Val Asn Glu Ile Phe Met
                165                 170                 175

Ala Ile Ala Lys Lys Leu Pro Lys Asn Glu Pro Gln Asn Ala Thr Gly
            180                 185                 190

Ala Pro Gly Arg Asn Arg Gly Val Asp Leu Gln Glu Asn Asn Pro Ala
        195                 200                 205

Ser Arg Ser Gln Cys Cys Ser Asn

210                215

<210> 71
<211> 1529
<212> DNA
<213> Homo sapiens

<220>
<223> Rab5c-like protein

<400> 71
   cttacactaa gtgcctcttt gcatagcacc agtccccacc cgcacgctct ctggaccact 60
   acagctggac gggcaatggc gggtcgggga ggcgcacgac gacccaatgg accagctgct 120
   gggaacaaga tctgtcaatt taagctggtt ctgctggggg agtctgcggt aggcaaatcc 180
   agcctcgtcc tccgctttgt caagggacag tttcacgagt accaggagag cacaattgga 240
   gcggccttcc tcacacagac tgtctgcctg gatgacacaa cagtcaagtt tgagatctgg 300
   gacacagctg gacaggagcg gtatcacagc ctggccccca tgtactatcg gggggcccag 360
   gctgccatcg tggtctatga catcaccaac acagatacat ttgcacgggc caagaactgg 420
   gtgaaggagc tacagaggca ggccagcccc aacatcgtca ttgcactcgc gggtaacaag 480
   gcagacctgg ccagcaagag agccgtggaa ttccaggaag cacaagccta tgcagacgac 540
   aacagtttgc tgttcatgga gacatcagca aagactgcaa tgaacgtgaa cgaaatcttc 600
   atggcaatag ctaagaagct tcccaagaac gagccccaga atgcaactgg tgctccaggc 660
   cgaaaccgag gtgtggacct ccaggagaac aacccagcca gccggagcca gtgctgcagc 720
   aactgagccc cccttgcctg cccgctgccc ccgcctcctc cgcctgaatg acccgactgg 780
   aatccactct aaccaatcgc acttaacgac tcgggccacc actggggggg cagggggagg 840
   ggtccaccat gatttctcca tataattttg atcataggcc ggagtgagtc attccacctg 900
   cacctttctg tacaaatact aattcaattt taagtcttaa gtcacttttt taatatatat 960
   gatcttctgc tcttcccact tcctcccctt tctactgctc tcccattttc ccttgctggg 1020
   agtagccaca tgctcttgcc ccccaaccct tgtatatggg gacagtgggg tcagtgcagc 1080
   taccctttct ttccctctgc ggaacagcgg acccagcaag agcatccaca tcctcacttt 1140
   gttcggagtg gtctttggtt tgggcggtgg ggcagacctt gggaaggggc ttaggaaggg 1200
   agaggcagct cttccttcag ctggctctca tcaggctgca gccccctccc cgctcccacc 1260
   tccctgctgg gaaaccacag cattatcaca gcattattgt gacagccacg aacccattgc 1320
   ccacaacccc tccaccctcg gtcaccccaa cctctggctc tgagccctgt tctgaccaaa 1380
   tcatgatgat gagtatttgg gggtgggtgg gtaagggggg gagtgggagg ggacggaacc 1440
   aactttttct gtattttgta ttgtatgttt tcttcaacat gtaaccaatc agtatcttgt 1500
   caatatagtc agccgatcga tcgacctca                                   1529

<210> 72
<211> 731
<212> PRT
<213> Mus musculus

<220>
<223> Gelsolin

<400> 72
Met Val Val Glu His Pro Glu Phe Leu Lys Ala Gly Lys Glu Pro Gly
1               5                   10                  15

Leu Gln Ile Trp Arg Val Glu Lys Phe Asp Leu Val Pro Val Pro Pro
            20                  25                  30

Asn Leu Tyr Gly Asp Phe Phe Thr Gly Asp Ala Tyr Val Ile Leu Lys
        35                  40                  45

Thr Val Gln Leu Arg Asn Gly Asn Leu Gln Tyr Asp Leu His Tyr Trp
    50                  55                  60

Leu Gly Asn Glu Cys Ser Gln Asp Glu Ser Gly Ala Ala Ala Ile Phe

65                  70                  75                  80

Thr Val Gln Leu Asp Asp Tyr Leu Asn Gly Arg Ala Val Gln His Arg
                85                  90                  95

Glu Val Gln Gly Phe Glu Ser Ser Thr Phe Ser Gly Tyr Phe Lys Ser
            100                 105                 110

Gly Leu Lys Tyr Lys Lys Gly Gly Val Ala Ser Gly Phe Lys His Val
        115                 120                 125

Val Pro Asn Glu Val Val Val Gln Arg Leu Phe Gln Val Lys Gly Arg
    130                 135                 140

Arg Val Val Arg Ala Thr Glu Val Pro Val Ser Trp Asp Ser Phe Asn
145                 150                 155                 160

Asn Gly Asp Cys Phe Ile Leu Asp Leu Gly Asn Asn Ile Tyr Gln Trp
                165                 170                 175

Cys Gly Ser Gly Ser Asn Lys Phe Glu Arg Leu Lys Ala Thr Gln Val
            180                 185                 190

Ser Lys Gly Ile Arg Asp Asn Glu Arg Ser Gly Arg Ala Gln Val His
        195                 200                 205

Val Ser Glu Glu Glu Thr Glu Pro Glu Ala Met Leu Gln Val Leu Gly
    210                 215                 220

Pro Lys Pro Ala Leu Pro Glu Gly Thr Glu Asp Thr Ala Lys Glu Asp
225                 230                 235                 240

Ala Ala Asn Arg Lys Leu Ala Lys Leu Tyr Lys Val Ser Asn Gly Ala
                245                 250                 255

Gly Ser Met Ser Val Ser Leu Val Ala Asp Glu Asn Pro Phe Ala Gln
            260                 265                 270

Gly Pro Leu Arg Ser Glu Asp Cys Phe Ile Leu Asp His Gly Arg Asp
        275                 280                 285

Gly Lys Ile Phe Val Trp Lys Gly Lys Gln Ala Asn Met Glu Glu Arg
    290                 295                 300

Lys Ala Ala Leu Lys Thr Ala Ser Asp Phe Ile Ser Lys Met Gln Tyr
305                 310                 315                 320

Pro Arg Gln Thr Gln Val Ser Val Leu Pro Glu Gly Gly Glu Thr Pro
                325                 330                 335

Leu Phe Lys Gln Phe Phe Lys Asn Trp Arg Asp Pro Asp Gln Thr Asp
            340                 345                 350

Gly Pro Gly Leu Gly Tyr Leu Ser Ser His Ile Ala Asn Val Glu Arg
        355                 360                 365

Val Pro Phe Asp Ala Gly Thr Leu His Thr Ser Thr Ala Met Ala Ala
    370                 375                 380

Gln His Gly Met Asp Asp Asp Gly Thr Gly Gln Lys Gln Ile Trp Arg
385                 390                 395                 400

Ile Glu Gly Ser Asn Lys Val Pro Val Asp Pro Ala Thr Tyr Gly Gln
                405                 410                 415

Phe Tyr Gly Gly Asp Ser Tyr Ile Ile Leu Tyr Asn Tyr Arg His Gly
            420                 425                 430

Gly Arg Gln Gly Gln Ile Ile Tyr Asn Trp Gln Gly Ala Gln Ser Thr
        435                 440                 445

Gln Asp Glu Val Ala Ala Ser Ala Ile Leu Thr Ala Gln Leu Asp Glu
    450                 455                 460

Glu Leu Gly Gly Thr Pro Val Gln Ser Arg Val Val Gln Gly Lys Glu
465                 470                 475                 480

Pro Ala His Leu Met Ser Leu Phe Gly Gly Lys Pro Met Ile Ile Tyr
                485                 490                 495

Lys Gly Gly Thr Ser Arg Asp Gly Gly Gln Thr Ala Pro Ala Ser Ile
            500                 505                 510

Arg Leu Phe Gln Val Arg Ala Ser Ser Ser Gly Ala Thr Arg Ala Val
        515                 520                 525

Glu Val Met Pro Lys Ser Gly Ala Leu Asn Ser Asn Asp Ala Phe Val
    530                 535                 540

Leu Lys Thr Pro Ser Ala Ala Tyr Leu Trp Val Gly Ala Gly Ala Ser
545                 550                 555                 560

Glu Ala Glu Lys Thr Ala Ala Gln Glu Leu Leu Lys Val Leu Arg Ser
                565                 570                 575

Gln His Val Gln Val Glu Glu Gly Ser Glu Pro Asp Gly Phe Trp Glu
            580                 585                 590

Ala Leu Gly Gly Lys Thr Ser Tyr Arg Thr Ser Pro Arg Leu Lys Asp
        595                 600                 605

Lys Lys Met Asp Ala His Pro Pro Arg Leu Phe Ala Cys Ser Asn Arg
    610                 615                 620

Ile Gly Arg Phe Val Ile Glu Glu Val Pro Gly Glu Leu Met Gln Glu
625                 630                 635                 640

Asp Leu Ala Thr Asp Asp Val Met Leu Leu Asp Thr Trp Asp Gln Val
                645                 650                 655

Phe Val Trp Val Gly Lys Asp Ser Gln Glu Glu Glu Lys Thr Glu Ala
            660                 665                 670

Leu Thr Ser Ala Lys Arg Tyr Ile Glu Thr Asp Pro Ala Asn Arg Asp
        675                 680                 685

Arg Arg Thr Pro Ile Thr Val Val Arg Gln Gly Phe Glu Pro Pro Ser
    690                 695                 700

Phe Val Gly Trp Phe Leu Gly Trp Asp Asn Asn Tyr Trp Ser Val Asp
705                 710                 715                 720

Pro Leu Asp Arg Ala Leu Ala Glu Leu Ala Ala
                725                 730

<210> 73
<211> 2447
<212> DNA
<213> Mus musculus

<220>
<223> Gelsolin

<400> 73
   tgagcgcggc ccagcactat ggtggtggag caccccgaat tcctgaaggc agggaaggag 60
   cctggcctgc agatctggcg tgtggagaag tttgacctgg tgcctgtgcc ccccaacctc 120
   tatggagact tcttcacggg tgatgcctat gtcatcctga agactgtgca gctgaggaat 180
   gggaatctgc agtatgacct ccactattgg ctgggcaatg aatgcagcca ggatgagagc 240
   ggggctgctg ccatctttac tgtgcaactg gatgactacc tgaacggccg ggctgtgcag 300
   caccgtgagg ttcagggctt tgagtcgtcc accttctccg gctacttcaa gtctggactt 360
   aagtacaaga aaggaggtgt ggcatctgga ttcaaacacg tggtacccaa tgaggtggtg 420
   gtccagaggc tcttccaggt caaaggacgc cgtgtagtcc gtgctactga ggtacctgtg 480
   tcctgggaca gtttcaacaa tggcgactgc ttcattctgg acctgggaaa caatatctat 540
   cagtggtgtg gctctggcag caacaaattt gaaaggctga aggccacaca ggtgtccaag 600
   ggcatccggg acaacgagag gagtggccgt gctcaagtac acgtgtctga agaggagact 660
   gagcccgagg cgatgctgca ggtgctgggc cccaagccgg ctctgcctga aggtaccgag 720
   gacacagcca aggaagatgc agccaaccgc aagctggcca agctctacaa ggtctccaac 780
   ggtgcaggta gcatgtcagt ctccctagtg gctgatgaga accccttcgc ccagggggcc 840
   ctgagatctg aggactgctt catcctggac catggcagag atgggaaaat ctttgtttgg 900
   aaaggcaagc aggccaacat ggaggagcgg aaggctgccc tcaaaacagc ctctgacttc 960
   atctccaaga tgcagtaccc caggcagacc caggtttcag ttctcccaga gggcggtgag 1020
   acccctctct ttaagcagtt cttcaagaac tggcgggacc cagaccagac agatggcccc 1080
   ggcctgggct acctctccag ccacattgcc aacgtggagc gcgtaccttt cgatgccggc 1140
   acgctgcaca cctccaccgc catggccgct cagcacggca tggatgatga tggaactggc 1200
   cagaaacaga tctggagaat tgaaggttcc aacaaggtgc cagtggaccc tgccacatac 1260
   ggacagttct atggaggcga cagctacatc attctgtaca actaccgcca cggtggccgc 1320
   cagggacaga tcatctacaa ctggcagggt gctcagtcta cccaggatga ggttgctgct 1380
   tctgccatcc tgactgccca gctggatgag gagctgggag gaactcctgt ccagagccga 1440
   gtggtccaag gcaaagagcc tgcacacctc atgagcttgt ttggcgggaa gcccatgatc 1500
   atctacaagg gtggcacctc ccgtgatggt gggcagacag ctcctgccag tatccgcctc 1560
   ttccaagtgc gtgccagcag ctctggagcc accagggctg tggaggtgat gcctaagtct 1620
   ggtgctctga actccaacga tgcctttgtg ctgaaaaccc cctccgctgc ctacctgtgg 1680
   gtgggcgcag gagccagtga ggcagagaag acggcggccc aggagcttct gaaggtcctt 1740
   cggtcccagc atgtgcaggt ggaagaaggc agtgagccag atggcttctg ggaggctctg 1800
   ggcgggaaga cgtcctaccg cacatccccc aggcttaagg acaagaagat ggatgcccat 1860
   cctcctcgac tctttgcctg ctccaacagg atcggacgct ttgtgatcga agaggttcct 1920
   ggcgagctta tgcaggaaga cctggctact gatgacgtca tgctcctgga cacctgggac 1980
   caggtctttg tctgggttgg aaaagactcc caggaagaag aaaagacgga agccttgact 2040
   tctgctaagc ggtacatcga gacagatcca gcaaatcggg acaggcggac ccccatcaca 2100
   gtcgttaggc agggctttga gcctccttcc ttcgtgggct ggttcctcgg ctgggacaac 2160
   aactactggt cggtggatcc tttggaccgg gccttggctg agctggctgc ctgagtaagg 2220
   accaagccat caatgtcacc aatcagtgcc tttgagggtt gtccatctcc caaagacatc 2280
   atatggcaag caggaaaact atgatgtgtg cgcgcgtgtt tttgtttttg ttttttacgg 2340
   tagccaaaac aagcccttgt ggaaactcag ggtctttaca gaattgcttc aaatgtctgt 2400
   actttggaaa tgaaagccaa taaaagcttt ttgaagtgaa aaaaaaa 2447


<210> 74
<211> 236
<212> PRT
<213> Rattus norvegicus

<220>
<223> Cd81 antigen

<400> 74
Met Gly Val Glu Gly Cys Thr Lys Cys Ile Lys Tyr Leu Leu Phe Val
1               5                   10                  15

Phe Asn Phe Val Phe Trp Leu Ala Gly Gly Val Ile Leu Gly Val Ala
            20                  25                  30

Leu Trp Leu Arg His Asp Pro Gln Thr Thr Thr Leu Leu Tyr Leu Glu
        35                  40                  45

Leu Gly Asp Lys Pro Ala Pro Ser Thr Phe Tyr Val Gly Ile Tyr Ile
    50                  55                  60

Leu Ile Ala Val Gly Ala Val Met Met Phe Val Gly Phe Leu Gly Cys
65                  70                  75                  80

Tyr Gly Ala Ile Gln Glu Ser Gln Cys Leu Leu Gly Thr Phe Phe Thr
                85                  90                  95

Cys Leu Val Ile Leu Phe Ala Cys Glu Val Ala Ala Gly Ile Trp Gly
            100                 105                 110

Phe Val Asn Lys Asp Gln Ile Ala Lys Asp Val Lys Gln Phe Tyr Asp
        115                 120                 125

Gln Ala Leu Gln Gln Ala Val Met Asp Asp Asp Ala Asn Asn Ala Lys
    130                 135                 140

Ala Val Val Lys Thr Phe His Glu Thr Leu Asn Cys Cys Gly Ser Asn
145                 150                 155                 160

Thr Leu Thr Thr Leu Thr Thr Ala Val Leu Arg Asn Ser Leu Cys Pro
                165                 170                 175

Ser Ser Ser Asn Ser Phe Thr Gln Leu Leu Lys Glu Asp Cys His Gln
            180                 185                 190

Lys Ile Asp Glu Leu Phe Ser Gly Lys Leu Tyr Leu Ile Gly Ile Ala
        195                 200                 205

Ala Ile Val Val Ala Val Ile Met Ile Phe Glu Met Ile Leu Ser Met
    210                 215                 220

Val Leu Cys Cys Gly Ile Arg Asn Ser Ser Val Tyr
225                 230                 235


<210> 75
<211> 1303
<212> DNA
<213> Rattus norvegicus

<220>
<223> Cd81 antigen

<400> 75
gtggcggtct gagcgagcgc gcccttgctt caaagcggta gcgactctag cgcctccggc 60
taggctccaa cccttctcta ccctacgtct caatagccac accgcagtct ccggcctcaa 120
gcgacccagg ctatcttcca gtcccgggac tccgggtact gcggtcccca gaaccgcccg 180
tccagggacc aacccaagtt ccgcaggccg cggcggccgc caatgggggt ggagggctgc 240
accaaatgca tcaaatacct gctcttcgtc ttcaatttcg tcttctggct ggctggaggt 300
gtgatcctag gtgtagctct gtggttgcgc catgatccac agaccaccac cttgctctac 360

ctggaactgg gagacaaacc agcacctagc accttctatg tgggcatcta cattctcatt 420
gctgtgggag ctgtgatgat gtttgtaggc ttcctggggt gctatggggc catccaggag 480
tcccagtgcc tgctggggac gttcttcact tgccttgtga tcctgtttgc ctgtgaggta 540
gctgcgggca tctggggctt tgtaaacaaa gaccagatcg ccaaggatgt gaagcagttc 600
tacgaccagg cccttcagca ggctgtgatg gatgatgatg ccaacaatgc caaggcagtg 660
gtgaagacct tccatgagac gctcaactgt tgtggctcca atacgctgac cacactgacc 720
accgccgtgc tgaggaacag cctgtgtccc tcaagcagca actcattcac tcagcttctg 780
aaggaagact gccatcagaa aatcgatgag ctcttttctg ggaagctgta cctcattgga 840
attgcagcca ttgtggtagc tgtcattatg atcttcgaga tgattctgag catggtgctg 900
tgctgtggca tccggaacag ctccgtgtac tgaggcccct cgcttcgcac cagaggtccc 960
ctggaatgac cagagggcac cttggggggc atggcctgtg tatatactat ttctgtatta 1020
ctctgctaca cttagccttt ttacttttga gtcttttttg ttctgaactt ttcctgtcac 1080
cttttggagc cgacaccaca cataggtggc gtatgtgggg acgtagaggt ggagctggcc 1140
ctagctggca gggccctgta cttctgggac ccctggagag ttctgcctgc tgagccaaac 1200
ctcctccata gctacttgtc cagaggccac atagccaagc tagaaggcca tgtccaccca 1260
ctctgcccac tgtgggtcac atcgctccca tctttttaat ccg 1303

<210> 76
<211> 81
<212> PRT
<213> Rattus norvegicus

<220>
<223> MOBP-81 protein

<400> 76
Met Ser Gln Lys Val Ala Lys Glu Gly Pro Arg Leu Ser Lys Asn Gln
1               5                   10                  15

Lys Phe Ser Glu His Phe Ser Ile His Cys Cys Pro Pro Phe Thr Phe
            20                  25                  30

Leu Asn Ser Lys Arg Glu Ile Val Asp Arg Lys Tyr Ser Ile Cys Lys
        35                  40                  45

Ser Gly Cys Phe Tyr Gln Lys Lys Glu Glu Asp Trp Ile Cys Cys Ala
    50                  55                  60

Cys Gln Lys Thr Arg Leu Arg Arg Arg Ser Arg Ser Thr Pro Arg Lys
65                  70                  75                  80

Lys

<210> 77
<211> 2405
<212> DNA
<213> Rattus norvegicus

<220>
<223> MOBP-81 protein

<400> 77
ctttcacagc agccaatacc tgcagggcaa caaagaatca aatgagagcg agacaagctg 60
ggaatgaagt cctggttgcc agatgggagc ttgaaaacac agtaagatga gtcaaaaagt 120
ggccaaggag ggccccaggc tctccaagaa ccagaagttc tcagagcact tcagcatcca 180
ctgctgccca cccttcacct tcctcaactc caagcgtgag atcgtggacc gcaagtacag 240
catctgcaag agcggttgct tttaccagaa gaaggaggag gactggatct gctgtgcctg 300
ccagaagacc agattgagaa ggaggagcag gtcaacccca agaaaaaagt gaccgagaag 360
ggacttctcg tgaatggaca gcctctgctt gtggatttac tgcttcaccg caacccatct 420

gccctcagac ttatctggcc tcgagtatga cgcaggggtg gacacctgct gatgtaacaa 480
ctacagatgc cctcagtccc catggtgtgg gagccttagg gcagcctgcc tggagcactt 540
ggacgatgcc actccatcac ctccctctat aaccgccgaa gaggcatctc tgtccacctt 600
ctgaccctcc atggttgttc agtctgaaga ttcctgtgtc ttcagggatg tgcaaaacaa 660
ggtcatggtg tgcgaccatc caaatggcaa atataaaaaa gaaattgtca agtgttggcg 720
gtgacgcaag aaaaatccta gaagccacag atggggattt atacactgtc gcaacaccgt 780
tgagatgtac tctggggaca ctatcaaggc aaagtatctt aaaacctcta atcaagcgac 840
tgtaatttta ggcatccatc ctggggaaaa ctcacaccct tttagagaaa ggagaaatgc 900
ccaaatttgt ttaaaagatc gtggtttgta gttaagagag gtagaatgag attaaccaaa 960
atgtcggtcc acgggagagc aagagagtca atcttggtat gtccacaaag tcctaagagc 1020
agttaaaagc caatctgtca gacagaccca tgccgagcag gtaaatctag aaagcaaacc 1080
aaggactata aaagtcaagc accaggcagt aaatccagaa taagagccta tgcatacata 1140
ttgacagctc acaatcctgt actggttatt gcttacataa ggagaagcca cagatctcat 1200
tctttcttct agaatgcaat agaatttaaa aatgacagca gaacaaaagg acactagaaa 1260
ttatcctctc agaaataaaa gtataagaaa taatttctgg cttgaggaaa taacccaggc 1320
taaaagctac acaggcctgg cttaaccata ccagcagggc atacctttgt taggagaggg 1380
tcaattcgaa gtttgtgaac agcctgggct gtttccgagg ctgcataaat cttaaacacc 1440
aaaaatgtgt gttttgaatt aactttgggt ctggtctaca gagtccggct tgtgatggct 1500
cctgacacgg gagaaaaggg gataagttaa tataaggctc tgggattttc ctttgaggaa 1560
caggcaggta ggaaaggaca ctagaatgtt ggggaatgtg gagggatgta aaacagacag 1620
tattatgatg gggggaactc aggaaggaac ggtaaggaca ccagaatggc ctcgtgctgg 1680
gagccagggt ggccaagccc aggcagatgc acaaagaccc caaaggccac cattgcctca 1740
caaccagacc cagcaaagtt cagatcaagt gcataaatgg gggtcacgtc agtggaaact 1800
gtccggagca tggagcaggg ggtgaggaag acagtcagaa ggaagcaaga gatggcttag 1860
agtgttgcat ggcagcaaga agaagcagtc caaatatcat cccaaggcag ccatggcttc 1920
ctctcccgag gacactgtgg ccctgtggaa gtgccaaggc agaggcagag gaatttaacc 1980
gcacacaaac tacatgcaga aactaaggtg ctaaatgaac atctacgtga caacctttct 2040
gaggaccagg ttccagtgag cggcccaggg ccgttatctt cgcgatggta actgagctga 2100
gaagaatggg aaccggatag gagcacacag tagcccactg gtgagacaaa tccacgggcg 2160
agcctcaacc aacccactaa ggaaacttca cgccttttca tctagttttc attttggcaa 2220
agcaaagcca tcctgagtgc ttgcttgtct ccgccctcca cgccaccccc gagacacaga 2280
gcatgcgcat taacccagag cgcgctaaca catgcacagc gttggacatt agcctattaa 2340
tgggaataac cagaaactac taaattttgt ttgttgtaga aataaaacct ggggatggat 2400
caaaa 2405

<210> 78
<211> 590
<212> PRT
<213> Homo sapiens

<220>
<223> Syntaxin binding protein 2

<400> 78
Met Ala Pro Ser Gly Leu Lys Ala Val Val Gly Glu Lys Ile Leu Ser
1               5                   10                  15

Gly Val Ile Arg Ser Val Lys Lys Asp Gly Glu Trp Lys Val Leu Ile
            20                  25                  30

Met Asp His Pro Ser Met Arg Ile Leu Ser Ser Cys Cys Lys Met Ser
        35                  40                  45

Asp Ile Leu Ala Glu Gly Ile Thr Ile Val Glu Asp Ile Asn Lys Arg
    50                  55                  60

Arg Glu Pro Ile Pro Ser Leu Glu Ala Ile Tyr Leu Leu Ser Pro Thr
65                  70                  75                  80

Glu Lys Ala Leu Ile Lys Asp Phe Gln Gly Thr Pro Thr Phe Thr Tyr
                85                  90                  95

Lys Ala Ala His Ile Phe Phe Thr Asp Thr Cys Pro Glu Pro Leu Phe
            100                 105                 110

Ser Glu Leu Gly Arg Ser Arg Leu Ala Lys Val Val Lys Thr Leu Lys
        115                 120                 125

Glu Ile His Leu Ala Phe Leu Pro Tyr Glu Ala Gln Val Phe Ser Leu
    130                 135                 140

Asp Ala Pro His Ser Thr Tyr Asn Leu Tyr Cys Pro Phe Arg Ala Glu
145                 150                 155                 160

Glu Arg Thr Arg Gln Leu Glu Val Leu Ala Gln Gln Ile Ala Thr Leu
                165                 170                 175

Cys Ala Thr Leu Gln Glu Tyr Pro Ala Ile Arg Tyr Arg Lys Gly Pro
            180                 185                 190

Glu Asp Thr Ala Gln Leu Ala His Ala Val Leu Ala Lys Leu Asn Ala
        195                 200                 205

Phe Lys Ala Asp Thr Pro Ser Leu Gly Glu Gly Pro Glu Lys Thr Arg
    210                 215                 220

Ser Gln Leu Leu Ile Met Asp Arg Ala Ala Asp Pro Val Ser Pro Leu
225                 230                 235                 240

Leu His Glu Leu Thr Phe Gln Ala Met Ala Tyr Asp Leu Leu Asp Ile
                245                 250                 255

Glu Gln Asp Thr Tyr Arg Tyr Glu Thr Thr Gly Leu Ser Glu Ala Arg
            260                 265                 270

Glu Lys Ala Val Leu Leu Asp Glu Asp Asp Asp Leu Trp Val Glu Leu
        275                 280                 285

Arg His Met His Ile Ala Asp Val Ser Lys Lys Val Thr Glu Leu Leu
    290                 295                 300

Arg Thr Phe Cys Glu Ser Lys Arg Leu Thr Thr Asp Lys Ala Asn Ile
305                 310                 315                 320

Lys Asp Leu Ser Gln Ile Leu Lys Lys Met Pro Gln Tyr Gln Lys Glu
                325                 330                 335

Leu Asn Lys Tyr Ser Thr His Leu His Leu Ala Asp Asp Cys Met Lys
            340                 345                 350

His Phe Lys Gly Ser Val Glu Lys Leu Cys Ser Val Glu Gln Asp Leu
        355                 360                 365

Ala Met Gly Ser Asp Ala Glu Gly Glu Lys Ile Lys Asp Ser Met Lys
    370                 375                 380

Leu Ile Val Pro Val Leu Leu Asp Ala Ala Val Pro Ala Tyr Asp Lys
385                 390                 395                 400

Ile Arg Val Leu Leu Leu Tyr Ile Leu Leu Arg Asn Gly Val Ser Glu
                405                 410                 415

Glu Asn Leu Ala Lys Leu Ile Gln His Ala Asn Val Gln Ala His Ser

420                 425                 430

Ser Leu Ile Arg Asn Leu Glu Gln Leu Gly Gly Thr Val Thr Asn Pro
        435                 440                 445

Gly Gly Ser Gly Thr Ser Ser Arg Leu Glu Pro Arg Glu Arg Met Glu
    450                 455                 460

Pro Thr Tyr Gln Leu Ser Arg Trp Thr Pro Val Ile Lys Asp Val Met
465                 470                 475                 480

Glu Asp Ala Val Glu Asp Arg Leu Asp Arg Asn Leu Trp Pro Phe Val
                485                 490                 495

Ser Asp Pro Ala Pro Thr Ala Ser Ser Gln Ala Ala Val Ser Ala Arg
            500                 505                 510

Phe Gly His Trp His Lys Asn Lys Ala Gly Ile Glu Ala Arg Ala Gly
        515                 520                 525

Pro Arg Leu Ile Val Tyr Val Met Gly Gly Val Ala Met Ser Glu Met
    530                 535                 540

Arg Ala Ala Tyr Glu Val Thr Arg Ala Thr Glu Gly Lys Trp Glu Val
545                 550                 555                 560

Leu Ile Gly Ser Ser His Ile Leu Thr Pro Thr Arg Phe Leu Asp Asp
                565                 570                 575

Leu Lys Ala Leu Asp Lys Lys Leu Glu Asp Ile Ala Leu Pro
            580                 585                 590


<210> 79
<211> 1888
<212> DNA
<213> Homo sapiens

<220>
<223> Syntaxin binding protein 2

<400> 79
ggcacgaggg gcggcggcgc ccctcgggga agatggcgcc ctcggggctg aaggcggtgg 60
tgggggaaaa aattctgagc ggagttattc ggagtgtcaa gaaggatggg gagtggaagg 120
tgcttatcat ggatcaccca agcatgcgca tcttgtcttc ctgctgcaaa atgtcagata 180
tcctggctga gggcatcacc attgttgaag acatcaacaa acggcgggaa cccattccca 240
gtctggaggc catttatttg ctgagcccca cggagaaggc cctgatcaaa gacttccagg 300
ggaccccgac tttcacctac aaagcggccc atatcttctt caccgacacc tgccccgagc 360
ccctgttcag tgagctaggc cgctctcgtc tggcaaaggt ggtgaagacg ttgaaggaga 420
ttcaccttgc cttcctcccc tacgaggccc aggtgttctc cctcgatgct ccccacagca 480
cctacaacct ctactgcccc ttccgggcag aggagcgcac gcggcagctc gaggtgctgg 540
cccagcagat tgccacgctg tgcgccaccc tgcaggagta cccggccatc cgctaccgca 600
agggcccaga ggacacagcc cagttggccc acgccgtcct ggccaagctg aacgccttca 660
aggcagacac tcccagtctg ggcgagggcc cagagaaaac ccgctcccag ctgctgataa 720
tggaccgggc agctgacccc gtgtccccac tactgcatga gctcacgttc caggccatgg 780
cgtatgatct gctggacata gagcaggaca catacaggta tgagaccacc gggctgagcg 840
aggcgcggga gaaggccgtc ttgctggacg aggacgatga cttgtgggtg gagcttcgcc 900
acatgcatat cgcagatgtg tccaagaagg tcacggagct cctgaggacc ttctgtgaga 960
gcaagaggct gaccacggac aaggcgaaca tcaaagacct atcccagatc ctgaaaaaga 1020
tgccgcagta ccagaaggag ctgaataagt attctacgca cctgcatcta gcagatgatt 1080
gtatgaagca cttcaagggc tcggtggaga agctgtgtag tgtggagcag gacctggcca 1140
tgggctccga cgcagagggg gagaagatca aggactccat gaagctgatc gttccggtgc 1200

tgctggacgc ggcggtgccc gcctacgaca agatccgggt cctgctgctc tacatcctcc 1260
ttcggaatgg tgtgagtgag gagaacctgg ccaagctgat ccagcatgcc aatgtacagg 1320
cgcacagcag cctcatccgt aacctggagc agctgggagg cactgtcacc aaccccgggg 1380
gctcggggac ctccagccgg ctggagccga gagaacgcat ggagcccacc tatcagctgt 1440
cccgctggac cccggtcatc aaggatgtaa tggaggacgc cgtggaggac cggctggaca 1500
ggaacctgtg gcccttcgta tccgaccccg cccccacggc cagctcccag gccgctgtca 1560
gtgcccgctt cggtcactgg cacaagaaca aggctggcat agaagcccgg gcgggccccc 1620
ggctcatcgt gtatgtcatg ggcggtgtgg ccatgtcaga gatgagggcc gcctacgagg 1680
tgaccagggc caccgagggc aagtgggagg tgctcattgg ctcctcacac atcctcaccc 1740
cgacccgctt cctggatgac ctgaaggcac tggacaagaa gctggaggac attgccctgc 1800
cctgacccct ggccccgccc cctacccctc cctttccaga gaaataaact cttcccgtcg 1860
ctctgccaaa aaaaaaaaaa aaaaaaa 1888

<210> 80
<211> 505
<212> PRT
<213> Rattus norvegicus

<220>
<223> Alpha-internexin

<400> 80
Met Ser Phe Gly Ser Glu His Tyr Leu Cys Ser Ala Ser Ser Tyr Arg
1               5                   10                  15

Lys Val Phe Gly Asp Gly Ser Arg Leu Ser Ala Arg Leu Ser Gly Pro
            20                  25                  30

Gly Ala Ser Gly Ser Phe Arg Ser Gln Ser Leu Ser Arg Ser Asn Val
        35                  40                  45

Ala Ser Thr Ala Ala Cys Ser Ser Ala Ser Ser Leu Gly Leu Gly Leu
    50                  55                  60

Ala Tyr Arg Arg Leu Pro Ala Ser Asp Gly Leu Asp Leu Ser Gln Ala
65                  70                  75                  80

Ala Ala Arg Thr Asn Glu Tyr Lys Ile Ile Arg Thr Asn Glu Lys Glu
                85                  90                  95

Gln Leu Gln Gly Leu Asn Asp Arg Phe Ala Val Phe Ile Glu Lys Val
            100                 105                 110

His Gln Leu Glu Thr Gln Asn Arg Ala Leu Glu Ala Glu Leu Ala Ala
        115                 120                 125

Leu Arg Gln Arg His Ala Glu Pro Ser Arg Val Gly Glu Leu Phe Gln
    130                 135                 140

Arg Glu Leu Arg Glu Leu Arg Ala Gln Leu Glu Glu Ala Ser Ser Ala
145                 150                 155                 160

Arg Ala Gln Ala Leu Leu Glu Arg Asp Gly Leu Ala Glu Glu Val Gln
                165                 170                 175

Arg Leu Arg Ala Arg Cys Glu Glu Glu Ser Arg Gly Arg Glu Gly Ala
            180                 185                 190

Glu Arg Ala Leu Lys Ala Gln Gln Arg Asp Val Asp Gly Ala Thr Leu
        195                 200                 205

Ala Arg Leu Asp Leu Glu Lys Lys Val Glu Ser Leu Leu Asp Glu Leu
    210                 215                 220

Ala Phe Val Arg Gln Val His Asp Glu Glu Val Ala Glu Leu Leu Ala
225                 230                 235                 240

Thr Leu Gln Ala Ser Ser Gln Ala Ala Ala Glu Val Asp Val Ala Val
                245                 250                 255

Ala Lys Pro Asp Leu Thr Ser Ala Leu Arg Glu Ile Arg Ala Gln Tyr
            260                 265                 270

Glu Ser Leu Ala Ala Lys Asn Leu Gln Ser Ala Glu Glu Trp Tyr Lys
        275                 280                 285

Ser Lys Phe Ala Asn Leu Asn Glu Gln Ala Ala Arg Ser Thr Glu Ala
    290                 295                 300

Ile Arg Ala Ser Arg Glu Glu Ile His Glu Tyr Arg Arg Gln Leu Gln
305                 310                 315                 320

Ala Arg Thr Ile Glu Ile Glu Gly Leu Arg Gly Ala Asn Glu Ser Leu
                325                 330                 335

Glu Arg Gln Ile Leu Glu Leu Glu Glu Arg His Ser Ala Glu Val Ala
            340                 345                 350

Gly Tyr Gln Asp Ser Ile Gly Gln Leu Glu Ser Asp Leu Arg Asn Thr
        355                 360                 365

Lys Ser Glu Met Ala Arg His Leu Arg Glu Tyr Gln Asp Leu Leu Asn
    370                 375                 380

Val Lys Met Ala Leu Asp Ile Glu Ile Ala Ala Tyr Arg Lys Leu Leu
385                 390                 395                 400

Glu Gly Glu Glu Thr Arg Phe Ser Thr Ser Gly Leu Ser Ile Ser Gly
                405                 410                 415

Leu Asn Pro Leu Pro Asn Pro Ser Tyr Leu Leu Pro Pro Arg Ile Leu
            420                 425                 430

Ser Ser Thr Thr Ser Lys Val Ser Ser Ala Gly Leu Ser Leu Lys Lys
        435                 440                 445

Glu Glu Glu Glu Glu Glu Glu Glu Glu Glu Gly Ala Ser Lys Glu Val
    450                 455                 460

Thr Lys Lys Thr Ser Lys Val Gly Glu Ser Phe Glu Glu Thr Leu Glu
465                 470                 475                 480

Glu Thr Val Val Ser Thr Lys Lys Thr Glu Lys Ser Thr Ile Glu Glu
                485                 490                 495

Ile Thr Thr Ser Ser Ser Gln Lys Met
            500                 505


<210> 81
<211> 4593
<212> DNA
<213> Rattus norvegicus

<220>
<223> Alpha-internexin

<400> 81
gaattctgaa ctacaaagca aagcgcttca catttccctg acttgtgcca taggctggaa 60
ggtaaactca gacaactgag ccatccattg ttcaggctct actctgtcct taccgggtgt 120
ggcgagggtg gccagttctg tcagccgtgc tcctgcttca ctcctggaat tgaatcaaag 180
actaatcccc acagaaaatg gttctcccat ctaattcaaa atggaatgtc aaacgcatac 240
agggccagac agatgaaaag acgaggtgga aagggtagct ctggagtgct gggagctgtg 300
aggaacaccg cacttaaaca aaggcactca cttccgccct ctgcagtcat cctaaacctt 360
ctactttcaa gagtggcagg ggctgggcat taaaaggtca aaatcaatcc agtctctttt 420
tagatgaaac ctcctgacta aaaattcatt aaaaaaaaac attttgaaat ctattgtttt 480
gttggtttgg gtctttttgt ttaaaccaaa gcctgcagac tcctttctac cagcctgcgg 540
tcctgggatg cgcggttgat tggaggtcta gagatgaacc ctgctaattc tcaacgcttt 600
gctccttttc caggcctagc ctctttccct ccccgtaacc cttcgcaccc ttggaaataa 660
gcctcttgtt cttcaccaat gtccggagca tctcacgctc tctgcgaatc ctcccgttta 720
ctgtggctgc ggagctgaga gatgctctct gaagtggacc cactttctcc gaaaaggacc 780
aggagacacc taagtccaga agataaagag gagggtgctt ctccccctga aaagggtggg 840
gacagggcct cacccttggg aactctggga tgcagatgag aacagagaca cctttcagaa 900
aaacctactc tccttggggg ataagcacag aaaaggaagg acaggctcta aagccatcaa 960
agagctgagt gctctgggct gggaagccaa ggttgttggg gcactccctc ccctccggtc 1020
attccctagg cagattcttg gggcacaggc gcagcggcca gggacggccc aggtcggtgg 1080
cgcgcagctg cggtggcact gcgcttcccc gcccccgcag gccccccttc ctctccccgc 1140
tagatctgaa gatgaagctc cacccctagc gcgtcgcccc agccccgcgc cttaaaagcc 1200
ccgcacaccg ccccgccgca cccagccttg ccgcaccttc gcgtcctcgc caggtccgcc 1260
gcagccgcgc acccggcccc gaccccggca ccatgagctt cggatcagag cactacttgt 1320
gctccgcctc ctcctaccgc aaggtgttcg gggatggctc gcgcctgtct gcgcgcctgt 1380
ccgggccggg agcgtccggc agctttcgct cgcagtcgct gtcccgcagc aatgtggcct 1440
ccacggccgc ttgctcctcg gcctcgtctc tcggcctggg cctggcctac cgccgcctgc 1500
cggcctccga cgggctggac ctgagccaag cagcggcgcg caccaacgag tacaagatca 1560
tccgcactaa cgagaaggag cagctgcagg gcctcaacga ccgcttcgcg gtgttcatcg 1620
aaaaggtgca ccagctggag acgcagaacc gcgcgctcga ggccgagctg gccgcgctgc 1680
gccagcgcca cgccgagccg tcgcgcgtcg gcgagctctt ccagcgcgag ctgcgcgagc 1740
tgcgcgcgca gctggaggag gcgagctccg cgcgcgcgca ggccctgctg gagcgcgacg 1800
ggctggccga ggaggtgcag cgactgcggg cgcgctgcga ggaggagagc cgcgggcgcg 1860
aaggcgcgga gcgcgccctg aaggcgcagc agcgcgacgt ggacggcgcc actctggccc 1920
gcctggatct ggagaagaag gtggagtcgc tgctggacga gctggccttc gtgcgccagg 1980
tgcacgacga ggaggtggcc gagctcctgg ccacgctgca ggcgtcttcg caagccgccg 2040
ccgaggtgga cgtggctgtg gctaaaccag acctgacttc ggcgctgagg gagatccgcg 2100
cccagtatga gtccttggcc gctaagaacc tgcagtcggc cgaggagtgg tacaagtcca 2160
agttcgccaa cctgaacgag caggctgcgc gcagcacgga agccatccga gccagccgag 2220
aggagatcca cgagtaccgg cgccagctcc aggctcgtac cattgagata gagggcctgc 2280
gcggagccaa tgaatccctg gagaggcaga tcttggaact ggaggagcgg cacagcgctg 2340
aggtggccgg ctaccaggat agcattgggc agctggagag tgacctgagg aacaccaaga 2400
gcgagatggc ccgccacctt cgggaatacc aggacttgct caatgtcaaa atggctcttg 2460
acattgagat agcagcttac aggaagctgc tggaaggcga agagacacga tttagcacca 2520
gcgggttaag catctcaggg ctgaatccac ttcccaatcc cagttatttg ctccctccta 2580
gaatccttag ttctacaacc tccaaagtct catccgctgg gctgtccctg aaaaaggagg 2640
aggaggagga ggaggaagag gaagaagggg cttctaagga agtcactaag aaaacatcca 2700
aggtagggga aagtttcgaa gaaacactgg aggaaacggt ggtatctacg aagaaaaccg 2760
aaaagtcaac tatagaagaa atcaccacca gttccagcca aaaaatgtaa tgctgttgct 2820
taaaaaaaaa aaacaaaaaa aggcgatgcc taagagggaa tgcacttggc ttattcacag 2880
cttactcctg aaccacacct accaccgcaa tagaatgtct tcaaggcttc aatgtcatag 2940
tctgtaccga atcctcgctc tctccagtag gaaggccact gtagctggga gcaaactgca 3000
gtgctggagg aggagcagga ggagctctct aagatcacag ctttcctacc tacagctcag 3060
gcctcatagt gacaggtgac ccaggtgcag aggaggctca aacgaaggtg ctaaatctgg 3120
tcatcgctcg ctgtgagctg aaatcgaaac ctgtttctcc accgtacccc gccagtaccc 3180
agccatagaa cctcactgta gatgtctaaa aatatacagt cactgccgag gacaagctag 3240
tgctccagac accattctcc catccccctc tgatagagct gagtcacaaa acgacggtcg 3300
tctctcatgg gcaggtttcc ttcctctctc ttcaatcccg tattgccatt tctcccacct 3360
gagaggaagg aacaaagttt agtgtggtat ccccttctag acacgcatag aacttacgcc 3420

ctttagtcct cactccgtgg gtaaattaga tgcatgttga ccattcccgt gatttgtata 3480
acgcaccctt gtccatttca tgacctttca tgtccgtagc ttaaaaaata gaaagtgatt 3540
aatgataatg gcagtgattg gttttaaggg ttttcctaaa ataatactaa ttataataat 3600
aatcataaaa gggatctcta gactttaggc tttcagctta tccagatcca cttaattaga 3660
gaggaaaaaa attaccttaa atgatgtagc atttcacctt aaatttagag gcacggtgat 3720
agttgcccta ctctgcgctt aagctgacct tagacctcgt atcgtaagta gagtgagtga 3780
gtgagtgctc tgaaagggaa agaacaaggt ctcctctgtg ttggtttcta tgtagtgcta 3840
tagaattagc tttctcatac ccttgcccta ggatctacat aattcccttc ccattagggt 3900
agtatgctat gtataggaca ttttatcacc agacacaacg ctactgccca aaagtgaaaa 3960
acacacactt ctggtcaatc aacaccacca gcaaatattt aaaaaaaata aaaaaggaag 4020
gaagaaagaa agaaagaaag aaagaaagaa agagagagag agagagaaag agagaaagaa 4080
agagaaagat tgattctcag agtcttttcc taacagggtt acattgcttc agttggtgga 4140
tgtccctccc ctgaaggaaa gacagaaaca ttattcaggg tccaggaggg gtatgcacac 4200
ttctccccca cctcaagaag gcaggctgga aagtcagctc ttcgtgcact caactgtggc 4260
atccacatga cctgtctgcc tcgcgtgcca aacttgatgc agggtttgcc ctgtctctgc 4320
tcactcccac caagctgtag aactgtctgt cctgtccccg cccaccccat cgcaagctct 4380
gtgctgataa ggacatgatg cttctactga attcacattc tagcacgtgc ttcaatatag 4440
ctcaaggaac cggaaagaca cttcgttttt cctgacccat cccctcttga cccccccccc 4500
acactgtaag acattagtaa tattaagaaa ttaaaagtca ttaatcctat gtggttcttg 4560
tctgtggtct ctatagctcc tgaggtgtgc att 4593

<210> 82
<211> 320
<212> PRT
<213> Mus musculus

<220>
<223> Beta-sarcoglycan

<400> 82
Met Ala Ala Ala Ala Ala Ala Ala Ala Ala Thr Glu Gln Gln Gly Ser
1               5                   10                  15

Asn Gly Pro Val Lys Lys Ser Met Arg Glu Lys Ala Val Glu Arg Arg
            20                  25                  30

Asn Val Asn Lys Glu His Asn Ser Asn Phe Lys Ala Gly Tyr Ile Pro
        35                  40                  45

Ile Asp Glu Asp Arg Leu His Lys Thr Gly Leu Arg Gly Arg Lys Gly
    50                  55                  60

Asn Leu Ala Ile Cys Val Ile Val Leu Leu Phe Ile Leu Ala Val Ile
65                  70                  75                  80

Asn Leu Leu Ile Thr Leu Val Ile Trp Ala Val Ile Arg Ile Gly Pro
                85                  90                  95

Asn Gly Cys Asp Ser Met Glu Phe His Glu Ser Gly Leu Leu Arg Phe
            100                 105                 110

Lys Gln Val Ser Asp Met Gly Val Ile His Pro Leu Tyr Lys Ser Thr
        115                 120                 125

Val Gly Gly Arg Arg Asn Glu Asn Leu Val Ile Thr Gly Asn Asn Gln
    130                 135                 140

Pro Ile Val Phe Gln Gln Gly Thr Thr Lys Leu Ser Val Glu Lys Asn
145                 150                 155                 160

Lys Thr Ser Ile Thr Ser Asp Ile Gly Met Gln Phe Phe Asp Pro Arg

                165                 170                 175

Thr His Asn Ile Leu Phe Ser Thr Asp Tyr Glu Thr His Glu Phe His
            180                 185                 190

Leu Pro Ser Gly Val Lys Ser Leu Asn Val Gln Lys Ala Ser Thr Glu
        195                 200                 205

Arg Ile Thr Ser Asn Ala Thr Ser Asp Leu Asn Ile Lys Val Asp Gly
    210                 215                 220

Arg Ala Ile Val Arg Gly Asn Glu Gly Val Phe Ile Met Gly Lys Thr
225                 230                 235                 240

Ile Glu Phe His Met Gly Gly Asp Val Glu Leu Lys Ala Glu Asn Ser
                245                 250                 255

Ile Ile Leu Asn Gly Thr Val Met Val Ser Pro Thr Arg Leu Pro Ser
            260                 265                 270

Ser Ser Ser Gly Asp Gln Ser Gly Ser Gly Asp Trp Val Arg Tyr Lys
        275                 280                 285

Leu Cys Met Cys Ala Asp Gly Thr Leu Phe Lys Val Gln Val Thr Gly
    290                 295                 300

His Asn Met Gly Cys Gln Val Ser Asp Asn Pro Cys Gly Asn Thr His
305                 310                 315                 320

<210> 83
<211> 1237
<212> DNA
<213> Mus musculus

<220>
<223> Beta-sarcoglycan

<400> 83
gagagcgcaa gcgcgggctg cggcgggcca agatggcggc agcggcggcg gcggcggcag 60
cgaccgagca gcaagggtcc aacgggccag tgaagaaatc catgcgggag aaggctgtgg 120
agcggaggaa tgttaataaa gagcacaaca gcaatttcaa agctggatat atccccatcg 180
atgaggaccg gctccataag actgggctga gggggcgaaa gggcaactta gccatctgcg 240
tgatcgtcct cctgtttatc ctggccgtca tcaatctact cattacactt gtcatctggg 300
cggtgatccg cattggccca aatgggtgtg atagcatgga gttccacgag agcggtctgc 360
tgaggttcaa gcaagtgtct gacatgggcg tcatccaccc gctttataag agcacggtgg 420
gaggacggcg gaatgaaaac ctggtcatca ctggcaacaa ccagccaatt gtcttccagc 480
aagggacgac caagctgagt gttgaaaaga acaagacctc catcaccagc gacatcggga 540
tgcagttttt tgacccaagg acacacaata tcctgttcag cacggactat gagacgcacg 600
agtttcatct gccaagtggg gtgaaaagtt tgaacgttca gaaagcatcg actgaaagga 660
ttaccagcaa tgctaccagt gacttaaaca tcaaagtcga tgggcgtgca attgtgcgag 720
gcaacgaagg cgtcttcatc atgggcaaaa ccattgaatt tcacatggga ggagatgtgg 780
agttaaaagc tgaaaacagc atcatcctca acggcacggt gatggtcagc cctacgcgcc 840
tgcccagttc ctccagcggg gaccagtcag ggagtggtga ctgggtgcgc tacaagctct 900
gcatgtgtgc agatggcaca ctcttcaaag tacaagtaac aggtcacaac atgggctgcc 960
aggtctcaga caacccttgt gggaacactc attagacggt gcatccgcag gctcagcgcg 1020
ctcagatctg acttgacctt ccccttttta agaatgcatg tgaaccatgt ttgtacagcg 1080
tttgccataa cttgtaatta ttttacctgg ggagcaccac tgtgtctatt atgtactctc 1140
catatttaag ggatcttgag tgcctacatt ctaatacatt ttagtaagtt gcactgatac 1200

tcaacagttc tctaaaacaa tgatgagaat taataaa                                1237

<210> 84
<211> 251
<212> PRT
<213> Homo sapiens

<220>
<223> CGI-78 protein

<400> 84
Met Gly Ala Ala Val Phe Phe Gly Cys Thr Phe Val Ala Phe Gly Pro
1               5                   10                  15

Ala Phe Ala Leu Phe Leu Ile Thr Val Ala Gly Asp Pro Leu Arg Val
            20                  25                  30

Ile Ile Leu Val Ala Gly Ala Phe Phe Trp Leu Val Ser Leu Leu Leu
        35                  40                  45

Ala Ser Val Val Trp Phe Ile Leu Val His Val Thr Asp Arg Ser Asp
    50                  55                  60

Ala Arg Leu Gln Tyr Gly Leu Leu Ile Phe Gly Ala Ala Val Ser Val
65                  70                  75                  80

Leu Leu Gln Glu Val Phe Arg Phe Ala Tyr Tyr Lys Leu Leu Lys Lys
                85                  90                  95

Ala Asp Glu Gly Leu Ala Ser Leu Ser Glu Asp Gly Arg Ser Pro Ile
            100                 105                 110

Ser Ile Arg Gln Met Ala Tyr Val Ser Gly Leu Ser Phe Gly Ile Ile
        115                 120                 125

Ser Gly Val Phe Ser Val Ile Asn Ile Leu Ala Asp Ala Leu Gly Pro
    130                 135                 140

Gly Val Val Gly Ile His Gly Asp Ser Pro Tyr Tyr Phe Leu Thr Ser
145                 150                 155                 160

Ala Phe Leu Thr Ala Ala Ile Ile Leu Leu His Thr Phe Trp Gly Val
                165                 170                 175

Val Phe Phe Asp Ala Cys Glu Arg Arg Arg Tyr Trp Ala Leu Gly Leu
            180                 185                 190

Val Val Gly Ser His Leu Leu Thr Ser Gly Leu Thr Phe Leu Asn Pro
        195                 200                 205

Trp Tyr Glu Ala Ser Leu Leu Pro Ile Tyr Ala Val Thr Val Ser Met
    210                 215                 220

Gly Leu Trp Ala Phe Ile Thr Ala Gly Gly Ser Leu Arg Ser Ile Gln
225                 230                 235                 240

Arg Ser Ser Cys Val Arg Thr Asp Tyr Leu Asp
                245                 250

<210> 85

<211> 1967
<212> DNA
<213> Homo sapiens

<220>
<223> CGI-78 protein

<400> 85
gtggggtcgc gttgccaccc cacgcggact ccccagctgg cgcgcccctc ccatttgcct 60
gtcctggtca ggcccccacc ccccttccca cctgaccagc catgggggct gcggtgtttt 120
tcggctgcac tttcgtcgcg ttcggcccgg ccttcgcgct tttcttgatc actgtggctg 180
gggacccgct tcgcgttatc atcctggtcg caggggcatt tttctggctg gtctccctgc 240
tcctggcctc tgtggtctgg ttcatcttgg tccatgtgac cgaccggtca gatgcccggc 300
tccagtacgg cctcctgatt tttggtgctg ctgtctctgt ccttctacag gaggtgttcc 360
gctttgccta ctacaagctg cttaagaagg cagatgaggg gttagcatcg ctgagtgagg 420
acggaagatc acccatctcc atccgccaga tggcctatgt ttctggtctc tccttcggta 480
tcatcagtgg tgtcttctct gttatcaata ttttggctga tgcacttggg ccaggtgtgg 540
ttgggatcca tggagactca ccctattact tcctgacttc agcctttctg acagcagcca 600
ttatcctgct ccataccttt tggggagttg tgttctttga tgcctgtgag aggagacggt 660
actgggcttt gggcctggtg gttgggagtc acctactgac atcgggactg acattcctga 720
acccctggta tgaggccagc ctgctgccca tctatgcagt cactgtttcc atggggctct 780
gggccttcat cacagctgga gggtccctcc gaagtattca gcgcagctct tgtgtaagga 840
ctgactacct ggactgatcg cctgacagat cccacctgcc tgtccactgc ccatgactga 900
gcccagcccc agcccggggt ccattgccca cattctctgt ctccttctcg tcggtctacc 960
ccactacctc cagggttttg ctttgtcctt ttgtgaccgt tagtctctaa gctttaccag 1020
gagcagcctg ggttcagcca gtcagtgact ggtgggtttg aatctgcact tatccccacc 1080
acctggggac ccccttgttg tgtccaggac tccccctgtg tcagtgctct gctctcaccc 1140
tgcccaagac tcacctccct tcccctctgc aggccgacgg caggaggaca gtcgggtgat 1200
ggtgtattct gccctgcgca tcccacccga ggactgaggg aacctagggg ggacccctgg 1260
gcctggggtg ccctcctgat gtcctcgccc tgtatttctc catctccagt tctggacagt 1320
gcaggttgcc aagaaaaggg acctagttta gccattgccc tggagatgaa attaatggag 1380
gctcaaggat agatgagctc tgagtttctc agtactccct caagactgga catcttggtc 1440
tttttctcag gcctgagggg gaaccatttt tggtgtgata aataccctaa actgcctttt 1500
tttctttttt gaggtggggg gagggaggag gtatattgga actcttctaa cctccttggg 1560
ctatattttc tctcctcgag ttgctcctca tggctgggct catttcggtc cctttctcct 1620
tggtcccaga ccttggggga aaggaaggaa gtgcatgttt gggaactggc attactggaa 1680
ctaatggttt taacctcctt aaccaccagc atccctcctc tccccaaggt gaagtggagg 1740
gtgctgtggt gagctggcca ctccagagct gcagtgccac tggaggagtc agactaccat 1800
gacatcgtag ggaaggaggg gagatttttt tgtagttttt aattggggtg tgggaggggc 1860
ggggaggttt tctataaact gtatcatttt ctgctgaggg tggagtgtcc catcctttta 1920
atcaaggtga ttgtgatttt gactaataaa aaagaatttg taaaaaa 1967


<210> 86
<211> 885
<212> PRT
<213> Homo sapiens

<220>
<223> KIAA0143 protein

<400> 86
Ala Gly Gly Gly Ser Cys Arg Pro Leu Gly Cys Val Thr Ala Gly Ser
1               5                   10                  15

Ala Ser Ala Pro Ser Thr Leu Arg Pro Ser Pro Phe Ala Ser Ser Arg
            20                  25                  30

Pro Pro Arg Pro Ser Asn Gly Arg His Gly Ala Val Gly Ala Pro Cys
        35                  40                  45

Ala Ala Pro Leu Ser Leu Gly Ala Ala Ala Ser Ala Val Glu Ile Ala

50                  55                  60

Met Pro Thr Arg Val Cys Cys Cys Cys Ser Ala Leu Arg Pro Arg Tyr
65                  70                  75                  80

Lys Arg Leu Val Asp Asn Ile Phe Pro Glu Asp Pro Lys Asp Gly Leu
                85                  90                  95

Val Lys Thr Asp Met Glu Lys Leu Thr Phe Tyr Ala Val Ser Ala Pro
            100                 105                 110

Glu Lys Leu Asp Arg Ile Gly Ser Tyr Leu Ala Glu Arg Leu Ser Arg
        115                 120                 125

Asp Val Val Arg His Arg Ser Gly Tyr Val Leu Ile Ala Met Glu Ala
    130                 135                 140

Leu Asp Gln Leu Leu Met Ala Cys His Ser Gln Ser Ile Lys Pro Phe
145                 150                 155                 160

Val Glu Ser Phe Leu His Met Val Ala Lys Leu Leu Glu Ser Gly Glu
                165                 170                 175

Pro Lys Leu Gln Val Leu Gly Thr Asn Ser Phe Val Lys Phe Ala Asn
            180                 185                 190

Ile Glu Glu Asp Thr Pro Ser Tyr His Arg Arg Tyr Asp Phe Phe Val
        195                 200                 205

Ser Arg Phe Ser Ala Met Cys His Ser Cys His Ser Asp Pro Glu Ile
    210                 215                 220

Arg Thr Glu Ile Arg Ile Ala Gly Ile Arg Gly Ile Gln Gly Val Val
225                 230                 235                 240

Arg Lys Thr Val Asn Asp Glu Leu Arg Ala Thr Ile Trp Glu Pro Gln
                245                 250                 255

His Met Asp Lys Ile Val Pro Ser Leu Leu Phe Asn Met Gln Lys Ile
            260                 265                 270

Glu Glu Val Asp Ser Arg Ile Gly Pro Pro Ser Ser Pro Ser Ala Thr
        275                 280                 285

Asp Lys Glu Glu Asn Pro Ala Val Leu Ala Glu Asn Cys Phe Arg Glu
    290                 295                 300

Leu Leu Gly Arg Ala Thr Phe Gly Asn Met Asn Asn Ala Val Arg Pro
305                 310                 315                 320

Val Phe Ala His Leu Asp His His Lys Leu Trp Asp Pro Asn Glu Phe
                325                 330                 335

Ala Val His Cys Phe Lys Ile Ile Met Tyr Ser Ile Gln Ala Gln Tyr
            340                 345                 350

Ser His His Val Ile Gln Glu Ile Leu Gly His Leu Asp Ala Arg Lys
        355                 360                 365

Lys Asp Ala Pro Arg Val Arg Ala Gly Ile Ile Gln Val Leu Leu Glu
    370                 375                 380

Ala Val Ala Ile Ala Ala Lys Gly Ser Ile Gly Pro Thr Val Leu Glu
385                 390                 395                 400

Val Phe Asn Thr Leu Leu Lys His Leu Arg Leu Ser Val Glu Phe Glu
                405                 410                 415

Ala Asn Asp Leu Gln Gly Gly Ser Val Gly Ser Val Asn Leu Asn Thr
            420                 425                 430

Ser Ser Lys Asp Asn Asp Glu Lys Ile Val Gln Asn Ala Ile Ile Gln
        435                 440                 445

Thr Ile Gly Phe Phe Gly Ser Asn Leu Pro Asp Tyr Gln Arg Ser Glu
    450                 455                 460

Ile Met Met Phe Ile Met Gly Lys Val Pro Val Phe Gly Thr Ser Thr
465                 470                 475                 480

His Thr Leu Asp Ile Ser Gln Leu Gly Asp Leu Gly Thr Arg Arg Ile
                485                 490                 495

Gln Ile Met Leu Leu Arg Ser Leu Leu Met Val Thr Ser Gly Tyr Lys
            500                 505                 510

Ala Lys Thr Ile Val Thr Ala Leu Pro Gly Ser Phe Leu Asp Pro Leu
        515                 520                 525

Leu Ser Pro Ser Leu Met Glu Asp Tyr Glu Leu Arg Gln Leu Val Leu
    530                 535                 540

Glu Val Met His Asn Leu Met Asp Arg His Asp Asn Arg Ala Lys Leu
545                 550                 555                 560

Arg Gly Ile Arg Ile Ile Pro Asp Val Ala Asp Leu Lys Ile Lys Arg
                565                 570                 575

Glu Lys Ile Cys Arg Gln Asp Thr Ser Phe Met Lys Lys Asn Gly Gln
            580                 585                 590

Gln Leu Tyr Arg His Ile Tyr Leu Gly Cys Lys Glu Glu Asp Asn Val
        595                 600                 605

Gln Lys Asn Tyr Glu Leu Leu Tyr Thr Ser Leu Ala Leu Ile Thr Ile
    610                 615                 620

Glu Leu Ala Asn Glu Glu Val Val Ile Asp Leu Ile Arg Leu Ala Ile
625                 630                 635                 640

Ala Leu Gln Asp Ser Ala Ile Ile Asn Glu Asp Asn Leu Pro Met Phe
                645                 650                 655

His Arg Cys Gly Ile Met Ala Leu Val Ala Ala Tyr Leu Asn Phe Val
            660                 665                 670

Ser Gln Met Ile Ala Val Pro Ala Phe Cys Gln His Val Ser Lys Val
        675                 680                 685

Ile Glu Ile Arg Thr Met Glu Ala Pro Tyr Phe Leu Pro Glu His Ile
    690                 695                 700

Phe Arg Asp Lys Cys Met Leu Pro Lys Ser Leu Glu Lys His Glu Lys
705                 710                 715                 720

Asp Leu Tyr Phe Leu Thr Asn Lys Ile Ala Glu Ser Leu Gly Gly Ser
                725                 730                 735

Gly Tyr Ser Val Glu Arg Leu Ser Val Pro Tyr Val Pro Gln Val Thr
            740                 745                 750

Asp Glu Asp Arg Leu Ser Arg Arg Lys Ser Ile Val Asp Thr Val Ser
        755                 760                 765

Ile Gln Val Asp Ile Leu Ser Asn Asn Val Pro Ser Asp Asp Val Val
    770                 775                 780

Ser Asn Thr Glu Glu Ile Thr Phe Glu Ala Leu Lys Lys Ala Ile Asp
785                 790                 795                 800

Thr Ser Gly Met Glu Glu Gln Glu Lys Glu Lys Arg Arg Leu Val Ile
                805                 810                 815

Glu Lys Phe Gln Lys Ala Pro Phe Glu Glu Ile Ala Ala Gln Cys Glu
            820                 825                 830

Ser Lys Ala Asn Leu Leu His Asp Arg Leu Ala Gln Ile Leu Glu Leu
        835                 840                 845

Thr Ile Arg Pro Pro Pro Ser Pro Ser Gly Thr Leu Thr Ile Thr Ser
    850                 855                 860

Gly His Ala Gln Tyr Gln Ser Val Pro Val Tyr Glu Met Lys Phe Pro
865                 870                 875                 880

Asp Leu Cys Val Tyr
                885


<210> 87
<211> 5286
<212> DNA
<213> Homo sapiens

<220>
<223> KIAA0143 protein


<400> 87
ggctggcggc ggtagctgtc gcccgcttgg ttgcgtgacc gcggggtccg cgtccgctcc 60
ctccaccctt cgcccttcgc ccttcgcctc gtcccggcct ccgcggccca gcaacggccg 120
tcatggtgcc gtcggcgctc cctgcgcggc cccgctgagc ctcggtgcgg cggcgagcgc 180
ggtcgagatc gccatgccta cccgagtatg ctgctgctgt tccgctttgc gtcctcgcta 240
caaacgcctg gtggacaaca tattccctga agatccaaaa gatggccttg tgaaaactga 300
tatggagaaa ttgacatttt atgcagtatc tgctccagag aaactggatc gaattggttc 360
ttacctggca gaaaggttga gcagggatgt tgtcagacat cgttctgggt atgttttgat 420
tgctatggag gcactggacc aacttctcat ggcttgccat tctcaaagca ttaagccatt 480
tgtagaaagc tttcttcata tggtggcaaa gctgctggaa tcgggggaac caaagcttca 540
agttcttgga acaaattctt ttgtcaaatt tgcaaatatt gaagaagaca caccatccta 600
tcacagacgt tatgactttt ttgtgtctcg attcagtgcc atgtgccatt cctgtcatag 660
tgatccagaa atacgaacag agatacgaat tgctggaatt agaggtattc aaggtgtggt 720
tcgcaaaaca gtcaacgatg aacttcgggc caccatttgg gaacctcagc atatggataa 780
gattgttcca tccctcctgt ttaacatgca aaagatagaa gaagttgaca gtcgcatagg 840
ccctccttct tctccttctg caactgacaa agaagagaat cctgctgtgc tggctgaaaa 900
ctgtttcaga gaactgctgg gtcgagcaac ttttgggaat atgaataatg ctgttagacc 960
agtttttgcg catttagatc atcacaaact gtgggatccc aatgaatttg cagttcactg 1020

ctttaaaatt ataatgtatt ccattcaggc tcagtattct caccatgtga tccaggagat 1080
tctaggacac cttgatgctc gtaaaaaaga tgctccccgg gttcgagcag gtattattca 1140
ggttctgtta gaggctgttg ccattgctgc taaaggttcc ataggtccga cagtgctgga 1200
agtcttcaat acccttttga aacatctgcg tctcagcgtt gaattcgaag caaatgattt 1260
acagggggga tctgtaggca gtgtcaactt aaatacaagt tccaaagaca atgatgagaa 1320
gattgtgcag aatgctatca tccaaacaat aggatttttt ggaagtaacc taccagatta 1380
tcagaggtca gaaatcatga tgttcattat ggggaaagta cctgtctttg gaacatctac 1440
ccatactttg gatatcagtc aactagggga tttgggaacc aggagaattc agataatgtt 1500
gctgagatct ttgcttatgg tgacctctgg atataaagcg aagacgattg ttactgcact 1560
gccagggtct ttcctggatc ctttgttatc accatctctc atggaggact acgaactgag 1620
acagttggtc ttggaagtaa tgcataatct catggatcgt catgacaata gggcaaagct 1680
tcgagggatc agaataatac cggatgtagc tgacctaaag ataaaaagag aaaaaatttg 1740
cagacaagac acaagtttca tgaaaaagaa tgggcaacag ctgtatcggc acatatattt 1800
gggttgtaaa gaggaagaca acgttcagaa aaactatgaa ctactttata cttctcttgc 1860
tcttataact attgaactgg ctaatgaaga agtagttatt gatctcattc gactggccat 1920
tgctttacag gacagtgcaa ttatcaatga ggataatttg ccaatgttcc atcgttgtgg 1980
aatcatggca ctggttgcag catacctcaa ctttgtaagt cagatgatag ctgtccctgc 2040
attttgccag catgttagca aggttattga aattcgaact atggaagccc cttattttct 2100
accagagcat atcttcagag ataagtgcat gcttccaaaa tctttagaga agcatgaaaa 2160
agatttgtac tttctgacca acaagattgc agagtcgcta ggtggaagtg gatatagtgt 2220
tgagagattg tcagttccgt atgtaccaca agtaacagat gaagatcgac tttctagaag 2280
aaaaagcatt gtggacaccg tatccattca ggtggatatt ttatccaaca atgttccttc 2340
tgatgatgtg gttagtaaca ctgaagaaat cacttttgaa gcattgaaga aagcaattga 2400
taccagtgga atggaagaac aggaaaagga aaagaggcgt cttgtgatag agaaatttca 2460
gaaagcacct tttgaagaaa tagcagcaca gtgtgaatcc aaagcaaatt tgcttcatga 2520
tagacttgcc caaatattgg aactcaccat acgtcctcct cccagtccat caggaacact 2580
gaccattact tctgggcatg cccaatacca atctgtccca gtctatgaga tgaagtttcc 2640
agatctgtgt gtgtactgat cggcgcatga agacctcagc atatgatttg taaagcctaa 2700
aaattaaggc caagctgagc tttcagggtt tacttaatgt gtattaacat acttcttgaa 2760
aataatgatg gaacatatct ttaaccaaat gtttggcata ccatattaga agttttggag 2820
ctatatataa tttcgagtac tttcaaagat agatttatgc catgttaatt tgctttgagg 2880
ttcctgttgc ctttttaagt tgaacatgtt ttggtttcac tttattccac tgttaagtag 2940
tatgttttaa acttttcaca aatgtaatgt tttttaaaaa gtaagccttc agaggattga 3000
aactgtataa attgtttatc tcttaaacat ctacacagcc gcttagatgt agaatttttg 3060
ttgttgtttt ctgcaaaggc agatacattt aaatatattc ctagtcctgg ggctgcaaaa 3120
ctgttcggtg gctttttgtc cccatgcttt agataagctg ggataggcac cttgctattc 3180
agttactata ataatatgtg ataggcattc ctcatcttct tcacaataat aggacatctg 3240
ttgaatagca ttcctcgaat ataaccctaa aaacgccata ctttaaattg tctggttctt 3300
gtaatattgt gtttcctgcc aagagtttga ccattctgct tgagaagtgt agagcttact 3360
cttggagtac caaactgtgc aatattttta catcataaga tgtattagtt tacaggctgt 3420
gctttgaaat tatagtagta ttttgctgtg gctccattaa ttaaatgaga tatatattta 3480
gtgcagaaaa aagacattta aaacagctat tagttcacct gtgaagagtc tgtacatttt 3540
gatttctatt aagagcacat ttatttacat ttgtatatta ttttaaatcc tcatagtcaa 3600
aaaattctcc agatggactt ttaatttgta agatttgaac tgtgacttgt atacatctgt 3660
ttagaatcaa ttatatttga aaagctgcct gtgttttaac agtcaagtgt gctaaagttt 3720
gtcaatttaa gctgcttttg atttcagcta ccaagattac aggtgcactc tacacataac 3780
actgacagac ccataacatt acatacatct tagtgaattc tatcacatgg taaaatgaac 3840
agctttcttt gtaactcata aaattctctt aggacatttt tataaagtca cctgtttata 3900
gttctatctt ttcagattcc atttcttttt acataaaaca gcatacatat caaaaactgt 3960
agcctagaat acagtttaat ttttggcttt tgtttttgtt taaaaaattg cagtgaagaa 4020
tgggatgttt gtgtttatgg ctatttgggc acctttagta gaaacagaca aaagtaagga 4080
aacgataata ggacaagcat acttgaaaat ttctgaatac ttaaacaaaa gcgcaacatc 4140
ttgaaaacca gtctagtcat tgaaacctat gaaatgacac tgaaagatcc tgggctgctt 4200
cttaaataag tagatcagca agacttgttt cagagtgaca gtggagtcgt taccgctgga 4260
ggactaaagg gccctgtggc agctgtcact ggaactttgc ctcttgatca ggaaaaatgc 4320
ttcaccagtc cgtaaagcca agttgtattt tttttattgc ccttttttcc ttctgtattt 4380
ttaaagaagg atgttaattt ttgactatat attttaaaaa aatctaagca gggggacatg 4440
caaaaacaat catcatccac ttggatgtca ttttatagat taacactgtg tgcttttgta 4500
tggaaaaaat atatataatt taatagtata aaaaataaaa tatatattca tttgcactta 4560
cgtgaaacac aaactttgct ctacaaaatt tcgtgtttct tagtgatttt aaaatgcatg 4620
tattgcatgt aaaggaaaac cattacaatt aatgtttatc acacctttat cttggtcttt 4680
gttgatttgg gttttgttgg ggtttttgtt actgttttta aattacagta ggcttctata 4740

tatcctggat ttctgaactg gtcttgttga caaggattcc caagaaatgg atcttttcac 4800
tggctgactc tcccatatct gcaagagatt ctgcaggaac tgggtgtgca cacggtgttg 4860
tagccagttc aggtactgaa tatttaggat ttggtgaagt tcactgtatt gctatatttt 4920
tgtagataaa taaaactcaa taaattgtta atcattctct ttttgctgta tagaattgct 4980
tatatcactc tttctttcat gacattggtt aacatttaaa tgttcctcct gtacttgtgt 5040
tgtctgtgac cgcttataga gttttattgt tattggtgtt tacctgaata cctatgcgta 5100
cacacacaca tatttcctta atacttctga actcattatc ttttagaata ataatactac 5160
tacactttac cagcaattaa cttctcccta cccaaaatgt ttttcttcct gtctgaaaat 5220
ggaactaatt tgtcttattc gtgcttatat ctgtattaaa tgcaataaag ttagtttttg 5280
aaatgt 5286

<210> 88
<211> 424
<212> PRT
<213> Homo sapiens

<220>
<223> Septin 2

<400> 88
Thr Asp Ile Ala Arg Gln Val Gly Glu Gly Cys Arg Thr Val Pro Leu
1               5                   10                  15

Ala Gly His Val Gly Phe Asp Ser Leu Pro Asp Gln Leu Val Asn Lys
            20                  25                  30

Ser Val Ser Gln Gly Phe Cys Phe Asn Ile Leu Cys Val Gly Glu Thr
        35                  40                  45

Gly Leu Gly Lys Ser Thr Leu Met Asp Thr Leu Phe Asn Thr Lys Phe
    50                  55                  60

Glu Gly Glu Pro Ala Thr His Thr Gln Pro Gly Val Gln Leu Gln Ser
65                  70                  75                  80

Asn Thr Tyr Asp Leu Gln Glu Ser Asn Val Arg Leu Lys Leu Thr Ile
                85                  90                  95

Val Ser Thr Val Gly Phe Gly Asp Gln Ile Asn Lys Glu Asp Ser Tyr
            100                 105                 110

Lys Pro Ile Val Glu Phe Ile Asp Ala Gln Phe Glu Ala Tyr Leu Gln
        115                 120                 125

Glu Glu Leu Lys Ile Arg Arg Val Leu His Thr Tyr His Asp Ser Arg
    130                 135                 140

Ile His Val Cys Leu Tyr Phe Ile Ala Pro Thr Gly His Ser Leu Lys
145                 150                 155                 160

Ser Leu Asp Leu Val Thr Met Lys Lys Leu Asp Ser Lys Val Asn Ile
                165                 170                 175

Ile Pro Ile Ile Ala Lys Ala Asp Ala Ile Ser Lys Ser Glu Leu Thr
            180                 185                 190

Lys Phe Lys Ile Lys Ile Thr Ser Glu Leu Val Ser Asn Gly Val Gln
        195                 200                 205

Ile Tyr Gln Phe Pro Thr Asp Asp Glu Ser Val Ala Glu Ile Asn Gly
    210                 215                 220

Thr Met Asn Ala His Leu Pro Phe Ala Val Ile Gly Ser Thr Glu Glu
225                 230                 235                 240

Leu Lys Ile Gly Asn Lys Met Met Arg Ala Arg Gln Tyr Pro Trp Gly
                245                 250                 255

Thr Val Gln Val Glu Asn Glu Ala His Cys Asp Phe Val Lys Leu Arg
            260                 265                 270

Glu Met Leu Ile Arg Val Asn Met Glu Asp Leu Arg Glu Gln Thr His
        275                 280                 285

Thr Arg His Tyr Glu Leu Tyr Arg Arg Cys Lys Leu Glu Glu Met Gly
    290                 295                 300

Phe Lys Asp Thr Asp Pro Asp Ser Lys Pro Phe Ser Leu Gln Glu Thr
305                 310                 315                 320

Tyr Glu Ala Lys Arg Asn Glu Phe Leu Gly Glu Leu Gln Lys Lys Glu
                325                 330                 335

Glu Glu Met Arg Gln Met Phe Val Gln Arg Val Lys Glu Lys Glu Ala
            340                 345                 350

Glu Leu Lys Glu Ala Glu Lys Glu Leu His Glu Lys Phe Asp Arg Leu
        355                 360                 365

Lys Lys Leu His Gln Asp Glu Lys Lys Lys Leu Glu Asp Lys Lys Lys
    370                 375                 380

Ser Leu Asp Asp Glu Val Asn Ala Phe Lys Gln Arg Lys Thr Ala Ala
385                 390                 395                 400

Glu Leu Leu Gln Ser Gln Gly Ser Gln Ala Gly Gly Ser Gln Thr Leu
                405                 410                 415

Lys Arg Asp Lys Glu Lys Lys Asn
            420


<210> 89
<211> 4612
<212> DNA
<213> Homo sapiens

<220>
<223> Septin 2

<400> 89
cgaccgatat agctcgccag gtgggtgaag gttgccgaac tgtccccctg gctggacatg 60
tggggtttga cagcttgcct gaccagctgg tgaataagtc cgtcagccag ggcttctgct 120
tcaacatcct gtgcgtggga gagacaggtt tgggcaagtc caccctcatg gacaccctgt 180
tcaacaccaa attcgaaggg gagccagcca cccacacaca gccgggtgtc cagctccagt 240
ctaataccta tgacctccaa gagagcaacg tgaggctaaa gctcacgatc gttagcacag 300
ttggctttgg ggaccagatc aacaaagagg acagctacaa gcctatcgtg gaattcatcg 360
atgcacaatt cgaggcctac ctgcaggaag agctaaagat ccgaagagtg ctacacacct 420
accatgactc ccgaatccat gtctgcttgt atttcattgc ccccacgggt cattccctga 480
agtctctgga cctagtgact atgaagaagc tggacagtaa ggtgaacatc atccccatca 540
ttgccaaagc agatgccatt tcgaagagtg agctaacaaa gttcaaaatc aaaatcacca 600
gcgagcttgt cagcaacgga gtccagatct atcagtttcc tacagatgat gagtcggtgg 660
cagagatcaa tggaaccatg aacgcccacc tgccgtttgc tgtcattggc agcacagaag 720

aactgaagat aggcaacaag atgatgaggg cgcggcagta tccttggggc actgtgcagg 780
ttgaaaacga ggcccactgc gactttgtga agctgcggga gatgctgatt cgggtcaaca 840
tggaggatct gcgggagcag acccacaccc ggcactatga gctgtatcgc cgctgtaagc 900
tggaggagat gggcttcaag gacaccgacc ctgacagcaa acccttcagt ttacaggaga 960
catatgaggc caaaaggaac gagttcctag gggaactcca gaaaaaagaa gaggagatga 1020
gacagatgtt cgtccagcga gtcaaagaga aagaagcgga gctcaaagag gcagagaaag 1080
agctgcacga gaagtttgac cgtctgaaga aactgcacca ggacgagaag aagaaactgg 1140
aggataagaa gaaatccctg gatgatgaag tgaatgcttt caagcaaaga aagacggcgg 1200
ctgagctgct ccagtcccag ggctcccagg ctggaggctc acagactctg aagagagaca 1260
aagagaagaa aaattaactc tgctgtttgc tgcatgctgc atgagaccca gggtcctgtt 1320
tgggcttcct gtagacaccc ttttcctgcg caacagagct gggcctccct ttctctaatt 1380
tcccccttaa catgcctggg gggcatacaa tccaacccgc gccctctcct ctcttcctgc 1440
caaggtttat agaaacctga gaatctgagg gtgatgtctg gccgctggtc aagaagccaa 1500
cagtcatgtg gctcgcagat gcatcctgca tcccagtccc cctcccagca cccccagcca 1560
tcccccctgt cttcccccac atctttgcca gaggtgtgac atggtcaggg ggcccatctg 1620
ctactctttc ccaccagctc ccctgttcca gttctggttg ctgttagttt ccctgaggta 1680
tttgcaacca ccatggctgg gtaaccaccg atcagcacag ctgtcccctt ggtctcctgt 1740
atcccagtca ctagtcctcc ctggtccacc ccaccctcat cctcaggagc cacagccatt 1800
tcttagaggg tttcaaaagg acagcctttg gcgccttttc cttctaacct ttgagtccag 1860
ccctttccag ttttcattca ctcgaagtaa ctgcactcaa gctgtgctca aaatcggcaa 1920
cgcatttatt tacaccaagc ccttcccata aaacacaact gctgaagaaa atagcagacg 1980
tttcccctct ctctaactct gggtatccca cagatgcaaa agggagaata aacctgaata 2040
ttattaccag cctagagtct tgaatgatag ccttaccgaa ttcttcttgt gaggtatttc 2100
agcatctcgg ggggtaattt ccggaagggc tccatactgt cccaataagg tgaggccagt 2160
agcaggaata ataaatccca ctttgtaggc tggaaaactg agctgtcaaa agaatcaagt 2220
gtttgggggt ttgctctgat gagtcttcta gttcatttgg tgaatgtcat gatgattttt 2280
aacatgcatt ttgcatgcat cccccaataa gaagagatga gactcggctg gagagaagaa 2340
aaggccctta actttctttc caatttaagg agttgagagt ttaaaaatat tccagcccta 2400
agtttttatc atgggtccca tctgatagtg gctttgggaa cctctgtgaa gtagagagcc 2460
ctcccttgtc agggttatga ggcacagtgg cctttggtgt ttggccagtg acagtgtgag 2520
agatggagtt gacctggcaa tgatctgtgg ctaacatgcc gtctctctgc ccttcctttg 2580
cagtaatcca tggctgtgta ctgaatagta ttccccgcta cagctggact ggactccatt 2640
tagcctttta agccgaggtt cctattttaa ctgacagctt tcctttgggg tgccaggcag 2700
cgaggccccc cacccctatc ctgccatgta cttcaagctc acttcttctt tttgagttcc 2760
gcaacttgct cctgcctccc agccccactg gcactgacca tgaccaccta cttctatttt 2820
ttttttagag tttctttttt tgatcactta ctttcaaagc acacagtcaa acaaggttat 2880
gccaaatttc caggcctttt tgaagtattg agaaggggaa ggggatttct cacttcaatt 2940
atagatcata ataggaagca aaaagaaaaa aatgaaaagc aaacatatgc acgcactttt 3000
cttgttgaca aagcaagaat ataggtttgc tgtgtaggtt tggtgctcta ttgattggtg 3060
agtgaccaga gcaagtatga aggtgatgct gccaaagcac aagccttttt gaagtattga 3120
gaaggggaag gggatttctc acttcaatta tagatcataa taggaagcaa aaagaaaaaa 3180
atgaaaagca aacatatgca cgcacttttc ttgttgacaa agcaagaata taggtttgct 3240
gtgtaggttt ggtgctctat tgattggtga gtgaccagag caagtatgaa ggtgatgctg 3300
ccaaagcaca agccagtttc ttgggaaaat tcaagttaca gtggagtatt tttttgaaga 3360
ccatatgctt ggaggtagaa acaaaccaac gaccaaaaaa aaaaaaaaaa aatctgctca 3420
gatactcagc cagtagctca gagagatgct gagttaggcc tgtcaggtct ccttgggaaa 3480
ggcttcatat ttgcaacttt gatgattcta tgtccagctt cagagctgct ttcccagaaa 3540
ttcacgctta aacaaccaac cggtaaccac cacttcccca caccgccgcc cggtaattat 3600
ttgcattaca aaccggaggc gccctcattt gcatttgtgt acagattaac tagttaaggc 3660
ttgagaagct ctgaataatt caaaagtatt agacccacac agccttggag agaccttcag 3720
aaactaagga ggagttttat attaagggag acattttagt cagtaagacg atataaccta 3780
cttactccgt aaggggaaat gaaggcccag agaagggaag ggacttgacc gaggtcccac 3840
ttctgtttcg aggcagaagc cagactaatt ttcatgcctc ctgactccca atcagtttca 3900
caaagggatt caatctgttt atatacgtta cattcctgga tacgaggtct tttgatgttc 3960
agagtaactg actagttagt attagaagac cctcgaggtt tttttccaca gaaaaacatc 4020
tgaagatgga ttgggtgagg gctggcaaaa cgaaggcatg ccgggccagc tccttaaccc 4080
aatgacccag tgatgctgca aggctggaac ggggtccagg agactgtgtg taacaggtgc 4140
cctaggtgac ccttataatc agggaagttt ggtgaacaaa aatcgaaccc atgagtgaac 4200
ataaattaaa aagttgatca acctattaaa atgtgtattt cattgggtag cttttctcac 4260
tgtagacaga ttttttcctt cttcaatgaa aaggctttta aattagtaca actgttacta 4320
tttaaaaaaa aaatacccta agtactctgt ttacttctgg tgaaacaaaa ccagtcatta 4380
gaaatggtct gtgcttttat tttcccagac tggagtggct tttctgaaac acacacacac 4440

acacacacac acacacacac gtacacacat ccctcacttc tcttaagcca agaagtttgc 4500
tttccctagc tgcagtgtag atggctcttg tttttgtttt tttgttttaa tcatttggca 4560
ttcacatgtg gctgttaata tgtgcttgtt tttaattaaa acaagaagct tt 4612

<210> 90
<211> 459
<212> PRT
<213> Rattus norvegicus

<220>
<223> Nucleobindin

<400> 90
Met Pro Thr Ser Val Pro Arg Gly Ala Pro Phe Leu Leu Leu Pro Pro
1               5                   10                  15

Leu Leu Met Leu Ser Ala Val Leu Ala Val Pro Val Asp Arg Ala Ala
            20                  25                  30

Pro His Gln Glu Asp Asn Gln Ala Thr Glu Thr Pro Asp Thr Gly Leu
        35                  40                  45

Tyr Tyr His Arg Tyr Leu Gln Glu Val Ile Asn Val Leu Glu Thr Asp
    50                  55                  60

Gly His Phe Arg Glu Lys Leu Gln Ala Ala Asn Ala Glu Asp Ile Lys
65                  70                  75                  80

Ser Gly Lys Leu Ser Gln Glu Leu Asp Phe Val Ser His Asn Val Arg
                85                  90                  95

Thr Lys Leu Asp Glu Leu Lys Arg Gln Glu Val Ser Arg Leu Arg Met
            100                 105                 110

Leu Leu Lys Ala Lys Met Asp Ala Lys Gln Glu Pro Asn Leu Gln Val
        115                 120                 125

Asp His Met Asn Leu Leu Lys Gln Phe Glu His Leu Asp Pro Gln Asn
    130                 135                 140

Gln His Thr Phe Glu Ala Arg Asp Leu Glu Leu Leu Ile Gln Thr Ala
145                 150                 155                 160

Thr Arg Asp Leu Ala Gln Tyr Asp Ala Ala His His Glu Glu Phe Lys
                165                 170                 175

Arg Tyr Glu Met Leu Lys Glu His Glu Arg Arg Arg Tyr Leu Glu Ser
            180                 185                 190

Leu Gly Glu Glu Gln Arg Lys Glu Ala Glu Arg Lys Leu Gln Glu Gln
        195                 200                 205

Gln Arg Arg His Arg Glu His Pro Lys Val Asn Val Pro Gly Ser Gln
    210                 215                 220

Ala Gln Leu Lys Glu Val Trp Glu Glu Leu Asp Gly Leu Asp Pro Asn
225                 230                 235                 240

Arg Phe Asn Pro Lys Thr Phe Phe Ile Leu His Asp Ile Asn Ser Asp
                245                 250                 255

Gly Val Leu Asp Glu Gln Glu Leu Glu Ala Leu Phe Thr Lys Glu Leu
            260                 265                 270

Glu Lys Val Tyr Asp Pro Lys Asn Glu Glu Asp Asp Met Arg Glu Met
        275                 280                 285

Glu Glu Glu Arg Leu Arg Met Arg Glu His Val Met Lys Asn Val Asp
    290                 295                 300

Thr Asn Gln Asp Arg Leu Val Thr Leu Glu Glu Phe Leu Ala Ser Thr
305                 310                 315                 320

Gln Arg Lys Glu Phe Gly Glu Thr Ala Glu Gly Trp Lys Thr Val Glu
                325                 330                 335

Met Tyr Pro Ala Tyr Thr Glu Glu Glu Leu Lys Arg Phe Glu Glu Glu
            340                 345                 350

Leu Ala Ala Arg Glu Ala Glu Leu Asn Ala Arg Ala Gln Arg Leu Ser
        355                 360                 365

Gln Glu Thr Glu Ala Leu Gly Arg Ser Gln Asp Arg Leu Glu Ala Gln
    370                 375                 380

Lys Arg Glu Leu Gln Gln Ala Val Leu Gln Met Glu Gln Arg Lys Gln
385                 390                 395                 400

Gln Gln Gln Glu Gln Ser Ala Pro Pro Ser Gln Pro Asp Gly Gln Leu
                405                 410                 415

Gln Phe Arg Ala Asp Thr Gly Asp Ala Pro Val Pro Ala Pro Ala Gly
            420                 425                 430

Asp Gln Lys Asp Val Pro Ala Ser Glu Lys Lys Val Pro Glu Gln Pro
        435                 440                 445

Pro Val Leu Pro Gln Leu Asp Ser Gln His Leu
    450                 455


<210> 91
<211> 2152
<212> DNA
<213> Rattus norvegicus

<220>
<223> Nucleobindin

<400> 91
ccagaaacct ggcggaggtc gcgggctggg aaaacgccct ctgcgctagg agagaccagc 60
tacgatgcct acctctgtgc cccgcggggc gcctttcctt ctcctaccac ctttactgat 120
gctgtctgct gtgctggcag tgcctgtgga ccgcgcagca cctcatcagg aggacaacca 180
ggccactgag accccggaca caggcctgta ctaccatcgg tacctccagg aggtcatcaa 240
cgtgctagag acagatgggc acttccggga gaagctgcaa gctgccaacg ctgaggacat 300
taagagtgga aagctgagtc aagagctgga cttcgtcagc cacaacgtcc gcaccaagct 360
ggatgagctc aagcgacagg aggtatcaag gcttcggatg ctgctcaagg ccaagatgga 420
tgcaaagcag gagcctaact tgcaggtgga ccacatgaac cttctgaagc agtttgaaca 480
cctggaccct cagaaccagc acacgtttga ggctcgggac ctagagctgc tgatccagac 540
ggccacccga gacctcgccc agtatgacgc tgcacaccat gaagagttca aacgctacga 600
gatgctcaag gaacatgagc gaagacgtta cctggagtct ctgggagagg agcagcggaa 660
ggaggctgag aggaagctac aagagcagca gcgcagacac cgggaacacc ccaaagtcaa 720
tgttcctggc agccaagccc agttgaagga ggtgtgggag gagctagatg gattggaccc 780

caacaggttc aaccccaaga ccttcttcat actgcatgac atcaacagcg atggtgtcct 840
agatgagcaa gaactggaag ctctgtttac caaggagctg gagaaggtgt acgacccgaa 900
gaacgaggag gacgacatga gggagatgga ggaggagcgg ctgcgcatgc gggagcatgt 960
gatgaagaac gtggacacca accaggaccg acttgtgacc ctggaggaat tcctggcatc 1020
cacacagagg aaggagtttg gggaaactgc ggagggatgg aagacagtgg aaatgtatcc 1080
agcctacaca gaggaagagc tgaagcgttt tgaagaggag cttgctgccc gggaggctga 1140
gctaaatgcc agagcccagc gcctcagcca ggagacagag gccctggggc gctcccagga 1200
ccgcctagag gctcagaaga gagagctgca gcaggccgtt ctgcagatgg agcaaaggaa 1260
gcagcaacag caagagcaga gcgctccgcc ttcccaacct gatgggcagc tgcagttccg 1320
tgcagacaca ggggatgctc ctgtcccagc tccagcaggt gaccagaaag acgtgcctgc 1380
ttctgaaaag aaggttccag agcagccccc tgtgctgcca cagctggatt ctcagcattt 1440
ataagccctc agagatcccg gccctcaagt tcccacagac agtgatggaa gctggatgaa 1500
gtgtccttgc agcctttctg gagaccaaag ccttcatggc ctccagggga gaacagggcc 1560
accctgttcc tcagatgcgc ttctgcccat gtctaccagt ctgttctcac agcctctggg 1620
tcccagtcct gccttcctcc ctcccttcct tggcagggac tcaactgcag gagcccctca 1680
ccccacctga gtgcacaccc caggcctgcc tgccttccat gctttgctct tacctgtccc 1740
aggctcaacc tttctgcttc ctgtacctgt cagtgggtca caggagcttg acttggtctc 1800
tccaactctt tgccttgtga gaggccaatg atctcaattc cttttggtgg ctagaggccg 1860
ccacaatgtg gggtgtccta tcagctctgc ctgggactcc tgaactccac caccacccca 1920
ccagccttac cctaaccacc tgggcccaat acctacctga gggtcccaga aggggcccag 1980
atatggctac accccttact gaggtccagc ctcactccca cgtatggggg ctcctgaaaa 2040
ttggttcacc cacttccctg gcttcaggcc acactgctgt gtcctgctct gtgtcccagt 2100
ctcatccaac tatagcctgg cacaaataaa cattagatat ctgtgtgaat gt 2152

<210> 92
<211> 160
<212> PRT
<213> Rattus norvegicus

<220>
<223> Myelin structural protein SR 13

<400> 92
Met Leu Leu Leu Leu Leu Gly Ile Leu Phe Leu His Ile Ala Val Leu
1               5                   10                  15

Val Leu Leu Phe Val Ser Thr Ile Val Ser Gln Trp Leu Val Gly Asn
            20                  25                  30

Gly His Arg Thr Asp Leu Trp Gln Asn Cys Thr Thr Ser Ala Leu Gly
        35                  40                  45

Ala Val Gln His Cys Tyr Ser Ser Ser Val Ser Glu Trp Leu Gln Ser
    50                  55                  60

Val Gln Ala Thr Met Ile Leu Ser Val Ile Phe Ser Val Leu Ser Leu
65                  70                  75                  80

Phe Leu Phe Phe Cys Gln Leu Phe Thr Leu Thr Lys Gly Gly Arg Phe
                85                  90                  95

Tyr Ile Thr Gly Val Phe Gln Ile Leu Ala Gly Leu Cys Val Met Ser
            100                 105                 110

Ala Ala Ala Ile Tyr Thr Val Arg His Ser Glu Trp His Val Asn Asn
        115                 120                 125

Asp Tyr Ser Tyr Gly Phe Ala Tyr Ile Leu Ala Trp Val Ala Phe Pro
    130                 135                 140

Leu Ala Leu Leu Ser Gly Ile Ile Tyr Val Ile Leu Arg Lys Arg Glu

145                 150                 155                 160

<210> 93
<211> 1736
<212> DNA
<213> Rattus norvegicus

<220>
<223> Myelin structural protein SR 13

<400> 93
gggggaagcc agcaacctag aggacgcccc cgagtttgtg cctgaggcta ctccgctctg 60
agccggctgt ccctttgaac tgaaagcacc gctccaccga gcccgagccc aactccagcc 120
accatgcttc tactcttgtt ggggatcctg ttccttcaca tcgcggtgct agtgttgctc 180
ttcgtctcca ccatcgtcag ccaatggctc gtgggcaatg gacacaggac tgatctctgg 240
cagaactgta ccacatccgc cttgggagcc gtccagcact gctactcctc atctgtgagc 300
gaatggcttc agtctgtcca ggccaccatg atcctgtctg tcatcttcag cgtcctgtcc 360
ctgttcctgt tcttctgcca gctcttcact ctcaccaaag gcggccgctt ttacatcact 420
ggagtcttcc aaatccttgc tggtctgtgt gtgatgagtg cagcggccat ctacacagtg 480
agacacagtg agtggcatgt caacaacgac tactcctatg gctttgctta catcctggcc 540
tgggtggctt tcccgctggc cctccttagt ggcatcatct acgtgatcct gcggaaacgc 600
gaatgaggcg cccgacgcac catccgtcta ggctctgagc gtgcataggg tacacaggga 660
gggaggaagg aaaccagaaa accaaaccaa ccaacccaaa agagctagcc cccaaaccca 720
aacgcaagcc aaaccaaaca gaacacagtt gagtggggat tgctgtcgat tgaagatgta 780
tataatatct atggtttata aaacctattt ataacacttt ttacatacat gtacatagga 840
ttgtttgctt tttatgttga ccgtcagcct cgtgttgaat cttaaacgac tctacatcct 900
aacactataa ccaagctcag tattttcgtt ttgtttcgtt tttttcatct ttttgttttg 960
ctcagacata aaaaaaaaaa aaaaatccac gtggccccct ttcatctgaa agcagatccc 1020
tccctcccat tcaacctcat aggataacca aagtgcgggg acaaacccca gatggccaga 1080
ggccttcaca ctatgggtga cccagtgaat ttagcaggaa taatccgctg cccgaatcaa 1140
tgtgtgaagc cctaagcact cacagacgaa acgccctgac cagagccctc tgcgaaacca 1200
atagctggtg gctgcggaac acttgaccct gaaggcggag tactggggca catgtttaaa 1260
tgagacgtca gagacaagca atctgtgaaa tggtgctata gatttaccat tccttgttat 1320
tactaatcat ttaaaccact cactggaaac tcaattaaca gttttatgac ctacagcaga 1380
acagagaccc gatacaaacg gttcgtaact gctttcgtac atagctaggc tgttgttatt 1440
actacaataa ataaatctca aagccttcgt cactcccaca gttttctcac ggtcggagca 1500
tcaggacgag ggtctagacc cttgggacta gcaaattccc tggctttctg ggtctagagt 1560
gttctgtgcc tccaaggact gtctagcgat gacttgtatt ggccaccaac tgtagatgta 1620
tatacggtgt ccttctgatg ctaagactcc agacctttct tggttttgct ggctttttct 1680
gattttatac caactgtgtg gactaagatg cattaaaata aacatcagag taactc 1736

<210> 94
<211> 375
<212> PRT
<213> Rattus norvegicus

<220>
<223> Cytoplasmic beta-actin

<400> 94
Met Asp Asp Asp Ile Ala Ala Leu Val Val Asp Asn Gly Ser Gly Met
1               5                   10                  15

Cys Lys Ala Gly Phe Ala Gly Asp Asp Ala Pro Arg Ala Val Phe Pro
            20                  25                  30

Ser Ile Val Gly Arg Pro Arg His Gln Gly Val Met Val Gly Met Gly

35                  40                  45

Gln Lys Asp Ser Tyr Val Gly Asp Glu Ala Gln Ser Lys Arg Gly Ile
    50                  55                  60

Leu Thr Leu Lys Tyr Pro Ile Glu His Gly Ile Val Thr Asn Trp Asp
65                  70                  75                  80

Asp Met Glu Lys Ile Trp His His Thr Phe Tyr Asn Glu Leu Arg Val
                85                  90                  95

Ala Pro Glu Glu His Pro Val Leu Leu Thr Glu Ala Pro Leu Asn Pro
            100                 105                 110

Lys Ala Asn Arg Glu Lys Met Thr Gln Ile Met Phe Glu Thr Phe Asn
        115                 120                 125

Thr Pro Ala Met Tyr Val Ala Ile Gln Ala Val Leu Ser Leu Tyr Ala
    130                 135                 140

Ser Gly Arg Thr Thr Gly Ile Val Met Asp Ser Gly Asp Gly Val Thr
145                 150                 155                 160

His Thr Val Pro Ile Tyr Glu Gly Tyr Ala Leu Pro His Ala Ile Leu
                165                 170                 175

Arg Leu Asp Leu Ala Gly Arg Asp Leu Thr Asp Tyr Leu Met Lys Ile
            180                 185                 190

Leu Thr Glu Arg Gly Tyr Ser Phe Thr Thr Thr Ala Glu Arg Glu Ile
        195                 200                 205

Val Arg Asp Ile Lys Glu Lys Leu Cys Tyr Val Ala Leu Asp Phe Glu
    210                 215                 220

Gln Glu Met Ala Thr Ala Ala Ser Ser Ser Ser Leu Glu Lys Ser Tyr
225                 230                 235                 240

Glu Leu Pro Asp Gly Gln Val Ile Thr Ile Gly Asn Glu Arg Phe Arg
                245                 250                 255

Cys Pro Glu Ala Leu Phe Gln Pro Ser Phe Leu Gly Met Glu Ser Cys
            260                 265                 270

Gly Ile His Glu Thr Thr Phe Asn Ser Ile Met Lys Cys Asp Val Asp
        275                 280                 285

Ile Arg Lys Asp Leu Tyr Ala Asn Thr Val Leu Ser Gly Gly Thr Thr
    290                 295                 300

Met Tyr Pro Gly Ile Ala Asp Arg Met Gln Lys Glu Ile Thr Ala Leu
305                 310                 315                 320

Ala Pro Ser Thr Met Lys Ile Lys Ile Ile Ala Pro Pro Glu Arg Lys
                325                 330                 335

Tyr Ser Val Trp Ile Gly Gly Ser Ile Leu Ala Ser Leu Ser Thr Phe
            340                 345                 350

Gln Gln Met Trp Ile Ser Lys Gln Glu Tyr Asp Glu Ser Gly Pro Ser
        355                 360                 365

Ile Val His Arg Lys Cys Phe
    370                 375

<210> 95
<211> 4100
<212> DNA
<213> Rattus norvegicus

<220>
<221> misc_feature
<222> 3859
<223> n is a or g or c or t

<220>
<223> Cytoplasmic beta-actin

<400> 95
acctcttcct caactcactt ctctctactc tcactttttt ttttcttctt cttttttttt 60
tttttttttt tttttttttt tgcaaaagaa gggggtaaaa aaatgctgca ctgtcgggcg 120
aggccggtga gtgagcgagc cggagccaat cagcgcccgc cgttccgaaa ttgcctttta 180
tggctcgagt ggccgctgtg gcgtcctata aaacccggcg gcgcaacgcg cgccactgtc 240
gagtccgcgt ccacccgcga gtacaacctc cttgcagctc ctccgtcccg gtccacaccc 300
gccaccaggt aagcagggac gtcgggccca gcgggcccca actttacctt ggccactacc 360
tcgctgcagg atcgtgagga acactcagaa gggacaccgt agaggggtgg agcgtggtac 420
cgggccgcgg agcagctggc aaagcttaac tttcccggcc cctagggtgt agagtgtttg 480
cagtcgtatt cccgcggtgt ggacactcgt gggtacgctc ctgcttggtg cgcggggctt 540
ggggacacac tagagtcgcg gtgtgggcat ttggagagcc ggtgcggctg cgggtgttaa 600
gccgcatctg tccaccttga ggggcaacag tattggggtc aggcgttaca atcacgcttt 660
gatggcctat gggtctttgt ccaaaccggt tttgccattc ggcttggcgg gcgcggcggg 720
gccgctcggc cgggtggggg ctgggatgcc attgcgcgtg cgcgctctat cactgggcat 780
tggggccgtg cgcgctgggg agggaactct tcctctcccc ctcttccgag ttaagagttg 840
cgcgtgcgta ttgagactag gagcgcggcc gccccgggtt gggcgagggc gggccgtcca 900
ccggaagggg cggggtcgta gcggctaggc gcctgctcgc gcttcctgct gggtgtggtc 960
gcctcccgcg cgcgcactag ccgcccgtcg cctcagtgta ggcgggcctg tgcccgtttg 1020
gggaggggcg gaggcctggc ttcctgccgt gggtccgcct ccgggccagc gtttgccttt 1080
tatggtaata atgcggctgt cctgcgcttc ctttgtcccc tgagcttggg cgcgcccctg 1140
gcggctcgag gccgcggctc gccggaagtg ggcagggcgg cagcggctgc tcttggcggc 1200
tccgcggtga ccatagccct cttttgtgcc ttgatagttc gccatggatg acgatatcgc 1260
tgcgctcgtc gtcgacaacg gctccggcat gtgcaaggcc ggcttcgcgg gcgacgatgc 1320
tccccgggcc gtcttcccct ccatcgtggg ccgccctagg caccaggtaa gtgacccttt 1380
actttgggag tggcagccct agggttttct tgggggtcga tgccagtgct gagaacgttg 1440
ttctcctccg cagggtgtga tggtgggtat gggtcagaag gactcctacg tgggcgacga 1500
ggcccagagc aagagaggca tcctgaccct gaagtacccc attgaacacg gcattgtaac 1560
caactgggac gatatggaga agatttggca ccacactttc tacaatgagc tgcgtgtggc 1620
ccctgaggag caccctgtgc tgctcaccga ggcccctctg aaccctaagg ccaaccgtga 1680
aaagatgacc caggtcagta tcctgggtga ccctcccctt cttattgggt caacttctca 1740
gcacgccctt ctctaattgt ctttcttctg ccatgtccca taggactctc ttctatgagc 1800
tgagtctccc ttggaacttt gcagtttctg ctctttccca gatgaggtct tttttttctc 1860
tcgatcgcct ttctgactag gtgttttaaa cccctacagt gctgtgggtg taggtactaa 1920
caatggctcg tgtgacaaag ctaatgaggc tggtgataaa tggccttgga gtgtgtattc 1980
agtagatgac agtaggtcta aatggagccc ctgtcctgat actcccagca cacttaactt 2040
agctgtgttc ttgccctctt tgcatgtctc actcaaatct atccttacag tctcacctgc 2100
cctgagtgtt tcttgtggct ttaggagctt gacaatactg tattcctttc tctacagatc 2160
atgtttgaga ccttcaacac cccagccatg tacgtagcca tccaggctgt gttgtccctg 2220
tatgcctctg gtcgtaccac tggcattgtg atggactccg gagacggggt cacccacact 2280
gtgcccatct atgagggtta cgcgctccct catgccatcc tgcgtctgga cctggctggc 2340
cgggacctga cagactacct catgaagatc ctgaccgagc gtggctacag cttcaccacc 2400
acagctgaga gggaaatcgt gcgtgacatt aaagagaagc tgtgctatgt tgccctagac 2460
ttcgagcaag agatggccac tgccgcatcc tcttcctccc tggagaagag ctatgagctg 2520
cctgacggtc aggtcatcac tatcggcaat gagcggttcc gatgccccga ggctctcttc 2580
cagccttcct tcctgggtaa gttgtagtct cgtcccttct ccatctaaag gtgaccaatg 2640

ctggaggcca cactgtaact ctgatctctt gcttttcctt tcaggtatgg aatcctgtgg 2700
catccatgaa actacattca attccatcat gaagtgtgac gttgacatcc gtaaagacct 2760
ctatgccaac acagtgctgt ctggtggcac caccatgtac ccaggcatcg ctgacaggat 2820
gcagaaggag attactgccc tggctcctag caccatgaag atcaaggtaa gcagccttag 2880
cctggaccca tagtggggtg tggtcagccc tgtagttgta gccaactctc ttggcttaag 2940
gaacaaccca gcatccagaa tgctcacaat cactgtcttg ctttcttcag atcattgctc 3000
ctcctgagcg caagtactct gtgtggattg gtggctctat cctggcctca ctgtccacct 3060
tccagcagat gtggatcagc aagcaggagt acgatgagtc cggcccctcc atcgtgcacc 3120
gcaaatgctt ctaggcggac tgttactgag ctgcgtttta caccctttct ttgacaaaac 3180
ctaacttgcg cagaaaaaaa aaatgagaca tttggcatgg ctttattgtt tttttgtttt 3240
ttgtttttgt tttttttaaa tttttttttt aaaaaggttt tttttttttg ttttgttttg 3300
gcgcttttga ctcaaggatt taaaaactgg aacggtgaag gcgaccgcag ttggttggag 3360
caaacatccc ccaaagttct acaatgtggc tgaggacttt gattgtacat tgttttttgt 3420
ttttggtttt tttaatagtc actccaagta tccacggcat agatggttac aggaagtccc 3480
tcaccctccc aaaagccacc cccaactcct aaggggagga tggctgcatc catgccctga 3540
gtccacaccg ggaaggtgac agcattgctt ctgtgtaaat tatgtacttg caaacatttt 3600
tttaaatctt ccgccttaat acttcatttt tgtttttaat ttctgaatgg tcagccattc 3660
gtggcctgcc cctttttttt gtccccccaa cttgatgtat gaaggctttg gtctccctgg 3720
gagtggtttg aggtgttgag gcgccagggc tggcctgtac actgacgtga gaccgtttta 3780
ataaaagtgc acaccttaca aacaagtttg tggctctgtg gcttctactg ggtgtgggga 3840
gcaggctggg tgggtgtgna actccacgtg ggggaggggc aatttagggt gggctggcct 3900
tgcctgatag ctagtgggag ggctaaagga tcatgatctt taatgaggtc tcataaatac 3960
cccaccttcg gtcttgaggg agggtgagga gttgggaagg tatctccctg acacctggcc 4020
aagctggcct catcaaccct actttcctca gccaggggga ggtccacatc ctcccacctg 4080
catagcttgc gtctagaggc 4100

<210> 96
<211> 116
<212> PRT
<213> Mus musculus

<220>
<223> Ly6/neurotoxin homolog

<400> 96
Met Thr His Leu Leu Thr Val Phe Leu Val Ala Leu Met Gly Leu Pro
1               5                   10                  15

Val Ala Gln Ala Leu Glu Cys His Val Cys Ala Tyr Asn Gly Asp Asn
            20                  25                  30

Cys Phe Lys Pro Met Arg Cys Pro Ala Met Ala Thr Tyr Cys Met Thr
        35                  40                  45

Thr Arg Thr Tyr Phe Thr Pro Tyr Arg Met Lys Val Arg Lys Ser Cys
    50                  55                  60

Val Pro Ser Cys Phe Glu Thr Val Tyr Asp Gly Tyr Ser Lys His Ala
65                  70                  75                  80

Ser Ala Thr Ser Cys Cys Gln Tyr Tyr Leu Cys Asn Gly Ala Gly Phe
                85                  90                  95

Ala Thr Pro Val Thr Leu Ala Leu Val Pro Ala Leu Leu Ala Thr Phe
            100                 105                 110

Trp Ser Leu Leu
        115

<210> 97

<211> 3925
<212> DNA
<213> Mus musculus

<220>
<223> Ly6/neurotoxin homolog

<400> 97
taccaacacc gcacgaagtg tgtacagatt cccagttaga cagcaggagg gacctgggag 60
cggccagggg gatgttttat ctctaagaga ccaagagctc aggcagggct tctgtgccct 120
gcttcctccc tggcttgagc tggatcctgg accagctgct gacctcctgt tcactctggc 180
actgccctca cgtctccgtc atgacccatc tgctcacagt gttcctggtg gccctgatgg 240
gcctgcctgt ggcccaggct ctggagtgcc acgtgtgtgc ctacaatgga gacaactgct 300
tcaaacccat gcgctgccca gccatggcca cctactgtat gaccacacga acttacttca 360
ccccataccg gatgaaggtg aggaagtcct gtgtccccag ctgctttgaa accgtgtacg 420
atggctattc caagcatgca tctgccacct cctgttgcca gtactacctc tgcaacggtg 480
ctggctttgc taccccggtg accttggccc tggtcccagc actcctagct accttctgga 540
gcttgctgta aagctcggtt ccccaagcca gatccactca aacgcaacac tctcaaaaaa 600
cacagtttcc ctctctctcc caattcactc cacccaacgc tcttccttct gacactcctc 660
aactaccacg aggtcccatg gctacctacg aaagaactga tggcatccag atacctcact 720
ccaaggtcat tttcagaagg ctgacatgtg gacctgtaat gtgcccaccc atgggtgggg 780
caggctgggc ttctcctcta cccaagatca ggggcatctg ggagaatgtt tatggaggag 840
gggtcatcac tcaagtcaag gagcactgat ttgatagaat tagtagccaa actccacctt 900
cagaaccctg cctcagtcta cccagtagag gatgggtctg ctagaggtga ggggaggaga 960
gcggcggaga ataacgagct ggctagaagc agagaaagac tcagcagggc tgtctccgaa 1020
gatcagcgcg gcttgccaga gcaaatgtga tgtggaagcc atgtgaggaa gccctttgtc 1080
atttccactt atctgaggaa ctctgccaga cctgatgttg ggatagccat tggccaaggg 1140
ttcctagcaa cggcgtcatt tccataggcc actgaaatcc ctccagcccc agctcagcag 1200
gccccttgac ctccactaca gtccttcatt cacacaccag ctgctgggcc ttgaagttgg 1260
cagggacttg ggagcaggtg acccatgcta ttttttgtct ggcctgttat tctgggcatg 1320
gcaagaaggg atcagacgca ggtcagagca gggcagtagg gcgactgaga cagggaaaca 1380
gacttcagcc agtggcttcc caggtcccgt aggcagctcc tacatccttc agtctcttgt 1440
tacattcccg ggagacaaat atacagggag ccaagccgag tgctaggtga tgactgcctg 1500
tgaagtctat tgtggccaca gactgctggg taccaagtct caggagaacc cagcctagat 1560
ttaggagaca cagatctgcc tttcatgcag tgtagctgtc cttgggagcc ttaccatgct 1620
ctctaactag ttcctcaact cacatgtcac tgaggaaccc cctaacactg gcccagccca 1680
ggggtcggga tgctggccaa tgtccatgga gtgggactac ccctggagag tccttgggtc 1740
atcacatcac aaatgtttta ttccaacctc ccagtggtga gagctcggga cacaaaggtc 1800
catcctgggg accttcttcc tggttctagg cagacctgaa ctctgtctgc tgctagagct 1860
gatgtggttt tccgcctcag tttcctcctc cggggatagg ccaccggagg atttgggagg 1920
gtggggaggg catcctgctg atgggctcgc cgaggttctc aggaacagga acgggcgggg 1980
ctttagtaca caggtgagtt gggtgggaac tggcccggag ctgaggagac actgactggg 2040
cagagggaag atgagtctca agggagggca ggaaaaggga gggggagcgc gcatgcacat 2100
gtgcactcag tgcaggctac agagcccaaa aggcagcact ggctgtggtg tcccctgagg 2160
cccaggcaag atgctaggag gaagccaatg ctgcccccac ctgagctcac atggaacatg 2220
cacaccacca gcagcagcag caagcattga gactgacctg tggacgccat agggcactgg 2280
caaggagggt cagaggcggg tccctgactc agtgggtgag gcccgggaaa cattatcctg 2340
ttaccctgcg tgtgcaagat cattgtcccc agctagatgg cgtcctcaac caaaactgag 2400
aggagcccca gttcaggtcc tccctcctac cacaaggggg tggtgtggag gaggcttgat 2460
tgcccttgga gaagcaccgg tactgcagag ctgggggcca gcttctttca tctgtgtcta 2520
gacaccgacc agataggccc cacagtggca acactgccac acagtcctac aagaagccct 2580
gtgcctagct agcacagagc cccaaaaggt gctcaattaa tacagggcca agcctgccag 2640
tgggggggat gcagattagg ggaacagacc cagatggcct gtcctgaacc ctgtctgggg 2700
tggtgtgatg agcatctgtc tagcccactg caggtggctc tacacactcc acaacagttc 2760
tgcaaaagtg tatgaggtgg tcattactgc gcccctctca caggtaaagg cactgaggca 2820
cggaggagtg aggcacttca ttttcctggg ccattcaact ttccaggacc aacacattca 2880
actatgggta ctactccaat agctggggtt ctttgaggct gggccccctg aagatgatag 2940
tggcttcatc aaccagagaa tttcagagtg cagtgttgta ggagcctatg aacctgaaat 3000
gtcagaactg gaggtttgag ggctgagggg gtaggccagg ggtgtctggc cccttgtgtg 3060
gagacagaga gagagggaac atgggatggg gtagtagaga gaagtgcaaa ggagcgtcag 3120
cctttctcag ggctaatgct gtcagggacg agggctcaag cctgtgagtg ttctcacact 3180
gtgataaaca gtggcccctc aacacagacg gtgtccagag tggccggcag tggttatcta 3240

gagttgcaat ctggaagcct cttggtagtc actggagaga ggccgcttga tgggacagca 3300
ccaaatgtgt gtgcttctgt gggatgtgag gaagctgggt cagcgcatga agccaaagcg 3360
tccttcagag cagaggggtg gctggtctag tccaccagag acaagctatc cagtgagagt 3420
catactctgt caccgtctct gtgattacct taccccaaag cagacgggga cgggatgcag 3480
agcacccgtg tcttcatctt ctgcggcaag cacgtgagtt cacattctga aactctagaa 3540
agatttccag gagtggggtg tgcctttgct ttggtgcatg gttacttcct ggcaagcacc 3600
gtggcatccc gcagcactga gtgacctggg ctcctcaagc catctcattg gtgaaatgac 3660
agtgccagta ccctctcagc tggctcttgg aggcctgtgc atggggtctg cacagaggag 3720
gcccccaaac tatgcatgga cggacacgtg atgcctagca cttcccttgg ttgtgtctct 3780
gccaacccca ggctctcacc cagcaaggaa atgaaatcca cttttatgac acatctccct 3840
cccccagcca gctccattca cctatatgcc agggtggtcc ctttcaatgt ctgtccccca 3900
ttggatgaat aaacaagcga aggac 3925

<210> 98
<211> 130
<212> PRT
<213> Rattus norvegicus

<220>
<223> Astrocytic phosphoprotein

<400> 98
Met Ala Glu Tyr Gly Thr Leu Leu Gln Asp Leu Thr Asn Asn Ile Thr
1               5                   10                  15

Leu Glu Asp Leu Glu Gln Leu Lys Ser Ala Cys Lys Glu Asp Ile Pro
            20                  25                  30

Ser Glu Lys Ser Glu Glu Ile Thr Thr Gly Ser Ala Trp Phe Ser Phe
        35                  40                  45

Leu Glu Ser His Asn Lys Leu Asp Lys Asp Asn Leu Ser Tyr Ile Glu
    50                  55                  60

His Ile Phe Glu Ile Ser Arg Arg Pro Asp Leu Leu Thr Met Val Val
65                  70                  75                  80

Asp Tyr Arg Thr Arg Val Leu Lys Ile Ser Glu Glu Asp Glu Leu Asp
                85                  90                  95

Thr Lys Leu Thr Arg Ile Pro Ser Ala Lys Lys Tyr Lys Asp Ile Ile
            100                 105                 110

Arg Gln Pro Ser Glu Glu Glu Ile Ile Lys Leu Ala Pro Pro Pro Lys
        115                 120                 125

Lys Ala
    130

<210> 99
<211> 2341
<212> DNA
<213> Rattus norvegicus

<220>
<223> Astrocytic phosphoprotein

<400> 99
gcggaagagg cggcggcggc aggatcggcg gcagcggtgg ccggagccaa ggagcaggct 60
ccagacgctg cttaggaacc ggggatccgg gagtgcccgc accctgagct ctcagctccg 120

gaggcgtcat ggcagagtac gggactctcc ttcaggacct gaccaacaac atcacccttg 180
aagatctgga acagctcaag tcagcctgca aggaggacat ccccagtgag aagagtgaag 240
agatcactac aggcagtgcc tggtttagct tcctggagag ccacaacaag ctggacaaag 300
acaacctctc ctacattgag cacatctttg agatctcccg ccgtcctgac ctactcacga 360
tggtggttga ctacagaacc cgtgtgctga agatctctga ggaggatgag ctggacacca 420
agctaacccg tatccccagc gccaagaagt acaaagacat tatccggcag ccctctgagg 480
aagaaatcat caaattggct cccccaccaa agaaggcctg agcaaggggg aggaagagga 540
ggaaggttgg atcttcatca gaccactccc ttccccatcc tcaatgggag gggctagggc 600
aacaccctgc tccgtaccca cttactaact tggtcctaac ccttactatg cgcgtgtgtg 660
tgcgtgtgcg cacgctctgg ctgtctgtct gtctagctca tctagttcct cctcctcatg 720
aggggattgg agtcaaggga gggggcttta gctgccccca ctgtaagggg aggtggacgc 780
tttttctatg taaacagaaa ctggcacact cctcctcctc tcctctcctc cactcttccc 840
cacatcttta aagtatggaa gcagaaaggg cctgcgtttt cccacactga ggagctatgg 900
tgaaaatgag gtatggcgga aatgattaga tctaaagaaa accagtgggg aaggaaggtc 960
aagaggctac ccaacctcca cctgaagggg ctcctgagtg ccagcgggcc ctcctttatg 1020
cccttgttac gggcctggga tgtggataag cagagatcct aatgagaaga ccactagctc 1080
ctttcctagg tcccaagatc aaaccccagt ggagaagcca gcttttgatg tccccaactt 1140
ctgctctgga gagactatgc tgggaccatg cactgctgag cctgagccag ggatgcaggc 1200
agagacaggc ccactctcgc tcctcagttt ctagatacag actgttggga tatactgacc 1260
cggagcctag gaattgggga atgagatccc ttaggtttta accgcaacct gcagagtccc 1320
aaaatgggta ttgtaggcaa cctttccact ttccagttcg gaattctcca agcaagagat 1380
aagttaacga cagtgtttcc tttgcactga ctaccacccc attcaatcca ggaagggact 1440
ggtcaccttt ctcatttggg tttgtgaatg ccacacagcc aagccactag agtacagtga 1500
gtgaacccag cctcctccct gtcccaagat gcccttcccc atcttgaccg tgctaactgt 1560
gtgtacatat atattctaca tatatgtata ttaaacccgc actgccatgt ctgccctttt 1620
ttgtggtttt tagcattaac ttattgtcta ggccagagcg ggagtgggag gggaatgcca 1680
cagtaaaggg agtggcagag ccaaattgct aagttagtca aaacagaaaa catttccaac 1740
caatcacaac aaacattgca tttacatttg agctaagaga caatttagag aaagttgaag 1800
gtaggagctt ttaggatgtg ggagcaaaac ttcacaggga agggaggacc agctgtcaga 1860
agagggctct tgaagccaga ctggctagag caccctcact tcctagctga gcccagtttg 1920
cccatctcct ctggccactg ctgcagacac ctgccttaac acacacacct ctaagactcc 1980
agttttgcct taaaggccct tcctaagtct ccctagtcct gcctggcatc tcctttgaga 2040
caagaaatcc ttgtacaact gggagaaagg aactatgggc aacatccttc catctggggt 2100
gttacgtgag agcaggagtt aagacggagg ctttccttat gaagacacag gagagagaga 2160
gggtgcttct cagcagtttc tctgtaaggc aaaaaacaca aaccttaaac acactaacct 2220
gccctaatac actcacctac cctgctaccc tcgtgctgct gtgaggttgc ccctagcctg 2280
tgttcctctg tctgcagcgt gcacggcctt gttctaaccc tggaataaag gtgactgact 2340
g 2341

<210> 100
<211> 582
<212> PRT
<213> Mus musculus

<220>
<223> PLIC-1

<400> 100
Met Ala Glu Ser Ala Glu Ser Gly Gly Pro Pro Gly Ala Gln Asp Ser
1               5                   10                  15

Ala Ala Asp Ser Gly Pro Ala Glu Pro Lys Ile Met Lys Val Thr Val
            20                  25                  30

Lys Thr Pro Lys Glu Lys Glu Glu Phe Ala Val Pro Glu Asn Ser Ser
        35                  40                  45

Val Gln Gln Phe Lys Glu Glu Ile Ser Lys Arg Phe Lys Ser His Ile
    50                  55                  60

Asp Gln Leu Val Leu Ile Phe Ala Gly Lys Ile Leu Lys Asp Gln Asp

65                  70                  75                  80

Thr Leu Ser Gln His Gly Ile His Asp Gly Leu Thr Val His Leu Val
                85                  90                  95

Ile Lys Thr Gln Asn Arg Pro Gln Asp Asn Ser Ala Gln Gln Thr Asn
            100                 105                 110

Thr Thr Gly Asn Ser Val Thr Ser Ser Pro Ala Pro Asp Ser Asn Pro
        115                 120                 125

Thr Ser Gly Pro Ala Ala Asn Ser Ser Phe Gly Leu Gly Gly Leu Gly
    130                 135                 140

Gly Leu Ala Gly Leu Ser Ser Leu Gly Leu Asn Thr Thr Asn Phe Phe
145                 150                 155                 160

Glu Leu Gln Ser Gln Met Gln Arg Gln Leu Leu Ser Asn Pro Glu Met
                165                 170                 175

Met Val Gln Ile Met Glu Asn Pro Phe Val Gln Ser Met Leu Ser Asn
            180                 185                 190

Pro Asp Leu Met Arg Gln Leu Ile Met Ala Asn Pro Gln Met Gln Gln
        195                 200                 205

Leu Ile Gln Arg Asp Pro Glu Ile Ser His Met Leu Asn Asn Pro Asp
    210                 215                 220

Ile Met Gly Gln Thr Leu Glu Leu Ala Arg Asn Pro Ala Met Met Gln
225                 230                 235                 240

Glu Met Met Arg Asn Gln Asp Arg Ala Leu Ser Asn Val Glu Ser Ile
                245                 250                 255

Arg Gly Gly Tyr Asn Ala Leu Arg Arg Met Tyr Thr Asp Ile Gln Glu
            260                 265                 270

Pro Met Ile Asn Ala Ala Gln Glu Gln Phe Gly Gly Asn Pro Phe Gly
        275                 280                 285

Ser Leu Val Ser Ser Pro Ser Ser Ala Glu Gly Thr Gln Pro Ser Arg
    290                 295                 300

Thr Glu Asn Arg Asp Pro Leu Pro Asn Pro Trp Ala Pro Gln Thr Pro
305                 310                 315                 320

Gln Ser Ser Pro Ala Tyr Gly Ser Ser Gly Ser Thr Asn Asn Thr Val
                325                 330                 335

Ser Thr Ser Gly Gly Asn Ala Thr Ser Thr Pro Ala Gly Gln Gly Thr
            340                 345                 350

Ser Gly Pro Lys Leu Val Pro Gly Ala Gly Gly Ser Met Phe Asn Thr
        355                 360                 365

Pro Gly Met Gln Ser Leu Leu Gln Gln Ile Thr Glu Asn Pro Gln Leu
    370                 375                 380

Met Gln Asn Met Leu Ser Ala Pro Tyr Met Arg Ser Met Leu Gln Ser
385                 390                 395                 400

Leu Ser Gln Asn Pro Asp Leu Ala Ala Gln Met Met Leu Asn Asp Pro
                405                 410                 415

Leu Phe Ala Gly Asn Pro Gln Leu Gln Glu Gln Met Arg Gln Gln Leu
            420                 425                 430

Pro Thr Phe Leu Gln Gln Met Gln Asn Pro Asp Thr Leu Ser Ala Met
        435                 440                 445

Ser Asn Pro Arg Thr Met Gln Ala Leu Leu Gln Ile Gln Gln Gly Leu
    450                 455                 460

Gln Thr Leu Ala Thr Glu Ala Pro Gly Leu Ile Pro Gly Phe Thr Pro
465                 470                 475                 480

Gly Leu Ala Ala Gly Asn Ser Gly Gly Pro Ala Gly Thr Thr Ala Pro
                485                 490                 495

Ser Thr Ala Pro Gly Glu Asp Thr Asn Pro Gln Gly Gly Ala Ala Glu
            500                 505                 510

Pro Gly His Gln Gln Phe Ile Gln Gln Met Leu Gln Ala Leu Ala Gly
        515                 520                 525

Val Asn Pro Gln Leu Gln Ser Pro Glu Val Arg Phe Gln Gln Gln Leu
    530                 535                 540

Glu Gln Leu Ser Ala Met Gly Phe Leu Lys Arg Glu Pro Asn Leu Gln
545                 550                 555                 560

Ala Leu Ile Ala Thr Gly Gly Asp Ile Asn Ala Ala Ile Glu Arg Leu
                565                 570                 575

Leu Gly Ser Gln Pro Ser
            580


<210> 101
<211> 1843
<212> DNA
<213> Mus musculus

<220>
<223> PLIC-1

<400> 101
ccatggccga gagcgcagag agcggcggcc caccgggcgc tcaggacagt gcggccgaca 60
gcggccccgc agagcccaag atcatgaagg tcacggtgaa gacgcctaaa gagaaggaag 120
agttcgctgt gcccgagaac agctcggtcc agcagttcaa agaggaaatc tcaaaacgtt 180
tcaaatctca tattgaccaa cttgtgttga tatttgctgg aaaaatttta aaagatcaag 240
atactttgag tcagcatggg attcatgatg gacttacggt tcaccttgtc atcaaaaccc 300
aaaacaggcc gcaagataat tcagctcagc aaacaaatac cactggaaac agtgtgacca 360
gttcaccagc tcctgacagt aaccccacgt ctgggcctgc tgctaacagc tcctttggtt 420
taggtggact tggaggactt gcaggtctta gtagcttggg tttaaatacc accaacttct 480
ttgaactaca gagccaaatg cagcggcaac ttttgtctaa ccctgaaatg atggtccaga 540
tcatggaaaa cccctttgtg cagagcatgc tctcaaaccc tgacctgatg cggcagctga 600
tcatggccaa cccacagatg cagcagctga tacagagaga cccagagatc agtcatatgc 660
ttaataaccc agatataatg ggacagacat tggaacttgc cagaaaccca gcgatgatgc 720
aggaaatgat gagaaaccag gaccgagcct tgagcaacgt agaaagtatc cgtggagggt 780
acaacgcctt acggcgcatg tacacagata ttcaggagcc catgataaac gctgcgcaag 840
agcagtttgg tggtaatcca tttggttcct tagtgagcag tccgtcctca gcagaaggga 900
ctcagccttc tcgaacagaa aatagggatc cgctacctaa cccttgggct ccacagactc 960

cccagagctc tccagcctat ggcagcagtg gcagcaccaa taacacagtg agcacctctg 1020
gtggcaatgc caccagtacc ccggcgggac agggcacctc agggcccaag cttgtgcctg 1080
gagcaggagg tagtatgttc aacacccccg gaatgcagag cctgctgcag cagataactg 1140
agaacccaca gctcatgcag aacatgctgt ctgccccata catgaggagc atgctgcagt 1200
ccctgagcca gaaccccgac cttgctgcgc agatgatgct gaatgacccc ctatttgctg 1260
ggaaccctca gcttcaagag caaatgagac aacagctccc aacgttcctc caacaaatgc 1320
agaaccccga cacattgtca gccatgtcga accctagaac catgcaggcc ctgctgcaaa 1380
tccagcaggg cttgcagacg ttggccacag aagctcccgg cctcatccca gggtttactc 1440
ctggcttggc agcgggcaat tctggaggcc ctgcaggaac cactgcacct agcactgcac 1500
ctggtgagga tacgaacccc caagggggcg ctgctgagcc aggccaccag cagtttatcc 1560
agcaaatgct gcaggccctc gccggggtga accctcagct gcagagtcca gaagtcagat 1620
ttcaacagca actggaacag ctcagtgcaa tgggattctt gaagcgtgaa ccaaacttac 1680
aagcactgat agcaacaggg ggcgacatca atgcggccat tgaaaggttg ctgggttccc 1740
agccgtcata gcagcatttc tgtatcctga aaaaatgtaa tttatttttg ataacggctc 1800
ttaaatcttt aaaataacct gctttatttc attgggattc tgt 1843

<210> 102
<211> 618
<212> PRT
<213> Rattus norvegicus

<220>
<223> Nfxl

<400> 102
Met Ala Asp Glu Gly Lys Ser Tyr Asn Glu His Asp Asp Arg Val Ser
1               5                   10                  15

Phe Pro Gln Arg Arg Lys Lys Gly Arg Gly Pro Phe Arg Trp Lys Cys
            20                  25                  30

Gly Val Gly Asn Arg Arg Ser Gly Arg Gly Gly Ser Gly Ile Arg Ser
        35                  40                  45

Ser Arg Phe Glu Glu Asp Asp Gly Asp Val Ala Met Asn Asp Pro Gln
    50                  55                  60

Asp Gly Pro Arg Val Arg Phe Asn Pro Tyr Thr Thr Arg Pro Asn Arg
65                  70                  75                  80

Arg Arg Asp Thr Trp His Asp Arg Asp Arg Ile His Val Thr Val Arg
                85                  90                  95

Arg Asp Arg Ala Pro Gln Glu Arg Gly Gly Ala Gly Thr Ser Gln Asp
            100                 105                 110

Gly Thr Thr Lys Asn Trp Phe Lys Ile Thr Ile Pro Tyr Gly Lys Lys
        115                 120                 125

Tyr Asp Lys Met Trp Leu Leu Ser Met Ile Gln Ser Lys Cys Ser Val
    130                 135                 140

Pro Phe Asn Pro Ile Glu Phe His Tyr Glu Asn Thr Arg Ala His Phe
145                 150                 155                 160

Phe Val Glu Asn Ala Thr Thr Ala Ser Ala Leu Lys Ala Val Asn Tyr
                165                 170                 175

Lys Ile Gln Asp Arg Glu Asn Gly Arg Ile Ser Ile Ile Ile Asn Ser
            180                 185                 190

Ser Ala Pro Pro Tyr Ile Val Gln Asn Glu Leu Lys Pro Glu Gln Val
        195                 200                 205

Glu Gln Leu Lys Leu Ile Met Ser Lys Arg Tyr Asp Gly Ser Gln Gln
    210                 215                 220

Ala Leu Asp Leu Lys Gly Leu Arg Ser Asp Pro Asp Leu Val Ala Gln
225                 230                 235                 240

Asn Ile Asp Val Val Leu Asn Arg Arg Gly Cys Met Ala Ala Ala Leu
                245                 250                 255

Arg Ile Ile Glu Glu Asn Ile Pro Glu Leu Leu Ser Leu Asn Leu Ser
            260                 265                 270

Asn Asn Arg Leu Tyr Lys Leu Asp Asp Met Ser Ser Ile Val Gln Lys
        275                 280                 285

Ala Pro Asn Leu Lys Ile Leu Asn Leu Ser Gly Asn Glu Leu Lys Ser
    290                 295                 300

Glu Trp Glu Leu Asp Lys Ile Lys Gly Leu Lys Leu Glu Glu Leu Trp
305                 310                 315                 320

Leu Asp Arg Asn Pro Met Cys Asp Thr Phe Leu Asp Gln Ser Thr Tyr
                325                 330                 335

Ile Ser Thr Ile Arg Glu Arg Phe Pro Lys Leu Leu Arg Leu Asp Gly
            340                 345                 350

His Glu Leu Pro Pro Pro Ile Ala Phe Asp Val Glu Ala Pro Thr Met
        355                 360                 365

Leu Pro Pro Cys Lys Gly Ser Tyr Phe Gly Thr Glu Asn Leu Lys Ser
    370                 375                 380

Leu Val Leu His Phe Leu Gln Gln Tyr Tyr Ala Ile Tyr Asp Ser Gly
385                 390                 395                 400

Asp Arg Gln Gly Leu Leu Asp Ala Tyr His Asp Gly Ala Cys Cys Ser
                405                 410                 415

Leu Ser Thr Pro Ser Asn Pro Gln Asn Pro Val Arg His Asn Leu Ala
            420                 425                 430

Lys Tyr Phe Asn Asp Ser Arg Asn Val Lys Lys Ile Lys Asp Thr Thr
        435                 440                 445

Thr Arg Phe Arg Leu Leu Lys His Thr Arg Leu Asn Val Val Ala Phe
    450                 455                 460

Leu Asn Glu Leu Pro Lys Thr His His Asp Val Asn Ser Phe Val Val
465                 470                 475                 480

Asp Ile Ser Ala Gln Thr Ser Thr Leu Leu Cys Phe Ser Val Asn Gly
                485                 490                 495

Val Phe Lys Glu Val Asp Gly Lys Ser Arg Asp Ser Leu Arg Ala Phe
            500                 505                 510

Thr Arg Thr Phe Ile Ala Val Pro Ala Ser Asn Ser Gly Leu Cys Ile
        515                 520                 525

Val Asn Asp Glu Leu Phe Val Arg Asn Ala Ser Pro Glu Glu Ile Gln
    530                 535                 540

Arg Ala Phe Ala Met Pro Ala Pro Thr Pro Ser Ser Ser Pro Val Pro
545                 550                 555                 560

Thr Leu Ser Gln Glu Gln Gln Asp Met Leu Gln Ala Phe Ser Thr Gln
                565                 570                 575

Ser Gly Met Asn Leu Glu Trp Ser Gln Lys Cys Leu Gln Asp Asn Asn
            580                 585                 590

Trp Asp Tyr Thr Arg Ser Ala Gln Ala Phe Thr His Leu Lys Ala Lys
        595                 600                 605

Gly Glu Ile Pro Glu Val Ala Phe Met Lys
    610                 615

<210> 103
<211> 2304
<212> DNA
<213> Rattus norvegicus

<220>
<223> Nfxl

<400> 103
attccatcct aatacgactc actatagggc tcgagcggcc gcccgggcag gtccaaggcg 60
tccgggtgtt tggtggcgtc aggttctgcg cctgagcccg cccctgtacc tgcctgtgcc 120
atggcggacg aagggaagtc atacaacgaa catgatgacc gtgttagttt cccacaaaga 180
agaaagaaag gtcgggggcc tttccgatgg aagtgtggtg tggggaatcg taggtctgga 240
agaggtggtt ctggtatacg gtcttcccgg tttgaggaag atgatggaga tgtggcaatg 300
aatgatcccc aggatggccc ccgagtgaga ttcaacccct ataccacccg acctaaccgt 360
cggagagata cttggcatga tcgagatcgg attcatgtta ctgtacggag agacagagct 420
cctcaagaaa gaggaggtgc tggcaccagt caagatggga ccaccaagaa ctggttcaag 480
attacaattc cttatggcaa aaagtatgat aaaatgtggc ttctgagcat gattcaaagc 540
aagtgcagtg tccccttcaa ccccattgag tttcactatg aaaatacacg ggcccatttt 600
tttgtggaaa atgccactac cgcttctgca ttaaaggctg tcaactataa gattcaggat 660
cgagaaaacg gaaggatatc tatcatcatc aactcttctg ctccacccta tattgtacag 720
aatgaactga agcctgaaca agtagagcag ctcaagctga tcatgagcaa acgatatgat 780
ggctcccagc aagcacttga cctcaagggc ctccgctcag acccagattt ggtggcccag 840
aacatcgatg ttgttctaaa ccgtagaggc tgtatggcag ccgccctgag gatcattgaa 900
gagaacattc ctgagctatt gtctttgaac ttgagtaaca acaggctgta caagcttgat 960
gacatgtcca gcattgtaca gaaggcacca aacttaaaga tcctgaatct ctctggaaac 1020
gaattgaagt ccgagtggga attagacaag ataaaagggc tgaagctgga agagctgtgg 1080
cttgacagaa accccatgtg tgacaccttc ctagaccagt ccacctatat cagcaccatt 1140
cgcgaacgat ttcccaagtt attacgcctg gatggccatg agctaccccc tccaattgcc 1200
tttgatgtcg aagcccctac tatgttacca ccctgcaagg gaagctattt tggaacagag 1260
aacctaaaga gtctggtcct gcacttcttg cagcagtact atgcaattta tgactcagga 1320
gaccgccagg gtcttctgga tgcctaccat gatggcgcct gttgctcgct aagcactcct 1380
tccaaccctc agaaccctgt ccgacacaac ttagccaaat acttcaatga cagcagaaat 1440
gtgaagaaga ttaaagatac caccacgcgg ttccggctat taaagcatac acgtcttaat 1500
gtcgttgcct tcctcaatga gttacctaaa actcaccatg acgttaactc ctttgtggta 1560
gatataagtg cccaaacaag cacattgttg tgtttttctg tcaatggagt cttcaaggaa 1620
gtggacggga agtctcgaga ttctttacgt gctttcacga gaacgttcat tgctgttcct 1680
gccagcaatt cagggctatg tattgtaaat gacgaattat ttgtgcggaa cgccagccct 1740
gaagaaatcc aaagagcctt cgccatgcct gcacccacac cttcttccag cccagtaccc 1800
acactctccc aagaacagca ggatatgtta caagcatttt ctacccagtc tggcatgaac 1860
ctcgagtggt ctcagaagtg ccttcaagac aataactggg actacaccag atctgcccag 1920
gccttcactc atctcaaggc caagggagag attccagaag tagcgtttat gaaatgatct 1980

caagaaaagt ccccttgtaa atagcccttg gataatattg tctggctgtc atctgcggac 2040
tcctgtccag cccgaggcca cmctgtaact gtgactgggg aggaagggct gcytcatcck 2100
tcctcaccta ccttctggaa gmcttagcag aagacttccg gaagattgag cctctatggt 2160
gccaggaagc caaagcttac tttgtagagc tdgacrctaa attaccggaa ggacttaggt 2220
gctctgtgta cttaacccca gsattccctt tactttttaa gataaagagt gatattgtag 2280
tgtgtgtccc aaaaaaaaaa aaaa 2304

<210> 104
<211> 175
<212> PRT
<213> Rattus norvegicus

<220>
<223> Alpha B-crystallin

<400> 104
Met Asp Ile Ala Ile His His Pro Trp Ile Arg Arg Pro Phe Phe Pro
1               5                   10                  15

Phe His Ser Pro Ser Arg Leu Phe Asp Gln Phe Phe Gly Glu His Leu
            20                  25                  30

Leu Glu Ser Asp Leu Phe Ser Thr Ala Thr Ser Leu Ser Pro Phe Tyr
        35                  40                  45

Leu Arg Pro Pro Ser Phe Leu Arg Ala Pro Ser Trp Ile Asp Thr Gly
    50                  55                  60

Leu Ser Glu Met Arg Met Glu Lys Asp Arg Phe Ser Val Asn Leu Asp
65                  70                  75                  80

Val Lys His Phe Ser Pro Glu Glu Leu Lys Val Lys Val Leu Gly Asp
                85                  90                  95

Val Ile Glu Val His Gly Lys His Glu Glu Arg Gln Asp Glu His Gly
            100                 105                 110

Phe Ile Ser Arg Glu Phe His Arg Lys Tyr Arg Ile Pro Ala Asp Val
        115                 120                 125

Asp Pro Leu Thr Ile Thr Ser Ser Leu Ser Ser Asp Gly Val Leu Thr
    130                 135                 140

Val Asn Gly Pro Arg Lys Gln Ala Ser Gly Pro Glu Arg Thr Ile Pro
145                 150                 155                 160

Ile Thr Arg Glu Glu Lys Pro Ala Val Thr Ala Ala Pro Lys Lys
                165                 170                 175

<210> 105
<211> 6806
<212> DNA
<213> Rattus norvegicus

<220>
<223> Alpha B-crystallin

<400> 105
gggaagagca gcctatgact ttctgcccac acggacgcca ttcggtctac caggttacag 60
acaggagcag cacaaacaag gcccctagat tttggtttat ttagcctcac ccagaattaa 120

atcgaaggct acgattcatc aagggaccaa accatacact gtggaccatg cacctatccc 180
atctcccagg aatgctgctc cctctggggt gtggtggatt tctggaatca ccacgggagg 240
tagacagggg attcaccggg ttctgggtct ggcagtcagg gttcaactct ggctacctct 300
tcctcttcct cggggtcagg aggtgcagga agcagggaga tgtagacctc attgacttcc 360
gtgtccagat gccggccacc ccgcggtgcc tccaagttaa ggatgccatc atgggagaga 420
gcagctctga cccgccaggg gtccacatct gcaggcagga cataggttgc gacagaactc 480
tcgggacacg aagccgtggc gtccagacgt tgggggtgtc gggcagacac tctagcaggt 540
tgtccacagt cctcacggtc acctcatctg gggtaaagtg gctcacatcc agaaatgcct 600
ggaacttgcc ttcactgagc ctgagctctg aggcccctgc cctgccgccc tccccagctc 660
ctggcggccc ggggccgaac atagtagcca tggtagaggg tgggggtcag gatctcttct 720
ggcaggaggc ctgggagcag agaaagaaag agaaggcaag aggcaggatg aggaagaatg 780
cagcgggcac agacagggct gaaaggcgat atctggagat atggctagaa agagcaggtg 840
ggaaactgta gaggggctgg gaaaggagag gagatggtta cggaaaaagc gagctggaac 900
tggaggacag aagcaaaacc agaaaatact ctgaagcgtg caaggcccgg acaccgaaag 960
ccctgccgat ctaaagtttg gtcctgaagt ctgttagcaa gaccagagag agtcttgctt 1020
gaggggggag tctggggtgg gagatggctc acctcaggag ttcacttaag ccccaggtag 1080
agtggaggcc agtgaagctg ggcagtgagc ccagaatttc cggaggtggg ggtaggaagc 1140
atctgagcca taccttctcc gaagcgctgc tcgccccagg cggctagggt tggcaaattc 1200
gtactcggca gtggctgggt gggcgtgtgg cactgtgcgg cccgacatga ctgctgttgc 1260
gactagtagc cctgatccca agtgtagccc caacaagatc aaggcgaccc ctccagcttc 1320
agagggagag ccaggactca gtcagagtgg gggcggggtc tacaccaccc aaaatagtgc 1380
agagcgtctg ggggatgggg gaggggtggg agcctaagtc tagagtgcag ctatgagggt 1440
gtgatcagcg ctgcctggac cccctaagtc ccctcctctg tatacccttc agctgtcgca 1500
taggcctgga agaacagctg agggtcctgg tcctagagta agctgggaag agaaactggt 1560
gcgctgcagg agggcaagga gaggcctggt tgggcccttc accaattgta cattccacat 1620
caccctttgt ccttatcagt ctcaggcact gagcacatta cttagaaaaa aaaaaaaaaa 1680
gacaatgttc tatgagccac agaacgtcaa aaatacaaga aacattttct gtctttttaa 1740
tgtcagggtc ttctgaacct agatcaactc ggggttccag tcagacacct agttctgaca 1800
tattggtggt cacagctctc ctctgggact ccacaaagag ttaatgtccc tgggctcagc 1860
ccaggaagat tccagcctct gcccaggccc aagatagttg ctggctcaat tcccctggca 1920
tgcaagactg gagaggagga ggggcccacc agcagctgct tgggattcca gaccctgtcc 1980
tggctccaga gaacaaggat ggggtgggtg ggtgccacta ggtgtggaca gagagctagt 2040
gaaacaagac cgtgacaagt caccggccag ctcagccctg ccccgtgttt ctcttttctt 2100
agctcagtga gtactgggta tgtgtcaccc tgccaaatcc ctgatcacaa gtccccatga 2160
actgtcgggg agctgggata ataaaacccc tgacatcacc gttccagaag cttcacaaga 2220
ctgcatatat aaggggcagg ctgtagcagc ggctgaagga gttgaccggc taaccgactc 2280
tacactcatc tagccatcat ggacatagcc atccaccacc cctggatccg gcgtcccttc 2340
tttcctttcc actccccaag ccgcctcttt gaccagttct tcggagagca cctgttggag 2400
tctgacctct tctctacagc cacttccctg agccccttct accttcggcc accctccttc 2460
ctgcgggcac ctagctggat tgacactggg ctctcagagg taagtctccc ttgtgccagg 2520
gcaggaatac gtatccttcc tggtctctcc attcctttgc cctttcaccc tgcctaagtc 2580
attttaggca tgtgcgtgtg caaaggtaca gagaaatgag gttactgttc tcttcctgtg 2640
gcacctgttc tgttgatgct cctgttcctg gtccattttc tgtgcatggt gcctctttct 2700
cctctttcaa ctcctttacc cacctcttta atagtcccag tcctggcaga ttttatcatc 2760
tcaaactgta gagatcagat agaaacccac tcattctggg cttttcctgt cttctttcct 2820
ccccacttcc ttttgatgga gctggataca tcaatcaata ttctctctct ctctctctct 2880
ctctctctct ctctctctct ctctctcttc cttggcctat gactttccgg gttcctcggg 2940
gctgagacag tgcattgact tcagtccatg ccttaagagg accgctcttg ggaagccctg 3000
acttcttatc tcagcagcgt gggagataag attaacagag gttgtcacag ataacactgt 3060
ggtttaaaaa tattcgcgtg tgaggaaaca ggagctgagt gagcaagggc tttggaagga 3120
cagaacattc cagatcgagt gggttggaaa cttggcaggc aactgaacaa gaaaaagggg 3180
gcttttgttt tatagacaaa tgacaaagcc agcactgggt agagaggcag cagatgccat 3240
ccccagaccc attcttgtga ctagtctcct actgacccgt cttcttccct gtgcctctcg 3300
gtaaagtttg ggactgatgg atgactaaga ggtgtctttt gggtatcacc atggtggctc 3360
acatatacca gtgtcactag tcaataagag gatttttccc ccagcagcca ggtagaggat 3420
ggattaccag ggcaggaagt aaatctcctg gacgatgagc cttctccagc ttaatggaca 3480
ttctaccatc ttttctcctc cgcccccttg ttattctaga tgcgtatgga gaaggacagg 3540
ttctctgtga acctggacgt gaagcacttc tctccagagg aactcaaagt caaggttctg 3600
ggagacgtga ttgaggtgca cggcaagcac gaagagcgcc aggtgtgtgg acctttctgc 3660
cttctcttgt gaattcattt agtgccctgc tggatgccag ggactgccgc caccagcctc 3720
tgaggctggc agaattccag tccccaaaca taagactaga tcaggagtta tagatcaatg 3780
gctgggaaac aagttagggc tagatctgct gttagtgttg tggctgtgct gagagaactt 3840

tcccctggat gttccttttg attgccttgt cttgtccagt gacgtcagaa tagcagaaga 3900
attctcaaca ttcccaaccc tcagcacatc ctaggccaat tgaatctgaa gttttaggga 3960
catagctcag gcatcaacct ttaaacaaac aaacaaacaa actcactcct tgggtacttt 4020
ctgtacccca aactggcagc cattgctcta ggcacagtgt gctgtacata atggctctgc 4080
gaaatgacag tcaccacgga gtcgaaggca ataaaatctg tggcttttga atgttcagga 4140
ttccatgcct cttctgctca gtttaatgct atgctacttg gtttgtctgc ttttcagagc 4200
ttcaggctta tggctgtgag gaagttaatt gagatttgtt tgtgtggatg cttggggtac 4260
ttgggattca ataggtaaca ggattagcat ccacaattcc tcttgcattt ttagcatttt 4320
tttttcttga cttcctaaaa aggtgttatg attgcttctt aaaaattaat gacttggggg 4380
gctggggatt tagctcagtg gtagagcgct tacctaggaa gcgcaaggtc ctgggttcgg 4440
tccccagctc cgaaaaaaaa gaaccaaaaa aaaaaaaatt aatgacttgg ggttggggat 4500
ttagctcagc ggtagagcgt ttgcctagca agcgcaaggc cctgggttca gtccccagct 4560
ccgaaaaaaa gaaaagaaaa aaaaattaat gacttagggc ctggagagat ggctcagcag 4620
ttaaaagcac tgaccgctct tccagaggtc ctgcatacaa ttcccagcaa ccacatggtg 4680
gctcacaacc atctgtaatg ggatccaatg ccctcttctg gcatgcaggt gtacatgcag 4740
atagagtgct catacataaa attaaaaaaa tcttaacaaa aaataactca gctgtgccgt 4800
ggtggtgcat gcctttaatc ccaacatttg ggaggcagag gtagacagat ctaagttaga 4860
ggccagctta gactacagaa tgagttccag gagagcagga gctcaacaga gaaaccctac 4920
ttatctcaaa caaaacaaaa aacaaaaatc aatacctcgg ttttgctcat gatttaggac 4980
tggaccacag tgtcactgat agggtcacct tatataatat cagcaatatt atactagaaa 5040
cattctgaga cattcctgag caattagaac aatttatggg cttcctataa gatacagaca 5100
tactcggggt tggggatttg gctcagtggt agagcgtttg cctaggaagc gcaaggtcct 5160
gggttcggtc cccagccctg aaaaaaagaa aaaagaaaaa aaaaaaagat acagacatac 5220
tcaatgccaa agtgggagtc taaattccat ggtccagtca acatggattc attcctttgt 5280
ggaaatgccc tgaattgtag ttgtgtctcc gaatatgaag atctatgaaa ttcggggctg 5340
tttcagattt cctacactct gattggtagg tgtgtccatc tggacagttt attctagcct 5400
taaacctgga caaacagctc tggacttagc tatctgcata tttacagtgt tggaggtgaa 5460
ctcaggcctt tcactactag actgaatccc cagctccaga tattttttcc ccccttaggg 5520
ccttttgttc taacccaagg gcaggggatg tctagagtct ggggcaagtg gtggtatttc 5580
ctgctcttat ttctctccct tgcccttcat tctctcggat taggacgaac atggcttcat 5640
ctccagggag ttccacagga agtaccggat cccagccgac gtggatcctc tcaccattac 5700
ttcttccctg tcatcggatg gagtcctcac tgtgaatgga ccaaggaaac aggcctctgg 5760
ccctgagcgc accattccca tcacccgtga agagaagcct gctgtcactg cagcccctaa 5820
gaagtagatt ccctttcctc gttgcatttt ttaagacaag gaagtttccc atcagcgaat 5880
gaacatctgt gactagtgcc gaagcttact aatgctaagg gctggcccag attattaagc 5940
taataaaaaa tatcgttcag caacagacct gcctcgtctt tgcaaaccca gtgtgcttta 6000
aaccaatctg caaacatagc agatctgctg atccccaccc tgtgattcag ggggctccga 6060
ggatacgttt ggtcagagtg attggctcca gaactttgta cagtacaaac ccagggaaaa 6120
ccattcactc tcctggtatt ccagtcttca gtttataaca cactgtactg gttaggacag 6180
agtcttagga gtagactaag aagggagaaa tggtgacgtc ccagcagggt catgatgaat 6240
cagctctggc taactccaca taaaagactt cagtgcattt ctgaagatta attacttcac 6300
cagggggaca cggtggcctt ggtgttattt gaagagtgtc ctcagctaat tcacaggaca 6360
gtaaatagct cgtgggtggt gactgtcgaa cgagagagcc tctcgggaat ggcacagagt 6420
aatcaacaga taccttttaa accgagacca ttctctacca aacatgaaaa tgacaataaa 6480
gggtccagca aaaaaacaaa caccaaaatc agaagaacat cttcctccta aagttactaa 6540
cccctggggt ggggggaagc ccacacaaaa tagaaacccc ccaaaaacct ctctccattt 6600
taatgggtct agacatagtt tctgtcagat tacactgagc cctgctaccc gatatctggc 6660
aagcctgtag caccagttct catctctcct gtgttaactt taggttcact tgaggatgtg 6720
ccttttgaag atttttttta tttagtaatt attatttttt tataaaactt gataaaaaat 6780
agtatttcca actgtacagt cactag 6806

<210> 106
<211> 646
<212> PRT
<213> Rattus norvegicus

<220>
<223> Heat shock cognate protein 70

<400> 106
Met Ser Lys Gly Pro Ala Val Gly Ile Asp Leu Gly Thr Thr Tyr Ser

1               5                   10                  15

Cys Val Gly Val Phe Gln His Gly Lys Val Glu Ile Ile Ala Asn Asp
            20                  25                  30

Gln Gly Asn Arg Thr Thr Pro Ser Tyr Val Ala Phe Thr Asp Thr Glu
        35                  40                  45

Arg Leu Ile Gly Asp Ala Ala Lys Asn Gln Val Ala Met Asn Pro Thr
    50                  55                  60

Asn Thr Val Phe Asp Ala Lys Arg Leu Ile Gly Arg Arg Phe Asp Asp
65                  70                  75                  80

Ala Val Val Gln Ser Asp Met Lys His Trp Pro Phe Met Val Val Asn
                85                  90                  95

Asp Ala Gly Arg Pro Lys Val Gln Val Glu Tyr Lys Gly Glu Thr Lys
            100                 105                 110

Ser Phe Tyr Pro Glu Glu Val Ser Ser Met Val Leu Thr Lys Met Lys
        115                 120                 125

Glu Ile Ala Glu Ala Tyr Leu Gly Lys Thr Val Thr Asn Ala Val Val
    130                 135                 140

Thr Val Pro Ala Tyr Phe Asn Asp Ser Gln Arg Gln Ala Ala Lys Asp
145                 150                 155                 160

Ala Gly Thr Ile Ala Gly Leu Asn Val Leu Arg Ile Ile Asn Glu Pro
                165                 170                 175

Thr Ala Ala Ala Ile Ala Tyr Gly Leu Asp Lys Lys Val Arg Ala Glu
            180                 185                 190

Arg Asn Val Leu Ile Phe Asp Leu Gly Gly Gly Thr Phe Asp Val Ser
        195                 200                 205

Ile Leu Thr Thr Glu Asp Gly Ile Phe Glu Val Lys Ser Thr Ala Gly
    210                 215                 220

Asp Thr His Leu Gly Gly Glu Asp Phe Asp Asn Arg Met Val Asn His
225                 230                 235                 240

Phe Ile Ala Glu Phe Lys Arg Lys His Lys Lys Asp Ile Ser Glu Asn
                245                 250                 255

Lys Arg Ala Val Arg Arg Leu Arg Thr Ala Cys Glu Arg Ala Lys Arg
            260                 265                 270

Thr Leu Ser Ser Ser Thr Gln Ala Ser Ile Glu Ile Asp Ser Leu Tyr
        275                 280                 285

Glu Gly Ile Asp Phe Tyr Thr Ser Ile Thr Arg Ala Arg Phe Glu Glu
    290                 295                 300

Leu Asn Ala Asp Leu Phe Arg Gly Thr Leu Asp Pro Val Glu Lys Ala
305                 310                 315                 320

Leu Arg Asp Ala Lys Leu Asp Lys Ser Gln Ile His Asp Ile Val Leu
                325                 330                 335

Val Gly Gly Ser Thr Arg Ile Pro Lys Ile Gln Lys Leu Leu Gln Asp
            340                 345                 350

Phe Phe Asn Gly Lys Glu Leu Asn Lys Ser Ile Asn Pro Asp Glu Ala
        355                 360                 365

Val Ala Tyr Gly Ala Ala Val Gln Ala Ala Ile Leu Ser Gly Asp Lys
    370                 375                 380

Ser Glu Asn Val Gln Asp Leu Leu Leu Leu Asp Val Thr Pro Leu Ser
385                 390                 395                 400

Leu Gly Ile Glu Thr Ala Gly Gly Val Met Thr Val Leu Ile Lys Arg
                405                 410                 415

Asn Thr Thr Ile Pro Thr Lys Gln Thr Gln Thr Phe Thr Thr Tyr Ser
            420                 425                 430

Asp Asn Gln Pro Gly Val Leu Ile Gln Val Tyr Glu Gly Glu Arg Ala
        435                 440                 445

Met Thr Lys Asp Asn Asn Leu Leu Gly Lys Phe Glu Leu Thr Gly Ile
    450                 455                 460

Pro Pro Ala Pro Arg Gly Val Pro Gln Ile Glu Val Thr Phe Asp Ile
465                 470                 475                 480

Asp Ala Asn Gly Ile Leu Asn Val Ser Ala Val Asp Lys Ser Thr Gly
                485                 490                 495

Lys Glu Asn Lys Ile Thr Ile Thr Asn Asp Lys Gly Arg Leu Ser Lys
            500                 505                 510

Glu Asp Ile Glu Arg Met Val Gln Glu Ala Glu Lys Tyr Lys Ala Glu
        515                 520                 525

Asp Glu Lys Gln Arg Asp Lys Val Ser Ser Lys Asn Ser Leu Glu Ser
    530                 535                 540

Tyr Ala Phe Asn Met Lys Ala Thr Val Glu Asp Glu Lys Leu Gln Gly
545                 550                 555                 560

Lys Ile Asn Asp Glu Asp Lys Gln Lys Ile Leu Asp Lys Cys Asn Glu
                565                 570                 575

Ile Ile Ser Trp Leu Asp Lys Asn Gln Thr Ala Glu Lys Glu Glu Phe
            580                 585                 590

Glu His Gln Gln Lys Glu Leu Glu Lys Val Cys Asn Pro Ile Ile Thr
        595                 600                 605

Lys Leu Tyr Gln Ser Ala Gly Gly Met Pro Gly Gly Met Pro Gly Gly
    610                 615                 620

Phe Pro Gly Gly Gly Ala Pro Pro Ser Gly Gly Ala Ser Ser Gly Pro
625                 630                 635                 640

Thr Ile Glu Glu Val Asp
                645

<210> 107

<211> 2242
<212> DNA
<213> Rattus norvegicus

<220>
<223> Heat shock cognate protein 70

<400> 107
atgcactttc aaagtagtga gaaaattttg atgttttcta atacatagaa atgatatcat 60
taaaaggcag atgtggctca ttgaacgcag agccagctct cgggggtctc tgtgtggtct 120
cgtcatcagc acagctgggc ctacacgcaa gcaaccatgt ctaagggacc tgcagttggc 180
attgatcttg gcaccaccta ctcctgtgtg ggtgtcttcc agcatggaaa ggtggaaata 240
attgccaatg accagggtaa ccgcaccacg ccgagctatg ttgctttcac cgacacagaa 300
cgattaattg gggatgcggc caagaatcag gttgcaatga accccaccaa cacagttttt 360
gatgccaaac gtctgatcgg acgtaggttc gatgatgctg ttgttcagtc tgacatgaag 420
cactggccct tcatggtggt gaacgatgca ggcaggccca aggtccaagt cgaatacaaa 480
ggggagacaa aaagtttcta tcctgaggaa gtgtcttcaa tggttctgac aaaaatgaag 540
gaaattgcag aagcttacct tggaaagact gttaccaatg ccgtggtcac cgtgccagct 600
tacttcaatg actctcagcg acaggcagca aaagatgctg gaactattgc tggcctcaac 660
gtacttcgaa ttatcaatga gccaactgct gctgctattg cctatggctt agataagaag 720
gtcagggctg aaaggaatgt gctcattttt gacttgggag gtggcacttt tgatgtgtca 780
attctgacta ctgaggatgg aatttttgaa gtcaaatcaa cagctggaga cacccacttg 840
ggcggagaag actttgacaa ccgaatggtc aaccatttca ttgctgagtt taagcgaaag 900
cacaagaagg acatcagtga gaacaagaga gctgtcaggc gtctccgcac tgcctgtgag 960
cgggccaagc gcaccctctc ctccagcacc caggccagta ttgagattga ttctctctat 1020
gagggaattg acttctacac ctccattacc cgtgctcgat ttgaggagtt gaatgctgac 1080
ctgttccgtg gcacactgga ccctgtagag aaggcccttc gagatgccaa actagacaag 1140
tcacagatcc atgatattgt cctggtgggt ggttctacca gaatccccaa gatccagaaa 1200
cttctgcaag acttcttcaa tggaaaagag ctgaataaga gcattaaccc cgatgaagct 1260
gttgcctatg gtgcagctgt ccaggcagcc attctatctg gagacaagtc tgagaatgtt 1320
caggatttgc tgctcttgga tgtcactcct ctttcccttg ggattgaaac tgctggtgga 1380
gtcatgactg tcctcatcaa gcgcaatacc accattccca ccaagcagac ccagactttc 1440
accacctact ctgacaacca gccaggtgta ctcatccagg tgtatgaagg tgaaagggcc 1500
atgaccaagg acaacaacct gcttgggaag tttgagctca caggcatacc tccagcaccc 1560
cgtggggttc ctcagattga ggttactttt gacattgatg ccaatggcat cctcaatgtt 1620
tctgctgtag ataagagcac aggaaaggag aacaagatca ccatcaccaa tgacaagggc 1680
cgcttgagta aggaggatat tgagcgcatg gtccaagaag ctgagaagta caaagctgag 1740
gatgagaagc agagagataa ggtttcctct aagaactcgc tggagtctta tgctttcaac 1800
atgaaagcaa ctgttgagga tgagaaactt caaggcaaga tcaatgatga agacaaacag 1860
aagattcttg acaagtgcaa cgaaatcatc agctggctgg ataagaacca gactgcggag 1920
aaggaagaat ttgagcatca gcagaaagaa ctggagaagg tctgcaaccc tatcatcacc 1980
aagctgtacc agagtgctgg tggcatgcct ggaggaatgc ctggtggctt ccctggtgga 2040
ggagctcctc catctggtgg tgcttcttca ggccccacca ttgaagaggt cgattaagtc 2100
aaagtagagg gtgtagcatt gttccacagg gacccaaaac aagtaacatg gaataataaa 2160
actatttaaa ttggcacaaa aaaaaaaaaa aaaacggaga tgtggtgact cagctgtaat 2220
cccggcactt aggaagctga gg 2242

<210> 108
<211> 374
<212> PRT
<213> Rattus norvegicus

<220>
<223> Tau microtubule associated protein

<400> 108
Met Ala Glu Pro Arg Gln Glu Phe Asp Thr Met Glu Asp Gln Ala Gly
1               5                   10                  15

Asp Tyr Thr Met Leu Gln Asp Gln Glu Gly Asp Met Asp His Gly Leu
            20                  25                  30

Lys Ala Glu Glu Ala Gly Ile Gly Asp Thr Pro Asn Met Glu Asp Gln
        35                  40                  45

Ala Ala Gly His Val Thr Gln Ala Arg Val Ala Gly Val Ser Lys Asp
    50                  55                  60

Arg Thr Gly Asn Asp Glu Lys Lys Ala Lys Gly Ala Asp Gly Lys Thr
65                  70                  75                  80

Gly Ala Lys Ile Ala Thr Pro Arg Gly Ala Ala Thr Pro Gly Gln Lys
                85                  90                  95

Gly Thr Ser Asn Ala Thr Arg Ile Pro Ala Lys Thr Thr Pro Ser Pro
            100                 105                 110

Lys Thr Pro Pro Gly Ser Gly Glu Pro Pro Lys Ser Gly Glu Arg Ser
        115                 120                 125

Gly Tyr Ser Ser Pro Gly Ser Pro Gly Thr Pro Gly Ser Arg Ser Arg
    130                 135                 140

Thr Pro Ser Leu Pro Thr Pro Pro Thr Arg Glu Pro Lys Lys Val Ala
145                 150                 155                 160

Val Val Arg Thr Pro Pro Lys Ser Pro Ser Ala Ser Lys Ser Arg Leu
                165                 170                 175

Gln Thr Ala Pro Val Pro Met Pro Asp Leu Lys Asn Val Arg Ser Lys
            180                 185                 190

Ile Gly Ser Thr Glu Asn Leu Lys His Gln Pro Gly Gly Gly Lys Val
        195                 200                 205

Gln Ile Ile Asn Lys Lys Leu Asp Leu Ser Asn Val Gln Ser Lys Cys
    210                 215                 220

Gly Ser Lys Asp Asn Ile Lys His Val Pro Gly Gly Gly Ser Val Gln
225                 230                 235                 240

Ile Val Tyr Lys Pro Val Asp Leu Ser Lys Val Thr Ser Lys Cys Gly
                245                 250                 255

Ser Leu Gly Asn Ile His His Lys Pro Gly Gly Gly Gln Val Glu Val
            260                 265                 270

Lys Ser Glu Lys Leu Asp Phe Lys Asp Arg Val Gln Ser Lys Ile Gly
        275                 280                 285

Ser Leu Asp Asn Ile Thr His Val Pro Gly Gly Gly Asn Lys Lys Ile
    290                 295                 300

Glu Thr His Lys Leu Thr Phe Arg Glu Asn Ala Lys Ala Lys Thr Asp
305                 310                 315                 320

His Gly Ala Glu Ile Val His Lys Ser Pro Val Val Ser Gly Asp Thr
                325                 330                 335

Ser Pro Arg His Leu Ser Asn Val Ser Ser Thr Gly Ser Ile Asp Met
            340                 345                 350

Val Asp Ser Pro Gln Leu Ala Thr Leu Ala Asp Glu Val Ser Ala Ser

        355                 360                 365

Leu Ala Lys Gln Gly Leu
    370

<210> 109
<211> 5240
<212> DNA
<213> Rattus norvegicus

<220>
<223> Tau microtubule associated protein

<400> 109
cctcccctgg ggaggttcgc gttcccgtgg ctcgcgcctg ctgcccgccg gcctcgagaa 60
cgcgctctct cggccgcggc gctcacagtc tccgccaccc accagctcca gcaccagcag 120
cagcgccggc gccaccgccc accttctgct gtcgccgccg ccacaaccac cttcccctcc 180
gctgtcctct tctgtcctcg cctcctgtcg attatcaggc tttgaagcag catggctgaa 240
ccccgccagg agtttgacac aatggaagac caggccggag attacactat gctccaagac 300
caagaaggag acatggacca tggcttaaaa gctgaagaag caggcatcgg agacaccccg 360
aacatggagg accaagctgc tgggcatgtg actcaagctc gagtggccgg cgtaagcaaa 420
gacaggacag gaaatgacga gaagaaagcg aagggggccg atggcaaaac gggggcgaag 480
atcgccacac ctcggggagc agccactccg ggccagaaag gcacatccaa tgccaccagg 540
atcccagcca agaccacacc cagcccaaag actcctccag gatcaggtga accaccaaaa 600
tccggagaac gaagcggcta cagcagcccc ggctcgcccg gaacccctgg cagtcgctcc 660
cgtaccccat ccctaccaac gccgcccacc cgagagccca aaaaggtggc agtggttcgc 720
actcccccta agtcaccgtc tgccagtaag agccgcctac agactgcccc tgtgcccatg 780
ccagacctaa agaacgtcag gtccaagatt ggctccactg agaacctgaa gcaccagccg 840
ggaggcggca aggtgcagat aattaataag aagctggatc ttagcaacgt ccagtccaag 900
tgtggctcaa aggacaatat caaacacgtc ccgggcggag gcagtgtgca aatagtctac 960
aagccagtgg acctgagcaa ggtgacctcc aagtgtggtt ccttagggaa catccatcac 1020
aagccaggag gtggccaggt agaagtaaaa tcagagaagc tggacttcaa ggatagagtc 1080
cagtcgaaga ttggctcctt ggataacatc acccatgtcc ctggaggagg gaataagaag 1140
attgaaaccc acaagctgac cttcagggag aatgccaaag ccaagacaga ccatggagca 1200
gaaatcgtgc acaagtcacc tgtggtgtct ggggacacat ctccacggca cctcagcaac 1260
gtctcctcca cgggcagcat cgacatggtg gactctccac agcttgccac gttagccgat 1320
gaagtgtccg cctctttggc caagcagggt ttgtgatcag gcccctgggg ccgtcactga 1380
tcatggagag aagagagagt gagagtgtgg aaaaaaaaaa aaaaagaatg acctggcccc 1440
tcaccctctg ccctccccgc tgctcctcat agacaggctg accagcttgt cacctaacct 1500
gcttttgtgg ctcgggtttg gctcgggact tcaaaatcag tgatgggaaa aagtaaattt 1560
catctttcca aattgatttg tgggctagta ataaaatatt tttaaggaag gaaaaaaaaa 1620
acacgtaaaa ccatggccaa acaaaaccca acatttcctt ggcaattgtt attgaccccg 1680
cccccccctc tgagttttag agggtgaagg aggctttgga tagaggctgc ttctggggat 1740
tggctgaggg actagggcaa ctaattgccc acagccccat cttaggggca tcagggacag 1800
cggcagacat gaaagacttg ggacttggtg tgtttgtgga gccgtaaggc gtatgttaac 1860
tttgtgtggg tttgagggag gactgtgata gtgaaggctg agagatgggt gggctgggag 1920
tcagaggaga gaggtgagga agacaggttg ggagaggggg cattgcgtcc ttgccaagga 1980
gcttgggaag cacaggtagc cctggctgca gcagtcttag ctagcacaga tgcctgcctg 2040
agaaagcaca gtggggtaca gtgggtgtgt gtgccccttc tgaagggcag cccatgggag 2100
aaggggtatt gggcagaagg aaggtaggcc ccagaaggtg gcaccttgta gattggttct 2160
ctgaaggctg accttgccat cccagggcac tgctcccacc ctccaggagg aggtctgagc 2220
tgaggagctt ccttttcgat ctcacaggaa aacctgtgtt actgagttct gaagtttgga 2280
actacagcca tgattttggc caccatacag acctgggact ttagggctaa ccagttcttt 2340
gtaaggactt gtgcctcttg cgggaacatc tgcctgttct caagcctggt cctctggcac 2400
ttctgcagtg gtgagggatg ggggtggtat tctgggatgt gggtcccagg cctcccatcc 2460
ctcgcacagc cactgtatcc cctctacctg tcctatcatg cccacgtctg ccacgagagc 2520
cagtcactgc cgtccgtaca tcacgtctca ccgtcctgag tgcccagcct cccaagccca 2580
atccctggac ccctgggtag ttatggccaa tctgctctac actaggggtt ggagtccagg 2640
gaaggcaaag atttgggcct tggtctctag tcctacgttg ccagaatcca accagtgtgc 2700
ctcccacaag gaaccttaca accttgtttg gtttgctcca tcaggcgttt ggcgccatcg 2760
tggatggagt ccgtgtgtgc ctggagatta ccctggacac ctctgctttt ttttttttta 2820

ctttagcggt tgcctcctag gcctgactcc ttcccatgtt gaactggagg cagccaagtt 2880
aggtgtcaat gtcctggcat cagtatgaac agtcagtagt cccagggcag ggccacactt 2940
ctcccatctt ctgcttccac cccagcttgt gattgctagc ctcccagagc tcagccgcca 3000
ttaagtcccc atgcacgtaa tcagcccttc cataccccaa tttggggaac ataccccttg 3060
attgaaatgt tttccctcca gtcctatgga agcggtgctg cctgcctgct ggagcagcca 3120
gccatctcag agacgcagcc ctttctctcc tgtccgcacc ctgctgcgct gtagtcggat 3180
tcgtctgttt gtctgggttc accagagtga ctatgatagt gaaaagaaaa agaaaaagaa 3240
aaaagaaaaa agaaaaaaaa aaaaggacgc atgttatctt gaaatatttg tcaaaaggtt 3300
gtagcccacc gcagggattg gagggcctga tattccttgt cttcttcgtg acttaggtcc 3360
aggccggtcg agtgctaccc tgctggacat cccatgtttt gaagggtttc ttcttcatct 3420
gggacccctg cagacactgg attgtgacat tggaggtcta tacattggcc aaggctgaag 3480
cacaggaccc gttagaggca gcaggctccg actgtcaggg agagcttgtg gctggcctgt 3540
ttctctgagt gaagatggtc ctctctaatc acaacttcaa gtcccacagc agccctggca 3600
gacatctaag aactcctgca tcacaagaga aaaggacact agtaccagca gggagagctg 3660
tggccctaga aattccatga ctctccacta ctatccgtgg gtcctttcca agccttgcct 3720
cgtcaccaag ggcttgggat ggactgcccc actgatgaaa gggacatctt tggagacccc 3780
cttggtttcc aaggcgtcag ccccctgacc ttgcatgacc tcctacagct gaaggatgag 3840
gcctttaaag attaggaacc tcaggcccag gtcggccact ttgggcttgg gtacagttag 3900
ggacgatgcg gtagaaggag gtggccaacc tttccatata agagttctgt gtgcccagag 3960
ctaccctatt gtgagctccc cactgctgat ggactttagc tgtccttaga agtgaagagt 4020
ccaacggagg aaaaggaagt gtggtttgat ggtctgtggt cccttcatca tggttacctg 4080
ttgtggtttt ctctgtatac ccccatttac ccatcctgca gttcctgtcc ttgaataggg 4140
gtgggggtac tctgccatat ctcttgtagg cagtcagccc ccaagtcata gtttggagtg 4200
atctggtcag tgctaatagg cagtttacaa ggaattctgg cttgttactt cagtgaggac 4260
aatcccccaa ggccctggca cctgtcctgt ctttccatgg ctctccactg cagagccaat 4320
gtctttgggt gggctagata gggtgtacaa tttgcctgga acctccaagc tcttaatcca 4380
ctttatcaat agttccattt aaattgactt caatataaga gtgtatccat ttgagattgc 4440
ttgtgttgtg gggtaaaggg gggaggagga acatgttaag ataattgaca tgggcaaggg 4500
gaagtcttga agtgtagcag ttaaaccatc ttgtagcccc attcatgatg ttgaccactt 4560
gctagagaga agaggtgcca taaggctaga acctagaggc ttggctgtcc accaacaggc 4620
aggcttttgc aaggcagagg cagccagcta ggtccctgac ttcccagcca ggtgcagctc 4680
taagaactgc tcttgcctgc tgccttcttg tggtgtccag agcccacagc caatgcctcc 4740
tcaaaaccct ggcttccttc cttctaatcc actggcacat cagcatcacc tccggattga 4800
cttcagatcc acagcctaca ctactagcag tgggtaagac cacttccttt gtccttgtct 4860
gttctccaga aaagtgggca tggaggcggt gttaataact ataggtctgt ggctttatga 4920
gccttcaaac ttctctctag cttctgaaag ggttactttt gggcagtatt gcagtctcac 4980
cctccgatgg ctgtagcctg tgcagttgct gtactgggca tgatctccag tgcttgcaag 5040
tcccatgatt tctttggtgt tttgagggtg gggggaggga catgaatcat cttagcttag 5100
cttcctgtct gtgaatgtcc atatagtgta ctgtgtttta acaaacgatt tacactgact 5160
gttgctgtac aagtgaattt ggaaataaag ttattactct gattaaacaa aaaaaaaaaa 5220
aaaaaaaaaa aaaaaaaaaa 5240

<210> 110
<211> 248
<212> PRT
<213> Rattus norvegicus

<220>
<223> Schwann cell peripheral myelin

<400> 110
Met Ala Pro Gly Ala Pro Ser Ser Ser Pro Ser Pro Ile Leu Ala Ala
1               5                   10                  15

Leu Leu Phe Ser Ser Leu Val Leu Ser Pro Thr Leu Ala Ile Val Val
            20                  25                  30

Tyr Thr Asp Arg Glu Val Tyr Gly Ala Val Arg Ser Gln Val Thr Leu
        35                  40                  45

His Cys Ser Phe Trp Ser Ser Glu Trp Val Ser Asp Asp Ile Ser Phe

50                  55                  60

Thr Trp Arg Tyr Gln Pro Glu Gly Gly Arg Asp Ala Ile Ser Ile Phe
65                  70                  75                  80

His Tyr Ala Lys Gly Gln Pro Tyr Ile Asp Glu Val Gly Thr Phe Lys
                85                  90                  95

Glu Arg Ile Gln Trp Val Gly Asp Pro Ser Trp Lys Asp Gly Ser Ile
            100                 105                 110

Val Ile His Asn Leu Asp Tyr Ser Asp Asn Gly Thr Phe Thr Cys Asp
        115                 120                 125

Val Lys Asn Pro Pro Asp Ile Val Gly Lys Thr Ser Gln Val Thr Leu
    130                 135                 140

Tyr Val Phe Glu Lys Val Pro Thr Arg Tyr Gly Val Val Leu Gly Ala
145                 150                 155                 160

Val Ile Gly Gly Ile Leu Gly Val Val Leu Leu Leu Leu Leu Phe
                165                 170                 175

Tyr Leu Ile Arg Tyr Cys Trp Leu Arg Arg Gln Ala Ala Leu Gln Arg
            180                 185                 190

Arg Leu Ser Ala Met Glu Lys Gly Lys Phe His Lys Ser Ser Lys Asp
        195                 200                 205

Ser Ser Lys Arg Gly Arg Gln Thr Pro Val Leu Tyr Ala Met Leu Asp
    210                 215                 220

His Ser Arg Ser Thr Lys Ala Ala Ser Glu Lys Lys Ser Lys Gly Leu
225                 230                 235                 240

Gly Glu Ser Arg Lys Asp Lys Lys
                245


<210> 111
<211> 1029
<212> DNA
<213> Rattus norvegicus

<220>
<223> Schwann cell peripheral myelin

<400> 111
attcggctgg gcccttgccc ctaccccagc tatggctcct ggggctccct catccagccc 60
cagccctatc ctggctgccc tgctcttctc ttctttggtg ctgtccccaa ccctggccat 120
tgtggtttac acggacaggg aagtctatgg tgctgtgcgc tcccaggtca ccctgcactg 180
ctccttctgg tccagtgaat gggtctcaga tgacatctct tttacctggc gctaccagcc 240
tgaaggaggc cgagatgcca tttcaatctt ccactatgcc aagggtcaac cttacatcga 300
tgaggtgggg accttcaagg agcgcatcca gtgggtaggg gaccctagct ggaaggatgg 360
ctccattgtc atacacaacc tagactacag tgacaacggc actttcacat gtgatgtcaa 420
aaacccaccg gacatagtgg gcaagacgtc tcaggtcacg ctctatgtct ttgaaaaagt 480
gcccactagg tatggggtgg tgttgggagc cgtgatcggt ggcatcctcg gggtggtgct 540
gttgctgctg ttgctcttct acctgatccg gtactgctgg ctgcgcaggc aggctgccct 600
gcagaggagg ctcagtgcca tggagaaggg gaaatttcac aagtcttcta aggactcctc 660
gaagcgcggg cggcagacgc cagtgctgta tgccatgctg gaccacagcc gaagcaccaa 720
agctgccagt gagaagaaat ctaaagggct gggggagtct cgcaaggata agaaatagcg 780
gttagcgggc cgggcggggg gtcgggggtc tgcgatggag tcttccaaag gctctcaggt 840

ggtggtcatc gagatggagc ttcgcaaaga tgagcagagc tcggagcccc ggcctgcagt 900
caaatccccc agtagaacca gcctcaagaa cgccctcaag aacatgatgg gcctggactc 960
gaacaagtga ccgtcacctc caggctctgt cagagcaggg gacctaggct cctctttttc 1020
ccagtctag 1029

<210> 112
<211> 444
<212> PRT
<213> Rattus norvegicus

<220>
<223> Class 1 beta tubulin

<400> 112


<210> 113
<211> 2346
<212> DNA
<213> Rattus norvegicus

<220>
<223> Class 1 beta tubulin

<400> 113
ctcgcatctc tctgcagcag cctccggggc tacgttccat cctatcagtc tgagaccagc 60
ccagaagaaa aaaaccaaaa atccttaatt atcttctttt gctcggtacc tacattggga 120
ccatcaaaaa aaaattatta cagtaaaccg tagccatgag ggaaatcgtg cacatccagg 180
ccggacagtg tggcaaccag atcggtgcta agttctggga ggtgataagc gatgaacacg 240
gcatcgaccc caccggcacc taccacggag acagcgactt gcagctggac cgaatctctg 300
tgtactacaa tgaagctaca ggtggcaagt atgtccctcg agctatctta gtggatctag 360
aacccgggac tatggactcc gttcgctcag gtccttttgg ccagatcttc agaccggaca 420
actttgtttt tggtcagtct ggggcaggca acaactgggc taagggtcac tacacagagg 480
gagctgagct ggttgactct gtcttggatg tggtgcggaa ggaggcggag agctgtgact 540
gcctgcaagg ctttcagctg acccactcgc tgggtggagg cacgggctct ggcatgggca 600
ccctgctcat cagcaagatt cgagaagaat accccgaccg catcatgaac accttcagcg 660
tggtgccctc acccaaagtg tctgacaccg tggttgagcc ctacaacgcc accctgtccg 720
tccaccagtt ggtagagaac acagatgaga cctactgcat tgacaacgag gctctctacg 780
acatctgctt ccgtaccctt aagctcacca cgccaaccta cggagacctg aaccatctcg 840
tctctgccac catgagcggt gtcaccacct gcctccgttt cccgggccag ctgaacgccg 900

accttcggaa gctggctgtc aacatggtgc ccttcccgcg tctccacttc ttcatgcctg 960
gctttgcccc tctcaccagc cgtggaagcc agcagtaccg ggccctcact gtgcctgaac 1020
tcacccagca ggtcttcgac gccaagaaca tgatggccgc ctgtgaccca cgccacggcc 1080
ggtacctcac ggttgcggct gtcttccgtg gacggatgtc catgaaggag gtagatgagc 1140
aaatgctcaa cgtgcagaat aagaatagca gctacttcgt ggaatggatc cccaacaacg 1200
tcaagacggc tgtctgtgac atcccaccgc gaggcctcaa gatggcagtc accttcatcg 1260
gaaacagcac ggccatccag gagctcttca agcgcatctc tgagcagttt acggcaatgt 1320
tccgtcggaa ggctttcctc cactggtaca cgggtgaggg catggacgag atggagttca 1380
ccgaagctga gagcaacatg aatgacctcg tctctgagta tcagcagtac caggacgcca 1440
ccgcagaaga ggaagaggat ttcggagagg aggcagaaga agaggcctaa ggcggaagcc 1500
ctgcgtcacc tcaggctgct tagttccctt agcttttttc caactgccct ttgtttcttt 1560
ctgctgcctc tgtcctgttt ttgttccgtt ttgttttctc attggggggt aaacggtgcc 1620
tggcacgtgg caggcgctca ataaatattt ctttgtggaa tgtctccttt ctctttccga 1680
tctgacattc tgagcgttaa ccctgtgctt cctcctggta cccatttctt tattatgact 1740
gtccagttaa tattcttcca atatattctc caggaaccag cgttcggctc cagacccggg 1800
gtagaaccca gcccagttct aagaactcaa gagagtagcc accatcttaa agcaaagtag 1860
cgggtgggga aaaggtgggt agaggagatg ctacttaggg ggtgggattt tccagtcagg 1920
gccatttaga cacgggaatc gctggggctt tcagtcgatg ggctttcttt tttccctgc 1980
ttcatctctc ggcctcagga gaggtattaa cagtattatc accatttata tcctagctgt 2040
cctgagccaa atctgccatt ggaggtgtct tcccctgtgt tggttctcca agggacgttg 2100
tatggggatt tgtgggggca gggcagcaag gccttgttct ctaaatgtcc agaaacactc 2160
taattaccat ttccacctgg gtgcctcaca cactccccag aggggaggta acatctctgc 2220
cccatttcca tcctgttcct tggcttggtc attccctacc tttctatttt ctgttaaact 2280
tggctttttt tttttcatat tgaaaagatg acattgcccc gagagccaaa aataaatggg 2340
aattgg 2346

<210> 114
<211> 724
<212> PRT
<213> Rattus norvegicus

<220>
<223> Heat shock protein 90

<400> 114
Met Pro Glu Glu Val His His Gly Glu Glu Glu Val Glu Thr Phe Ala
1               5                   10                  15

Phe Gln Ala Glu Ile Ala Gln Leu Met Ser Leu Ile Ile Asn Thr Phe
            20                  25                  30

Tyr Ser Asn Lys Glu Ile Phe Leu Arg Glu Leu Ile Ser Asn Ala Ser
        35                  40                  45

Asp Ala Leu Asp Lys Ile Arg Tyr Glu Ser Leu Thr Asp Pro Ser Lys
    50                  55                  60

Leu Asp Ser Gly Lys Glu Leu Lys Ile Asp Ile Ile Pro Asn Pro Gln
65                  70                  75                  80

Glu Ala Thr Leu Thr Leu Val Asp Thr Gly Ile Gly Met Thr Lys Ala
                85                  90                  95

Asp Leu Ile Asn Asn Leu Gly Thr Ile Ala Lys Ser Gly Thr Lys Ala
            100                 105                 110

Phe Met Glu Ala Leu Gln Ala Gly Ala Asp Ile Ser Met Ile Gly Gln
        115                 120                 125

Phe Gly Val Gly Phe Tyr Ser Ala Tyr Leu Val Ala Glu Lys Val Val
    130                 135                 140

Val Ile Thr Lys His Asn Asp Asp Glu Gln Tyr Ala Trp Glu Ser Ser
145                 150                 155                 160

Ala Gly Gly Ser Phe Thr Val Arg Ala Asp His Gly Glu Pro Ile Gly
                165                 170                 175

Arg Gly Thr Lys Val Ile Leu His Leu Lys Glu Asp Gln Thr Glu Tyr
            180                 185                 190

Leu Glu Glu Arg Arg Val Lys Glu Val Val Lys Lys His Ser Gln Phe
        195                 200                 205

Ile Gly Tyr Pro Ile Thr Leu Tyr Leu Glu Lys Glu Arg Glu Lys Glu
    210                 215                 220

Ile Ser Asp Asp Glu Ala Glu Glu Glu Lys Gly Glu Lys Glu Glu Glu
225                 230                 235                 240

Asp Lys Glu Asp Glu Glu Lys Pro Lys Ile Glu Asp Val Gly Ser Asp
                245                 250                 255

Glu Glu Asp Asp Ser Gly Lys Asp Lys Lys Lys Lys Thr Lys Lys Ile
            260                 265                 270

Lys Glu Lys Tyr Ile Asp Gln Glu Glu Leu Asn Lys Thr Lys Pro Ile
        275                 280                 285

Trp Thr Arg Asn Pro Asp Asp Ile Thr Gln Glu Glu Tyr Gly Glu Phe
    290                 295                 300

Tyr Lys Ser Leu Thr Asn Asp Trp Glu Asp His Leu Ala Val Lys His
305                 310                 315                 320

Phe Ser Val Glu Gly Gln Leu Glu Phe Arg Ala Leu Leu Phe Ile Pro
                325                 330                 335

Arg Arg Ala Pro Phe Asp Leu Phe Glu Asn Lys Lys Lys Lys Asn Asn
            340                 345                 350

Ile Lys Leu Tyr Val Arg Arg Val Phe Ile Met Asp Ser Cys Asp Asp
        355                 360                 365

Leu Ile Pro Glu Tyr Leu Asn Phe Ile Arg Gly Val Val Asp Ser Glu
    370                 375                 380

Asp Leu Pro Leu Asn Ile Ser Arg Glu Met Leu Gln Gln Ser Lys Ile
385                 390                 395                 400

Leu Lys Val Ile Arg Lys Asn Ile Val Lys Lys Cys Leu Glu Leu Phe
                405                 410                 415

Ser Glu Leu Ala Glu Asp Lys Glu Asn Tyr Lys Lys Phe Tyr Glu Ala
            420                 425                 430

Phe Ser Lys Asn Leu Lys Leu Gly Ile His Glu Asp Ser Thr Asn Arg
        435                 440                 445

Arg Arg Leu Ser Glu Leu Leu Arg Tyr His Thr Ser Gln Ser Gly Asp
    450                 455                 460

Glu Met Thr Ser Leu Ser Glu Tyr Val Ser Arg Met Lys Glu Thr Gln

465                 470                 475                 480

Lys Ser Ile Tyr Tyr Ile Thr Gly Glu Ser Lys Glu Gln Val Ala Asn
                485                 490                 495

Ser Ala Phe Val Glu Arg Val Arg Lys Arg Gly Phe Glu Val Val Tyr
            500                 505                 510

Met Thr Glu Pro Ile Asp Glu Tyr Cys Val Gln Gln Leu Lys Glu Phe
        515                 520                 525

Asp Gly Lys Ser Leu Val Ser Val Thr Lys Glu Gly Leu Glu Leu Pro
    530                 535                 540

Glu Asp Glu Glu Glu Lys Lys Lys Met Glu Glu Ser Lys Ala Arg Phe
545                 550                 555                 560

Glu Asn Leu Cys Lys Leu Met Lys Glu Ile Leu Asp Lys Lys Val Glu
                565                 570                 575

Lys Val Thr Ile Ser Asn Arg Leu Val Ser Ser Pro Cys Cys Ile Val
            580                 585                 590

Thr Ser Thr Tyr Gly Trp Thr Ala Asn Met Glu Arg Ile Met Lys Ala
        595                 600                 605

Gln Ala Leu Arg Asp Asn Ser Thr Met Gly Tyr Met Met Ala Lys Lys
    610                 615                 620

His Leu Glu Ile Asn Pro Asp His Pro Ile Val Glu Thr Leu Arg Gln
625                 630                 635                 640

Lys Ala Glu Ala Asp Lys Asn Asp Lys Ala Val Lys Asp Leu Val Val
                645                 650                 655

Leu Leu Phe Glu Thr Ala Leu Ser Ser Leu Ala Ser His Phe Arg Arg
            660                 665                 670

Pro Lys Thr His Ser Asn Arg Ile Tyr Arg Met Ile Lys Leu Gly Leu
        675                 680                 685

Gly Ile Asp Glu Asp Glu Val Thr Ala Glu Glu Pro Ser Ala Ala Val
    690                 695                 700

Pro Asp Glu Ile Pro Pro Leu Glu Gly Asp Glu Asp Ala Ser Arg Met
705                 710                 715                 720

Glu Glu Val Asp


<210> 115
<211> 2524
<212> DNA
<213> Rattus norvegicus

<220>
<223> Heat shock protein 90

<400> 115
ggcctaggct tgccgtgcga gtcggacgtg gtccgggccc accctgctct gtactactac 60
tcggctttct cgtcaagatg cctgaggaag tgcaccatgg agaggaagag gtggagacct 120

tcgcctttca ggcagaaatt gcccagctga tgtccctcat catcaacact ttttactcaa 180
acaaagagat tttcctccgg gagttgatct ctaatgcttc tgatgccctg gacaagattc 240
ggtatgagag cctgactgac ccttccaagt tggacagcgg gaaagagctg aagattgaca 300
tcatccccaa ccctcaggaa gccacgctga ctttggtgga cacaggcatt ggcatgacca 360
aggctgacct cataaataac ttgggaacca ttgctaagtc tggtacaaag gcgttcatgg 420
aggctctcca ggctggtgca gacatctcca tgattgggca gtttggtgtc ggcttctact 480
ctgcctacct ggtggcagag aaagtggttg tgatcacgaa gcacaacgat gatgagcagt 540
atgcctggga gtcttctgct ggtggatcct tcactgtccg tgcagaccac ggtgagccca 600
ttggccgggg taccaaagtg atccttcacc tcaaagaaga ccagacagag tacttagagg 660
agaggagggt caaggaggtg gtgaagaaac actcacagtt cataggctac cccatcaccc 720
tctatctgga gaaggaacgc gagaaggaga tcagtgatga tgaggcagag gaagagaaag 780
gggagaagga ggaggaagat aaggaggatg aggagaagcc taagattgag gatgtgggat 840
ctgatgagga ggatgacagc ggcaaagata agaaaaagaa aacaaagaag atcaaggaga 900
agtacattga ccaggaagag ctgaataaga cgaagcccat ctggaccaga aatcctgatg 960
acatcactca agaggaatat ggtgaattct acaagagcct caccaatgac tgggaagacc 1020
acttggcagt caagcacttc tctgtagaag ggcagttgga attcagggca ttgctcttca 1080
ttccccggcg agctcccttt gacctctttg agaacaagaa gaagaagaac aacatcaaat 1140
tgtatgtccg tcgtgtgttc atcatggaca gctgtgatga cctgatacct gagtacctca 1200
acttcatccg tggtgtggtt gattccgagg acctgcctct gaacatctcc cgagaaatgc 1260
tccagcagag caagatcctg aaagtcatcc gcaaaaacat tgtgaagaag tgccttgagc 1320
tcttctctga gttggctgaa gacaaagaga actacaagaa attttatgag gcattctcta 1380
agaatttaaa gcttggaatc catgaggatt ccactaaccg tcgccgcctc tctgagctcc 1440
ttcgctacca tacctctcag tctggagatg agatgacttc cctgtcagag tatgtgtctc 1500
gcatgaagga gacacagaag tccatctact atatcactgg tgagagcaaa gagcaggtgg 1560
ccaactctgc cttcgtggag cgtgtgcgga aacggggctt tgaggtggta tacatgactg 1620
agcccattga tgagtactgc gtacagcagc tcaaggagtt cgatggcaag agcctggtct 1680
ccgtgaccaa ggagggcctg gagctaccag aggatgagga agagaagaaa aaaatggagg 1740
agagcaaggc aaggtttgag aatctctgca agctcatgaa ggagatcttg gacaagaagg 1800
ttgaaaaagt gactatctcc aataggcttg tgtcttcccc ctgctgcatt gtgaccagca 1860
cctacggctg gacagccaac atggaacgga ttatgaaggc ccaggcactg cgggacaact 1920
cgacaatggg ctacatgatg gccaagaaac acctagagat caaccctgac caccccattg 1980
tggagactct gcggcagaag gctgaggcag acaaaaacga caaagctgtc aaagacctgg 2040
tggtgctgct gtttgagact gctctgtctt ctctcgcttc tcactttagg aggcccaaaa 2100
cccactccaa ccgcatctac cgcatgatta aactaggcct gggcattgat gaagatgagg 2160
tcactgcaga agagcccagt gctgctgttc ccgatgagat ccccccactg gagggtgatg 2220
aggatgcctc tcgcatggaa gaagtggatt aaaggctcct gggaagcccc cgccctctgt 2280
atagtatccc tgtggctccc ccaacagcct tgactggccc tgacccacct gggctctctc 2340
tgctcatgtc tacaagaatc tcttctatcc tgtccttgtg ccttaaggca ggaagatccc 2400
ctcccacaga tagcagggtt gggtgttgtg tattgtgttt ttttgtttgt tttattttgt 2460
tctgaaatta aaagtatgca aaataaagat gatgcagttt taaaaaaaaa aaaaaaaaaa 2520
aagg 2524

<210> 116
<211> 541
<212> PRT
<213> Homo sapiens

<220>
<223> Putative ribonuclease III

<400> 116
Met Arg Arg Glu Val Thr Val Glu Leu Ser Ser Gln Gly Phe Trp Lys
1               5                   10                  15
Thr Gly Ile Arg Ser Asp Val Cys Gln His Ala Met Met Leu Pro Val
            20                  25                  30
Leu Thr His His Ile Arg Tyr His Gln Cys Leu Met His Leu Asp Lys
        35                  40                  45
Leu Ile Gly Tyr Thr Phe Gln Asp Arg Cys Leu Leu Gln Leu Ala Met
    50                  55                  60

Thr His Pro Ser His His Leu Asn Phe Gly Met Asn Pro Asp His Ala
65                  70                  75                  80
Arg Asn Ser Leu Ser Asn Cys Gly Ile Arg Gln Pro Lys Tyr Gly Asp
                85                  90                  95
Arg Lys Val His His Met His Met Arg Lys Lys Gly Ile Asn Thr Leu
            100                 105                 110
Ile Asn Ile Met Ser Arg Leu Gly Gln Asp Asp Pro Thr Pro Ser Arg
        115                 120                 125
Ile Asn His Asn Glu Arg Leu Glu Phe Leu Gly Asp Ala Val Val Glu
    130                 135                 140
Phe Leu Thr Ser Val His Leu Tyr Tyr Leu Phe Pro Ser Leu Glu Glu
145                 150                 155                 160
Gly Gly Leu Ala Thr Tyr Arg Thr Ala Ile Val Gln Asn Gln His Leu
                165                 170                 175
Ala Met Leu Ala Lys Lys Leu Glu Leu Asp Arg Phe Met Leu Tyr Ala
            180                 185                 190
His Gly Pro Asp Leu Cys Arg Glu Ser Asp Leu Arg His Ala Met Ala
        195                 200                 205
Asn Cys Phe Glu Ala Leu Ile Gly Ala Val Tyr Leu Glu Gly Ser Leu
    210                 215                 220
Glu Glu Ala Lys Gln Leu Phe Gly Arg Leu Leu Phe Asn Asp Pro Asp
225                 230                 235                 240
Leu Arg Glu Val Trp Leu Asn Tyr Pro Leu His Pro Leu Gln Leu Gln
                245                 250                 255
Glu Pro Asn Thr Asp Arg Gln Leu Ile Glu Thr Ser Pro Val Leu Gln
            260                 265                 270
Lys Leu Thr Glu Phe Glu Glu Ala Ile Gly Val Ile Phe Thr His Val
        275                 280                 285
Arg Leu Leu Ala Arg Ala Phe Thr Leu Arg Thr Val Gly Phe Asn His
    290                 295                 300
Leu Thr Leu Gly His Asn Gln Arg Met Glu Phe Leu Gly Asp Ser Ile
305                 310                 315                 320
Met Gln Leu Val Ala Thr Glu Tyr Leu Phe Ile His Phe Pro Asp His
                325                 330                 335
His Glu Gly His Leu Thr Leu Leu Arg Ser Ser Leu Val Asn Asn Arg
            340                 345                 350
Thr Gln Ala Lys Val Ala Glu Glu Leu Gly Met Gln Glu Tyr Ala Ile
        355                 360                 365
Thr Asn Asp Lys Thr Lys Arg Pro Val Ala Leu Arg Thr Lys Thr Leu
    370                 375                 380
Ala Asp Leu Leu Glu Ser Phe Ile Ala Ala Leu Tyr Ile Asp Lys Asp
385                 390                 395                 400
Leu Glu Tyr Val His Thr Phe Met Asn Val Cys Phe Phe Pro Arg Leu
                405                 410                 415
Lys Glu Phe Ile Leu Asn Gln Asp Trp Asn Asp Pro Lys Ser Gln Leu
            420                 425                 430
Gln Gln Cys Cys Leu Thr Leu Arg Thr Glu Gly Lys Glu Pro Asp Ile
        435                 440                 445
Pro Leu Tyr Lys Thr Leu Gln Thr Val Gly Pro Ser His Ala Arg Thr
    450                 455                 460
Tyr Thr Val Ala Val Tyr Phe Lys Gly Glu Arg Ile Gly Cys Gly Lys
465                 470                 475                 480
Gly Pro Ser Ile Gln Gln Ala Glu Met Gly Ala Ala Met Asp Ala Leu
                485                 490                 495
Glu Lys Tyr Asn Phe Pro Gln Met Ala His Gln Lys Arg Phe Ile Glu
            500                 505                 510
Arg Lys Tyr Arg Gln Glu Leu Lys Glu Met Arg Trp Glu Arg Glu His
        515                 520                 525
Gln Glu Arg Glu Pro Asp Glu Thr Glu Asp Ile Lys Lys
    530                 535                 540

<210> 117
<211> 1626
<212> PNA
<213> Homo sapiens

<220>
<223> Putative ribonuclease III

<400> 117
atgagacgag aagtaacggt ggagctaagt agccaaggat tctggaaaac tggcatccgt 60
tctgatgtct gtcagcatgc aatgatgcta cctgttctga cccatcatat ccgctaccac 120
caatgcctaa tgcatttgga caagttgata ggatatactt tccaagatcg ttgtctgttg 180
cagctggcca tgactcatcc aagtcatcat ttaaattttg gaatgaatcc tgatcatgcc 240
aggaattcat tatctaactg tggaattcgg cagcccaaat acggagacag aaaagttcat 300
cacatgcaca tgcggaagaa agggattaac accttgataa atatcatgtc acgccttggc 360
caagatgacc caactccctc gaggattaac cacaatgaac ggttggaatt cctgggtgat 420
gctgttgttg aatttctgac cagcgtccat ttgtactatt tgtttcctag tctggaagaa 480
ggaggattag caacctatcg gactgccatt gttcagaatc agcaccttgc catgctagca 540
aagaaacttg aactggatcg atttatgctg tatgctcacg ggcctgacct ttgtagagaa 600
tcggaccttc gacatgcaat ggccaattgt tttgaagcgt taataggagc tgtttacttg 660
gagggaagcc tggaggaagc caagcagtta tttggacgct tgctctttaa tgatccggac 720
ctgcgcgaag tctggctcaa ttatcctctc cacccactcc aactacaaga gccaaatact 780
gatcgacaac ttattgaaac ttctccggtt ctacaaaaac ttactgagtt tgaagaagca 840
attggagtaa tttttactca tgttcgactt ctggcaaggg cattcacatt gagaactgtg 900
ggatttaacc atctgaccct aggccacaat cagagaatgg aattcctagg tgactccata 960
atgcaactgg tagccacaga gtacttattc attcatttcc cagatcatca tgaaggacac 1020
ttaactttgt tgcgaagctc tttggtgaat aatagaactc aggccaaggt agcggaggag 1080
ctgggcatgc aggagtacgc cataaccaac gacaagacca agaggcctgt ggcgcttcgc 1140
accaagacct tggcggacct tttggaatca tttattgcag cgctgtacat tgataaggat 1200
ttggaatatg ttcatacttt catgaatgtc tgcttctttc cacgattgaa agagttcatt 1260
ttgaatcagg attggaatga ccccaaatcc cagcttcagc agtgttgctt gacacttagg 1320
acagaaggaa aagagccaga cattcctctg tacaagactc tgcagacagt gggcccatcc 1380
catgcccgaa cctacactgt ggctgtttat ttcaagggag aaagaatagg ctgtgggaaa 1440
ggaccaagta ttcagcaagc ggaaatggga gcagcaatgg atgcgcttga aaaatataat 1500
tttccccaga tggcccatca gaagcggttc atcgaacgga agtacagaca agagttaaaa 1560
gaaatgaggt gggaaagaga gcatcaagag agagagccag atgagactga agacatcaag 1620
aaataa 1626

## Description

### FIELD OF THE INVENTION

The present invention relates to nucleic acids, their expression products and pathways involved in pain, and their use in screening for molecules that can alleviate pain. The invention further relates to methods for the assay and diagnosis of pain in patients.

### BACKGROUND TO THE INVENTION

Pain is currently classified into four general types. Post-operative acute pain can be successfully treated with existing pain medications of e.g. the opioid and non-steroidal anti-inflammatory (NSAID) types, and is usually short-term and self-limiting. A second type of pain, present e.g. in cancer and arthritis, is also responsive to medication initially with a NSAID and in its later stages with opioids. Neuropathic pain arises from damage to the central or peripheral nerve systems, and is more effectively treated with antidepressants or anticonvulsants. A fourth type of pain called central sensitization results from changes in the central nervous system as a result of chronic pain, these changes often being irreversible and difficult to treat. Nerve pain from shingles or diabetes falls into this and the neuropathic category. Changes occur where pain is at first poorly controlled and gradually progress to the point where a person is sensitive to stimuli which would not normally cause pain, for example a light touch. People with pain of this kind often describe a widening of the pain area to include areas which had originally not been injured or which were thought not to be involved in pain. This classification is, however based on clinical symptoms rather than on the underlying pain mechanisms.

Opiates such as morphine belong to a traditional class of pain-relieving compounds that are now recognized as binding to opiate receptors. Naturally occurring polypeptides have also been found to have opiate-like effects on the central nervous system, and these include β-endorphin, met-enkephalin and leu-enkephalin.

Salicin was isolated at the beginning of the 19th century, and from that discovery a number of NSAIDs such as aspirin, paracetamol, ibuprofen, flurbiprofen and naproxen were developed. NSAIDs are by far the most widely used pain-relieving compounds, but can exhibit side effects, in particular irritation of the GI tract that can lead to the formation of ulcers, gastrointestinal bleeding and anemia.

Interest in the neurobiology of pain is developing: see a colloquium sponsored by the US National Academy of Sciences in December 1998 concerning the neurobiology of pain and reviewed in *The Scientist* **13[1],** 12, 1999. Many pain mechanisms were discussed including the role of the capsaicin receptors in pain, (M.J. Caterina *et al*., *Nature,* **389,** 816-824, 1997). Large dosages of capsaicin were reported to disable that receptor, (W.R. Robbins *et al*., *Anesthesia and Analgesia*, **86,** 579-583, 1998). Additionally, a tetrodotoxin-resistant sodium channel found in small diameter pain-sensing neurons (PN3) was discussed (A.N. Akopian *et al*., *Nature,* **379,** 257-262, 1986) and L. Sangameswaran *et al*., *Journal of Biological Chemistry,* **271,** 953-956, 1996). Its involvement in transmission and sensitization to pain signals has been reported, (S.D. Novakovic *et al*., *Journal of Neuroscience,* **18,** 2174-2187, 1998). A further tetrodotoxin-resistant sodium channel has been reported (S. Tate *et al*., *Nature Neuroscience,* **1,** 653-655 1998).

Second messenger systems have also been shown to be important since knockout-mice lacking protein kinase C (PKC) γ were reported to respond to acute pain e.g. from a hot surface, but not to respond to neuropathic pain when their spinal nerves are injured (Malmberg *et al*., *Science,* **278,** 279-283 (1997).

Present methods for identifying novel compounds that relieve pain of one or more of the types indicated above suffer from the defect that they are dependent either on the relatively limited number of receptors known to be involved in pain or on the empirical identification of new receptors which is an uncertain process. In relation to known receptors, for example the opioid receptor, research directed to improved compounds offers the possibility of screenining compounds that have a better therapeutic ratio and fewer side effects. This does not lead naturally to compounds for different pain receptors that have new modes of action and new and qualitatively different benefits. Even when newly identified additional receptors are taken into account, known receptors revolve around tens of gene products. However, there are between 30,000 and 40,000 genes in the genome of an animal and more of them are concerned with nervous system function than with peripheral function. We therefore concluded that a large number of receptors and pathways are important to the transduction of pain, but up to now have remained unknown.

### SUMMARY OF THE INVENTION

It is an object of the invention to provide sequences of genetic material for which no role in pain has previously been disclosed, and which are useful, for example, in:
- identifying metabolic pathways for the transduction of pain
- identifying from said metabolic pathways compounds having utility in the diagnosis or treatment of pain
- producing proteins and polypeptides with a role in the transduction of pain;
- producing genetically modified non-human animals that are useful in the screening of compounds having utility in the treatment or diagnosis of pain.
- Identifying ligand molecules for receptors involved in said metabolic pathways and having utility in the treatment of pain.

It is yet a further object of the invention to provide research tools, for example non-human animals and microorganisms, that can be used in screening compounds for pharmacological activity, especially pain-reducing activity.

The present invention is based on sequences that are down-regulated in two models of chronic pain, namely streptozocin-induced diabetes and chronic constrictive injury (CCI) to a nerve leading to the spine, for example the sciatic nerve.

In one aspect, the invention relates to the use in the screening of compounds that are effective in the treatment of pain, or in the diagnosis of pain, of:
(a) an isolated gene sequence that is down regulated in the spinal cord of a mammal in response to first and second models of pain, for example in response to streptozocin-induced diabetes and in response to a chronic constrictive injury to a nerve leading into the spine;
(b) an isolated gene sequence having at least 80% sequence identity with any of the nucleic acid sequences of Tables I - VI, preferably 85% sequence identity, more preferably 90%, increasingly preferably 95%, most preferably 99%.
(c) an isolated nucleic acid sequence comprising a sequence that is hybridizable to any of the gene sequences according to (a) or (b) under stringent hybridisation conditions;
(d) a recombinant vector comprising any of the gene sequences according to (a) to (c);
(e) a host cell containing the vector according to (d);
(f) a non-human animal, for use in the screening of compounds that are effective in the treatment of pain, or in the diagnosis of pain, having in its genome an introduced gene sequence or a removed or down-regulated nucleotide sequence, said sequence becoming down-regulated in the spinal cord of a mammal in response to first and second models of pain, particularly neuropathic or sensitisation pain, for example in response to streptozocin-induced diabetes and in response to a chronic constrictive injury to a nerve leading into the spine;
(g) an isolated polypeptide containing an amino acid sequence at least 90% identical to an amino acid sequence encoded by a nucleotide sequence according to any one of (a) to (d), or a variant thereof with sequential amino acid deletions from the C terminus and/or the N-terminus; or
(h) an isolated antibody that binds specifically to the isolated polypeptide according to (g).

The invention further provides a compound that is useful in the treatment or diagnosis of pain and that modulates the action of an expression product of a gene sequence that becomes down-regulated in the spinal cord of a mammal in response to first and second models of pain, for example being down-regulated both in response to streptozocin induced diabetes and in response to chronic constrictive injury to a nerve leading into the spine.

The invention also relates to the use of naturally occurring compounds such as peptide ligands of the expression products of the above gene sequences and their associated signal transduction pathways for the treatment of pain.

### DESCRIPTION OF PREFERRED EMBODIMENTS

### DEFINITIONS

Within the context of the present invention:
- "Comprising" means consisting of or including. Thus nucleic acid comprising a defined sequence includes nucleic acid that may contain a full-length gene or full-length cDNA. The gene may include any of the naturally occurring regulatory sequence(s), such as a transcription and translation start site, a promoter, a TATA box in the case of eukaryotes, and transcriptional and translational stop sites. Further, nucleic acid comprising a cDNA or gene may include any appropriate regulatory sequences for the efficient expression thereof *in vitro.*
- "Isolated" requires that the material be removed from its original environment (e.g. the natural environment if it is naturally occurring). For example, a naturally occurring polynucleotide or a peptide present in a living animal is not isolated, but the same polynucleotide or peptide, separated from some or all of the coexisting materials in the natural system, is isolated. Such polynucleotide can be part of a vector and/or such polynucleotide or peptide can be part of a composition, and still be isolated in that the vector or composition is not a part of its natural environment.
- "Mechanistically distinct" in relation to pain models implies that the pain is induced by mechanisms that differ in kind rather than being variants of a similar pain model. Thus diabetic pain and chronic constrictive pain models are mechanistically distinct, whereas spinal nerve ligation models and sciatic nerve ligation models which both work by ligation are not.
- "Purified" does not require absolute purity; instead it is intended as a relative definition. Purification of starting materials or natural materials from their native environment to at least one order of magnitude, preferably two or three orders, and more preferably four or five orders of magnitude is expressly contemplated.
- "Nucleic acid sequence" or "gene sequence" means a sequence of nucleotides or any variant or homologue thereof, or truncated or extended sequence thereof, and is preferably indicated by a Genebank accession number. Also within the scope of the present invention are down-regulated nucleic acid sequences which encode expression products which are components of signaling pathways. This invention also includes any variant or homologue or truncated or extended sequence of the down-regulated nucleotide sequence. Also within the scope of the present invention, the term "nucleic acid(s) product", or "expression product" or "gene product" or a combination of these terms refers without being biased, to any, protein(s), polypeptide(s), peptide(s) or fragment(s) encoded by the down-regulated nucleotide sequence.
- "Operably linked" refers to a linkage of polynucleotide elements in a functional relationship. For instance, a promoter or an enhancer is operably linked to a coding sequence if it regulates the transcription of the coding sequence. In particular, two DNA molecules (such as a polynucleotide containing a promoter region, and a polynucleotide encoding a desired polypeptide) are said to be "operably linked" if the nature of the linkage between the two polynucleotides does not (1) result in the introduction of a frame-shift mutation and (2) interfere with the ability of the polynucleotide containing the promoter to direct the transcription of the coding polynucleotide.
   "Gene product" refers to polypeptide - which is interchangeable with the term protein - which is encoded by a nucleotide sequence and includes single-chain polypeptide molecules as well as multiple-polypeptide complexes where individual constituent polypeptides are linked by covalent or non-covalent means. Polypeptides of the present invention may be produced by synthetic means (e.g. as described by Geysen *et al*., 1996) or by recombinant means.
   The terms "variant", "homologue", "fragment", "analogue" or "derivative" in relation to the amino acid sequence for the polypeptide of the present invention includes any substitution of, variation of, modification of, replacement of, deletion of or addition of one (or more) amino acid from or to the sequence providing the resultant polypeptide has the native gene product activity. In particular, the term "homologue" covers homology with respect to structure and/or function. With respect to sequence homology, there is at least 90%, more preferably at least 95% homology to an amino acid sequence encoded by the relevant nucleotide sequence shown in Tables I - VI, preferably there is at least 98% homology.
   Typically, for the variant, homologue or fragment of the present invention, the types of amino acid substitutions that could be made should maintain the hydrophobicity/hydrophilicity of the amino acid sequence. Amino acid substitutions may include the use of non-naturally occurring amino acid analogues.
   In addition, or in the alternative, the protein itself could be produced using chemical methods to synthesize a polypeptide, in whole or in part. For example, peptides can be synthesized by solid phase techniques, cleaved from the resin, and purified by preparative high performance liquid chromatography (e.g. Creighton (1983) Proteins Structures and Molecular Principles, WH Freeman and Co., New York, NY, USA). The composition of the synthetic peptides may be confirmed by amino acid analysis or sequencing (e.g. the Edman degradation procedure).
   Direct peptide synthesis can be performed using various solid-phase techniques (Roberge JY *et al* Science Vol 269 1995 202-204) and automated synthesis may be achieved, for example, using the ABI 431 A Peptide Synthesizer (Perkin Elmer) in accordance with the instructions provided by the manufacturer. Additionally, the amino acid sequence of a gene product, or any part thereof, may be altered during direct synthesis and/or combined using chemical methods with a sequence from other subunits, or any part thereof, to produce a variant polypeptide.
   In another embodiment of the invention, a gene product natural, modified or recombinant amino acid sequence may be ligated to a heterologous sequence to encode a fusion protein. For example, for screening of libraries for compounds and peptide agonists and antagonists of gene product activity, it may be useful to encode a chimeric gene product expressing a heterologous epitope that is recognised by a commercially available antibody. A fusion protein may also be engineered to contain a cleavage site located between a gene product sequence and the heterologous protein sequence, so that the gene product may be cleaved and purified away from the heterologous moiety.
   The gene product may also be expressed as a recombinant protein with one or more additional polypeptide domains added to facilitate protein purification. Such purification facilitating domains include, but are not limited to, metal chelating peptides such as histidine-tryptophan modules that allow purification on immobilised metals (Porath J, Protein Expr Purif Vol 3 1992 p263-281), protein A domains that allow purification on immobilised immunoglobulin, and the domain utilised in the FLAGS extension/affinity purification system (Immunex Corp, Seattle, WA, USA). The inclusion of a cleavable linker sequence such as Factor XA or enterokinase (Invitrogen, San Diego, CA, USA) between the purification domain and the gene product is useful to facilitate purification.
- "Pain" includes chronic pain, neuropathic pain, pain arising from central sensitisation, and in particular diabetic pain.
- "Stringent hybridization conditions" is a recognized term in the art and for a given nucleic acid sequence refers to those conditions which permit hybridization of that sequence to its complementary sequence and closely homologous sequences. Conditions of high stringency may be illustrated in relation to filter-bound DNA as for example 2X SSC, 65°C (where SSC = 0.15M sodium chloride, 0.015M sodium citrate, pH 7.2), or as 0.5M NaHPO₄, 7% sodium dodecyl sulfate (SDS), 1mM EDTA, at 65°C, and washing in 0.1xSCC/0.1% SDS at 68°C (Ausubel F.M. *et al*., eds, 1989, *Current Protocols in Molecular Biology,* Vol. I, Green Publishing Associates, Inc., and John Wiley & Sons Inc., New York, at p. 2. 10.3). Hybridization conditions can be rendered highly stringent by raising the temperature and/or by the addition of increasing amounts of formamide, to destabilize the hybrid duplex of non-homologous nucleic acid sequence relative to homologous and closely homologous nucleic acid sequences. Thus, particular hybridisation conditions can be readily manipulated, and will generally be chosen depending on the desired results.
- "Variants or homologues" include (a) sequence variations of naturally existing gene(s) resulting from polymorphism(s), mutation(s), or other alteration(s) as compared to the above identified sequences, and which do not deprive the encoded protein of function (b) recombinant DNA molecules, such as cDNA molecules encoding genes indicated by the relevant Genebank accession numbers and (c) any sequence that hybridizes with the above nucleic acids under stringent conditions and encodes a functional protein or fragment thereof.

### IDENTIFIED SEQUENCES

The inventors have identified nucleotide sequences that give rise to expression products listed in Tables I - VI, that become differentially expressed in the spinal cord in response to two distinct chronic pain stimuli, for example neuropathic and/or central sensitization pain stimuli, and that are believed to be involved in the transduction of pain. In Tables I - VI, * denotes more preferred nucleic acid sequences and ** denotes most preferred nucleic acid sequences. These nucleic acid sequences have not previously been implicated in the transduction of pain.

The validity of the present experimental procedure was confirmed by the fact that nucleotide sequences were obtained as a result of the investigation whose function in the transduction of pain has been previously confirmed and established. These nucleic acid sequences are not part of this invention. Any of the nucleic acid sequences and expression products can be used to develop screening technologies for the identification of novel molecules for the prevention or treatment of pain. These screening technologies could also be used to ascribe new pain therapeutic indications to molecules that have not previously been identified as being useful for the prevention or treatment of pain. Furthermore, the said nucleic acid sequences can be used as diagnostic tools and for the development of diagnostic tools.

**Table I -**

| **Sequences whose expression products are kinases** | | | | | |
|---|---|---|---|---|---|
| **Expression product or name (Reference)** | **Rat Accession Number** | **Mouse accession number** | **Human Accession Number** | **Reaction** | **Assay** |
| Pyruvate kinase, M1 and M2 subunit s (Refs :1 - 2) | M24359 (SEQ ID No's 1-3) | X97047 | X56494 | Tissue-specific promoter; Carbohydrate kinase | Kinase |
| | | | | | |

**Table II -**

| **Sequences whose expression products are receptors** | | | | | |
|---|---|---|---|---|---|
| **Expression Product or Name (Reference)** | **Rat Accession Number** | **Mouse accession Number** | **Human Accession Number** | **Reaction** | **Assay** |
| Dopamine receptor D.sub.1** (Ref : 3) | I58000 (SEQ ID NO 4) | | | Dopamine receptor | Receptor |
| Putative GABA-B1a receptor** (Ref : 4) | | AF114168 (SEQ ID No's 5-6) | | GABA-B Receptor | Receptor |

**Table III -**

| **Sequences whose expression products are transporters** | | | | | |
|---|---|---|---|---|---|
| **Expression Product or Name** | **Rat Accession Number** | **Mouse Accession Number** | **Human Accession Number** | **Reaction** | **Assay** |
| Differentiation-associated Na-dependent inorganic phosphate cotransporter (Ref : N/A) | AF271235 (SEQ ID NO's 7-8) | | | Trans-membrane phosphate transport | Transporter |
| Putative vacuolar assembly protein VSP41 gene (Ref : N/A) | U87309 | | AAM47563. 1 (SEQ ID No 9) | Vacuolar assembly and traffic | Transporter |

**Table IV -**

| **Sequences whose expression products are G-protein coupled receptor proteins** | | | | | |
|---|---|---|---|---|---|
| **Expression product or Name** | **Rat Accession Number** | **Mouse Accession Number** | **Human Accession Number** | **Function** | **Assay** |
| Git1 (G-protein-coupled receptor kinase-interactor 1 ; GPCR kinase-associated ADP-ribosylation factor) (Ref : 5) | AF085693 (SEQ ID NO 10-11) | | | Regulation of activity of ARF6 in phosphatidyl-inisitol 3-kinase signalling pathways ; β2 adrenergic receptor regulation | Ligand binding |

**Table V -**

| **Sequences whose expression products are DNA-binding proteins** | | | | | |
|---|---|---|---|---|---|
| **Expression Product or Name** | **Rat Accession Number** | **Mouse Accession Number** | **Human Accession Number** | **Reaction** | **Assay** |
| Putative histone H3.3A (Ref: 6) | | X91866 | M11354(SEQ ID NO 12-13) | DNA binding | DNA binding |
| | | | | | |

**Table VI -**

| **Sequences whose expression products are other enzymes** | | | | | |
|---|---|---|---|---|---|
| **Expression product or Name** | **Rat Accession Number** | **Mouse Accession Number** | **Human Accession Number** | **Reaction** | **Assay** |
| 3-Hydroxy 3-methylglutaryl coenzyme A synthase, cytosolic * (Ref: 7) | X52625 (SEQ ID No's 14-15) | | | Cholesterol biosynthesis | Ligase |
| Acyl-CoA synthetase II, brain (Ref : N/A) | D360666 (N/A) | | | Fatty acid metabolism | Ligase |
| Farnesyl diphosphate synthase ** (Ref : 8) | M34477 (Seq ID No's 16-17) | | | Isoprene biosynthesis | Ligase |
| Bendless protein (Ref : N /A) | AB032739 (Seq ID No's 18-19) | | E12457 | Protein degradation | Ligase |
| fatty acid synthase (Ref: 9) | X62888 (SEQ ID No's 20-21) | | | Fatty acid synthesis | Ligase |
| Glutamine synthetase (EC | M91652 (SEQ ID | | | Amino acid metabolism | Ligase |
| 6.3.1.2) ** (Ref : 10) | NO's 22-23) | | | | |
| Putative seryl-tRNA synthetase (Ref No : 11) | | | X91257 (SEQ ID NO's 24-25) | Ligase | Ligase |
| Enolase, alpha alpha, non-neuronal (NNE) (REF NO 12) | X02610 | X52379 | M14328 (SEQ ID NO's 26-27) | Glycolysis | Lyase |
| Aldose reductase, lens (AREC 11.1.21) (REF : 13) | X05884 (SEQ ID NO's 28-29) | | | Reduces carbonyl | Oxidoreductase |
| Cytochrome-c oxidase I, mitochondrial (Ref : 14) | S79304 (SEQ ID NO's 30-31) | | | Mitochrondrial energy metabloism | Oxidoreductase |
| Lactate dehydrogenase-B (LDH-B) (Ref : 15) | U07181 (SEQ ID NO's 32-33) | X51905 | Y00711 | Glycolysis | Oxidoreductase |
| Putative cytochrome c oxidase VIB (EC 1.9.3.1) (Ref : 16) | | | X13923 (SEQ ID NO's 34-35) | Mitochrondrial energy metabolism | Oxidoreductase |
| Putative NADH: ubiquinone oxidoreductasePG IV subunit (Ref : 17) | | | AF044953 (SEQ ID No's 36-37) | Mitochrondrial energy metabolism | Oxidoreductase |
| Putative succinate dehydrogenase flavoprotein (Ref: N/A) | | AF095938 (SEQ ID No's 38-39) | AF171022 | TCA cycle | Oxidoreductase |
| Putative ubiquinol-cytochrome-c reductase (EC 1.10.2.2) core protein II (REF: 18) | | | J04973 (SEQ ID No's 40-41) | Mitochrondrial energy metabolism | Oxidoreductase |
| Stearoyl-coA desaturase 2 * (Ref: N/A) | AB032243 (SEQ ID N0's 42-43) | M26270 | | Fatty acid biosynthesis | Oxidoreductase |
| Ribophorin I * (Ref: 19) | X05300 (SEQ ID No's 44-45) | | | Glycosolation | Transferase |
| Sulfotransferase-like protein (REF : 20) | AF188699 (SEQ ID No's 46-47) | | | Transferase | Transferase |
| ATP synthase, H+, alpha subunit, mitochondrial (EC 3.6.1.34) (Ref: N/A) | X56133 (SEQ ID No's 48-49) | | | Mitochrondrial energy metabolism | Hydrolase |
| F1F0 ATPase | U00926 | | | Mitochrondrial | Hydrolase |
| delta subunit (REF : 21) | (SEQ ID No's 50-51) | | | energy metabolism | |
| Putative dihydropyrimidin ase related protein * (REF: 22) | | | D78013 (SEQ ID NO's 52-53) | Pyrimidine degradation | Hydrolase |
| Heat shock protein 90 (REF : 23) | S45392 (SEQ ID NO's 114-115) | M18186 | M16660 | Cell protection | Hydrolase |
| Myelin basic protein S (MBP S) (REF :24) | K00512 SEQ ID No 54) | | | Myelin structural protein | Ligand binding |
| Transferrin (Ref 25) | D38380 (SEQ ID No's 55-56) | | | Iron transport | Ligand binding |
| Neurofilament, light molecular weight (NF-L) (Ref : 26) | AF031880 (SEQ ID No's 57-58) | | | Cytoskeleton | Ligand binding |
| Myelin-associayted glycoprotein (MAG) (Ref : 27) | M1680 (SEQ ID NO's 59-60) | M31811 | | Cell adhesion molecule for postnatal neural development | Ligand binding |
| NF-M middle molecular weight neurofilament protein (Ref : 28) | M18628 (SEQ ID No's 61-62) | | | Cytoskeleton | Ligand binding |
| Neuro-degeneration associated-protein 1 (Ref: 29) | D32249 (SEQ ID No's 63-64) | | | Protein sorting; synaptic communication and plasticity | Ligand binding |
| S-100 protein β-subunit (REF: 30) | X01090 (SEQ ID No 65) | | | Zinc and calcium binding | Ligand binding |
| Microtubule-associated protein 1b (Map 1b) (Ref: 31) | X60370 (SEQ ID No's 66-67) | | L06237 | Cytoskeleton protein, neuronal growth/ regeneration; microtubule binding protein | Ligand binding |
| Putative cdc 37 homolog (Ref: 32) | D26564 (Seq ID No's 68-69) | | | Cell signalling ; cell cycle protein | Ligand binding |
| Putative ras-related protein Rab-5c (Ref : 33) | | | U11293 (SEQ ID No's 70-71) | Small GTP binding protein | Ligand binding |
| Putative gelsolin (Ref: 34) | | J04953 (SEQ ID No's 72-73) | | Cytoskeleton protein | Ligand binding |
| Cd81 antigen (target of antiproliferative antibody 1) (Ref : 35) | U19894 (SEQ ID No's 74-75) | X59047 | M33680 | Regulator for neuron-induced astrocyte differentiation; microglial | Ligand binding |
| | | | | effector functions | |
| Mobp81 (Myelin-associated/Oligo-dendrocytic basic protein 81) (Ref : 36) | X87900 (SEQ ID No's 76-77) | | | Myelin compaction | Ligand binding |
| Syntaxin binding protein n-sec1, sec1 homolog (Ref : N/A) | | | BC002869 (Seq ID No's 78-79) | Neuro-transmission | Ligand binding |
| Alpha-internexin (Ref: 37) | M73049 (SEQ ID No's 80-81) | | | Cytoskeleton protein; neuronal intermediate filament that can self-assemble | Ligand binding |
| Putative β-sarcoglycan A3b (Ref: N/a) | | AB024921 (SEQ ID No's 82-83) | | Cytoskeleton protein | Ligand binding |
| CGI-78 protein Ref: 38) | AF151835 | | AF151835 (SEQ ID No's 84-85) | Unknown | Ligand binding |
| KIAA0143 (Ref: 39) | | | D63477 (SEQ ID NO's 86-87) | Transmembrane protein | Ligand binding |
| Septin 2 (KIAA0128 ; Ref 39) | | | D50918 (SEQ ID No's 88-89) | GTPase; cytokinesis | Ligand binding |
| Nucleobindin (Ref: 40) | Z36277 (SEQ ID No's 90-91) | | | Unknown | Ligand binding |
| Myelin protein SR13 (Ref: 41) | M69139 (SEQ ID NO's 92-93) | S55427 | | Myelin structural protein | Ligand binding |
| B-Actin, cytoplasmic (Ref: 42) | V01217 (SEQ ID NO's 94-95) | X03672 | | Structural protein | Ligand binding |
| Ly6/neurotoxin (Lynx1) homolog (Ref: 43) | | AF141377 (SEQ ID No's 96-97) | | Neuro-transmitter modulator | Ligand binding |
| Astrocytic phosphoprotein; PFA 15 gene (Ref: N/A) | AJ243949 (SEQ ID NO's 98-99) | X86694 | | PKC substrate | Ligand binding |
| PLIC-1 (Ref: N/A) | | AF177345 (SEQ ID NO 100-101) | | Cytoskeleton interaction | Ligand binding |
| Nfx1 (tip associating protein (TAP) gene) (Ref: N/A) | AF093139 (SEQ ID NO's 102-103) | AF093140 | | Unknown | Logand binding |
| Alpha-Crystallin B (Ref N/A) | U04320 SEQ ID NO's 104- | M73741 | M28638 | Stress response ; heat shock element | Ligand binding |
| | 105) | | | | |
| Heat shock-like protein 70 kD (Ref : 44) | X70065 (SEQ ID No's 106-107) | U73744 | Y00371 | Stress response | Ligand binding |
| Tau microtubule-associated protein (Ref : 45) | X79321 (SEQ ID NO's 108-109) | | | Microtubule associated protein expressed in neurons | Ligand binding |
| Myelin, Schwann cell, Peripheral (P-0) (Ref : 46) | K03242 (SEQ ID No's 110-111) | | | Myelin structural protein | Ligand binding |
| B-Tubulin class 1 (Ref: 47) | AB011679 (SEQ ID NO's 112-113) | X04663 | AF14139 | Structural protein | Ligand binding |
| Putative ribonuclease III (Ref : N/A) | | | AF116910 (SEQ ID NO's 116-117) | RNA hydrolysis | Hydrolase |

### PRODUCTION OF POLYPEPTIDES AND NUCLEIC ACIDS

### Vectors

Recombinant expression vectors comprising a nucleic acid can be employed to express any of the nucleic acid sequences of the invention. The expression products derived from such vector constructs can be used to develop screening technologies for the identification of molecules that can be used to prevent or treat pain, and in the development of diagnostic tools for the identification and characterization of pain. The expression vectors may also be used for constructing transgenic non-human animals.

Gene expression requires that appropriate signals be provided in the vectors, said signals including various regulatory elements such as enhancers/promoters from viral and/or mammalian sources that drive expression of the genes or nucleic acid sequences of interest in host cells. The regulatory sequences of the expression vectors used in the invention are operably linked to the nucleic acid sequence encoding the pain-associated protein of interest or a peptide fragment thereof.

Generally, recombinant expression vectors include origins of replication, selectable markers, and a promoter derived from a highly expressed gene to direct transcription of a downstream nucleoide sequence. The heterologous nucleotide sequence is assembled in an appropriate frame with the translation, initiation and termination sequences, and if applicable a leader sequence to direct the expression product into the periplasmic space, the extra-cellular medium or cell membrane.

In a specific embodiment wherein the vector is adapted for expressing desired sequences in mammalian host cells, preferred vectors will comprise an origin of replication from the desired host, a suitable promoter and an enhancer, and also any necessary ribosome binding sites, polyadenylation site, transcriptional termination sequences, and optionally 5'-flanking non-transcribed sequences. DNA sequences derived from the SV40 or CMV viral genomes, for example SV40 or CMV origin, early promoters, enhancers, and polyadenylation sites may be used to provide the required non-transcribed genetic elements.

A recombinant expression vector used in the invention advantageously also comprises an untranscribed polynucleotide region located at the 3'end of the coding sequence (ORF), this 3'-UTR (untranslated region) polynucleotide being useful for stabilizing the corresponding mRNA or for increasing the expression rate of the vector insert if this 3'-UTR harbours regulation signal elements such as enhancer sequences.

Suitable promoter regions used in the expression vectors are chosen taking into account the host cell in which the nucleic acid sequence is to be expressed. A suitable promoter may be heterologous with respect to the nucleic acid sequence for which it controls the expression, or alternatively can be endogenous to the native polynucleotide containing the coding sequence to be expressed. Additionally, the promoter is generally heterologous with respect to the recombinant vector sequences within which the construct promoter/coding sequence has been inserted. Preferred promoters are the LacI, LacZ, T3 or T7 bacteriophage RNA polymerase promoters, the lambda PR, PL and Trp promoters (see EP-0 036 776), the polyhedrin promoter, or the p10 protein promoter from *baculovirus* (kit Novagen; Smith *et al*., (1983); O'Reilly *et al*. (1992)).

Preferred selectable marker genes contained in the expression recombinant vectors used in the invention for selection of transformed host cells are preferably dehydrofolate reductase or neomycin resistance for eukaryotic cell culture, TRP1 for *S. cerevisiae* or tetracycline, rifampicin or ampicillin resistance in *E. coli,* or Levamsaccharase for *Mycobacteria,* this latter marker being a negative selection marker.

Preferred bacterial vectors are listed hereafter as illustrative but not limitative examples: pQE70, pQE60, pQE-9 (Quiagen), pD10, phagescript, psiX174, p.Bluescript SK, pNH8A, pNH16A, pNH18A, pNH46A (Stratagene); pKK223-3, pKK233-3, pDR540, pRIT5 (Pharmacia); pWLNEO, pSV2CAT, pOG44, pXT1, pSG (Stratagene); pSVK3, pBPV, pMSG, pSVL (Pharmacia); pQE-30 (QIA express).

Preferred bacteriophage recombinant vectors of the invention are P1 bacteriophage vectors such as described by Sternberg N.L. (1992;1994).

A suitable vector for the expression of any of the pain associated polypeptides used in the invention or fragments thereof, is a baculovirus vector that can be propagated in insect cells and in insect cell-lines. A specific suitable host vector system is the pVL 1392/1393 *baculovirus* transfer vector (Pharmingen) that is used to transfect the SF9 cell line (ATCC N°CRL 1711) that is derived from *Spodoptera frugiperda.*

The recombinant expression vectors of in the invention may also be derived from an adenovirus. Suitable adenoviruses are described by Feldman and Steig (1996) or Ohno *et al.* (1994). Another preferred recombinant adenovirus is the human adenovirus type two or five (Ad 2 or Ad 5) or an adenovirus of animal origin (Patent Application WO 94/26914)

Particularly preferred retrovirus for the preparation or construction of retroviral *in vitro* or *in vivo* gene delivery vehicles include retroviruses selected from the group consisting of Mink-Cell Focus Inducing Virus, Murine Sarcoma Virus, and Ross Sarcoma Virus. Other preferred retroviral vectors are those described in Roth *et al*. (1996), in PCT Application WO 93/25234, in PCT Application WO 94/06920, and also in Roux *et al*. (1989), Julan *et al*.(1992) and Nada *et al.* (1991).

Yet, another viral vector system that is contemplated is the Adeno Associated Viruses (AAV) such as those described by Flotte *et al.* (1992), Samulski *et al.* (1989) and McLaughlin *et al.* (1996).

### Host cells expressing pain associated polypeptides

Host cells that endogenously express pain associated polypeptides or have been transformed or transfected with one of the nucleic acid ssequences described herein, or with one of the recombinant vector described above, particularly a recombinant expression vector, can be used in the present invention. Also included are host cells that are transformed (prokaryotic cells) or are transfected (eukaryotic cells) with a recombinant vector such as one of those described above.

Preferred host cells used as recipients for the expression vectors used in the invention are the following:
(a) prokaryotic host cells: *Escherichia coli,* strains. (i.e. DH5-α, strain) *Bacillus subtilis, Salmonella typhimurium* and strains from species like *Pseudomonas, Streptomyces* and *Staphylococcus* for the expression of up and down-regulated nucleic acid sequence modulated by pain, characterized by having at least 80% sequence identity with any of the nucleic acid sequences of Tables I - VI. Plasmid propagation in these host cells can provide plasmids for transfecting other cells.
(b) eukaryotic host cells: HeLa cells (ATCC N°CCL2; N°CCL2.1; N°CCL2.2), Cv 1 cells (ATCC N°CCL70), COS cells (ATCC N°CRL 1650; N°CRL 1651), Sf-9 cells (ATCC N°CRL 1711), C127 cells (ATCC N°CRL-1804), 3T3 cells (ATCC N°CRL-6361), CHO cells (ATCC N°CCL-61), human kidney 293 cells (ATCC N° 45504; N°CRL-1573), BHK (ECACC N°84100 501; N°84111301), PC12 (ATCC N° CRL-1721), NT2, SHSYSY (ATCC N° CRL-2266), NG108 (ECACC N°88112302) and F11, SK-N-SH (ATCC N° CRL-HTB-11), SK-N-BE(2) (ATCC N° CRL-2271), IMR-32 (ATCC N° CCL-127). A preferred system to which the nucleic acids of the invention can be expressed are neuronal cell lines such as PC12, NT2, SHSY5Y, NG108 and F11, SK-N-SH, SK-N-BE(2) , IMR-32 cell lines, COS cells, 3T3 cells, HeLa cells, 292 cells and CHO cells. The above cell lines could be used for the expression of any of the nucleic acid sequences of Tables I - VI.

When a nucleic acid sequence of any of Tables I - X is expressed using a neuronal cell line, the sequence can be expressed through an endogenous promoter or native neuronal promoter, or an exogenous promoter. Suitable exogenous promoters include SV40 and CMV and eukaryotic promoters such as the tetracycline promoter. The preferred promoter when pain associated molecules are endogenously expressed is an endogenous promoter. A preferred promoter in a recombinant cell line is the CMV promoter.

In a specific embodiment of the host cells described above, these host cells have also been transfected or transformed with a polynucleotide or a recombinant vector for the expression of a natural ligand of any of the nucleic acid sequences of any of Tables I - VI or a modulator of these expression products.

### PROTEINS, POLYPEPTIDES AND FRAGMENTS

The expression products of the nucleic acid sequences of Tables I - VI or fragment(s) thereof can be prepared using recombinant technology, from cell lines or by chemical synthesis. Recombinant methods, chemical methods or chemical synthetic methods can be used to modify a gene in order to introduce into the gene product, or a fragment of the gene product, features such as recognition tags, cleavage sites or other modifications. For efficient polypeptide production, the endogenous expression system or recombinant expression system should allow the expression products to be expressed in a manner that will allow the production of a functional protein or fragment thereof which can be purified. Preferred cell lines are those that allow high levels of expression of polypeptide or fragments thereof. Such cell lines include cell lines which naturally express the nucleic acid sequence of Tables I - VI or common mammalian cell lines such as CHO cells or COS cells, etc, or more specific neuronal cell lines such as PC12. However, other cell types that are commonly used for recombinant protein production such as insect cells, amphibian cells such as oocytes, yeast and prokaryotic cell lines such as *E. coli* can also be used.

The expression products of Tables I - VI or fragments thereof can be utilized in screens to identify potential therapeutic ligands, either as a purified protein, as a protein chimera such as those produced in phage display, as a cell membrane (lipid or detergent) preparation, or in intact cells.

The invention also relates generally to the use of proteins, peptides and peptide fragments for the development of screening technologies for the identification of molecules for the prevention or treatment of pain, and the development of diagnostic tools for the identification and characterization of pain. These peptides include expression products of the nucleic acid sequences of Tables I - VI and purified or isolated polypeptides or fragments thereof having at least 90%, preferably 95%, more preferably 98% and most preferably 99% sequence identity with the any of the expression products of nucleic acid sequences of Tables I - VI. Expressed peptides and fragments of any of these nucleic acid sequences can be used to develop screening technologies for the identification of novel molecules for the prevention or treatment of pain. These screening technologies could also be used to ascribe new pain therapeutic indications to molecules, which have not previously been ascribed for the prevention or treatment of pain. Furthermore the said expressed peptides and fragments can be used as diagnostic tools and for the development of diagnostic tools.

### SCREENING METHODS

As discussed above, we have identified nucleic acid sequences whose expression is regulated by pain, particularly chronic pain and more particularly diabetic pain. The expression products of these nucleic acids can be used for screening ligand molecules for their ability to prevent or treat pain, and particularly, but not exclusively, chronic pain. The main types of screens that can be used are described below. The test compound can be a peptide, protein or chemical entity, either alone or in combination(s), or in a mixture with any substance. The test compound may even represent a library of compounds.

The expression products of any of the nucleic acid sequences of Tables I - VI or fragments thereof can be utilized in a ligand binding screen format, a functional screen format or invivo format. Examples of screening formats are provided.

### A) Ligand binding screen

In ligand binding screening a test compound with is contacted with an expression product of one of the sequences of Tables I - VI, and the ability of said test compound to interact with said expression product is determined, e.g. the ability of the test compound(s) to bind to the expression product is determined. The expression product can be a part of an intact cell, lipidic preparation or a purified polypeptide(s), optionally attached to a support, such as beads, a column or a plate etc.

Binding of the test compound is preferably performed in the presence of a ligand to allow an assessment of the binding activity of each test compound. The ligand may be contacted with the expression product either before, simultaneously or after the test compound. The ligand should be detectable and/or quantifiable. To achieve this, the ligand can be labelled in a number of ways, for example with a chromophore, radioactive, fluorescent, phosphorescent, enzymatic or antibody label. Methods of labelling are known to those in the art. If the ligand is not directly detectable it should be amenable to detection and quantification by secondary detection, which may employ the above technologies. Alternatively the expression product or fragment thereof can be detectable or quantifiable. This can be achieved in a similar manner to that described above.

Binding of the test compound modifies the interaction of the ligand with its binding site and changes the affinity or binding of the ligand for/to its binding site. The difference between the observed amount of ligand bound relative to the theoretical maximum amount of ligand bound (or to the ligand bound in the absence of a test compound under the same conditions) is a reflection of the binding ability (and optionally the amount and/or affinity) of a test compound to bind the expression product.

Alternatively, the amount of test compound bound to the expression product can be determined by a combination of chromatography and spectroscopy. This can be achieved with technologies such as Biacore (Amersham Pharmacia). The amount of test compound bound to the expression product can also be determined by direct measurement of the change in mass upon compound or ligand binding to the expression product. Alternatively, the expression product, compound or ligand can be fluorescently labelled and the association of expression product with the test compound can be followed by changes in Fluorescence Energy Transfer (FRET).

The invention therefore includes a method of screening for pain alleviating compounds, comprising:
a) contacting a test compound or test compounds in the presence of a ligand with an expression product of any of the nucleic acid sequences of Tables I - VI or with a cell expressing at least one copy of the expression product or with a lipidic matrix containing at least one copy of the expression product;
b) determining the binding of the test compound to the expression product, and
c) selecting test compounds on the basis of their binding abilities.

In the above method, the ligand may be added prior to, simultaneously with or after contact of the test compound with the expression product. Non limiting examples and methodology can be gained from the teachings of the *Molecular Probes handbook* and references therein (Molecular Probes, Inc., 4849 Pitchford Ave, Eugene, USA), *Methods in neurotransmitter receptor analysis* (Yamamura HI., Enna, SJ., and Kuhar, MJ., Raven Press New York, the *Glaxo Pocket Guide to Pharmacology*, Dr. Michael Sheehan, Glaxo Group Research Ltd, Ware, Herts SG12 0DP), *Bylund DB and Murrin LC* (2000, Life Sciences, 67 (24) 2897-911), *Owicki JC* (*2000, J. biomol Screen (5) 297-306), Alberts et al (1994, Molecular Biology of the Cell, 3*^{*rd*} *Edn, Garland Publication Inc), Butler JE, (2000 Methods 22(1):4-23, Sanberg SA* (2000, Curr Opin Biotechnol 11(1) 47-53), and *Christopoulos A* (1999, biochem Pharmacol 58(5) 735-48).

### B) Functional screening

### (a) Kinase assays

The expression product of any of the nucleic acid sequences of Tables I - VI which encode a kinase, and in particular the nucleic acid sequence listed in Table I, is amenable to screening using kinase assay technology.

Kinases have the ability to add phosphate molecules to specific residues in ligands such as binding peptides in the presence of a substrate such as adenosine triphosphate (ATP). Formation of a complex between the kinase, the ligand and substrate results in the transfer of a phosphate group from the substrate to the ligand. Compounds that modulate the activity of the kinase can be determined with a kinase functional screen. Functional screening for modulators of kinase activity therefore involves contacting one or more a test compounds with an expression product of one of the nucleic acid sequences of Tables I - VI which encodes a kinase, and determining the ability of said test compound to modulate the transfer of a phosphate group from the substrate to the ligand.

The expression product can be part of an intact cell or of a lipidic preparation or it can be a purified polypeptide(s), optionally attached to a support, for example beads, a column, or a plate. Binding is preferably performed in the presence of ligand and substrate to allow an assessment of the binding activity of each test compound.

The ligand should contain a specific kinase recognition sequence and it should not be phosphorylated at its phosphoryation site. The ligand and/or substrate may be contacted with the kinase either before, simultaneously or after the test compound. Optionally the substrate may be labelled with a kinase transferable labelled phosphate. The assay being monitored by the phosphorylation state of the substrate and/or the ligand. The ligand should be such that its phosphorylation state can be determined. An alternative method to do this is to label the ligand with a phosphorylation-state-sensitive molecule. To achieve this, the ligand can be labelled in a number of ways, for example with a chromophore, radioactive, fluorescent, phosphorescent, enzymatic or antibody label. If the ligand is not directly detectable it should be amenable to detection and quantification by secondary detection, which may employ the above technologies. Such technologies are known to those in the art.

Binding of the test compound to the kinase modifies its ability to transfer a phosphate group from the substrate to the ligand. The difference between the observed amount of phosphate transfer relative to the theoretical maximum amount of phosphate transfer is a reflection of the modulatory effect of the test compound. Alternatively, the degree of phosphate transfer can be determined by a combination of chromatography and spectroscopy. The extent of phosphorylation of the ligand peptide or dephosphorylation of the substrate can also be determined by direct measurement. This can be achieved with technologies such as Biacore (Amersham Pharmacia).

The invention also provides a method for screening compounds for the ability to relieve pain, which comprises:
(a) contacting one or more test compounds in the presence of ligand and substrate with an expression product of any of the sequences of Tables I - VI which is a kinase or with a cell containing at least 1 copy of the expression product or with a lipidic matrix containing at least 1 copy of an expression product;
(b) determining the amount of phosphate transfer from the substrate to the ligand; and
(c) selecting test compounds on the basis of their capacity to modulate phosphate transfer.

Optionally ligand, substrate and/or other essential molecules may be added prior to contacting the test compound with expression product of step (a) or after step (a). Non limiting examples and methodology can be gained from the teachings of the *Molecular Probes handbook* and references therein (Molecular Probes, Inc., 4849 Pitchford Ave, Eugene, USA), *Methods in Molecular Biology 2000*; 99: 191-201, *Oncogene* 2000 20; 19(49): 5690-701, and *FASAB Journal,* (10, 6, P55, P1458, 1996, Pocius D Amrein K *et al*).

### b) Receptor assays

An expression product of any of the nucleic acid sequences of Tables I - VI which encodes a receptor, and in particular any of the nucleic acid sequences listed in Table II, is amenable to screening using receptor assay technology.

Receptors are membrane associated proteins that initiate intracellular signalling upon ligand binding. Therefore, the identification of molecules for the prevention and treatment of pain can be achieved with the use of a ligand binding assay, as outlined above. Such an assay would utilize an endogenous or non-endogenous ligand as a component of the ligand binding assay. The binding of this ligand to the receptor in the presence of one or more test compounds would be measured as described above. Such is the nature of receptors that the assay is usually, but not exclusively performed with a receptor as an intact cell or membraneous preparation.

The invention therefore includes a method of screening for pain alleviating compounds, comprising:
a) contacting a test compound or test compounds in the presence of a ligand with a cell expressing at least one copy of the expression product of any of the sequences of Tables I - VI which is a receptor or with a lipidic matrix containing at least one copy of the expression product;
b) determining the binding of the test compound to the expression product, and
c) selecting test compounds on the basis of their binding abilities.

### c) Transporter protein assays

An expression product of any of the nucleic acid sequences of Tables I - VI which encodes a transporter protein, and in particular any of the nucleic acid sequences listed in Table III, is amenable to screening using transporter protein assay technology. Non limiting examples of technologies and methodologies are given by *Carroll FI, et al* (1995, Medical Research Review, Sep15 (5) p419-444), *Veldhuis JD and Johnson Ml* (1994, Neurosci. Biobehav Rep., winter 18(4) 605-12), *Hediger MA and Nussberger S* (1995, Expt Nephrol, July-Aug 3(4) p211-218, *Endou H and Kanai Y,* (1999, Nippon Yakurigaku Zasshi, Oct. 114 Suppl 1:1p-16p), *Olivier B et al* (2000, Prog. Drug Res., 54, 59-119), *Braun A et al* (2000, Eur J Pharm Sci, oct 11, Suppl 2 S51-60) and *Molecular Probes handbook and references therein* (Molecular Probes, Inc., 4849 Pitchford Ave, Eugene, USA).

The main function of transporter proteins is to facilitate the movement of molecules across a cellular membrane. Compounds that modulate the activity of transporter proteins can be determined with a transporter protein functional screen. Functional screening for modulators of transporter proteins comprises contacting at least one test compound with an expression product as aforesaid which is a transporter protein and determining the ability of said test compound to modulate the activity of said transporter protein. The expression product can be part of an intact cell, or lipidic preparation, optionally attached to a support, for example beads, a column or a plate. Binding is preferably performed in the presence of the molecule to be transported, which should only able to pass through a cell membrane or lipidic matrix with the aid of the transporter protein. The molecule to be transported should be able to be followed when it moves into a cell or through a lipidic matrix. Preferably the molecule to be transported is labelled to aid in characterization, e.g. with a chromophore, radioactive, fluorescent, phosphorescent, enzymatic or antibody label. If the molecule to be transported is not directly detectable it should be amenable to detection and quantification by secondary detection, which may employ the above technologies. The molecule to be transported may be contacted with the transporter protein before, simultaneously with or after the test compound. If binding of the test compound to the transporter protein modifies its ability to transport molecules through a membraneous or lipidic matrix, then the difference between the observed amount of transported molecule in a cell/or through a lipidic matrix relative to the theoretical maximum amount is a reflection of the modulatory effect of the test compound.

The invention further provides a method for screening compounds for their ability to relieve pain, comprising
a) contacting at least one test compound in the presence of transporter molecules with a cell containing at least one copy of an expression product of any of the sequences of Tables I - VI which is a transporter protein or with a lipidic matrix containing at least one copy of the expression product;
c) measuring the movement of transported molecules into or from the cell, or across the lipidic matrix; and
d) selecting test compounds on the basis of their ability to modulate the movement of transported molecules.

### d) G-protein coupled receptor protein assays

An expression product of any of the nucleic acid sequences of Tables I - VI that encodes a G-protein coupled receptor protein, and in particular any of the nucleic acid sequences listed in Table IV, is amenable to screening using G-protein coupled receptor protein assay technology.

G-protein coupled receptor proteins (GPCRs) are membrane associated proteins whose main function is to transduce a signal through a cellular membrane. Upon ligand binding, GPCRs undergo a conformational change that allows complexing of the GPCRs with a G-protein. G-proteins possess a GTP/GDP binding site. The formation of the G-protein/ligand complex allows exchange of GTP for GDP, resulting in a conformational change of the G-protein. This conformational change initiates signal transduction.

Functional screening for modulators of GPCRs comprises contacting at least one test compound with an expression product as aforesaid which is a G-protein coupled receptor protein, and assessing the ability of the test compound(s) to modulate the exchange of GTP for GDP or the modulation of the GPCR signal transduction pathway. The expression product can be part of an intact cell or lipidic preparation, optionally attached to a support, for example beads, a column or a plate. Binding is preferably performed in the presence of a ligand, G-protein and GTP/GDP to allow an assessment of the binding activity of each test compound. Alternatively components of the signaling pathway are also included. In particular, in a preferred embodiment, a labeled GTP is used and the ability of the test compound(s) to modulate the exchange of GTP to GDP is determined. A further optional characteristic of the assay can be the inclusion of a reporter molecule that enables monitoring the regulation of the signaling pathway. The ligand may be contacted with the GPCR before, simultaneously with, or after the test compound. Binding of the test compound to the GPCR modifies its ability to modulate the exchange of GTP for GDP and hence the modulation of signal transduction. The difference between the observed amount of GTP exchanged for GDP relative to the theoretical maximum amount of GTP is a reflection of the modulatory effect of the test compound. Likewise the relative activities of signal transduction reporter molecules are also a reflection of the modulatory effect of the test compound. Non limiting examples of technologies and methodologies can be found in *Molecular Probes handbook* and references therein (Molecular Probes, Inc., 4849 Pitchford Ave, Eugene, USA), *Glaxo Pocket Guide to Pharmacology,* (Michael Sheehan, Pharmacology Division staff, Glaxo Group Research Ltd., Ware, Herts SC12 0DP) and *Xing et al* (2000, J. Recept. Signal. Transduct. Res. 20(40 189-210).

The invention provides a method of screening compounds for their ability to relieve pain, comprising:
a) contacting at least one test compound in the presence of a ligand, GTP/GDP, G-protein with a cell containing at least one copy of an expression product of a sequence of Tables I - VI as aforesaid which is a G-protein coupled receptor protein or with a lipidic matrix containing at least one copy of the expression product;
b) measuring the exchange of GTP for GDP, and
c) selecting test compounds on the basis of their ability to modulate said exchange.
   In the above method, the ligand, GTP/GDP, G-protein and other essential molecules can be added before, simultaneously with or after the contacting of the test compound(s) with the cell line or lipidic matrix in step (a).

### e) DNA-binding protein assays

An expression product of any of the nucleic acid sequences of Tables I - VI that encodes a DNA-binding protein, and in particular any of the nucleic acid sequences listed in Table V, is amenable to screening using DNA-binding protein assay technology.

DNA binding proteins are proteins that are able to complex with DNA. The complexing of the DNA binding protein with the DNA in some instances requires a specific nucleic acid sequence. Screens can be developed in a similar manner to ligand binding screens as previously indicated and will utilise DNA as the ligand. DNA-binding protein assays can be carried using similar principles described in ligand binding assays as described above. Non limiting examples of methodology and technology can be found in the teachings of *Haukanes BI and Kvam* C (Biotechnology, 1993 Jan 11 60-63), *Alberts B et al* (Molecular Biology of the Cell, 1994, 3^{rd} Edn., Garland Publications Inc, *Kirigiti P and Machida CA* (2000 Methods Mol Biol, 126, 431-51) and *Molecular Probes handbook* and references therein (Molecular Probes, Inc., 4849 Pitchford Ave., Eugene, USA).

The invention therefore includes a method of screening for pain alleviating compounds, comprising:
a) contacting a test compound or test compounds in the presence of a plurality of nucleic acid sequences with an expression product of any of the nucleic acid sequences of Tables I - VI which is a DNA binding protein or with cells expressing at least one copy of the expression product or with a lipidic matrix containing at least one copy of the expression product;
b) determining the binding of the test compound to the expression product, and
c) selecting test compounds on the basis of their binding abilities.
   In the above method, the plurality of nucleic acid sequence may be added prior to, simultaneously with or after contact of the test compound with the expression product.

### f) Assays using other enzymes

Expression products of any of the nucleic acid sequences of Tables I - VI that encode other enzymes, e.g. ligases, lyases, oxidoreductases, transferases and hydrolases, and in particular any of the nucleic acid sequences listed in Table VI, is amenable to screening using appropriate assay technology.

Each class of enzyme has a defined function. Ligases have the property of being able to splice molecules together. This is achieved with the conversion of ATP substrate to AMP. Therefore, the activity of a ligase can be followed by monitoring the conversion of ATP to AMP. Such technologies are known to those in the art. Non limiting examples and methodologies are illustrated by *Ghee. T. Tan et al* (1996,Biochem J. 314, 993-1000, *Yang SW et al* (1992, 15: 89(6) 2227-31 and refrences therein, and in *Molecular Probes handbook* and references therein (Molecular Probes, Inc., 4849 Pitchford Ave, Eugene, USA).

The invention also provides a methods of screening for pain alleviating compounds, comprising;
a) contacting one or more test compounds in the presence of ATP with an expression product of any of the sequences of Tables I - VI which is a ligase or with a cell expressing at least 1 copy of a expression product or with a lipidic matrix containing at least 1 copy of an expression product which is a ligase;
b) determining the amount of ATP converted to AMP, and
c) selecting test compounds on the basis of their ability to modulate said conversion.

Lyases are enzymes which catalyse the cleavage of by reactions other than hydrolysis. These enzymes can be grouped into seven groups according to type of bond cleaved. These groups are carbon-carbon lyases (E.C. No 4.1), carbon-oxygene lyases (E.C. No 4.2), carbon-nitrogen lyases (E.C. No 4.3), carbon-sulphur lyase (E.C. No 4.4) carbon-halide lyases (E.C. No 4.5), phosphorous-oxygene lyases (E.C. No 4.6) and other lyases (E.C. No 4.99) (Analytical Biochemistry 3^{rd} Edn, David J. Holme and Hazel Peck, Longman press). The enzyme commission number (E.C.) of the International Union of Biochemistry relates to the type of reaction catalysed by the enzyme. Further *teachings on how to develop assays and screens for lyases can be obtained from Methods in Enzymology* (Academic Press).

Oxidoreductases are enzymes that catalyse the transfer of hydrogen or oxygen atoms or electrons. These enzymes can by sub-grouped into twenty categories according to their specific mode of action. These groups are oxidoreductases acting on the CH-OH group of donors (E.C. Nol.1 oxidoreductases acting on the aldehyde or oxo group of donors (E.C. No 1.2), oxidoreductases acting on the CH-CH group of the donor (E.C. No 1.3), oxidoreductases acting on the CH-NH2 group of donors (E.C. 1.4), oxidoreductases acting on the CH-NH group of donor (E.C. 1.5), oxidoreductases acting on the NADH or NADPH (E.C. No 1.6), oxidoreductases acting on other nitrogen compounds as donors (E.C. No 1.7), oxidoreductases acting on a sulphur group of donors (E.C. No 1.8), oxidoreductases acting on a haem group of donors (E.C. No1.9), oxidoreductases acting on diphenols and related substances as donors (E.C. 1.10), oxidoreductases acting on hydrogen peroxide as acceptor (E.C. No 1.11), oxidoreductases acting on hydrogen as donor (E.C. No 1.12), oxidoreductases acting on single doners with incorporation of molecular oxygen (E.C. No 1.13), oxidoreductases acting on paired donors with incorporation of molecular oxygen (E.C. No 1.14), oxidoreductases acting on superoxide radicals as acceptors (E.C. 1.15), oxidoreductases oxidizing metal ions (E.C. No 1.16), oxidoreductases acting on -CH2 groups (E.C. No 1.17), oxidoreductases acting on reduced ferredoxin as donor (E.C. No 1.18), oxidoreductases acting on reduced flavodoxin as donor (E.C. No 1.19) and other oxidoreductases (E.C. No 1.97) (Analytical Biochemistry 3^{rd} Edn, David J. Holme and Hazel Peck, Longman Press). The enzyme commission number (E.C.) of the International Union of Biochemistry relates to the type of reaction catalysed by the enzyme. Further teachings on how to develop assays and screens for oxidoreductases can be obtained from *Methods in Enzymology* (Academic Press) with special reference to volume 249.

Transferases are enzymes that catalyse the transfer of specific groups. They are classified into eight sub groups according to function, transferring one-carbon group (E.C. No 2.1), Transfering aldehyde or ketonic residues (E.C. No 2.2), Acetyltransferases (E.C. 2.3), glycosyltransferases (E.C. No 2.4), transferring alkyl or aryl groups other than methyl groups (E.C. No 2.5), transferring nitrogeneous groups (E.C. No 2.6), transferring phosphorous-containing groups (E.C. No 2.7) and transferring sulphur-containing groups (E.C. No 2.8) (Analytical Biochemistry 3^{rd} Edn, David J. Holme and Hazel Peck, Longman Press). The enzyme commission number (E.C.) of the International Union of Biochemistry relates to the type of reaction catalysed by the enzyme. Further teachings on how to develop assays and screens for transferases can be obtained from Methods in Enzymology (Academic Press).

Hydrolases are enzymes that catalyse hydrolytic reactions and are sub-grouped into eleven classes according to the type of reaction they carry out. Hydrolases acting on ester bonds (E.C. No 3.1), hydrolases acting on glycosyl compounds (E.C. No 3.2), hydrolases acting on ether bonds (E.C. No 3.3), hydrolases acting on peptide bonds (E.C. No 3.4), hydrolases acting on carbon-nitrogen bonds, other than peptide bonds (E.C. No 3.5), hydrolases acting on acid anhydrides (E.C. No 3.6), hydrolases acting on acid anhydrides (E.C. No 3.6), hydrolases acting on carbon-carbon bonds (E.C. No 3.7), hydrolases acting on halide bonds (E.C. No 3.8), hydrolases acting on phosphorous-nitrogen bonds (E.C. No 3.9), hydrolases acting on sulphur-nitrogen bonds (E.C. No 3.10) and hydrolases acting on carbon-phosphorous bonds (Analytical Biochemistry 3^{rd} Edn, David J. Holme and Hazel Peck, Longman Press). The enzyme commission number (E.C.) of the International Union of Biochemistry relates to the type of reaction catalysed by the enzyme. Further teachings on how to develop assays and screens for hydrolases can be obtained from Methods in Enzymology (Academic Press) with special reference to volume 249.

### C) In vivo functional screen

Any of the nucleotide sequences described in Tables I - VI or homologues thereof may be inserted by means of an appropriate vector into the genome of a lower vertebrate or of an invertebrate animal or may be inactivated or down regulated in the genome of said animal. The resulting genetically modified animal may be used for screening compounds for effectiveness in the regulation of pain. The invertebrate may, for example, be a nematode e.g. *Caenorhabditis elegans,* which is a favourable organism for the study of response to noxious stimuli. Its genome sequence has been determined, see *Science,* **282,** 2012 (1998), it can be bred and handled with the speed of a micro-organism (it is a self-fertilizing hermaphrodite) and can therefore be used in a high throughput screening format (WO 00/63424, WO 00/63425, WO 00/63426 and WO 00/63427), and it offers a full set of organ systems, including a simple nervous system and contains many similarly functioning genes and signaling pathways to mammals. A thermal avoidance model based on a reflexive withdrawal reaction to an acute heat stimulus has been described by Wittenburg *et al, Proc. Natl. Acad. Sci.USA,* **96,** 10477-10482 (1999), and allows the screening of compounds for the treatment of pain with the modulation of pain sensation as an endpoint.

The genome of *C. elegans* can be manipulated using homologous recombination technology which allows direct replacement of nucleic acids encoding *C. elegans* with their identified mammalian counterpart. Replacement of these nucleic acids with those nucleic acids outlined above would allow for the direct screening of test compound(s) with their expression products. Any of the pain-related genes described above may be ligated into a plasmid and introduced into oocytes of the worm by microinjection to produce germline transformants. Successful plasmid injection into *C. elegans* and expression of inserted sequences has been reported by Devgen B.V., Ghent, Belgium. It is also possible to produce by routine methods worms in which the target sequences are down-regulated or not expressed (knock-out worms). Further non limiting examples of methodology and technology can be found in the teachings of *Hazendonk et al* (1997, Nat genet. 17(1) 119-21), *Alberts et al,* (1994, Molecular Biology of the Cell 3^{rd} Ed. Garland Publishing Inc, *Caenorhabditis elegans* is anatomically and genetically simple), *Broverman S et al*, (1993, PNAS USA 15;90(10) 4359-63) and Mello et al (1991, 10(12)3959-70).

A further method for screening compounds for ability to modify response to pain, e.g. relieve pain, comprises:
(a) contacting one or more test compounds with at least one *C elegans* containing at least one copy of a sequence as set out above;
(b) subjecting the *C. elegans* to a nociceptive stimulus;
(c) observing the response of the *C. elegans* to said stimulus; and
d) selecting test compounds on the basis of their ability to modify the response of *C. elegans* to said stimulus.

### DIAGNOSTIC TOOLS AND KITS

### Affinity peptides, ligands and substrates

Pain associated polypeptides and fragments thereof can be detected at the tissue and cellular levels with the use of affinity peptides, ligands and substrates, which will enable a skilled person to define more precisely a patient's ailment and help in the prescription of a medicament. Such affinity peptides are characterized in that firstly they are able to bind specifically to a pain associated polypeptide, and secondly that they are capable of being detected. Such peptides can take the form of a peptide or polypeptide for example an antibody domain or fragment, or a peptide/polypeptide ligand or substrate, or a polypeptide complex such as an antibody.

The preparation of such peptides and polypeptides are known to those in the art. Antibodies, these may be polyclonal or monoclonal, and include antibodies derived from immunized animals or from hybridomas, or derivatives thereof such as humanized antibodies, Fab or F(ab')2 antibody fragments or any other antibody fragment retaining the antigen binding specificity.

Antibodies directed against pain-associated gene product molecules may be produced according to conventional techniques, including the immunization of a suitable mammal with the peptides or polypeptides or fragment thereof. Polyclonal antibodies can be obtained directly from the serum of immunized animals. Monoclonal antibodies are usually produced from hybridomas, resulting from a fusion between splenocytes of immunized animals and an immortalized cell line (such as a myeloma). Fragments of said antibodies can be produced by protease cleavage, according to known techniques. Single chain antibodies can be prepared according to the techniques described in US 4,946,778. Detection of these affinity peptides could be achieved by labeling. technologies which allow detection of peptides, such as enzymatic labeling, fluorescence labeling or radio-labeling are well known to those in the art. Optionally these affinity peptides, ligands and substrates could themselves be detected with the use of a molecule that has specific affinity to the peptides, ligands and substrates and is itself labeled.

The invention further provides a kit comprising;
(a) affinity peptide and/or ligand and/or substrate for an expression product of a gene sequence that is down-regulated in the spinal cord of a mammal in response to first and second models of neuropathic or central sensitization pain ; and
(b) a defined quantity of an expression product of a gene sequence that is down-regulated in the spinal cord of a mammal both in response to first and second models of neuropathic or central sensitization pain,
   for simultaneous, separate or sequential use in detecting and/or quantifying an expression product of a gene sequence that is down-regulated in the spinal cord of a mammal in response to first and second models of neuropathic or central sensitization pain.

### Complimentary nucleic acids

Pain associated nucleic acid sequences can be characterized at the tissue and cellular levels with the use of complimentary nucleic acid sequences. Detection of the level of expression of pain associated nucleic acid sequences can help in the prognosis of a pain condition and the prescription of a medicament. These complimentary nucleic acids are characterized in that they can hybridize to a pain associated nucleic acid sequence and their presence can be detected through various techniques. Such techniques are known to those in the art and may include detection by polymerase chain reaction or detection by labeling of complimentary nucleic acid sequences by enzymatic labeling, affinity labeling fluorescent labeling or radio labeling. Complimentary strand nucleic acid sequences of this invention are 10 to 50 bases long, more preferably 15 to 50 bases long and most preferably 15 to 30 bases long, and hybridize to the coding sequence of the nucleic acid sequence.

A further aspect of this invention is a kit that comprises:
(a) nucleic acid sequences capable of hybridization to a nucleic acid sequence that is down-regulated in the spinal cord of a mammal in response to first and second models of neuropathic or central sensitization pain; and
(b) a defined quantity of one or more nucleic acid sequences capable of hybridization to a nucleic acid sequence that is down-regulated in the spinal cord of a mammal in response to first and second models of neuropathic or central sensitization pain, for simultaneous, separate or sequential use in detecting and/or quantifying a gene sequence that is down-regulated in the spinal cord of a mammal in response to first and second models of neuropathic or central sensitization pain.

### IDENTIFICATION AND VALIDATION

Subtractive hybridization enables the identification of nucleic acid sequences whose expression profiles are modified by a stimulus. Upon stimulation of a system (in the case of this invention a nociceptive stimulus on an animal model) all observed changes in the level of nucleic acid sequence expression are due to the reaction of the system to the stimulus. Characterization of these changes in expression by way of identification of nucleic acid sequence and level of expression is both identification and validation.

The inventors have developed a four step process which allows for the simultaneous identification and validation of nucleic acid sequences whose expression are regulated by a pain stimulus, preferably a chronic pain stimulus, and more preferably a diabetic pain stimulus. This process may comprise the following steps:
(a) induction of a nociceptive stimulus in test animals;
(b) extraction of nucleic acids from specific neuronal tissue of test and control animals;
(c) selective amplification of differentially expressed nucleic acid sequences; and
(d) identification and characterization of differentially expressed gene products that are modulated by a nociceptive stimulus.

The above process is described in more detail below.

### (a) Induction of nociceptive stimulus

The effect of the selected nociceptive stimulus on the test animal needs to be confirmable. The test subjects are therefore a species that has a "developed" nervous system, preferably similar to that of humans, most preferably rats or mice. Advantageously, the nociceptive stimulus is analogous to known pain paradigms in humans. One such paradigm of pain is the pain associated with diabetes, which can be induced in rodents with the use of streptozotocin (STZ). The present application requires the sequences to be down-regulated in two pain models which may be, but are not limited to models of neuropathic pain and/or central sensitization, and in which diabetic pain provides the first model and mechanical damage e.g. to a nerve leading into the spine can provide an appropriate second model.

Streptozotocin (STZ) induces hyperglycemia and Type 1 diabetes mellitus in rats. In particular, STZ contains a glucose analogue that allows it to be taken up by the glucose transporter 2 present on the surface of pancreatic β cells, the site of insulin synthesis. Once inside the cell, STZ causes a reduction in the level of nicotinamide adenine dinucleotide (NAD⁺). The decrease in NAD⁺ levels eventually leads to necrosis of the pancreatic β cell, causing a reduction in insulin levels and then diabetes, leading to neuropathy (diabetic) and neuropathic pain (R. B. Weiss, *Cancer Treat. Rep.,* **66,** 427-438 1982, Guy *et al, Diabetologica*, **28,** 131-137 1985; Ziegler *et al, Pain,* **34,** 1-10 1988; Archer *et al, J. Neural. Neurosurgeon. Psychiatry,* **46,** 491-499 1983). The diabetic rat model has been shown to be a reliable model of hyperalgesia. We have used an STZ-induced diabetic rat model to create a state of hyperlagesia that can be compared with control animals (Courteix *et al, Pain,* **53*,*** 81-88 1993).

Three models of neuropathic and/or central sensitization pain in rats, which involve nerve injury, may be used, see Ralston, DD (1998) Present models of neuropathic pain. *Pain Rev.* **5:** 83-100. The injuries are caused by (1) loosely tying four chromic gut sutures around the sciatic nerve (CCI model developed by Bennett,GJ and Y-K Xie, A peripheral mononeuropathy in rat that produces disorders of pain sensation like those seen in man, *Pain* **33:** 87-107 (1988), (2) tightly ligating one third to one half of the fibers in the sciatic nerve (model developed by Seltzer, Z, R Dubner, Y Shire, A novel behavioral model of neuropathic pain disorders produced in rats by partial sciatic nerve injury, *Pain* **43:** 205-218, 1990), and (3) tightly ligating the dorsal spinal nerve of a rat at the L5 or L5 and L6 levels (L5 model developed by Kim, SH and JM Chung, An experimental model for peripheral neuropathy produced by segmental spinal nerve ligation in the rat, *Pain* **50**:355-363, 1992).

### (b) Extraction of nucleic acids from neuronal tissue of test and control animals

The inventors have determined that RNA extraction of whole spinal cord nervous tissue would provide a way of identifying nucleic acid sequences whose expression in spinal tissue is modulated by streptozotocin induced diabetes or by a mechanical nerve damage model for neuropathic and/or central sensitization pain e.g. CCI. Test (subjected to the nociceptive stimulus) and control animals were sacrificed, and the tissue to be studied e.g. neural tissue separated. Techniques for so doing vary widely from animal to animal and will be familiar to skilled persons.

A cDNA library can be prepared from total RNA extracted from neural tissue of the test and control animals. Where possible, however, it is preferred to isolate the mRNA from the total RNA of the test and control animals, by affinity chromatography on oligo (dT)-cellulose, and then reverse transcribe the mRNA from the test and control animals to give test and control cDNA. Converting mRNA from the test and control animals to corresponding cDNA may be carried out by any suitable reverse transcription method, e.g. a method as described by Gubler & Hoffman, *Gene,* 25, 263-269 (1983). If desired a proprietary kit may be used e.g. the CapFinder PCR cDNA Library Construction Kit (Life Technologies) which is based on long-distance PCR and permits the construction of cDNA libraries from nanograms of total RNA.

### (c) Selective amplification of differentially expressed nucleic acids

The reverse transcribed cDNA of the test and control animals is subjected to subtractive hybridisation and amplification so that differentially expressed sequences become selectively amplified and commonly expressed sequences become suppressed, so as to over-produce DNA associated with said nociceptic stimulus. A wide range of subtractive hybridisation methods can be used, but the preferred method is so-called suppression subtractive hybridisation, see US-A-5565340 and Diatchenko *et al, Proc.* *Nat. Acad. Sci. USA,* 93, 6025-6030 (1996), the disclosures of which are herein incorporated by reference. Kits for carrying out this method are available from CLONTECH Laboratories, Inc.

### (d) Cloning and Sequencing the differentially expressed cDNA

The differentially expressed cDNA is ligated into a cloning vector, after which cells of *E. coli* are transformed with the vector and cultured. Positive clones are selected and lysed to release plasmids containing the cDNA insert. The plasmids are primed using forward and reverse primers to either side of the cloning site and the cDNA insert is sequenced. Vector and adaptor sequences are then removed from the output data from the sequencer, leaving only the nucleotide sequence of the differentially expressed gene. The sequence is then checked against data held in a database for homology to known nucleotide sequences and genes, including expressed sequence tags (ESTs) and coding sequences for proteins.

### (e) Validation of the above method

The importance of the sequences that we have identified in pain is confirmed by the fact that genes have been identified using this method that represent nucleic acid sequences which have previously been implicated in pain, including Calmodulin (pRCM1, Genebank X13933), Enkephalin (Genebank Y07503) and Neurotensin receptor type 2 (Genebank X97121).

The inventors have identified nucleic acid sequences of the MAP kinase pathway, a previously non pain-associated biological pathway. The inventors have subsequently shown that intra-spinal injection of a MEK inhibitor (MEK is part of the MAP kinase pathway) produces a powerful inhibition of pain (Patent application No US 60/144292). Subsequently, it has been shown that the MAP kinase is also implicated in acute inflammatory pain (Woolf *et al*, Nature Neuroscience 1999).

The invention will now be further described in the following Example.

### EXAMPLE

### Induction of diabetes

Diabetes was induced in adult (150-200g) male Sprague-Dawley rats (n=6) as described by Courteix *et al (supra).* Animals were injected intraperitoneally with streptozotocin (STZ)(50 mg/kg) dissolved in distilled water. Control or sham animals (age-matched animals, n=6) were injected with distilled water only.

### Chronic Constrictive Injury (CCI)

Rats were anaesthetized with i.p. sodium phenobarbital, after which the common left sciatic nerve was exposed at the level of the middle of the thigh by blunt dissection through the biceps femoris and proximal to the sciatic trifurcation. Four ligatures (4.0 braided silk) were tied loosely around it with about 1mm spacing. The muscle was closed in layers and two wound clips were applied to close the skin incision. The wound was then covered with topical antibiotics.

### Nociceptive testing

Static allodynia (a form of hyperlagesia) was measured using a method described by Chaplan *let al,* "Quantitative assessment of tactile allodinya in the rat paw", *J. Neurosci. Methods,* **53,** 55-63 (1994). A series of von Frey filaments of different buckling weight (i.e. the load required for the filament to bend) were applied to the plantar surface of the right hand paw. The starting filament had a buckling weight of 20g. Lifting of the paw was taken to be a positive result, in which case a filament with the next lowest buckling weight was used for the next measurement. The test was continued until a filament was found for which there was an absence of response for longer than 5 seconds whereas a re-test with the next heaviest filament gave a positive response. Animals were considered hyperalgesic if their thresholds were found to be <4g of those of comparable untreated rats, see Calcutt & Chaplan, "Spinal pharmacology of tactile allodynia in diabetic rats", *British J. Pharmacol*, **122,** 1478-1482 (1997).

### Tissue Extraction

STZ-treated, CCI-treated and control animals were anaesthetized with 4% halothane and perfused with ice-cold 0.9% saline containing 1% citric acid (pH adjusted to 7.4 with NaOH). The animals were decapitated and the lumbar spinal cord exposed. A 2-centimetre length of spinal cord ending at L6 (lumbar-6 forward) was removed from the spinal column. Attached dorsal root ganglia and contaminating spinal connective tissues were removed. The spinal cord tissue was snap frozen in dry ice and isopentane. In the experiments that follow, procedures on the streptozocin-treated and control groups of animals are disclosed. It will be understood that for identification of CCI-treated animals the same experiments are performed, but using tissues from the CCI-treated animals and from control animals.

### Total RNA Extraction

Total RNA was extracted from the pooled male rat tissues of the streptozocin-treated and control groups using the TRIZOL Reagent Kit (Life Technologies). Briefly, tissue samples were homogenised fully using a Polytron homogenizer in 1ml of TRIZOL reagent per 50-100mg of tissue. Homogenized samples were then incubated at room temperature for 5 minutes and phase separated using 0.2ml chloroform per 1ml TRIZOL reagent followed by centrifugation at 3,000g. The aqueous phase was transferred to a fresh tube and the RNA precipitated with an equal volume of isopropyl alcohol and followed by centrifugation at 10,000g. The RNA pellet was washed in 75% ethanol and re-centrifuged. The pellet was then air dried and re-suspended in water.

### mRNA Extraction

In contrast to ribosomal RNA and transfer RNA, the vast majority of mRNAs of mammalian cells carry tracts of poly(A⁺) at their 3' termini. mRNAs can therefore be separated from the bulk of cellular RNA by affinity chromatography on oligo (dT)-cellulose. mRNA was extracted from Total RNA using the MESSAGEMAKER Kit (Life Technologies) in which mRNA (previously heated to 65°C in order to disrupt secondary structures and so expose the poly (A⁺) tails) was bound to oligo(dT) cellulose under high salt concentrations (0.5M NaCl) in a filter syringe. Unbound RNA was then washed away and the poly(A⁺) mRNA eluted in distilled water. A tenth of the volume of 7.5 M Ammonium Acetate, 50µg of glycogen/ml mRNA sample and 2 volumes of absolute alcohol were then added to the samples which were placed at -20°C overnight. Following precipitation, the mRNA was spun down at 12,000g for 30 minutes at 4°C. RNAase free water was used to re-suspend the pellets, which were then and stored at -80°C.

### PCR SELECT

The technique used was based on that of the CLONTECH PCR Select Subtraction Kit. The following protocol was performed using STZ-treated lumbar spinal cord Poly A ⁺ RNA as the 'Tester' and Sham lumbar spinal cord poly A⁺ RNA as the 'Driver' (Forward Subtraction). A second subtraction experiment using the Sham lumbar spinal cord mRNA as the 'Tester' and STZ treated lumbar spinal cord mRNA as the 'Driver' (Reverse Subtraction) was performed in parallel using the same reagents and protocol. As a control for both experiments, the subtraction was also carried out using human skeletal muscle mRNA that had been provided by the manufacturer.

### First-Strand cDNA Synthesis

2 µg of PolyA⁺ RNA and 1 µl of cDNA synthesis primer (10 µM) were combined in a 0.5ml Eppendorf tube and sterile H₂O was added where necessary to achieve a final volume of 5 µl. The contents were mixed gently and incubated in a thermal cycler at 70°C for 2 min. The tubes were then cooled on ice for two minutes, after which 2 µl of 5X First-strand buffer, 1 µl of dNTP mix (10 mM each), sterile H₂O and 1 µl of AMV reverse transcriptase (20 units/µl) was also added. The tubes were then placed at 42°C for 1.5 hr in an air incubator. First-strand cDNA synthesis was terminated by placing the tubes on ice. (the human skeletal muscle cDNA produced by this step was used as the 'control driver' in later steps).

### Second-Strand cDNA Synthesis

48.4 µl of Sterile H₂O, 16.0 µl of 5X Second-strand buffer, 1.6 µl of dNTP mix (10 mM) and 4.0 µl of 20X Second-strand enzyme cocktail were added to each of the first-strand synthesis reaction tubes. The contents were then mixed and incubated at 16°C in a thermal cycler for 2 hr. 6 units (2 µl) of T4 DNA polymerase was then introduced and the tubes were incubated for a further 30 min at 16°C. In order to terminate second-strand synthesis, 4 µl of 20X EDTA/glycogen mix was added. A phenol:chloroform extraction was then carried out using the following protocol:-

100 µl of phenol:chloroform:isoamyl alcohol (25:24:1) was added to the tubes which were then vortexed thoroughly and centrifuged at 14,000 rpm for 10 min at room temperature. The top aqueous layer was removed and placed in a fresh tube. 100 µl of chloroform:isoamyl alcohol (24:1) was then added to the aqueous layer and the tubes were again vortexed and centrifuged at 14,000 rpm for 10 min. 40 µl of 4 M NH₄OAc and 300 µl of 95% ethanol were then added and the tubes centrifuged at 14,000 rpm for 20 min. The supernatant was removed carefully, then 500 µl of 80% ethanol was added to the pellet. The tubes were centrifuged at 14,000 rpm for 10 min and the supernatant was removed so that the pellet could be air-dried. The precipitate was then dissolved in 50 µl of H₂O. 6 µl was transferred to a fresh microcentrifuge tube. The remainder of the sample was stored at -20°C until needed.

### Rsa I Digestion

All products of the above procedures were subjected to a restriction digest, using the restriction endonuclease *Rsa* I, in order to generate ds cDNA fragments that are short and thus are optimal for subtraction hybridisation due to the standard kinetics of the hybridisation. Also, as *Rsa* I makes a double stranded cut in the middle of a recognition sequence, 'blunt ends' of a known nucleotide sequence are produced allowing ligation of adaptors onto these ends in a later step. The following reagents were added to the 6 µl product of the second hybridisation (see above): 43.5 µl of ds cDNA, 5.0 µl 10X *Rsa* I restriction buffer and 1.5 µl of *Rsa* I (10 units/ µl). The reaction mixture was incubated at 37°C for 1.5 hr. 2.5 µl of 20X EDTA/glycogen mix was used to terminate the reaction. A phenol:chloroform extraction was then performed as above (second-strand cDNA synthesis section). The pellet produced was then dissolved in 5.5 µl of H₂O and stored at -20°C until needed. The preparation of the experimental 'Driver' cDNAs and the control skeletal muscle cDNA was thus completed.

### Adaptor Ligation

The adaptors were not ligated to the driver cDNA.

1 µl of each *Rsa* I-digested experimental cDNA (from the *Rsa* I Digestion above) was diluted with 5 µl of sterile water. Preparation of the control skeletal muscle tester cDNA was then undertaken by briefly centrifuging the tube containing control DNA (*Hae* III-digest of φX174 DNA [3 ng/ µl]) and diluting 2 µl of the DNA with 38 µl of sterile water (to 150 ng/ml). 1 µl of control skeletal muscle cDNA (from the *Rsa* I Digestion) was then mixed with 5 µl of the diluted φX174/ *Hae* III DNA (150 ng/ml) in order to produce the control skeletal muscle tester cDNA.

### Preparation of the adaptor-ligated tester cDNA

A ligation master mix was prepared by combining 3 µl of sterile water, 2 µl of 5X ligation buffer and 1 µl T4 DNA ligase (400 units/µl) per reaction. 2 µl of adaptor 1 (10 µM) was then added to 2 µl of the diluted tester cDNA. To this, 6 µl of the ligation master mix was also added. The tube was therefore labeled Tester 1-1. In a separate tube, 2 µl of the adaptor 2R (10 µM) was mixed with 2 µl of the diluted tester cDNA and 6 µl of the master mix. This tube was named Tester 1-2.

2 µl of Tester 1-1 and 2 µl of Tester 1-2 were then placed into fresh tubes. These would later be used as the unsubtracted tester control. The remainder of the contents of Tester 1-1 and Tester 1-2 tubes were then centrifuged briefly and incubated at 16°C overnight. The ligation reaction was stopped by adding 1 µl of EDTA/glycogen mix and the samples were heated at 72°C for 5 min in order to inactivate the ligase. In doing so, preparation of the experimental and control skeletal muscle adaptor-ligated tester cDNAs was complete.

1 µl from each unsubtracted tester control was then removed and diluted into 1 ml of water. These samples were set aside as they were to be used later for PCR (see below). All of the samples were stored at -20°C

### Analysis of Ligation efficiency

1 µl of each ligated cDNA was diluted into 200 µl of water and the following reagents were then combined in four separate tubes:

| **Component** | **Tube:** | **1** | **2** | **3** | **4** |
|---|---|---|---|---|---|
| Tester 1-1 (ligated to Adaptor 1) | | 1 | 1 | - | - |
| Tester 1-2 (ligated to Adaptor 2R) | | - | - | 1 | 1 |
| G3PDH 3' primer (10 µM) | | 1 | 1 | 1 | 1 |
| G3PDH 5' primer (10 µM) | | - | 1 | - | 1 |
| PCR primer 1 (10 µM) | | 1 | - | 1 | - |
| Total volume µl | | 3 | 3 | 3 | 3 |

A master mix for all of the reaction tubes plus one additional tube was made up by adding 18.5 µl of sterile H₂O, 2.5 µl of 10X PCR reaction buffer, 0.5 µl of dNTP mix (10 mM), and 0.5 µl of 50X Advantage cDNA Polymerase Mix, per reaction, into a fresh tube. 22 µl of this master mix was then aliquotted into each of the 4 reaction tubes prepared above. The contents of the tubes were overlaid with 50 µl of mineral oil. The reaction mix was incubated in a thermal cycler at 75°C for 5 min in order to extend the adaptors. The following protocol was then carried out immediately in a thermal cycler (Perkin-Elmer GeneAmp PCR Systems 2400): 94°C for 30 sec (1 cycle), 94°C 10 sec, 65°C 30 sec and then 68°C 2.5 min (25 cycles)

### First Hybridisation

1.5 µl of the Adaptor 1-ligated Tester 1-1 was combined with 1.5 µl of the *Rsa* I-digested driver cDNA, prepared earlier and 1 µl of 4X Hybridisation buffer. This process was then repeated combining the Adaptor 2R-ligated Tester 1-2 with the *Rsa* I-digested driver cDNA and 4X hybridisation buffer. The samples were incubated in a thermal cycler at 98°C for 1.5 min followed by incubation at 68°C for 8 hr.

### Second Hybridisation

1 µl of Driver cDNA (i.e. the *Rsa* I-digested cDNA (see above)), 1 µl 4X Hybridisation buffer and 2 µl Sterile H₂O were all combined in a fresh tube. 1 µl of this mix was then removed and placed in a new tube, overlaid with 1 drop of mineral oil and incubated at 98°C for 1.5 min in order to denature the driver. The following procedure was used to simultaneously mix the driver with hybridisation samples 1 and 2 (prepared in the first hybridisation), thus ensuring that the two hybridisation samples were mixed together only in the presence of freshly denatured driver: A micropipettor was set at 15 µl. The pipette tip was then touched onto the mineral oil/sample interface of the tube containing hybridisation sample 2. The entire sample was drawn partway into the tip before it was removed from the tube in order to draw a small amount of air into the tip. The pipette tip was then touched onto the interface of the tube containing the freshly denatured driver (i.e. the tip contained both samples separated by a small pocket of air) before the entire mixture was transferred to the tube containing hybridisation sample 1. The reaction was then incubated at 68°C overnight. 200 µl of dilution buffer was added to the tube, which was then heated in a thermal cycler at 68°C for 7 min. The product of this second hybridisation was stored at -20°C.

### PCR Amplification

Seven PCR reactions were set up: (1) The forward-subtracted experimental cDNA, (2) the unsubtracted tester control (see preparation of the adaptor ligated tester cDNA), (3) the reverse-subtracted experimental cDNA, (4) the unsubtracted tester control for the reverse subtraction, (5) the subtracted control skeletal muscle cDNA, (6) the unsubtracted tester control for the control subtraction, and (7) the PCR control subtracted cDNA (provided in the kit). The PCR control subtracted cDNA was required to provide a positive PCR control as it contained a successfully subtracted mixture of *Hae* III-digested φX174 DNA.

The PCR templates were prepared by aliquotting 1 µl of each diluted cDNA (i.e., each subtracted sample from the second hybridisation and the corresponding diluted unsubtracted tester control produced by the adaptor ligation see above) into an appropriately labeled tube. 1 µl of the PCR control subtracted cDNA was placed into a fresh tube. A master mix for all of the primary PCR tubes, plus one additional reaction, was then prepared by combining 19.5 µl of sterile water, 2.5 µl of 10X PCR reaction buffer, 0.5 µl of dNTP Mix (10 mM), 1.0 µl of PCR primer 1 (10 µM) and 0.5 µl of 50X Advantage cDNA Polymerase Mix. 24 µl of Master Mix was then aliquotted into each of the 7 reaction tubes prepared above and the mixture was overlaid with 50 µl of mineral oil, before being incubated in a thermal cycler at 75°C for 5 min in order to extend the adaptors. Thermal cycling was then immediately started using the following protocol: 94°C 25 sec (1 cycle), 94°C 10 sec, 66°C 30 sec and 72°C 1.5 min (32 cycles).

3 µl of each primary PCR mixture was then diluted in 27 µl of H₂O, 1 µl of each of these dilutions was then placed into a fresh tube.

A master mix for the secondary PCRs, (plus an additional reaction) was set up by combining 18.5 µl of sterile water, 2.5 µl of 10X PCR reaction buffer, 1.0 µl of Nested PCR primer 1 (10 µM), 1.0 µl of Nested PCR primer 2R (10 µM), 0.5 µl of dNTP Mix (10 mM) and 0.5 µl of 50X Advantage cDNA Polymerase Mix per reaction. 24 µl of this Master Mix was then added into each reaction tube containing the 1 µl diluted primary PCR mixture. The following PCR protocol was then carried out: 94°C 10 sec, 68°C 30 sec and 72°C 1.5 min (12 cycles). The reaction products were then stored at -20°C.

### Ligation into a Vector/ Transformation & PCR

The products of the PCR amplification (enriched for differentially expressed cDNAs) were ligated into the pCR2.1-TOPO vector using a T/A cloning kit (Invitrogen), transformed into TOPO One Shot competent cells according to the manufacturers protocol and grown up on LB (Luria-Bertani) Agar plates overnight at 37°C. 1,000 colonies were then individually picked (using fresh sterile tips) and dipped into 5 µl of sterile water which had been aliquotted previously into 96 well PCR plates. The water/colonies were heated in a thermal cycler at 100°C for 10 minutes in order to burst the cells, thus releasing the plasmids containing a differentially expressed cDNA insert. The 5 µl of water/plasmid was then used as a template in a PCR reaction (see below) using M13 Forward and Reverse primers (10 ng/µl), complementary to the M13 site present on either side of the cloning site on the vector. 5 µl of the PCR product was then run on a 2% agarose gel and stained by ethidium bromide. PCR products of an amplified insert were identified and 5 µl of the remainder of the PCR product (i.e. from the 15 µl that had not been run on the gel) was diluted 1/10 with water. 5 µl of the diluted PCR product was then used as a template in a sequencing reaction.

### Sequencing

A sequencing reaction containing M13 primer (3.2 pmol/µl), 'BigDye' reaction mix (i.e. AmpliTaq® DNA polymerase, MgCl_{2,} buffer and fluorescent dNTPs [each of the four deoxynucleoside triphosphates is linked to a specific fluorescent donor dye which in turn is attached to a specific acceptor dye]) and cDNA template (diluted PCR product) was set up. The reaction was carried out on a thermal cycler for 25 cycles of 10 seconds at 96°C, 20 seconds at 50°C and 4 minutes at 60°C. Each reaction product was then purified through a hydrated Centri-Sep column, and lyophilised. The pellets were resuspended in Template Supression Reagent and sequenced on an ABI Prism 310 Genetic Analyser. The analyser uses an ion laser to excite the specific donor dye that transfers its energy to the acceptor dye, which emits a specific energy spectrum that can be read by the sequencer.

The differentially expressed genes of the streptozocin-induced diabetes experiment and of the CCI experiment were sequenced at Parke-Davis, Cambridge and at the applicant's core sequencing facility in Ann Arbor, MI, USA.

### Bioinformatics

The sequencing results were analysed using the computer program CHROMAS in which the vector and adaptor sequences were clipped off, leaving only the nucleotide sequence of the differentially expressed gene. Each sequence was then checked for homology to known genes, Expressed Sequence Tags (ESTs) and Proteins using various Basic Local Alignment Search Tool (BLAST) searches against the Genbank sequence database at the National Centre for Biotechnology Information, Bethesda, Maryland, USA (NCBI).

Lists were derived called STZup and STZdown and CCIup and CCIdown that contain the nucleic acid sequences from the forward and back subtracted libraries respectively. In each list there are given accession numbers and descriptions for the known rat genes identified, and where available corresponding mouse or human genes. Sequences that are considered to be of interest and that are down-regulated both in a streptozocin-induced diabetes model and in a chronic constrictive injury pain model are identified in Tables I - VI above and are listed below.

References have been given where available for the sequences that have been found, and sequence listings have been given in the form in which they currently appear in publicly searchable databases e.g. the NCBI databaase (National Center for Biotechnology Information, National Library of Medicine, National Institutes of Health, Bethesda, Maryland, MD 20894, USA, www.ncbi.nlm.nih.gov). These sequence listings are given for the purposes of identification only. The invention includes the use of subsequently revised versions of the above sequences (which may incorporate small differences to the version set out herein) and homologous sequences or similar proteins in other species as determined by a high percentage identity (e.g. above 50%, preferably above 90%), length of alignment and functional equivalence.
1. Noguchi, T., Inoue, H. and Tanaka, T., The M-1- and M-2-type isozymes of rat pyruvate kinase are produced from the same gene by alternative RNA splicing, *J. Biol. Chem*. **261,** 13807-13812 (1986).
2. Takenaka, M., Noguchi, T., Inoue, H., Yamada, K., Matsuda ,T. and Tanaka, T., Rat pyruvate kinase M gene: Its complete structure and characterization of the 5'-flanking region, *J. Biol. Chem*. **264,** 2363-2367 (1989).
3. Zhou, Q.Y., Grandy, D.K., Thambi, L., Kushner, J.A., Van Tol, H.H., Cone R., Pribnow, D., Salon, J., Bunzow, J.R. and Civelli, O., Cloning and expression of human and rat D1 dopamine receptors, ***Nature,* 347** (6288), 76-80 (1990)
4. Sullivan, R., Chateauneuf, A., Coulombe, N., Kolakowski, L.F. Jr., Johnson, M.P., Hebert, T.E., Ethier, N., Belley, M., Metters, K., Abramovitz, M., O'Neill, G.P. and Ng, G.Y.K., Coexpression of full-length gamma-aminobutyric acid(B) (GABA(B)) receptors with truncated receptors and metabotropic glutamate receptor 4 supports the GABA(B) heterodimer as the functional receptor, *J. Pharmacol. Exp. Ther.* **293 (2),** 460-467 (2000)
5. Premont, R.T., Claing, A., Vitale, N., Freeman, J.L., Pitcher, J.A., Patton, W.A., Moss, J., Vaughan, M. and Lefkowitz, R.J., beta2-Adrenergic receptor regulation by GIT1, a G protein-coupled receptor kinase-associated ADP ribosylation factor GTPase-activating protein, *Proc. Natl. Acad. Sci. U.S.A.,* **95 (24),** 14082-14087 (1998).
6. Wells, D. and Kedes, L., Structure of a human histone cDNA: evidence that basally expressed histone genes have intervening sequences and encode polyadenylylated mRNAs, *Proc. Natl. Acad. Sci. U.S.A.,* **82 (9),** 2834-2838 (1985).
7. Ayte, J., Gil-Gomez, G. and Hegardt, F.G., Nucleotide sequence of a rat liver cDNA encoding the cytosolic 3-hydroxy-3-methylglutaryl coenzyme A synthase, *Nucleic Acids Res.,* **18 (12),** 3642 (1990).
8. Teruya, J.H., Kutsunai, S.Y., Spear, D.H., Edwards, P.A. and Clarke, C.F., Testis-specific transcriptional initiation sites of rat farnesyl pyrophosphate synthetase mRNA, ***Mol. Cell. Biol.,* 10,** 2315-2326 (1990).
9. Beck, K.F., Schreglmann, R., Stathopulos, I., Klein, H., Hoch, J. and Schweizer, M., The fatty acid synthase (FAS) gene and its promoter in Rattus norvegicus, *DNA Seq.,* **2 (6),** 359-386 (1992).
10. Mill, J.F., Mearow, K.M., Purohit, H.J., Haleem-Smith, H., King, R. and Freese, E., Cloning and functional characterization of the rat glutamine synthetase gene, *Brain Res. Mol. Brain Res.,* **9 (3),** 197-207 (1991).
11. Vincent, C., Tarbouriech, N. and Hartlein, M., Genomic organization, cDNA sequence, bacterial expression, and purification of human seryl-tRNA synthase, *Eur. J. Biochem*., **250 (1),** 77-84 (1997).
12. Giallongo, A., Feo, S., Moore, R., Croce, C.M. and Showe, L.C., Molecular cloning and nucleotide sequence of a full-length cDNA for human alpha enolase, *Proc. Natl. Acad. Sci. U.S.A.,* **83 (18),** 6741-6745 (1986).
13. Carper, D., Nishimura, C., Shinohara, T., Dietzchold, B., Wistow, G., Craft, C., Kador, P. and Kinoshita, J.H., Aldose reductase and p-crystallin belong to the same protein superfamily as aldehyde reductase, *FEBS Lett.* **220 (1),** 209-213 (1987).
14. Ku, C.Y., Lu, Q., Ussuf, K.K., Weinstock, G.M. and Sanborn, B.M., Hormonal regulation of cytochrome oxidase subunit messenger RNAs in rat Sertoli cells, *Mol. Endocrinol.* **5 (11),** 1669-1676 (1991).
15. Tsuji, S., Qureshi, M.A., Hou, E.W., Fitch, W.M. and Li, S.S.-L., Evolutionary relationships of lactate dehydrogenases from mammals, birds, an amphibian, fish, barley, and bacteria: LDH cDNA sequences from Xenopus, pig and rat, *Proc. Natl. Acad. Sci. U.S.A.* **91,** 9392-9396 (1994).
16. Taanman, J.W., Schrage, C., Ponne, N., Bolhuis, P., de Vries, H. and Agsteribbe,E., Nucleotide sequence of cDNA encoding subunit VIb of human cytochrome c oxidase, *Nucleic acids research.* **17 (4),** 1766 (1989).
17. Triepels, R., van den Heuvel, L., Loeffen, J., Smeets, R., Trijbels, F. and Smeitink, J., The nuclear-encoded human NADH: ubiquinone oxidoreductase NDUFA8 subunit: cDNA cloning, chromosomal localization, tissue distribution, and mutation detection in complex-I-deficient patients, *Hum. Genet.* **103 (5),** 557-563 (1998).
18. Hosokawa, Y., Suzuki, H., Toda, H., Nishikimi, M. and Ozawa, T. Complementary DNA encoding core protein II of human mitochondrial cytochrome bc-1 complex: Substantial diversity in deduced primary structure from its yeast counterpart, *J. Biol. Chem.* **264,** 13483-13488 (1989).
19. Harnik-Ort, V., Prakash, K., Marcantonio, E., Colman, D.R., Rosenfeld, M.G., Adesnik, M., Sabatini, D.D. and Kreibich, G. Isolation and characterization of cDNA clones for rat ribophorin I: complete coding sequence and in vitro synthesis and insertion of the encoded product into endoplasmic reticulum membranes, *J. Cell Biol.* **104**(4), 855-863 (1987).
20. Falany, C.N., Xie, X., Wang, J., Ferrer, J. and Falany, J.L., Molecular cloning and expression of novel sulphotransferase-like cDNAs from human and rat brain, *Biochem. J.* **346** Pt 3, 857-864 (2000).
21. Pan, W., Ko, Y.H. and Pedersen, P.L., Delta subunit of rat liver mitochondrial ATP synthase: molecular description and novel insights into the nature of its association with the F1-moiety, *Biochemistry* **37** (19), 6911-6923 (1998).
22. Hamajima, N., Matsuda, K., Sakata, S., Tamaki, N., Sasaki, M. and Nonaka, M., A novel gene family defined by human dihydropyrimidinase and three related proteins with differential tissue distribution, *Gene* **180** (1-2), 157-163 (1996).
23. McGuire, J.A., Poellinger, L., Wikstrom, A.C. and Gustafsson, J.A., Cloning and regulation by glucocorticoid receptor ligands of a rat hsp90 *J. Steroid Biochem. Mol. Biol.* **42** (8), 813-822 (1992).
24. Roach, A., Boylan, K.B., Horvath, S., Prusiner, S.B. and Hood, L.E., Characterization of cloned cdna representing rat myelin basic protein: absence of expression in brain of shiverer mutant mice, *Cell* **34,** 799-806 (1983)
25. Hoshino, A., Hisayasu, S. and Shimada, T., Complete sequence analysis of rat transferrin and expression of transferrin but not lactoferrin in the digestive glands, *Comp. Biochem. Physiol. B* **113,** 491-497 (1996).
26. Chin, S.S. and Liem, R.K., Expression of rat neurofilament proteins NF-L and NF-M in transfected non-neuronal cells, Eur. J. Cell Biol. 50 (2), 475-490 (1989).
27. Lai, C., Brow, M.A.D., Nave, K.-A., Noronha, A.B., Quarles, R.H., Bloom, F.E., Milner, R.J. and Sutcliffe, J.G., Two forms of lB236/myelin-associated glycoprotein, a cell adhesion molecule for postnatal neural development, are produced by alternative splicing, *Proc. Natl. Acad. Sci. U.S.A.* **84,** 4337-4341 (1987).
28. Napolitano, E.W., Chin, S.S.M., Colman, D.R. and Liem, R.K.H., Complete amino acid sequence and in vitro expression of rat NF-M, the middle molecular weight neurofilament protein, *J. Neurosci*. **7,** 2590-2599 (1987).
29. Nakayama, M., Miyake, T., Gahara, Y., Ohara, O. and Kitamura, T., A novel RING-H2 motif protein downregulated by axotomy: its characteristic localization at the postsynaptic density of axosomatic synapse, *J. Neurosci.* **15** (7 Pt 2), 5238-5248 (1995).
30. Kuwano, R., Usui, H., Maeda, T., Fukui, T., Yamanari, N., Ohtsuka, E., Ikehara, M. and Takahashi, Y., Molecular cloning and the complete nucleotide sequence of cDNA to mRNA for S-100 protein of rat brain, *Nucleic Acids Res.* **12** (19), 7455-7465 (1984).
31. Zauner, W., Kratz, J., Staunton, J., Feick, P. and Wiche, G., Identification of two distinct microtubule binding domains on recombinant rat MAP 1B, *Eur. J. Cell Biol.* **57** (1), 66-74 (1992).
32. Ozaki, T., Irie, K. and Sakiyama, S., Molecular cloning and cell cycle-dependent expression of a novel gene that is homologous to cdc37, *DNA Cell Biol.* **14** (12), 1017-1023 (1995).
33. Albertsen, H.M., Smith, S.A., Mazoyer, S.S., Fujimoto, E., Stevens, J., Williams, B., Rodriguez, P., Cropp, C.S., Slijepcevic, P. and Carlson,M., A physical map and candidate genes in the BRCA1 region on chromosome 17q12-21, *Nature Genet.* **7** (4), 472-479 (1994).
34. Dieffenbach, C.W., SenGupta, D.N., Krause, D., Sawzak, D. and Silverman,R.H., Cloning of murine gelsolin and its regulation during differentiation of embryonal carcinoma cells, *J. Biol. Chem.* **264,** 13281-13288 (1989).
35. Geisert, E.E. Jr., Murphy, T.P., Irwin, M.H. and Larjava, H., A novel cell adhesion molecule, G-CAM, found on cultured rat glia , *Neurosci. Lett.* **133** (2), 262-266 (1991); Irwin, .H. and Geisert, .E. Jr., The upregulation of a glial cell surface antigen at the astrocytic scar in the rat, *Neurosci. Lett.* **154** (1-2), 57-60 (1993); Geisert, E.E. Jr., Yang, L. and Irwin,M.H., Astrocyte growth, reactivity, and the target of the antiproliferative antibody, TAPA, *J. Neurosci.* **16** (17), 5478-5487 (1996).
36. Holz, A., Schaeren-Wiemers, N., Schaefer, C., Pott, U., Colello, R.J. and Schwab,M.E., Molecular and developmental characterization of novel cDNAs of the myelin-associated/oligodendrocytic basic protein, *J. Neurosci.* **16** (2), 467-477 (1996).
37. Ching, G.Y. and Liem, R.K.H., Structure of the gene for the neuronal intermediate filament protein alpha-internexin and functional analysis of its promoter, *J. Biol. Chem.* **266,** 19459-19468 (1991).
38. Lai, C.H., Chou, C.Y., Ch'ang, L.Y., Liu, C.S. and Lin, Wc., Identification of novel human genes evolutionarily conserved in caenorhabditis elegans by comparative proteomics, *Genome Res.* **10** (5), 703-713 (2000).
39. Nagase, T., Seki, N., Tanaka, A., Ishikawa, K. and Nomura, N., Prediction of the coding sequences of unidentified human genes. IV. The coding sequences of 40 new genes (KIAA0121-KIAA0160) deduced by analysis of cDNA clones from human cell line KG-1, *DNA Res.* **2** (4), 167-174 (1995).
40. Wendel, M., Sommarin, Y., Bergman, T. and Heinegard, D., Isolation, characterization, and primary structure of a calcium-binding 63-kDa bone protein, *J. Biol. Chem.* **270** (11), 6125-6133 (1995).
41. Welcher, A.A., Suter, U., De Leon, M., Snipes, G.J. and Shooter, E.M., A myelin protein is encoded by the homologue of a growth arrest-specific gene, *Proc. Natl. Acad. Sci. U.S.A.* **88,** 7195-7199 (1991).
42. Nudel, U., Zakut, R., Shani, M., Neuman, S., Levy, Z. and Yaffe, D., The nucleotide sequence of the rat cytoplasmic beta-actin gene, *Nucleic Acids Res.* **11** (6), 1759-1771 (1983).
43. Miwa, J.M., Ibanez-Tallon, I., Crabtree, G.W., Sanchez, R., Sali, A., Role, L.W. and Heintz,N., lynx1, an endogenous toxin-like modulator of nicotinic acetylcholine receptors in the mammalian CNS, *Neuron* **23** (1), 105-114 (1999).
44. Rothermel, E., Detzler, E., Walter, L., Levan, G. and Gunther, E., Characterization and mapping of a highly conserved processed pseudogene and an intron-carrying gene of the heat shock cognate protein 70 (Hsc70) gene family in the rat, *Mamm. Genome* **6** (9), 602-606 (1995).
45. Sadot, E., Marx, R., Barg, J., Behar, L. and Ginzburg, I., Complete sequence of 3'-untranslated region of Tau from rat central nervous system. Implications for mRNA heterogeneity, *J. Mol. Biol.* **241** (2), 325-331 (1994).
46. Lemke, G. and Axel, R., Isolation and sequence of a cDNA encoding the major structural protein of peripheral myelin, *Cell* **40,** 501-508 (1985).

## Claims

1. Use of:
(a) an isolated gene sequence that is down-regulated in the spinal cord of a mammal in response to mechanistically distinct first and second models of neuropathic or central sensitization pain;
(b) an isolated gene sequence comprising a nucleic acid sequence of any of Tables I to VI;
(c) an isolated gene sequence having at least 80% sequence identity with a nucleic acid sequence of any of Tables I to VI;
(d) an isolated nucleic acid sequence that is hybridizable to any of the gene sequences according to (a), (b) or (c) under stringent hybridisation conditions;
(e) a recombinant vector comprising a gene sequence or nucleic acid sequence according to any one of (a) to (d);
(f) a host cell containing the vector according to (e);
(g) a non-human animal having in its genome an introduced gene sequence or nucleic acid sequence or a removed or down-regulated gene sequence or nucleic acid sequence according to any one of (a) to (d);
(h) an isolated polypeptide comprising an amino acid sequence at least 90% identical to an amino acid sequence encoded by a nucleotide sequence according to any one of (a) to (d), or a polypeptide variant thereof with sequential amino acid deletions from the C terminus and/or the N-terminus;
(i) an isolated polypeptide encoded by a nucleotide sequence according to any one of (a) to (d); or
(j) an isolated antibody that binds specifically to a polypeptide according to (h) or (i);
in the screening of compounds for the treatment of pain, or for the diagnosis of pain.

2. Use according to claim 1, wherein the isolated gene sequence is down-regulated both in response to streptozocin-induced diabetes and in response to surgical injury of a nerve leading to the spine.

3. Use according to claim 1 or 2 wherein the isolated gene sequence encodes a kinase.

4. Use according to claim 1, 2 or 3, wherein the isolated gene sequence encodes an expression product or fragment thereof of pyruvate kinase, M1 and M2 subunits (M24359 ; X97047; X56494).

5. Use according to claim 1 or 2, wherein the isolated gene sequence encodes an expression product or fragment thereof of a receptor.

6. Use according to claim 1, 2 or 5, wherein the isolated gene sequence encodes dopamine receptor D.sub.1 (I58000).

7. Use according to claim 1 or 2, wherein the isolated gene sequence encodes a transporter.

8. Use according to claim 1, 2 or 7, wherein the isolated gene sequence encodes differentiation-associated Na⁺-dependent inorganic phosphate cotransporter (AF271235) or putative vacuolar assembly protein VSP41 gene (U87309).

9. Use according to claim 1 or 2, wherein the isolated gene sequence encodes a G-protein coupled receptor protein.

10. Use according to claim 1, 2 or 9, wherein the isolated gene sequence encodes Git1 (G-protein-coupled receptor kinase-interactor 1 ; GPCR kinase-associated ADP-ribosylation factor) (AF085693).

11. Use according to claim 1 or 2, wherein the isolated gene sequence encodes a DNA-binding protein.

12. Use according to claim 1, 2 or 11, wherein the isolated gene sequence encodes putative histone H3.3A (X91866 ; M11354).

13. Use according to claim 1 or 2, wherein the isolated gene sequence encodes a ligase.

14. Use according to claim 1, 2 or 13, wherein the isolated gene sequence encodes 3-Hydroxy 3-methylglutaryl coenzyme A synthase, cytosolic (X52625), acyl-CoA synthetase II, brain (D360666) famesyl diphosphate synthase (M34477), bendless protein (AB032739; E12457), fatty acid synthase (X62888), glutamine synthetase (EC 6.3.1.2) (M91652), or putative seryl-tRNA synthetase (X91257).

15. Use according to claim 1 or 2, wherein the isolated gene sequence encodes a lyase.

16. Use according to claim 1, 2 or 15, wherein the lyase is enolase, alpha alpha, non-neuronal (NNE) (X02610; X52379; M14328).

17. Use according to claim 1 or 2, wherein the isolated gene sequence encodes an oxidoreductase.

18. Use according to claim 1, 2 or 17, wherein the isolated gene sequence encodes aldose reductase, lens (AREC 11.1.21) (X05884) cytochrome-c oxidase I, mitochondrial (S79304), lactate dehydrogenase-B (LDH-B) (U07181; X51905; Y00711), putative cytochrome c oxidase VIB (EC 1.9.3.1) (X13923), putative NADH: ubiquinone oxidoreductase PGIV subunit (AF044953), putative succinate dehydrogenase flavoprotein (AF095938; AF171022), putative ubiquinol―cytochrome-c reductase (EC 1.10.2.2) core protein II (J04973) or stearoyl-coA desaturase 2 (AB032243; M26270).

19. Use according to claim 1 or 2, wherein the isolated sequence encodes a transferase.

20. Use according to claim 1, 2 or 19, wherein the isolated sequence encodes ribophorin I (X05300) or sulfotransferase-like protein (AF188699).

21. Use according to claim 1 or 2, wherein the isolated sequence encodes a hydrolase.

22. Use according to claim 1, 2 or 21, wherein the isolated sequence encodes ATP synthase, H+, alpha subunit, mitochondrial (EC 3.6.1.34) (X56133), F1F0 ATPase delta subunit (U00926), putative dihydropyrimidinase related protein (D78013), heat shock protein 90 (S45392; M18186; M16660), or putative ribonuclease III (AF116910).

23. Use according to claim 1 or 2, wherein the isolated sequence encodes myelin basic protein S (MBP S) (K00512), transferring (D38380), neurofilament, light molecular weight (NF-L) (AF031880), myelin-associated glycoprotein (MAG) (M16800; M31811), NF-M middle molecular weight neurofilament protein (M18628) neuro-degeneration associated- protein 1 (D32249), S-100 protein β-subunit (X01090), microtubule-associated protein 1b (Map 1b) (X60370; L06237), putative cdc 37 homolog (D26564), putative ras-related protein Rab-5c (U11293), putative gelsolin (J04953), Cd81 antigen (target of antiproliferative antibody 1) (U19894; X59047; M33680), Mobp81 (Myelin-associated/Oligo-dendrocytic basic protein 81) (X87900), syntaxin binding protein n-secl, sec1 homolog, A-internexin (M73049), putative β-sarcoglycan A3b (AB024921), CGI-78 protein (AF151835), KIAA0143 (D63477), septin 2 (D50918), Nucleobindin (Z36277), myelin protein SR13 (M69139; S55427), B-Actin, cytoplasmic (V01217; X03672), ly6/neurotoxin (Lynx1) homolog (AD141377), astrocytic phosphoprotein ; PFA 15 gene (AJ243949; X86694), PLIC-1 (AF177345), Nfx1 (tip associating protein (TAP) gene) (AF093139; AF093140), A-Crystallin B (U04320; M73741 ; M28638), heat shock-like protein 70 kD (X70065; U73744; Y00371), tau microtubule-associated protein (X79321), myelin, Schwann cell, Perioheral (P-0) (K03242 ), B-Tubulin class 1 (AB011679; X04663; AF14139), putative long-chain polyunsaturated fatty acid elongation enzyme (Helo1) or peptidylglycine alpha-amidating monooxygenase 1.

24. A non-human animal having in its genome an introduced gene sequence or a removed or down-regulated gene sequence, said sequence being down-regulated in the spinal cord of a mammal in response to first and second models of neuropathic or central sensitisation pain.

25. A non-human animal according to claim 24, wherein said gene sequence becomes down regulated both in response to streptozocin induced diabetes and in response to chronic constriction injury.

26. A non-human animal according to claim 24 or 25, wherein the introduced gene sequence is according to any of claims 1 to 23.

27. A non-human animal according to any one of claims 24 to 26 which is *C. elegans.*

28. A kit comprising;
(a) affinity peptide and/or ligand and/or substrate for an expression product of a gene sequence that is down-regulated in the spinal cord of a mammal in response to a mechanistically distinct first and second models of neuropathic or central sensitization pain ; and
(b) a defined quantity of an expression product of a gene sequence that is down-regulated in the spinal cord of a mammal both in response to first and second models of neuropathic or central sensitization pain,
for simultaneous, separate or sequential use in detecting and/or quantifying down-regulation of a gene sequence in the spinal cord of a mammal in response to first and second models of neuropathic or central sensitization pain.

29. A kit according to claim 28, wherein the gene sequence is defined in any one of claims 1 to 23.

30. A kit comprising:
(a) nucleic acid sequences capable of hybridization to a nucleic acid sequence that is down-regulated in the spinal cord of a mammal in response to first and second models of neuropathic or central sensitization pain; and
(b) a defined quantity of one or more nucleic acid sequences capable of hybridization to a nucleic acid sequence that is down-regulated in the spinal cord of a mammal in response to first and second models of neuropathic or central sensitization pain,
for simultaneous, separate or sequential use in detecting and/or quantifying down-regulation of a gene sequence in the spinal cord of a mammal in response to first and second models of neuropathic or central sensitization pain.

31. The kit of claim 30, wherein the gene sequence is according to any of claims 1 to 23.

32. A compound that modulates the action of an expression product of a gene sequence that is down-regulated in the spinal cord of a mammal in response to first and second models of neuropathic or central sensitization pain.

33. A compound according to claim 32 wherein the gene sequence is listed in Tables I to VI.

34. A compound according to claim 32 or 33 wherein the nucleotide sequence is according to any one of claims 1 to 23.

35. A compound according to any one of claims 32 to 34 for use as a medicament.

36. A compound according to any one of claims 32 to 35 for the treatment or diagnosis of pain.

37. A pharmaceutical composition comprising a compound according to any one of claims 32 to 36 and a pharmaceutically acceptable carrier or diluent.

38. Use of a compound according to any one of claims 32 to 36 in the manufacture of a medicament for the treatment or diagnosis of pain.

39. Use of a compound according to any one of claims 32 to 36 in the manufacture of a medicament for the treatment or diagnosis of chronic pain.

40. A method of treatment of pain, which comprises administering to a patient an effective amount of a compound according to any one of claims 32 to 36.
